# EUROPEAN PATENT APPLICATION

(11) **EP 4 663 632 A1**
(43) Date of publication of application: **17.12.2025**
(21) Application number: 24182120.6
(22) Date of filing: 13.06.2024
(51) Int. Cl.: C07D 401/14, C07D 403/14, C07D 417/14, A61P 35/00

(54) **COMPOUNDS USEFUL FOR THE MODULATION OF THE ACTIVITY OF STING**

(71) Applicant: Sulis Therapeutics ApS, 8000 Aarhus C (DK)
(72) Inventor: The designation of the inventor has not yet been filed
(74) Representative: Mewburn Ellis LLP

(57) **Abstract**

The invention relates to agents capable of degrading STING, composition comprising said agents and medical uses thereof.

## Description

### Field of the Invention

The present invention relates to compounds useful for the modulation of the activity of Stimulator of Interferon Genes (STING) and particularly, although not exclusively, to proteolysis targeting chimeric (PROTAC^{®}) compounds (also referred to herein as degraders/degrader compounds) which induce degradation of STING by binding to both STING and an E3 ubiquitin ligase, and uses of such compounds.

### Background

Traditional small molecule drugs reversibly (or sometimes irreversibly) bind to a target protein as a means of modulating a given biological activity. In contrast, degraders bind to their target proteins, but then bring about the target protein's degradation. Having achieved this effect, the degrader is in theory able to repeat this process with another target protein. Accordingly, unlike with "traditional small molecule" inhibitors, the degrader-driven degradation mechanism can in theory operate in a sub-stoichiometric manner - meaning that more modest exposures of a degrader compound could still achieve a desired level of efficacy in vivo. In practice this can mean that the degradation power (DC50 and Dmax) of a degrader can have an improved effect than that reflected only by its binding affinity. While many degrader molecules utilize ligands that have a functional effect independently of their function as part of the degrader, this is not necessary. That is, the ligand moiety may have no effect on a target protein independent of the degrader as a whole.

At a simplistic level, degrader molecules are often described as having three parts - (1) a part that is capable of binding to the target protein to be degraded, (2) a second part that is capable of binding to an E3 ubiquitin ligase, and finally, a linker that connects (1) and (2) together.

In use, the degrader binds to both the target protein and E3 ubiquitin ligase simultaneously to form a ternary complex. The E3 ligase then recruits an E2 conjugating enzyme to the ternary complex, which ubiquitinates the target protein. This has the effect of labelling the target protein for degradation by the cell's proteasome machinery. The degrader can then dissociate from the target protein and initiate another cycle of this process in a catalytic manner. Meanwhile, the ubiquitinated target proteins are recognized and degraded by the cell's proteasome machinery.

Proteolysis targeting chimeric (PROTAC^{®}) compounds are bifunctional molecules comprising a target protein ligand joined to a E3 ubiquitin ligase ligand by a linker (Békés et al., 2022). These molecules are used as modulators of targeted ubiquitination of proteins. Formation of a ternary complex between the degrader, the target protein and the E3 ligase leads to the specific polyubiquitination of the target protein and its subsequent intracellular degradation via the proteasome. Degrader compounds have been shown to be capable of the targeted degradation of a range of proteins.

Over 600 human E3 ubiquitin ligases are known, however among these there are a more limited selection of ubiquitin ligases which have been identified for use in degrader compounds.

Cereblon (CRBN) is a substrate specificity receptor that associates with the Cullin-4-RING E3 ubiquitin ligase (CRL4) complex. Known ligands for CRBN include thalidomide and other immunomodulatory imide drugs (IMiDs). CRBN has been widely used as an E3 ligase in degrader compounds targeting a range of proteins.

The Von Hippel-Lindau (VHL) protein is a part of a multiprotein complex which possesses an E3 ubiquitin ligase activity. Similarly to CRBN, VHL ligands have been used extensively in the design of degrader compounds targeting a range of proteins.

The Inhibitor of Apoptosis Proteins (lAPs) family includes antiapoptotic proteins, which are commonly overexpressed in some cancer cells and promote their survival, as well as the survival of neuronal cells. Among this family, certain lAPs possess E3 ubiquitin ligase activity, such as cellular lAPs 1 and 2 (clAP-1/2) and ligands for these molecules have been incorporated into the design of degrader compounds.

Stimulator of Interferon Genes (STING) is an intracellular adaptor protein which plays an important role in innate immunity. STING induces type I interferon (IFN) production in addition to other proinflammatory mediators such as cytokines in response to infection or cellular damage. Cytosolic STING is activated upon binding cyclic dinucleotides (CDNs), which can be derived from bacterial sources or 2',3'-cyclic GMP-AMP (2',3'-cGAMP) generated by cyclic GMP-AMP synthase (cGAS), an enzyme that is activated by binding to double-stranded DNA in the cytoplasm. In addition to the canonical CDN-dependent activation, STING can also be activated in a non-canonical CDN-independent manner, for example through gain-of-function mutations (Decout et al., 2021).

Activation of STING leads to up-regulation of type I interferon production. Activation of type I interferon production is an approach which has been explored in the treatment of some cancers, and numerous STING agonists have been designed for this purpose. Examples of STING agonists include benzimidazoles, for example as described in WO2017/175147, WO2019/069270, WO2020/132582, WO2020/132566, and WO2020/132549 the content of each of which is hereby incorporated by reference.

WO2013/106643 describes compounds and methods for the enhanced degradation of targeted proteins and other polypeptides by an E3 ubiquitin ligase.

WO2016/197032 describes imide-based compounds, including bifunctional compounds comprising the same, which find utility as modulators of targeted ubiquitination, especially inhibitors of a variety of polypeptides and other proteins which are degraded and/or otherwise inhibited by bifunctional compounds.

WO2016/169989 describes PROTAC^{®}/degrader compounds incorporating a selective E3 Ligase IAP binding moiety (IAP binding moiety) as the degradation component, functioning to recruit target proteins to the E3 ubiquitin ligase IAP for degradation.

WO2018/066545 describes drugs that can decompose a target intracellular protein specifically and is effective for the prevention or treatment of diseases associated with the target protein. A SNIPER compound produced by linking a specific IAP ligand, through a linker, to a ligand capable of specifically binding to a target intracellular protein.

In contrast, many monogenic autoinflammatory syndromes, autoimmune diseases, inflammatory diseases, neurological disorders, metabolic diseases, cardiovascular diseases, and cancers have been linked the activation of cyclic GMP-AMP synthase leading to activation of the cGAS-STING pathway, or direct activation of STING itself.

Monogenic autoinflammatory diseases are associated with mutations in genes that participate in innate immunity or that control innate immune homeostasis. STING-associated vasculopathy with onset in infancy (SAVI) is one such disease. SAVI is characterized by recurrent fevers, ulcerative skin lesions, vasculitis and interstitial lung disease and is mostly caused by autosomal dominant mutations in STING1 (Decout et al., 2021).

Another monogenic autoinflammatory disease is COPA syndrome, a rare early-onset autosomal dominant disease which can affect the lungs, kidneys, and joints, caused by missense mutations in the COPA gene, which encodes the COPα protein subunit of the COPI complex. This mutation impairs the binding and sorting of proteins targeted for ER retrieval, and promotes ligand-independent activation of STING-mediated signaling (Decout et al., 2021).

Aicardi-Goutières syndrome (AGS) is a further example of a monogenic autoinflammatory disease, which is characterised by early onset of progressive encephalopathy and skin lesions (known as chilblains). Mutations in a subset of genes associated with nucleic acid sensing or metabolism - specifically TREX1, and genes encoding the three RNase H2 endonuclease subunits, RNASEH2A, RNASEH2B, and RNASEH2C and the dNTPase SAMHD1 - have been associated with disturbed self-DNA metabolism and constitutive activation of the cGAS-STING pathway. TREX1 deficiency is therefore another disease linked to STING and activation of the cyclic GMP-AMP synthase pathway (Decout et al., 2021). Similarly, DNase II deficiency is a further autoinflammatory disease linked to STING and activation of the cyclic GMP-AMP synthase pathway (Rodero et al., 2017). Familial chilblain lupus is a further monogenic autoinflammatory disease which is a form of cutaneous lupus erythematosus caused by a gain-of-function in STING, or loss-of-function mutations in the nuclease genes TREX1 and SAMHD1 (Konig et al., 2017).

The autoimmune disease systemic lupus erythematosus (SLE) has also been linked with cGAS pathway activity and type I interferon induction via STING, providing a mechanism for how cGAS-STING may amplify or accelerate disease symptoms. Sjögren's syndrome is a further autoimmune disorder characterized by an increased type 1 interferon gene signature (Decout et al., 2021).

Inflammation is also a prominent hallmark of several neurodegenerative diseases, and elevated levels of type I interferons and contributions of STING have also been observed in models and disease samples of Huntington disease (Decout et al., 2021).

Other diseases and conditions linked to STING and activation of the cyclic GMP-AMP synthase pathway include rheumatoid arthritis, ischaemic brain injury, Parkinson disease, general neurodegeneration, amyotrophic lateral sclerosis and frontotemporal dementia, systemic sclerosis, age-dependent macular degeneration, traumatic brain injury, metabolic dysfunction-associated steatohepatitis (previously termed non-alcoholic steatohepatitis), alcoholic liver disease, acute pancreatitis, silica-induced fibrosis, sepsis, cardiovascular diseases, myocardial infarction, chronic heart failure, colorectal cancer, skin cancer, metastases, senescence, and aging (Decout et al., 2021, Paul et al., 2022 and Seok et al., 2023).

The identification of the link to these diseases and conditions has led increased focus on STING as a potential therapeutic target. The search for STING inhibitors has yielded several small molecules targeting STING which have anti-inflammatory effects. So far there have been two main categories of STING inhibitor design. The first uses molecules which bind non-covalently to STING and act as competitive antagonists of STING activators. Examples of this type of inhibitor molecule are isoquinolones as described in WO2019/182886, the content of which is hereby incorporated by reference. Isoquinolone compounds have been shown to bind to STING in a 2:1 stoichiometry, wherein two of their small molecules bind one STING dimer (Siu et al. 2019). Secondly, inhibitors have been designed that bind covalently to cysteine residues in the transmembrane domain of STING. Cysteine-reactive molecules are prone to being non-specific, which is due to covalent binding to cysteine in other non-target proteins, meaning STING inhibitors which bind covalently have a higher risk of off-target activities when used therapeutically. More recently, targeted STING degradation using 'molecular glue' degraders has been explored, with a one compound being identified which can induce STING degradation (Mutlu et al. 2024). However, while targeted STING degradation was demonstrated in this study, the potency of the reported degrader is relatively low.

An ongoing need exists in the art for effective treatments of disease associated with unwanted or uncontrolled activation of STING. However, STING antagonists rely on high systemic drug exposures to maintain sufficient STING inhibition, which increases the risk of unwanted off-target effects. Furthermore, existing STING antagonists are less effective in the treatment of diseases driven by constitutive (agonist-free) activation of STING.

The present invention has been devised in light of the above considerations.

### Summary of the Invention

A first aspect provides a compound of formula (I):

***S1-L-E*** (I),

wherein:
S1 is a STING ligand;
L is a Linker;
E is a E3 ubiquitin ligase ligand,
and pharmaceutically acceptable salts thereof,
wherein the STING ligand does not form a covalent bond with STING and
wherein the STING ligand does not exhibit STING agonist activity.

In some embodiments, the compound is suitable for the degradation of Stimulator of Interferon Genes (STING).

In some embodiments, the E3 ubiquitin ligase ligand is a Cereblon (CRBN) E3 ubiquitin ligase ligand.

In some embodiments, the E3 ubiquitin ligase ligand is a von Hippel-Lindau (VHL) E3 ubiquitin ligase ligand

In some embodiments, the E3 ubiquitin ligase ligand is an Inhibitor of Apoptosis (IAP) E3 ubiquitin ligase ligand.

In some embodiments, L connects the STING ligand to the E3 ubiquitin ligase ligand via a chain of atoms having 10-40 atoms in shortest length.

In some embodiments, the compound is of formula (I-1): 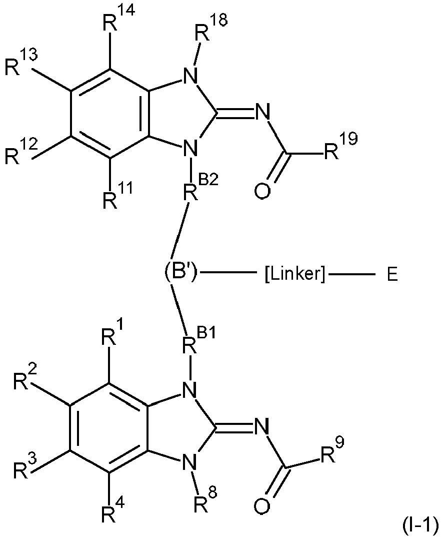
or a tautomer or pharmaceutically acceptable salt thereof,
wherein R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl);
R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -O-P(O)(OH)₂, C₁₋₄ alkoxy, NH₂, NH(C₁₋₆ alkyl), N(C₁₋₆ alkyl)₂, CO₂(C₁₋₆ alkyl) and halogen;
R³ and R¹³ are each independently selected from H, C(=O)NH₂; optionally substituted - C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, NH(C₁₋₆ alkyl), N(C₁₋₆ alkyl)₂, NH₂, CO₂(C₁₋₆ alkyl) and halogen;
R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH(C₁₋₆ alkyl), NH₂, CO₂(C₁₋₆ alkyl) and halogen;
R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -O-P(O)(OH)₂, C₁₋₄ alkoxy, N(C₁₋₆ alkyl)₂, NH(C₁₋₆ alkyl), NH₂, CO₂(C₁₋₆ alkyl) and halogen;
R⁸ and R¹⁸ are each independently selected from H, and C₁₋₄ alkyl;
B' taken together with R^{B1} and R^{B2} forms a linking group having 3-7 atoms in shortest length;
B' is the point of attachment to the Linker group L as follows: 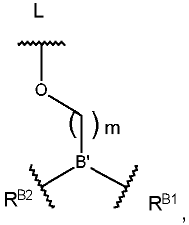
wherein m is 0 or 1;
the Linker (L) is a saturated or a partially or fully unsaturated framework comprising C and H atoms and at least one heteroatom, wherein said framework has a minimum length of from 10 to 40 atoms between B' and E; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-CO-R^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, C!, and CN;
wherein each R^{L} is independently selected from C₁₋₆ alkyl, C₂₋₆ alkenyl, C₂₋₆ alkynyl, C₃₋₆ cycloalkyl, C₃₋₆ carbocyclyl, C₅₋₆ heterocyclyl, C₅₋₆ aryl, and C₅₋₆ heteroaryl; and
E is an E3 ubiquitin ligase ligand.

In some embodiments, R^{B1} and R^{B2} are each independently selected from (-CH₂-)₁₋₂ or a bond and B' taken together with R^{B1} and R^{B2} forms an alkyl linking group having 3-7 atoms in shortest length; and B' is the point of attachment to the Linker group L as follows: 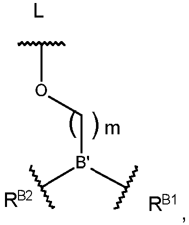
wherein B' is CH or C₁₋₅ alkyl;
wherein m is 0 or 1.

In a second aspect, the present invention provides the compound of the first aspect, or a pharmaceutically acceptable salt thereof, for use in a method of treatment of a disorder or condition in a subject, the method comprising administering a therapeutically effective amount of the compound to a subject in need thereof.

In a third aspect, the present invention provides a compound according to the first aspect or a pharmaceutically acceptable salt thereof, for use as a medicament.

In a fourth aspect, the present invention provides a use of a compound according to the first aspect, or a pharmaceutically acceptable salt thereof, for the manufacture of a medicament for the treatment of a disease or condition a subject in need thereof.

In a fifth aspect, the present invention provides a method of treatment of a disease or condition in a subject comprising administering a therapeutically effective amount of a compound of the first aspect, or a pharmaceutically acceptable salt thereof, to a subject in need thereof.

In a sixth aspect, the present invention provides a use of the compound of the first aspect, or a pharmaceutically acceptable salt thereof, in the manufacture of a medicament for the treatment of a disease or condition in a subject in need thereof.

In a seventh aspect, the present invention provides a pharmaceutical composition comprising the compound according to any one of the preceding claims, or a pharmaceutically acceptable salt thereof, and a pharmaceutically acceptable excipient.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

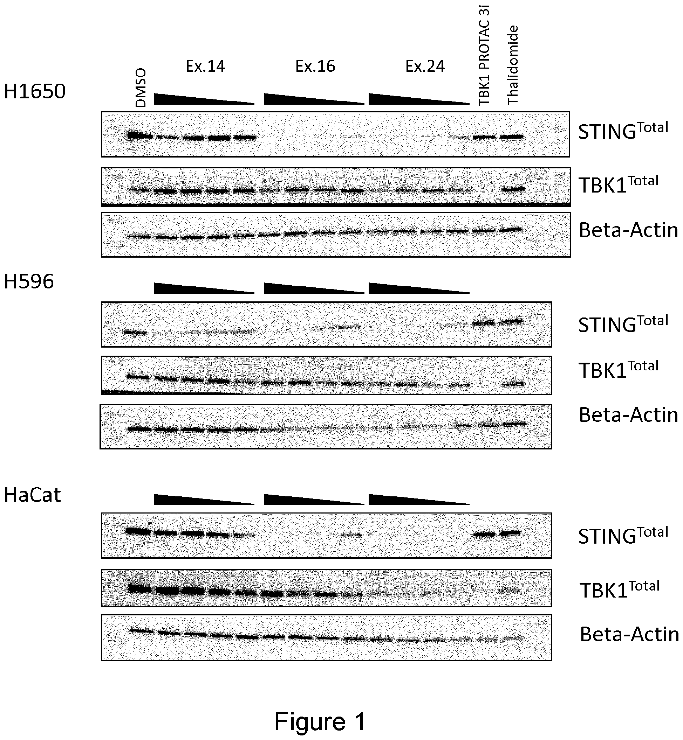

**Figure 1****.** Western Blot analysis of STING protein degradation in H1650 cells (top panel), H596 cells (middle panel), and HaCat cells (bottom panel) following treatment with vehicle control (DMSO), Example compound 14, Example compound 16, Example compound 24, positive control TBK1 PROTAC^{®} 3i (CAS-no: 2052306-13-3; Bio-Techne, cat.# 7259) or 2µM thalidomide (MedChemExpress, cat.# HY-14658). Cells were treated with 0.22 µM, 0.67 µM, 2 µM, or 6 µM of Example compounds 14, 16 or 24. Example compounds 16 and 24 induced STING degradation in all cell lines tested at all concentrations tested. Example compound 14 induced visible degradation in H1650 and H596 at higher concentrations.

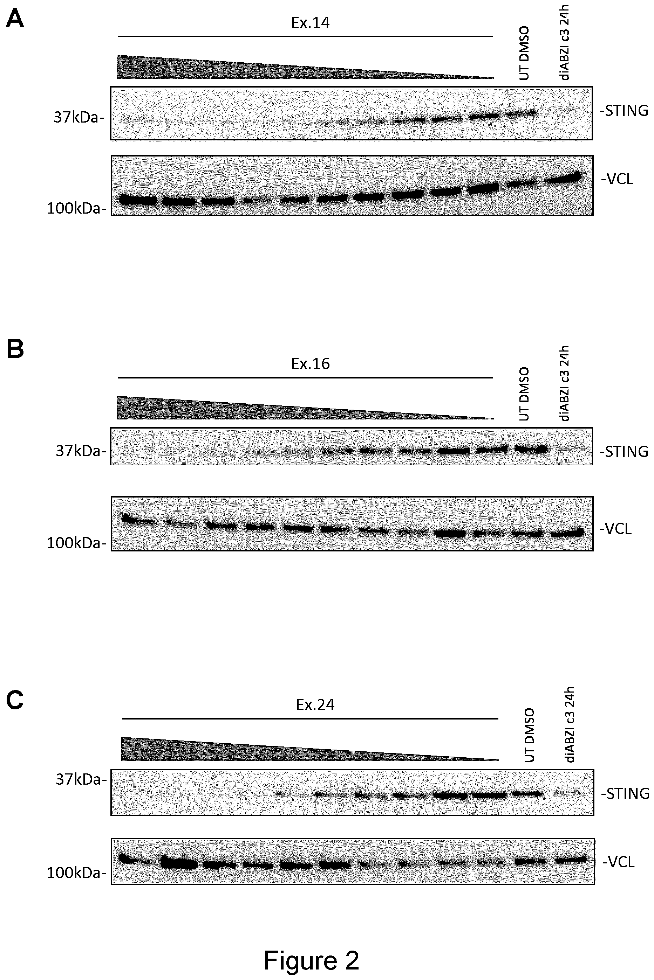

**Figure 2****.** Western Blots analysis of STING protein degradation in mouse RAW264.7 cells. RAW264.7 cells were treated with 0.02 µM - 10 µM of either (A) Example compound 14; (B) Example compound 16; or (C) Example compound 24. Controls in (A) - (C) included UT (DMSO) and 1.8µM diABZI treated cells. Figures 2A-C demonstrate dose-dependent STING degradation upon treatment with increasing concentrations of Example compounds. UT DMSO = untreated DMSO.

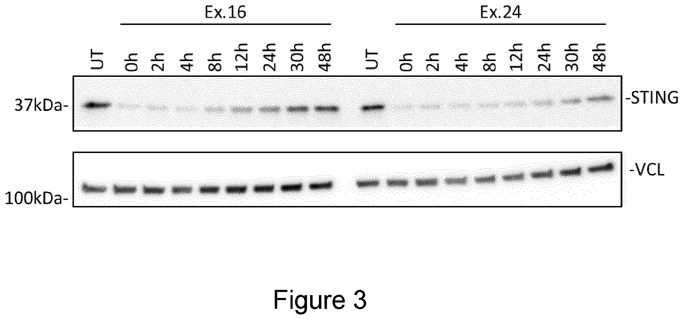

**Figure 3****.** Western blot analysis of STING protein recovery following 24 hours treatment with, and washout of, Example compound 16 or Example compound 24. 48 hours after Example compound washout, STING protein levels failed to recover to untreated (UT DMSO) levels, indicating that Example compounds persisted in cells for an extended period and exerted prolonged protein degradative effects. VCL = vinculin.

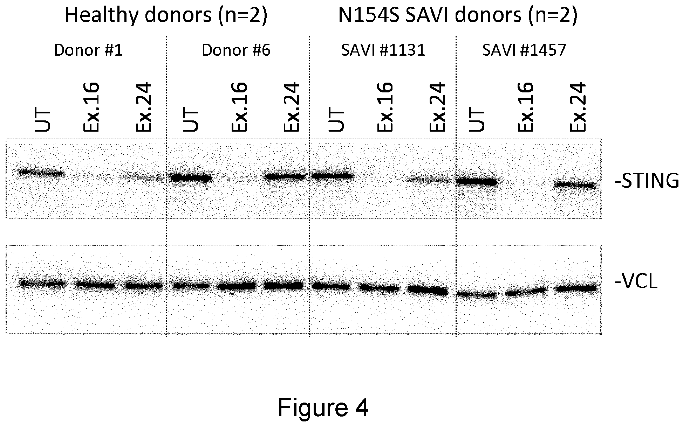

**Figure 4****.** Western blot analysis of STING protein degradation in healthy donor fibroblasts (Donor #1 and Donor #2) and STING-associated vasculopathy with onset in infancy (SAVI) patient fibroblast (SAVI 1131 and SAVI 1457) comprising a constitutively active STING protein harboring a N154S mutation. Both Example compound 16 and Example compound 24 induced STING degradation in healthy and patient fibroblast cells, with Example compound 16 inducing near complete degradation. UT = untreated. VCL = vinculin.

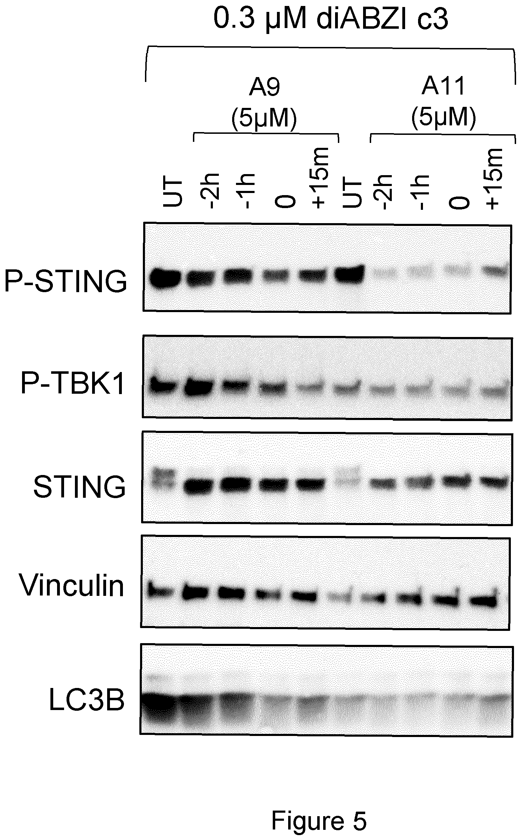

**Figure 5****.** Western Blot analysis of S1 STING ligand antagonism in human skin fibroblast. Cells were treated with 5 µM of A9 or A11 compound either before, simultaneously with, or after activation with 0.3 µM diABZI. Cells were either left untreated with Example compound (UT), pre-treated for 2 hours (-2h) before activation, pre-treated for 1 hour (-1h), treated with A9 or A11 simultaneously with diABZI c3 (0), or treated with A9 or A11 15 minutes after activation (+15m). The WB membrane was probed for p-STING, p-TBK1, STING, Vinculin and LC3B. Pretreatment, simultaneous treatment or post-treatment with either A9 or A11 inhibits diABZI c3-induced STING activation, as shown by a decrease in P-STING relative to cells treated only with diABZI c2 (UT). A9 was further demonstrated to inhibit diABZI c3-induced STING activation by a decrease in LC3B lipidation, a downstream effector of STING.

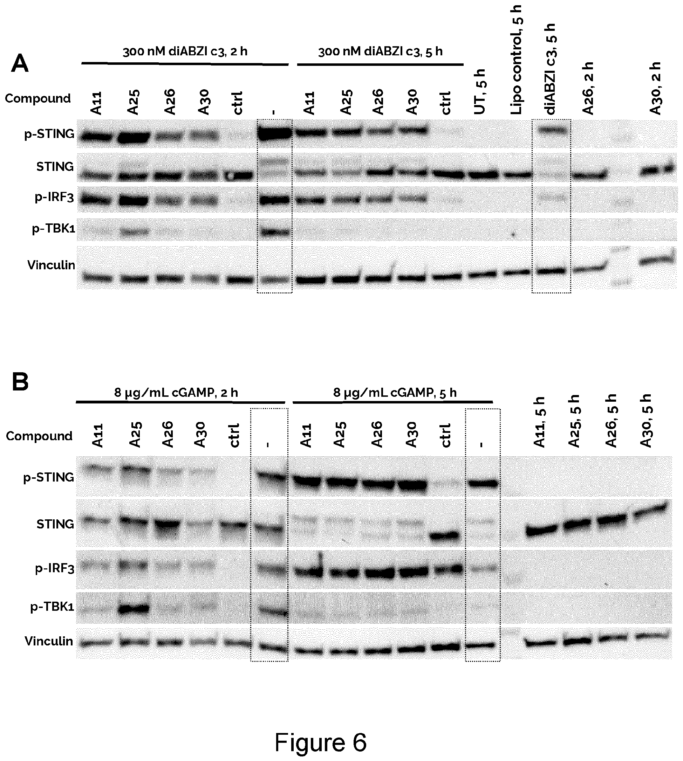

**Figure 6****.** Western Blot analysis of inhibition of STING activation in HFF-1 cells. (A) HFF-1 cells treated with 5 µM of STING binder compounds A11, A25, A26, A30, or 0.5 µM positive control compound for 15 min followed by 300 nM diABZI and incubated for 2 h or 5 h. As controls, cells were left untreated before activation with 300 nM diABZI and incubated for 2 h (-), left untreated (UT), treated with Lipofectamine (Lipo control), A26 or A30 as single agent. (B) shows HFF-1 cells treated with 5 µM of STING binder compounds A11, A25, A26, A30, or 0.5 µM STING antagonist for 15 min followed with 8 µg/mL Lipo-cGAMP and incubated for 2 h or 5 h.. As controls, cells were left untreated before activation with 8 µg/mL Lipo-cGAMP and incubated for 2 h (-), or treated with A11, A25, A26 or A30 as single agent. Western Blot membranes were probed for p-STING, STING, p-IRF3, p-TBK1, and Vinculin. Compared to cells which were left untreated prior to activation with diABZI c3 or cGAMP, STING binder compounds A11, A26 and A30 inhibited STING, IRF3 and TBK1 phosphorylation and activation. A11, A25, A26, A30 do not induce STING, IRF3 or TBK1 phosphorylation and activation alone.

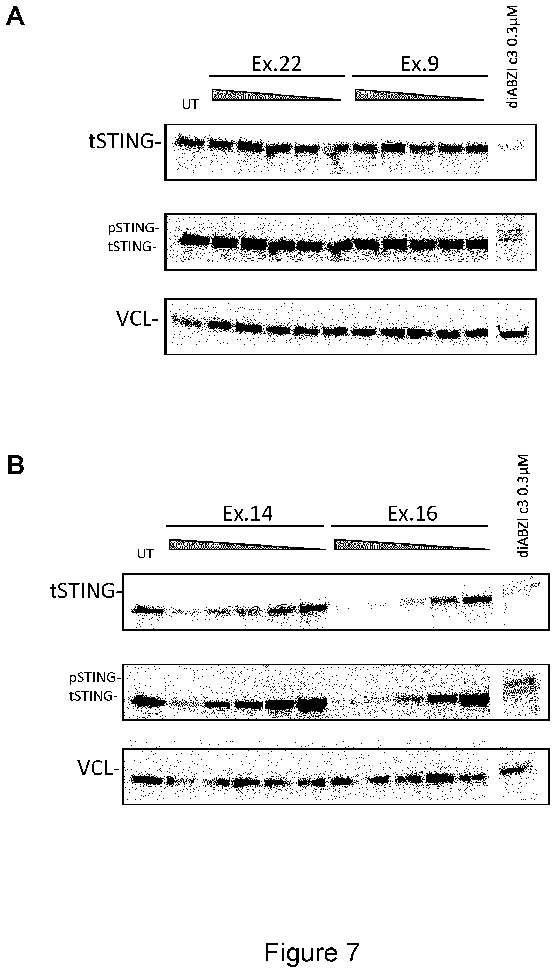

**Figure 7****.** Western Blot analysis of STING phosphorylation in human fibroblast cells following treatment with (top panel) Example compound 22 or Example compound 9 and (bottom panel) Example compound 14 or Example compound 16. Cells were incubated in 3.6 nM to 7.2 µM Example compound or 0.3 µM diABZI c3 for 24 hours prior to lysis. No Example compound induced STING phosphorylation indicating that these compounds are not STING agonists.

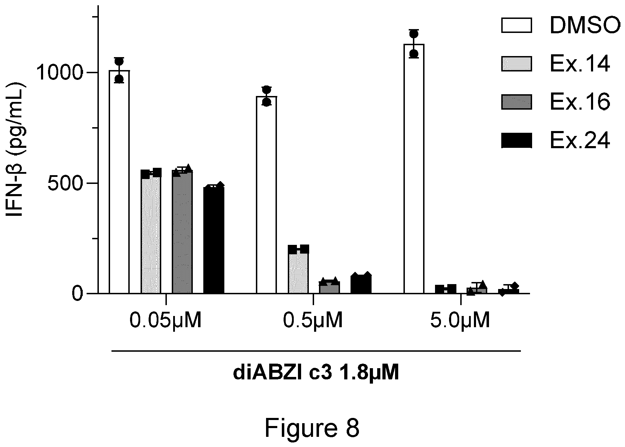

**Figure 8****.** Analysis of IFN-β secretion from RAW264.7 mouse cells treated with (from left to right), 1.8 µM diABZI only, 1.8 µM diABZI + 0.05 µM Example compound 14, 1.8 µM diABZI + 0.05 µM Example compound 16, 1.8 µM diABZI + 0.05 µM Example compound 24, 1.8 µM diABZI only, 1.8 µM diABZI + 0.5 µM Example compound 14, 1.8 µM diABZI + 0.5 µM Example compound 16, 1.8 µM diABZI + 0.5 µM Example compound 24, 1.8 µM diABZI only, 1.8 µM diABZI + 5 µM Example compound 14, 1.8 µM diABZI + 5 µM Example compound 16, 1.8 µM diABZI + 5 µM Example compound 24. Example compounds reduce STING-induced secretion of IFN-β in mouse RAW246.7 cells.

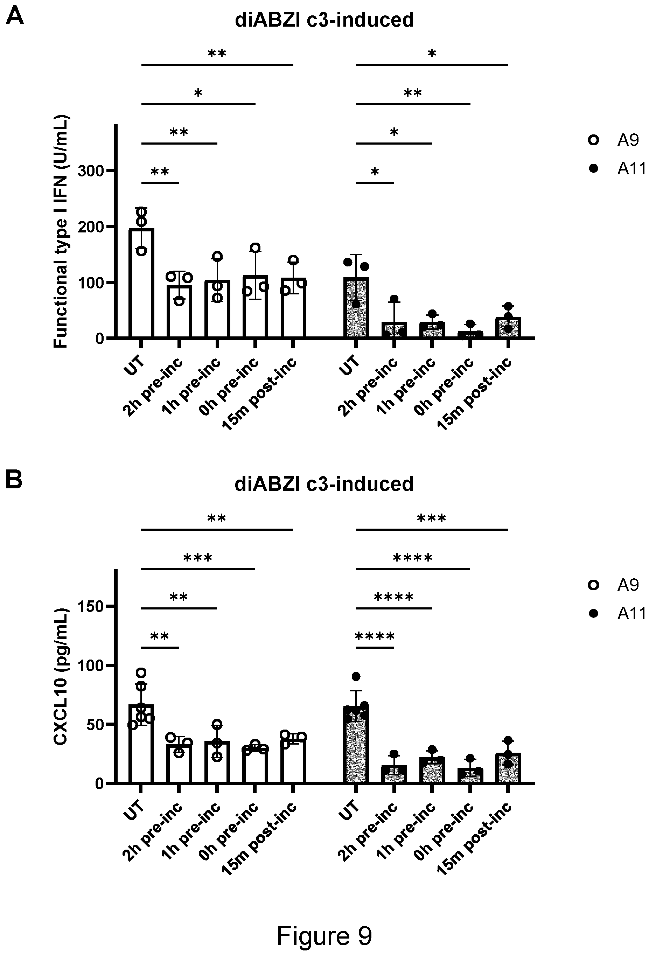

**Figure 9****.** Analysis of secretion of functional IFN-β (A) and CXCL10 (B) from human skin fibroblasts. Cells were treated with 5 µM of A9 or A11 compound either before, simultaneously with, or after activation with 0.3 µM diABZI. Cells were either left untreated with Example compound (UT), pre-treated for 2 hours before activation (-2hr), pre-treated for 1 hour before activation (-1hr), treated with A9 or A11simulataneously with diABZI c3 (0), or treated with A9 or A11 15 minutes after activation (+15m). Pretreatment, simultaneous treatment, or post-treatment with either A9 or A11 inhibited diABZI c3-induced STING activation and consequently inhibits secretion of Functional Type I IFN (A) and CXCL10 (B).

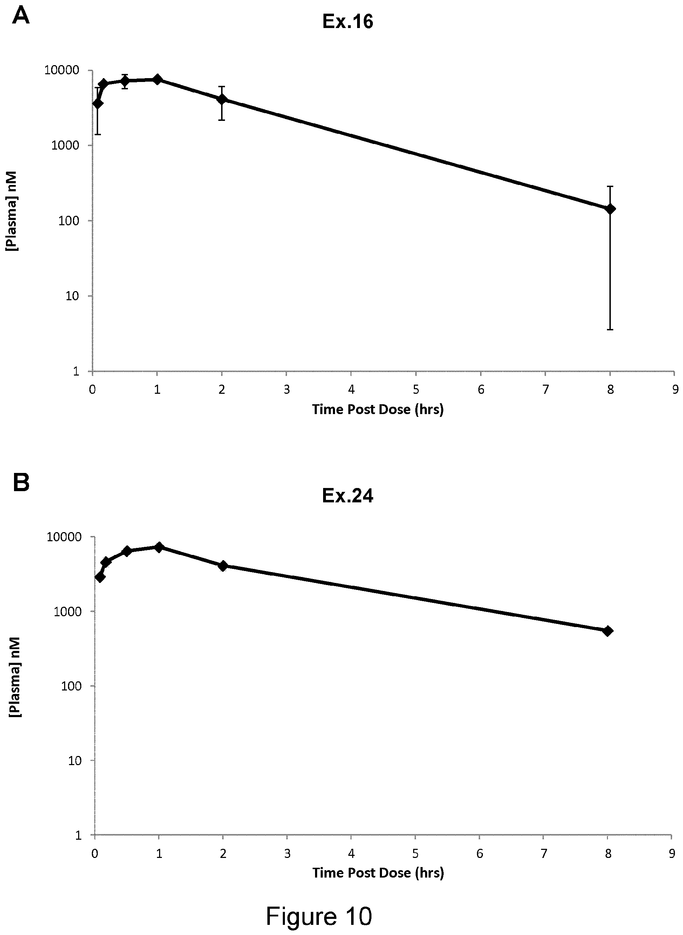

**Figure 10****.** Pharmacokinetic profiling in mice injected with (A) 10 mg/kg Example compound 16 or (B) 8 mg/kg Example compound 24. Compounds were subcutaneously (SC) injected, and plasma concentrations of the compounds measured over the course of 8 hours.

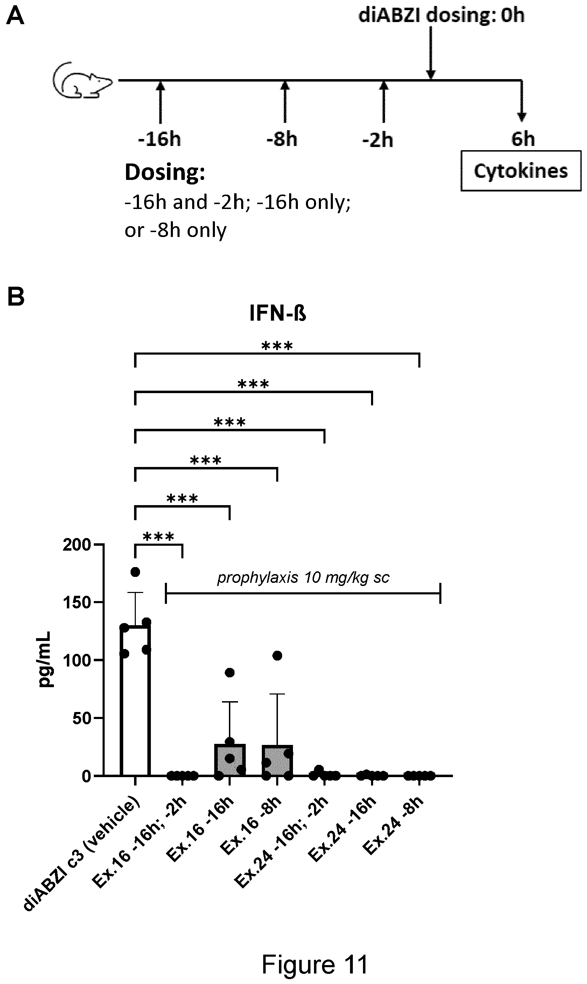

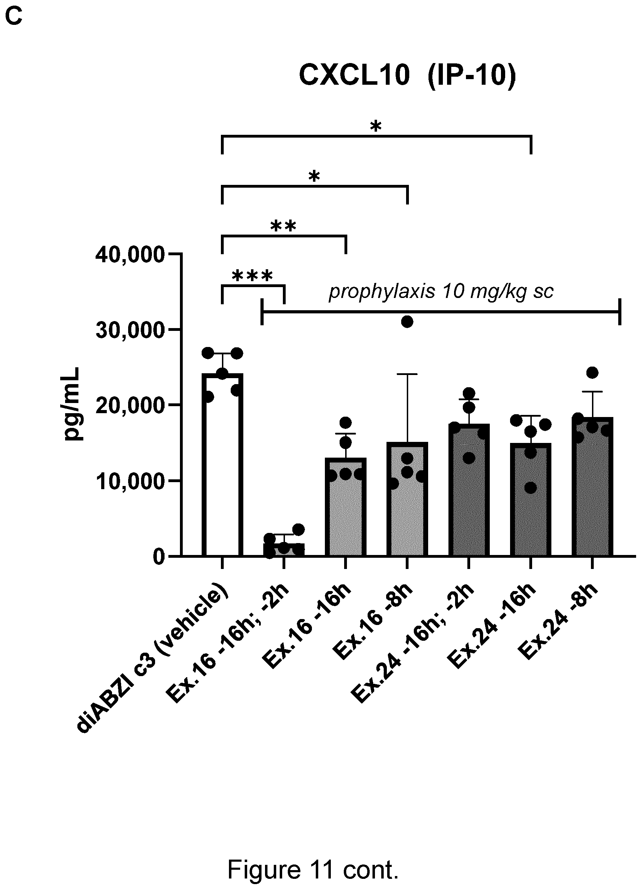

**Figure 11****.** (A) Schematic diagram showing the dosing arms of mice pre-treated with Example compound 16 followed by 1 mg/kg diABZi treatment at t = 0 hours. Dosing arms are as followings; i) t = -16h and t = -2h; ii) t = - 16h only; and iii) t = -8h only. This dosing arm scheme was used for both Example compound 16 and Example compound 24 treatment arms. (B) and (C) show the reduction in IFN-β and CXCL10, respectively, in mice treated with 10 mg/kg Example compound 16 or 10 mg/kg Example compound 24 compared to mice treated with 1 mg/kg diABZi alone.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Definitions

### Substituents

The phrase "optionally substituted" as used herein, pertains to a parent group which may be un substituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

Examples of substituents are described in more detail below.

Unless otherwise stated, halogen or halo is selected from chloro (Cl), fluoro (F), bromo (Br) and iodo (I), such as fluoro.
Hydroxy: -OH.
Oxo: =O (oxygen double bonded to the rest of the molecule).

C₁₋₁₂ hydrocarbon: The term "C₁₋₁₂ hydrocarbon" as used herein pertains to a monovalent moiety obtained by removing a hydrogen atom from a carbon atom of a hydrocarbon compound having from 1 to 12 carbon atoms, which may be aliphatic or alicyclic, which may be saturated or unsaturated (e.g. partially unsaturated, fully unsaturated) and may also be branched. Thus, the term "hydrocarbon" includes the terms alkyl, alkenyl, alkynyl, cycloalkyl, etc., discussed below.

C₁₋₈ alkyl: The term "C₁₋₈ alkyl" as used herein, pertains to a monovalent moiety obtained by removing a hydrogen atom from a carbon atom of a hydrocarbon compound having from 1 to 8 carbon atoms, which may be aliphatic or alicyclic, and which are saturated and may also be branched. The term "C₁₋₆ alkyl" as used herein, pertains to a monovalent moiety obtained by removing a hydrogen atom from a carbon atom of a hydrocarbon compound having from 1 to 6 carbon atoms, which may be aliphatic or alicyclic, and which are saturated. The term "C₁₋₄ alkyl" as used herein, pertains to a monovalent moiety obtained by removing a hydrogen atom from a carbon atom of a hydrocarbon compound having from 1 to 4 carbon atoms, which may be aliphatic or alicyclic, and which are saturated. The term "C₁₋₈ alkyl" as used herein encompasses both linear and branched alkyl groups. Thus, the term "alkyl" includes the sub-classes cycloalkyl, discussed below.
Me: methyl
Et: ethyl.

Examples of saturated alkyl groups include, but are not limited to, methyl (C₁), ethyl (C₂), propyl (C₃), butyl (C₄), pentyl (C₅) hexyl (C₆), heptyl (C₇), octyl (C₈).

Examples of saturated linear alkyl groups include, but are not limited to, methyl (C₁), ethyl (C₂), n-propyl (C₃), n-butyl (C₄), n-pentyl (amyl) (C₅) and n-hexyl (C₆).

Examples of saturated branched alkyl groups include iso-propyl (C₃), iso-butyl (C₄), sec-butyl (C₄), tert-butyl (C₄), iso-pentyl (C₅), and neo-pentyl (C₅).

C₃₋₁₀ cycloalkyl: The term "C₃₋₁₀ cycloalkyl" as used herein, pertains to an alkyl group which is also a cyclyl group; that is, a monovalent moiety obtained by removing a hydrogen atom from a ring atom of a cyclic hydrocarbon (carbocyclic) compound, which moiety has from 3 to 10 carbon atoms, including from 3 to 10 ring atoms. The carbocyclic ring may be saturated or unsaturated and may be bridged or unbridged. The ring may be a fused ring or a single ring.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) methylcyclohexane (C₇), cyclooctane (C₈), cyclononane (C₉) and cyclodecane (C₁₀);
unsaturated monocyclic hydrocarbon compounds:
   cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

C₂₋₈ alkenyl: The term "C₂₋₈ alkenyl" as used herein, pertains to a non-aromatic hydrocarbon group having one or more carbon-carbon double bonds.

Examples of unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₈alkynyl: C₂₋₈ alkynyl: The term "C₂₋₈ alkynyl" as used herein, pertains to a hydrocarbon group having one or more carbon-carbon triple bonds.

Examples of unsaturated alkynyl groups include, but are not limited to, ethynyl (-C=CH), 1-propynyl (-C≡CCH₃) and 2-propynyl (propargyl, -CH₂C≡CH).

Ether: -OR, or -R-O-R- wherein R is an ether substituent, for example, a C₁₋₁₂ hydrocarbon group (also referred to as a C₁₋₁₂ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

C₁₋₆ alkoxy: The term C₁₋₆ alkoxy as used herein, pertains to an OR group, wherein R is an C₁₋₆ alkyl group. Examples of C₁₋₆ alkoxy groups include, but are not limited to, OMe, Oet (ethoxy), -O(nPr) (n-propoxy), -O(iPr) (isopropoxy), O(nBu) (n-butoxy), O(sBu) (sec-butoxy), O(iBu) (isobutoxy), and O(tBu) (tert-butoxy).

C₃₋₄ alkenoxy: The term C₃₋₄ alkenoxy as used herein, pertains to an OR group, wherein R is an C₃₋₄ alkenyl group. Examples of C₃₋₄ alkenoxy include O-CH=CH-CH₃, O-C(CH₃)=CH₂, or O-butenyl.

C₃₋₄ alkynoxy: The term C₃₋₄ alkynoxy as used herein, pertains to an OR group, wherein R is a C₃₋₄alkynyl group. Examples of C₃₋₄ alkynoxy groups include O-CHzC=CH, OCH₂CH₂C≡CH or O-C=CCHs.

C₃₋₁₀ heteroalkyl; The term C₃₋₁₀ heteroalkyl as used herein pertains to a C₃₋₁₀ alkyl group which chain is interrupted by 1 to 3 heteroatoms selected from O, S and N. In other words, a C₃₋₁₀ alkyl chain, which chain includes 1 to 3 heteroatoms selected from O, S and N. Examples of C₃₋₁₀ heteroalkyl groups include but are not limited to CH₂CH₂OCH₃, CH₂CH₂OCH₂CH₃, CH₂CH₂OCH₂CH₂CH₃, CH₂OCH₂CH₃, CH₂OCH₂CH₂CH₃, CH₂OCH₂CH₂CH₂CH₃, CH₂CH₂OCH₂CH₂CH₂CH₂CH₃, CH₂OCH₂CH₂CH₂CH₂CH₃, CH₂CH₂CH₂OCH₃, CH₂CH₂CH₂CH₂CH₂CH₂OCH₃, CH₂CH₂SCH₃, CH₂CH₂SCH₂CH₃, CH₂CH₂SCH₂CH₂CH₃, CH₂SCH₂CH₃, CH₂SCH₂CH₂CH₃, CH₂SCH₂CH₂CH₂CH₃, CH₂CH₂SCH₂CH₂CH₂CH₂CH₃, CH₂SCH₂CH₂CH₂CH₂CH₃, CH₂CH₂CH₂SCH₃, CH₂CH₂CH₂CH₂CH₂CH₂SCH₃, CH₂CH₂NHCH₃, CH₂CH₂NHCH₂CH₃, CH₂CH₂NHCH₂CH₂CH₃, CH₂NHCH₂CH₃, CH₂NHCH₂CH₂CH₃, CH₂NHCH₂CH₂CH₂CH₃, CH₂CH₂NHCH₂CH₂CH₂CH₂CH₃, CH₂NHCH₂CH₂CH₂CH₂CH₃, CH₂CH₂CH₂NHCH₃, CH₂CH₂CH₂CH₂CH₂CH₂NHCH₃, CH₂CH₂N(CH₂)₂, CH₂N(CH₂CH₂)₂ CH₂N(CH₂CH₂CH₂)₂, CH₂NHCH₂CH₂CH₂OCH₂, CH₂NHCH₂CH₂OCH₃, CH₂NHCH₂CH₂OCH₂CH₃, CH₂CH₂NHCH₂CH₂CH₂OCH₂CH₃, CH₂NHCH₂CH₂CH₂OCH₂CH₃, CH₂OCH₂CH₂NHCH₃, CH₂CH₂CH₂OCH₂CH₂CH₂NHCH₃, CH₂OCH₂N(CH₂)₂, CH₂N(CH₂CH₂OCH₂)₂, CH₂NHCH₂SCH₃, CH₂NHCH₂CH₂SCH₃, CH₂NHCH₂CH₂SCH₂CH₃, CH₂CH₂NHCH₂CH₂CH₂SCH₂CH₃, CH₂NHCH₂CH₂SCH₂CH₂CH₃, CH₂SCH₂CH₂NHCH₃, CH₂CH₂SCH₂CH₂CH₂CH₂NHCH₃, CH₃NHCH₂CH₂CH₂OCH₃

C₃₋₁₀ heteroalkenyl; The term C₃₋₁₀ heteroalkenyl as used herein pertains to a C₃₋₁₀ alkenyl group which chain is interrupted by 1 to 3 heteroatoms selected from O, S and N. In other words, a C₃₋₁₀ alkenyl chain, which chain includes 1 to 3 heteroatoms selected from O, S and N. Examples of C₃₋₁₀ heteroalkenyl groups include but are not limited to CH=CHOCH₃, CH=CHOCH₂CH₃, CH=CHOCH₂CH₂CH₃, CH₂OCH=CH₂, CH₂OCH₂CH=CH₂, CH₂OCH₂CH=CHCH₃, CH₂CH₂OCH₂CH=CHCH₂CH₃, CH₂OCH₂CH₂CH₂CH=CH₂, CH=CHCH₂OCH₃, CH₂CH₂CH=CHCH₂CH₂OCH₃, CH=CHSCH₃, CH=CHSCH₂CH₃, CH=CHSCH₂CH₂CH₃, CH₂SCH=CH₂, CH₂SCH₂CH=CH₂, CH₂SCH₂CH=CHCH₃, CH₂CH₂SCH₂CH=CHCH₂CH₃, CH₂SCH₂CH₂CH₂CH=CH₂, CH=CHCH₂SCH₃, CH₂CH₂CH=CHCH₂CH₂SCH₃, CH=CHNHCH₃, CH=CHNHCH₂CH₃, CH=CHNHCH₂CH₂CH₃, CH₂NHCH=CH₂, CH₂NHCH₂CH=CH₂, CH₂NHCH₂CH₂CH=CH₂, CH₂CH₂NHCH₂CH₂CH=CHCH₃, CH₂NHCH₂CH₂CH=CHCH₃, CH₂CH₌CHNHCH₃, CH₂CH₌CHCH₂CH₂CH₂NHCH₃, CH₌CHN(CH₂)₂, CH₂N(CH₌CH)₂ CH₂N(CH₂CH₌CH)₂, CH₂NHCH₂OCH₂CH=CH₂, CH₂OCH₂NHCH₂CH₂CH₌CHCH₃, CH₂NHCH₂OCH₂CH=CHCH₃, CH₂CH₌CHNHOCH₃, CH₂CH₌CHCH₂OCH₂CH₂NHCH₃

C₃₋₁₀ heteroalkynyl; The term C₃₋₁₀ heteroalkynyl as used herein pertains to a C₃₋₁₀ alkynyl group which chain is interrupted by 1 to 3 heteroatoms selected from O, S and N. In other words, a C₃₋₁₀ alkynyl chain, which chain includes 1 to 3 heteroatoms selected from O, S and N. Examples of C₃₋₁₀ heteroalkynyl groups include but are not limited to CH≡COCH₃, CH≡COCH₂CH₃, CH≡COCH₂CH₂CH₃, CH₂OC≡CH, CH₂OCH₂C≡CH, CH₂OCH₂C≡CCH₃, CH₂CH₂OCH₂C≡CCH₂CH₃, CH₂OCH₂CH₂CH₂C≡CH, CH≡CCH₂OCH₃, CH₂CH₂C≡CCH₂CH₂OCH₃, CH≡CSCH₃, CH≡CSCH₂CH₃, CH≡CSCH₂CH₂CH₃, CH₂SC≡CH, CH₂SCH₂C≡CH, CH₂SCH₂C≡CCH₃, CH₂CH₂SCH₂C≡CCH₂CH₃, CH₂SCH₂CH₂CH₂C≡CH, CH≡CCH₂SCH₃, CH₂CH₂C≡CCH₂CH₂SCH₃, CH≡CNHCH₃, CH≡CNHCH₂CH₃, CH≡CNHCH₂CH₂CH₃, CH₂NHC≡CH, CH₂NHCH₂C≡CH, CH₂NHCH₂CH₂C≡CH, CH₂CH₂NHCH₂CH₂C≡CCH₃, CH₂NHCH₂CH₂C≡CCH₃, CH₂C≡CNHCH₃, CH₂C≡CCH₂CH₂CH₂NHCH₃, CH≡CN(CH₂)₂, CH₂N(C≡CH)₂ CH₂N(CH₂C≡CH)₂.

Amino: -NR¹R², wherein R¹ and R² are independently amino substituents, for example, hydrogen, a C₁₋₆ hydrocarbon group (also referred to as C₁₋₆ alkylamino or C₁₋₆ dialkylamino), or, in the case of a "cyclic" amino group, R¹ and R², taken together with the nitrogen atom to which they are attached, form a heterocyclic ring having from 4 to 6 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to -NH₂, -NHCH₃, -NHC(CH₃)₂, -N(CH₃)₂, -N(CH₂CH₃)₂, N(C₁₋₆ alkyl)₂ and -NHPh. Examples of cyclic amino groups include, but are not limited to, aziridino, azetidino, pyrrolidino, piperidino, piperazino, morpholino, and thiomorpholino.

C₁₋₆ alkyl amido: The term C₁₋₆ alkyl amido (also referred to as carbamoyl, carbamyl, aminocarbonyl, carboxamide) has the structure -C(=O)NR¹R², wherein R¹ and R² are independently amino substituents, as defined for amino groups for example, hydrogen, a C₁₋₆ hydrocarbon group (also referred to as C₁₋₆ alkylamido or C₁₋₆ dialkylamido), or, in the case of a "cyclic" amido group, R¹ and R², taken together with the nitrogen atom to which they are attached, form a heterocyclic ring having from 4 to 6 ring atoms. Examples of amido groups include, but are not limited to, -C(=O)NH₂, -C(=O)N(C₁₋₆ alkyl)₂, C(=O)NH(C₁₋₆ alkyl), such as -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Carbonyl: a carbonyl functional group refers to a C=O bond such as the following structure R-C(=O)-R' wherein R and R¹ are independently carbonyl substituents which can be organic groups or H atoms for example a C₁₋₆hydrocarbon group , C₆₋₁₀ aryl, C₃₋₆ cycloalkyl, C₄₋₁₀ heterocyclyl, C₄₋₁₀ heterocyclyl or H. For example carbonyl groups include C(=O)-(C₁₋₄ alkyl), -C(=O)-(C₂₋₄ alkenyl), -C(=O)-(C₂₋₄ alkynyl), - C(=O)-(C₆₋₁₀ aryl), -C(=O)-(C₃₋₆ cycloalkyl), -C(=O)-(C₄₋₁₀ heterocyclyl), -C(=O)-( C₄₋₁₀ heterocyclyl), - C(=O)-(C₁₋₄ alkoxy), -C(=O)-(C₃₋₄ alkenoxy), -C(=O)-(C₃₋₄ alkynoxy), -C(=O)-(C₃₋₆ heteroalkyl), -C(=O)-(C₄₋₆ heteroalkenyl), or -C(=O)-(C₄₋₆ heteroalkynyl).

### Carboxy (carboxylic acid): -C(=O)OH (CO₂H).

Ester: If not stated otherwise, the term "ester" in this document refers to a carboxylic ester functional group with the formula R-C(=O)-OR' wherein R and R' are independently substituents which are organic groups. For example a C₁₋₆ hydrocarbon group. R can also be hydrogen. For example an ester group can be -CO₂(C₁₋₆ alkyl).

C₁₋₆ alkyl ester: The term C₁₋₆ alkyl ester (carboxylate, carboxylic acid ester, oxycarbonyl) has the structure -C(=O)OR, wherein R is a C₁₋₆ hydrocarbon group. For example, CO₂(C₁₋₆ alkyl) or CO₂C₁₋₄ alkyl. Examples of ester groups include, but are not limited to, -C(=O)OCH₃, -C(=O)OCH₂CH₃ and -C(=O)OC(CH₃)₃.

Amide: an amide functional group which has the structure R-C(=O)N(R')(R") wherein R, R' and R" are independently amide substituents which can be organic groups or H atoms for example a C₁₋₆hydrocarbon group or H.

Aminocarbonyloxy: -OC(=O)NR¹R², wherein R¹ and R² are independently amino substituents, as defined for amino groups. Examples of aminocarbonyloxy groups include, but are not limited to, -OC(=O)NH₂, - OC(=O)NH-CH₃, -OC(=O)N(CH₃)₂, and -OC(=O)N(CH₂-CH₃)₂.

### Phosphoric acid (phosphonooxy): -OP(=O)(OH)₂.

C₄₋₁₂ heterocyclyl: The term "C₄₋₁₂ heterocyclyl" as used herein, pertains to a monovalent moiety obtained by removing a hydrogen atom from a ring atom of a heterocyclic compound, which moiety has from 4 to 12 ring atoms, of which from 1 to 5 are ring heteroatoms. In certain embodiments, each ring has from 5 to 7 ring atoms, of which from 1 to 4 are ring heteroatoms. The ring may be saturated or unsaturated and may be bridged or unbridged. The ring may be a fused ring or a single ring. For the avoidance of doubt, substituents on the heterocyclyl ring may be linked via either a carbon atom or a heteroatom.

In this context, the prefixes (e.g. C₅₋₁₀ C₅₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrrolidine (C₅), pyrroline (e.g., 3-pyrroline, 2,5-dihydropyrrole) (C₅), 2H-pyrrole or 3H-pyrrole (C₅), piperidine (C₆), dihydropyridine (C₆), tetrahydropyridine (C₆), azepine (C₇), 1 ,2,3,4,-tetrahydroquinolone (C₁₀);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), pyrazole (C₅), piperazine (C₆), pyrimidine (C₆);
N₃: triazole (C₅)
N₄: tetrazole (C₅)
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂S₁: 6,7-dihydro-4H-thiazolo[5,4-c]pyridine-5-yl (C₉);
N₂O₁: oxadiazine (C₆);
O₁S₁: oxathiole (C₅) and oxathiane (thioxane) (C₆); and,
N₁O₁S₁: oxathiazine (C₆).

Examples of bicyclic heterocyclyl groups include, but are not limited to those derived from:

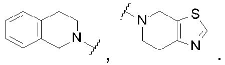

C₆₋₁₀ carboaryl: The term "C₆₋₁₀ carboaryl", as used herein, pertains to a monovalent moiety obtained by removing a hydrogen atom from an aromatic ring atom of an aromatic compound, which moiety has from 6 to 10 ring atoms and the ring atoms are all carbon atoms, as in "carboaryl groups". The ring may be a fused ring or a single ring. Examples of carboaryl groups include, but are not limited to, those derived from benzene (i.e. phenyl) (C₆), naphthalene (C₁₀) and azulene (C₁₀).

In this context, the prefixes (e.g. C₅₋₇, C₅₋₆, C₅₋₁₀, etc.) denote the number of ring atoms, or range of number of ring atoms. For example, the term "C₅₋₆ aryl" as used herein, pertains to an aryl group having 5 or 6 ring atoms.

Examples of carboaryl groups which comprise fused rings, at least one of which is an aromatic ring, include, but are not limited to, groups derived from indane (e.g. 2,3-dihydro-1H-indene) (C₉), indene (C₉), isoindene (C₉) and tetraline (1,2,3,4-tetrahydronaphthalene) (C₁₀). A tetralin has the following structure:

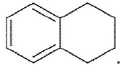

C₅₋₁₂ heteroaryl: The term "C₅₋₁₂ heteroaryl", as used herein, pertains to a monovalent moiety obtained by removing a hydrogen atom from an aromatic ring atom of an aromatic compound, which moiety has from 5 to 12 ring atoms of which from 1 to 5 are ring heteroatoms. In certain embodiments, each ring has from 5 to 7 ring atoms, of which from 1 to 4 are ring heteroatoms. For the avoidance of doubt, substituents on the heteroaryl ring may be linked via either a carbon atom or a heteroatom. The ring may be a fused ring or a single ring.

Examples of monocyclic heteroaryl groups include, but are not limited to, those derived from:
N₁: pyrrole (C₅), pyridine (C₆);
O₁: furan ((C₅);
S₁: thiophene (C₅);
N₁O₁ : oxazole (C₅), isoxazole (C₅)
N₂O₁: oxadiazole, such as 1 ,2,5-oxadiazole (furazan) (C₅), ;
N₃O₁: oxatriazole (C₅);
N₁S₁: 1 ,3-thiazole (C₅), 1 ,2-thiazole (isothiazole) (C₅);
N₂: imidazole (C₅), pyrazole (C₅), pyridazine (1 ,2-diazine) (C₆), pyrimidine (1 ,3-diazine) (C₆) (e.g., cytosine, thymine, uracil), pyrazine (1 ,4-diazine) (C₆);
N₃: triazole (C₅), triazine (C₆); and,
N₄: tetrazole (C₅).

For the avoidance of doubt, where multiple substituents are independently selected from a given group, the selected substituents may comprise the same substituents or different substituents from within the given group.

### Linker

The degrader/PROTAC^{®} linker connects the two functional motifs of a degrader/PROTAC^{®}, a target protein binder and an E3 ligase recruiter. An optimal linker will allow for maximal interaction between the target protein and the E3 ligase resulting in efficient ubiquitination of the target protein and its ultimate degradation. The linker length can vary from 1-45 atoms in length. The length and chemical composition of the linker affects the structural rigidity, hydrophobicity and solubility of a degrader/PROTAC^{®}. The structure of the linker is described in more detail below.

### Pharmaceutically acceptable salt

The term "pharmaceutically acceptable" is used to specify that an object (for example a salt, dosage form or excipient) is suitable for use in patients. An example list of pharmaceutically acceptable salts can be found in the Handbook of Pharmaceutical Salts: Properties, Selection and Use, P. H. Stahl and C. G. Wermuth, editors, Weinheim/Zürich: Wiley-VCH/VHCA, 2002. A suitable pharmaceutically acceptable salt of a compound of Formula (I-b) is, for example, an acid-addition salt. An acid addition salt of a compound of Formula (I-b) may be formed by bringing the compound into contact with a suitable inorganic or organic acid under conditions known to the skilled person. An acid addition salt may for example be formed using an inorganic acid selected from the group consisting of hydrochloric acid, hydrobromic acid, sulphuric acid and phosphoric acid. An acid addition salt may also be formed using an organic acid selected from the group consisting of trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid and para-toluenesulfonic acid.

Therefore, in one embodiment there is provided a compound of Formula (I), (I-1), (I-2), (I-2b), (I-2c) or (I2-d) or a pharmaceutically acceptable salt thereof, where the pharmaceutically acceptable salt is a hydrochloric acid, hydrobromic acid, sulphuric acid, phosphoric acid, trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid or para-toluenesulfonic acid salt. In one embodiment there is provided a compound of Formula (I), (I-1), (I-2), (I-2b), (I-2c) or (I2-d) or a pharmaceutically acceptable salt thereof, where the pharmaceutically acceptable salt is a methanesulfonic acid salt.

### Other forms

Compounds and salts described in this specification may exist in solvated forms and unsolvated forms. For example, a solvated form may be a hydrated form, such as a hemihydrate, a monohydrate, a dihydrate, a trihydrate or an alternative quantity thereof. The compounds of Formula (I-b) encompass all such solvated and unsolvated forms of compounds of Formula (I-b), particularly to the extent that such forms possess STING antagonist activity, as for example measured using the tests described herein.

Compounds and salts described in this specification include one or more chiral (i.e. asymmetric) centres. To the extent a structure or chemical name in this specification does not indicate the chirality, the structure or name is intended to encompass any single stereoisomer (i.e. any single chiral isomer) corresponding to that structure or name, as well as any mixture of stereoisomers (e.g. a racemate). In some embodiments, a single stereoisomer is obtained by isolating it from a mixture of isomers (e.g. a racemate) using, for example, chiral chromatographic separation. In other embodiments, a single stereoisomer is obtained through direct synthesis from, for example, a chiral starting material.

A particular enantiomer of a compound described herein may be more active than other enantiomers of the same compound.

According to one embodiment there is provided a compound of Formula (I-b), or a pharmaceutically acceptable salt thereof, which is a single enantiomer being in an enantiomeric excess (%ee) of ≥ 95, ≥ 98% or ≥ 99%. Conveniently, the single enantiomer is present in an enantiomeric excess (%ee) of ≥ 99%.

According to another embodiment there is provided a pharmaceutical composition, which comprises a compound of Formula (I-b), which is a single enantiomer being in an enantiomeric excess (%ee) of ≥ 95, ≥ 98% or ≥ 99% or a pharmaceutically acceptable salt thereof, in association with one or more pharmaceutically acceptable excipients. Conveniently, the single enantiomer is present in an enantiomeric excess (%ee) of ≥ 99%.

### Isotopes

Atoms of the compounds and salts described in this specification may exist as their isotopes. The compound of Formula (I-1) encompasses all compounds of Formula (I-1) where an atom is replaced by one or more of its isotopes (for example a compound of Formula (I-1) where one or more carbon atom is an ¹¹C or ¹³C carbon isotope, or where one or more hydrogen atoms is a ²H or ³H isotope).

### Tautomers

Compounds and salts described in this specification may exist as a mixture of tautomers. "Tautomers" are structural isomers that exist in equilibrium resulting from the migration of a hydrogen atom. The compound of Formula (I-1) includes all tautomers of compounds of Formula (I-1) particularly to the extent that such tautomers possess STING antagonist activity.

For example, the tautomeric form of compounds of Formula (I-1), when R⁸ and R¹⁸ are hydrogen (Formula (I-a2)), can be shown as Formula (I-a1):

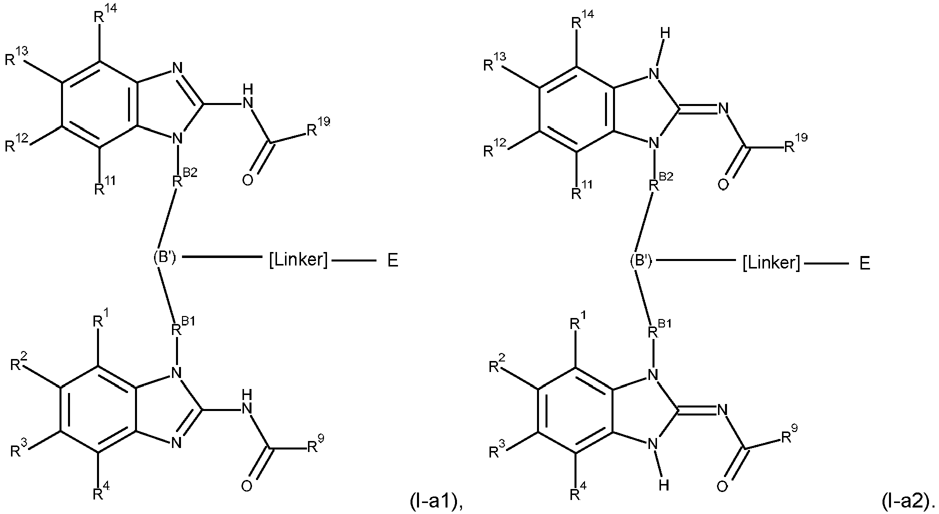

The tautomeric form of compounds of Formula (I-2), when R⁸ and R¹⁸ are hydrogen (Formula (I-a4)), can be shown as (I-a3):

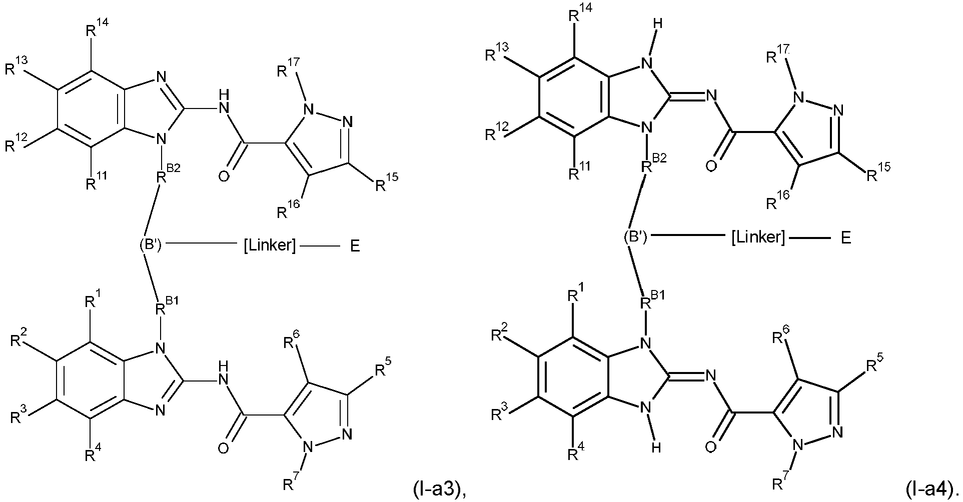

Where R⁸ and R¹⁸ are H, the tautomeric forms (I-a1) and (I-a3) might form.

### Crystalline forms

Compounds and salts described in this specification may be crystalline and may exhibit one or more crystalline forms. The compound of Formula (I-1) encompasses any crystalline or amorphous form of a compound of Formula (I-1), or mixture of such forms, which possesses STING antagonist activity.

It is generally known that crystalline materials may be characterised using conventional techniques such as X-Ray Powder Diffraction (XRPD), Differential Scanning Calorimetry (DSC), Thermal Gravimetric Analysis (TGA), Diffuse Reflectance Infrared Fourier Transform (DRIFT) spectroscopy, Near Infrared (NIR) spectroscopy, solution and/or solid state nuclear magnetic resonance spectroscopy. The water content of crystalline materials may be determined by Karl Fischer analysis.

### Therapy, prophylaxis and related terms

The term "therapy" is intended to have its normal meaning of dealing with a disease in order to entirely or partially relieve one, some or all of its symptoms, or to correct or compensate for the underlying pathology. The term "therapy" also includes "prophylaxis" unless there are specific indications to the contrary. The terms "therapeutic" and "therapeutically" should be interpreted in a corresponding manner.

The term "prophylaxis" is intended to have its normal meaning and includes primary prophylaxis to prevent the development of the disease and secondary prophylaxis whereby the disease has already developed and the patient is temporarily or permanently protected against exacerbation or worsening of the disease or the development of new symptoms associated with the disease.

The term "treatment" is used synonymously with "therapy". Similarly the term "treat" can be regarded as "applying therapy" where "therapy" is as defined herein.

The term "subject" to which administration is contemplated includes, but is not limited to, humans (i.e., a male or female of any age group, e.g., a paediatric subject (e.g., infant, child, adolescent) or adult subject (e.g., young adult, middle-aged adult or senior adult)) and/or other primates (e.g., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys. Preferred subjects are humans.

An "effective amount", as used herein, refers to an amount that is sufficient to achieve a desired biological effect. A "therapeutically effective amount", as used herein refers to an amount that is sufficient to achieve a desired therapeutic effect. For example, a therapeutically effective amount can refer to an amount that is sufficient to improve at least one sign or symptom of the disease to be treated.

### Pharmaceutical compositions

The compounds of Formula (I), and pharmaceutically acceptable salts thereof, may be administered as pharmaceutical compositions, comprising one or more pharmaceutically acceptable excipients.

Therefore, in one embodiment there is provided a pharmaceutical composition comprising a compound of Formula (I), or a pharmaceutically acceptable salt thereof, and a pharmaceutically acceptable excipient.

The excipient(s) selected for inclusion in a particular composition will depend on factors such as the mode of administration and the form of the composition provided. Suitable pharmaceutically acceptable excipients are well known to persons skilled in the art and are described, for example, in the Handbook of Pharmaceutical Excipients, Sixth edition, Pharmaceutical Press, edited by Rowe, Ray C; Sheskey, Paul J; Quinn, Marian. Pharmaceutically acceptable excipients may function as, for example, adjuvants, diluents, carriers, stabilisers, flavourings, colorants, fillers, binders, disintegrants, lubricants, glidants, thickening agents and coating agents. As persons skilled in the art will appreciate, certain pharmaceutically acceptable excipients may serve more than one function and may serve alternative functions depending on how much of the excipient is present in the composition and what other excipients are present in the composition.

The pharmaceutical compositions may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous or intramuscular dosing), or as a suppository for rectal dosing. The compositions may be obtained by conventional procedures well known in the art. Compositions intended for oral use may contain additional components, for example, one or more colouring, sweetening, flavouring and/or preservative agents.

Suitable daily doses of the compounds disclosed herein, or a pharmaceutically acceptable salt thereof, in therapeutic treatment of humans are about 0.0001-100 mg/kg body weight.

Pharmaceutical formulations as described herein may be formulated by methods known to those skilled in the art to provide doses of the active compound in the range of 0.1 mg to 1000 mg. The daily dose will necessarily be varied depending upon the host treated, the particular route of administration, any therapies being co-administered, and the severity of the illness being treated. Accordingly, the practitioner who is treating any particular patient may determine the optimum dosage.

The pharmaceutical compositions described herein comprise compounds of Formula (I), or a pharmaceutically acceptable salt thereof, and are therefore expected to be useful in therapy.

As such, in one embodiment there is provided a pharmaceutical composition for use in therapy, comprising a compound of Formula (I), or a pharmaceutically acceptable salt thereof, and at least one pharmaceutically acceptable excipient.

In one embodiment there is provided a pharmaceutical composition for use in the treatment of a disease associated with overactivation of STING, comprising a compound of Formula (I-1), or a pharmaceutically acceptable salt thereof, and at least one pharmaceutically acceptable excipient. In one embodiment there is provided a pharmaceutical composition for use in the treatment of a disease, comprising a compound of Formula (I), or a pharmaceutically acceptable salt thereof, and at least one pharmaceutically acceptable excipient. In one embodiment there is provided a pharmaceutical composition for use in the treatment of a disease associated with overactivation of STING, comprising a compound of Formula (I), or a pharmaceutically acceptable salt thereof, and at least one pharmaceutically acceptable excipient. In these embodiments, the E3 ubiquitin ligase ligand may optionally not be an Inhibitor of Apoptosis (IAP) E3 ubiquitin ligase ligand.

### Methods of Use

The compounds described herein may be used in a method of therapy. Also provided is a method of treatment, comprising administering to a subject in need of treatment a therapeutically effective amount of a compound of Formula (I). The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound may be administered alone or in combination with other treatments, either simultaneously or sequentially dependent upon the condition to be treated.

In one embodiment there is provided a compound of Formula (I), or a pharmaceutically acceptable salt thereof, or a pharmaceutical composition comprising the compound of Formula (I) for use in therapy. In one embodiment there is provided the use of the compound of Formula (I), or a pharmaceutically acceptable salt thereof, or a pharmaceutical composition comprising the compound of Formula (I) for the manufacture of a medicament for the treatment of a disease or condition in a subject in need thereof. In another embodiment, there is provided a compound of Formula (I), or a pharmaceutically acceptable salt thereof, or a pharmaceutical composition comprising the compound of Formula (I) for use in a method of treatment of a disorder or condition in a subject, the method comprising administering a therapeutically effective amount of the compound to a subject in need thereof. In another embodiment there is provided a method of treatment comprising administering to a subject the compound of Formula (I), or a pharmaceutically acceptable salt thereof, or a pharmaceutical composition comprising the compound of Formula (I). In another embodiment, there is provided a compound of Formula (I), or a pharmaceutically acceptable salt thereof, for use as a medicament.

In one embodiment there is provided a compound of Formula (I), or a pharmaceutically acceptable salt thereof, or a pharmaceutical composition comprising the compound of Formula (I) for use in treating a disorder. In some embodiments there is provided a method of treating a disorder or condition comprising administering to a subject the compound of Formula (I), or a pharmaceutically acceptable salt thereof, or a pharmaceutical composition comprising the compound of Formula (I).

In some embodiments, the disorder or condition is associated with overactivation of STING. In some embodiments, the disorder or condition is driven by constitutive STING activation. In some embodiments, the disorder or condition is driven by constitutive STING activation caused by a gain-of-function mutation. In these embodiments, the E3 ubiquitin ligase ligand may optionally not be an Inhibitor of Apoptosis (IAP) E3 ubiquitin ligase ligand. In some embodiments, upon non-covalent binding of S1 to STING and E to an E3 ubiquitin ligase, STING is degraded.

In some embodiments, the disorder or condition is a monogenic autoinflammatory syndrome, autoimmune disease, inflammatory disease, neurological disorder, metabolic disease, cardiovascular disease, or cancer. In these embodiments, the E3 ubiquitin ligase ligand may optionally not be an Inhibitor of Apoptosis (IAP) E3 ubiquitin ligase ligand.

In some embodiments, the disorder or condition is a monogenic autoinflammatory syndrome. In some embodiments, the disorder or condition is STING-associated vasculopathy with onset in infancy (SAVI), Aicardi-Goutieres Syndrome, COPA Syndrome, Familial Chilblain Lupus, Dnase II deficiency, or TREX1 deficiency. In these embodiments, the E3 ubiquitin ligase ligand may optionally not be an Inhibitor of Apoptosis (IAP) E3 ubiquitin ligase ligand.

In some embodiments, the disorder or condition is an autoimmune disease. In some embodiments, the disorder or condition is systemic lupus erythematosus (SLE), Sjögren's syndrome, or rheumatoid arthritis. In some embodiments, the disorder or condition is systemic lupus erythematosus (SLE).

In some embodiments, the disorder or condition is an inflammatory disease. In some embodiments, the disorder or condition is silica-induced fibrosis or sepsis.

In some embodiments, the disorder or condition is a neurological disorder. In some embodiments, the disorder or condition is ischaemic brain injury, Parkinson disease, general neurodegeneration, Huntington disease, amyotrophic lateral sclerosis and frontotemporal dementia, age-dependent macular degeneration, or traumatic brain injury.

In some embodiments, the disorder or condition is a metabolic disease. In some embodiments, the disease is non-alcoholic steatohepatitis, alcoholic liver disease, or acute pancreatitis.

In some embodiments, the disorder or condition is a cardiovascular disease. In some embodiments, the disorder or condition is myocardial infarction or chronic heart failure.

In some embodiments, the disorder or condition is cancer. In some embodiments, the disorder or condition is colorectal cancer, skin cancer, or metastases.

In some embodiments the disorder or condition is selected from monogenic autoinflammatory syndrome, STING-associated vasculopathy with onset in infancy (SAVI), Aicardi-Goutieres Syndrome, COPA Syndrome, Familial Chilblain Lupus, DNase II deficiency, or TREX1 deficiency, an autoimmune disease, systemic lupus erythematosus (SLE), Sjögren's syndrome or a cardiovascular disease.

In some embodiments, the disorder or condition is senescence or aging. In some embodiments, the subject is a mammal. In some embodiments, the mammal is a human.

In a further aspect, the invention provides a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently, for use as a medicament.

In a further aspect, the invention provides a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently, for the manufacture of a medicament for the treatment of a disease or condition.

In a further aspect, the invention provides a method of treatment of a disease or condition in a subject comprising administering a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently.

In a further aspect, the invention provides a use of a compound in the manufacture of a medicament for the treatment of a disease or condition, wherein the bifunctional compound is suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently.

In a further aspect, the invention provides a pharmaceutical composition comprising a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently, and a pharmaceutically acceptable excipient.

In a further aspect, the invention provides a method of inhibiting signaling driven by constitutive activation of STING, the method comprising administration of a therapeutically effective amount of a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand does not form a covalent bond with STING.

### Further embodiments /preferences

The following embodiments may apply to all aspects as described above or may relate to a single aspect. The embodiments may be combined together in any combination.

### S1

S1 is a STING ligand. The STING ligand does not form a covalent bond with STING. The STING ligand does not exhibit STING agonist activity. Exhibiting STING agonist activity means inducing cytokine induction or inducing phospho-STING. Therefore, the STING ligand S1 does not induce cytokine induction nor induce phospho-STING. Any standard method known in the art may be used to determine STING agonist activity. For example, STING agonist activity (or lack thereof) can be measured as described in Examples 48 and/or Example 51. For example, following the method described in Example 48, cells may be incubated with a compound comprising a S1 STING ligand. The cells may be lysed and Western Blotting performed. Western blots may be probed for phospho-STING and/or phospho-TBK1. Cells incubated with STING ligands which are not agonists will not result in detectable phospho-STING or phospho-TBK1 using the methods outlined above and in Examples 48. In another example, following the method described in Example 51, human skin fibroblasts may be pretreated for 2hours with a serial dilution of a test compounds, for example a compound comprising an S1 ligand, prior to stimulation with a STING agonist (e.g. 300 nM GSK STING agonist). Supernatants may then be harvested after STING agonist stimulation (e.g. after 20 h stimulation). IFN-a/b may be measured in the supernatants using HEK-Blue IFN-α/β bioassay (Invivogen) to determine a half-maximal inhibitory concentration of a test compound. A compound comprising an S1 ligand having an IC50 value against STING indicates that the compound is not a STING agonist.

### STING degradation

STING degradation may be quantified using any standard method in the art. For example, STING degradation may be measured using Western Blot analysis as described in Example 11 followed by quantification of protein band signal intensity (e.g. densiometric analysis). Quantification of may be performed using any commonly available software, for example Image Lab software (Biorad, Version 6.0.1). In some embodiments, incubating cells with 1 µM S1-L-E compound for 16 hours reduces relative STING protein abundance by at least 30%, at least 35%, at least 40%, at least 45%, 50%, at least 55%, at least 60%, at least 65%, at least 70%, at least 75%, at least 80%, at least 85%, at least 90%, at least 95%, at least 96%, at least 97%, at least 98%, at least 99% as compared to cells not treated with S1-L-E compound. In some embodiments, incubating cells with 5 µM S1-L-E compound for 16 hours reduces relative STING protein abundance by at least 50%, at least 55%, at least 60%, at least 65%, at least 70%, at least 75%, at least 80%, at least 85%, at least 90%, at least 95%, at least 96%, at least 97%, at least 98%, at least 99% as compared to cells not treated with S1-L-E compound. In some embodiments, incubating cells with 7.5 µM S1-L-E compound for 16 hours reduces relative STING protein abundance by at least 55%, at least 60%, at least 65%, at least 70%, at least 75%, 80%, at least 85%, at least 90%, at least 95%, at least 96%, at least 97%, at least 98%, at least 99% as compared to cells not treated with S1-L-E compound. In some embodiments, incubating cells with 10 µM S1-L-E compound for 16 hours reduces relative STING protein abundance by at least 80%, at least 85%, at least 90%, at least 95%, at least 96%, at least 97%, at least 98%, at least 99% as compared to cells not treated with S1-L-E compound. In some embodiments, the cells incubated with the S1-L-E compounds are selected from the following: H1650 cells, H596 cells, HaCat cells, HFF-1 cells, RAW264.7 cells, primary fibroblasts, primary SAVI patient fibroblasts, primary human skin fibroblasts, and THP-1 cells.

### R⁹ and R¹⁹

In some embodiments R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl).

In some embodiments, R⁹ and R¹⁹ are each independently selected from an optionally substituted C₅₋₆ heteroaryl or optionally substituted C₅₋₆ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl).

In further embodiments R⁹ and R¹⁹ are each independently selected from an optionally substituted C₅₋₆ heteroaryl or optionally substituted C₅₋₆ heterocyclyl, wherein the optional substituents are independently selected from H, halogen, C₃₋₅ cycloalkyl, optionally substituted C₁₋₄ alkyl wherein said optionally substituted alkyl is optionally substituted by one or more substituents independently selected from halogen, halo(C₁₋₄ alkyl), OH, NH₂, C₁₋₄ alkoxy, CO₂H, CO₂C₁₋₆ alkyl, N(C₁₋₆ alkyl)₂, OCONH₂ and CONH₂.

When R⁹ or R¹⁹ is an optionally substituted C₅₋₆ heteroaryl or optionally substituted C₅₋₆ heterocyclyl the optional substituent is selected from optionally substituted methyl, optionally substituted ethyl, optionally substituted propyl wherein the optional substituents are OH, NH₂, halogen or C₁₋₄ alkoxy. In further embodiments when R⁹ or R¹⁹ is an optionally substituted C₅₋₆ heteroaryl or optionally substituted C₅₋₆ heterocyclyl the optional substituent is selected from methyl and ethyl.

In some embodiments formula (I) is of formula (I-2): 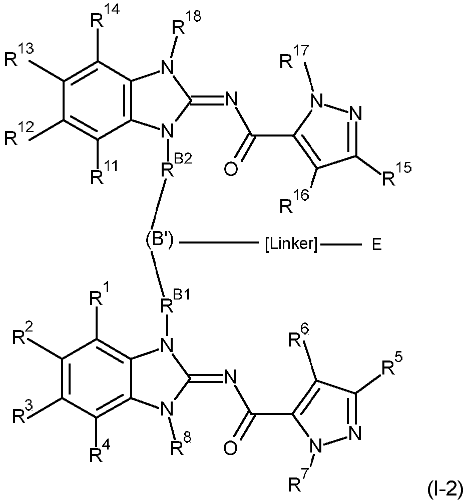
wherein R¹, R², R³, R⁴, R⁸, R¹¹, R¹², R¹³, R¹⁴, R¹⁸, R^{B1}, R^{B2}, B', Linker and E are as defined herein,
R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy;
R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy; and
R⁵ and R¹⁵ are each independently selected from H, cyclopropyl and C₁₋₄ alkyl.

### R¹ and R¹¹

In some embodiments R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -OP(O)(OH)₂, C₁₋₄ alkoxy, NH₂, NH(C₁₋₆ alkyl), N(C₁₋₆ alkyl)₂, CO₂(C₁₋₆ alkyl) and halogen.

When R¹ or R¹¹ are optionally substituted C₁₋₆ alkyl, the optional substituents are selected from OH, F, Br, methoxy and ethoxy. In some embodiments when R¹ or R¹¹ are optionally substituted C₁₋₆ alkyl, they are optionally substituted methyl or optionally substituted ethyl. In further embodiments, they are optionally substituted methyl. In further embodiments, they are unsubstituted methyl.

When R¹ or R¹¹ are halo, in some embodiments they are F, Br or Cl.

In some embodiments R¹ and R¹¹ are independently selected from H, OH, F and methyl.

In some embodiments R¹ is identical to R¹¹.

In some embodiments R¹ and R¹¹ are H.

### R² and R¹²

In some embodiments R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -OP(O)(OH)₂, C₁₋₄ alkoxy, N(C₁₋₆ alkyl)₂, NH₂, NH(C₁₋₆ alkyl), CO₂(C₁₋₆ alkyl) and halogen.

When R² or R¹² are optionally substituted C₁₋₆ alkyl, the optional substituents are selected from OH, F, Br, methoxy and ethoxy. In some embodiments when R² or R¹² are optionally substituted C₁₋₆ alkyl, they are optionally substituted methyl or optionally substituted ethyl. In further embodiments, they are optionally substituted methyl. In further embodiments, they are unsubstituted methyl.

When R² or R¹² are halo, in some embodiments they are F, Br or Cl.

In some embodiments R² and R¹² are independently selected from H, OH, F or methyl.

In some embodiments R² is identical to R¹².

In some embodiments R² and R¹² are H.

### R³ and R¹³

In some embodiments R³ and R¹³ are each independently selected from H, C(=O)NH₂; optionally substituted -C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, NH(C₁₋₆ alkyl), N(C₁₋₆ alkyl)₂, NH₂, CO₂(C₁₋₆ alkyl) and halogen.

When R³ or R¹³ are optionally substituted -C(=O)NH(C₁₋₆ alkyl), optionally substituted C₁₋₆ alkoxy or optionally substituted CO₂C₁₋₆ alkyl, the optional substituents are selected from OH, -O-P(O)(OH)₂, methoxy, ethoxy, N(CH₃)₂, NH(CH₃), NH₂, CO₂(CH₃) and halogen.

When R³ or R¹³ are -C(=O)NH(C₁₋₆ alkyl) in some embodiments they are each independently - C(=O)NHCH₃, -C(=O)NHCH₂CH₃, -C(=O)NHCH₂CH₂CH₃.

In some embodiments R³ and R¹³ are each independently selected from H, -C(=O)NH₂ -C(=O)NHCH₃, - C(=O)NHCH₂CH₃, or -C(=O)NHCH₂CH₂CH₃.

In some embodiments R³ and R¹³ are each independently selected from -C(=O)NH(C₁₋₃ alkyl) and - C(=O)NH₂.

In some embodiments R³ is identical to R¹³.

In some embodiments R³ and R¹³ are -C(=O)NH₂.

### R⁴ and R¹⁴

In some embodiments R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH(C₁₋₆ alkyl), NH₂, CO₂(C₁₋₆ alkyl) and halogen.

When R⁴ or R¹⁴ are optionally substituted C₁₋₆ alkyl, the optional substituents are selected from OH, F, Br, methoxy and ethoxy. In some embodiments when R⁴ or R¹⁴ are optionally substituted C₁₋₆ alkyl, they are optionally substituted methyl or optionally substituted ethyl. In further embodiments, they are optionally substituted methyl. In further embodiments they are unsubstituted methyl.

When R⁴ or R¹⁴ are halo, in some embodiments they are F, Br or Cl.

In some embodiments R⁴ and R¹⁴ are each independently selected from H, OH, F or methyl.

In some embodiments R⁴ is identical to R¹⁴.

In some embodiments R⁴ and R¹⁴ are H.

### R⁵ and R¹⁵

In some embodiments, R⁵ and R¹⁵ are each independently selected from H, C₁₋₄ alkyl or C₁₋₄ alkoxy.

In some embodiments, R⁵ and R¹⁵ are each independently selected from C₁₋₄ alkyl or C₁₋₄ alkoxy.

In some embodiments R⁵ and R¹⁵ are each independently selected from H, cyclopropyl and C₁₋₄ alkyl.

In some embodiments R⁵ and R¹⁵ are both H.

In some embodiments R⁵ and R¹⁵ are both cyclopropyl.

When R⁵ or R¹⁵ are each independently C₁₋₄ alkyl they are methyl, ethyl, propyl or butyl. In some embodiments they are methyl or ethyl. In further embodiments they are methyl.

In some embodiments R⁵ is identical to R¹⁵.

In some embodiments R⁵ and R¹⁵ are methyl.

### R⁶ and R¹⁶

In some embodiments R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy.

When R⁶ or R¹⁶ are optionally substituted C₁₋₆ alkyl, the optional substituents are selected from OH, F, Br, methoxy and ethoxy. In some embodiments when R⁶ or R¹⁶ are optionally substituted C₁₋₆ alkyl, they are optionally substituted methyl or optionally substituted ethyl. In further embodiments, they are optionally substituted methyl. In further embodiments, they are unsubstituted methyl.

When R⁶ or R¹⁶ are halo, in some embodiments they are F, Br or Cl.

In some embodiments R⁶ and R¹⁶ are each independently selected from H, OCH₃, halogen or C₁₋₃ alkyl.

In some embodiments R⁶ and R¹⁶ are each independently selected from H, OCH₃, F or methyl.

In some embodiments R⁶ is identical to R¹⁶.

In some embodiments R⁶ and R¹⁶ are H.

### R⁷ and R¹⁷

In some embodiments R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy.

When R⁷ or R¹⁷ are each independently optionally substituted C₁₋₄ alkyl it is an optionally substituted methyl, ethyl, propyl or butyl. When R⁷ or R¹⁷ are each independently selected from optionally substituted C₁₋₄ alkyl the optional substituents are selected from OH, halogen, methoxy and ethoxy. When R⁷ and R¹⁷ are each independently optionally substituted C₁₋₄ alkyl, in some embodiments they are methyl or ethyl.

In some embodiments R⁷ is identical to R¹⁷.

In some embodiments R⁷ and R¹⁷ are both C₁₋₄ alkyl.

In some embodiments R⁷ and R¹⁷ are ethyl.

### R⁸ and R¹⁸

In some embodiments R⁸ and R¹⁸ are each independently selected from H, and C₁₋₄ alkyl.

In some embodiments when R⁸ and R¹⁸ are C₁₋₄ alkyl they are selected from methyl, ethyl, propyl or butyl.

In some embodiments R⁸ and R¹⁸ are methyl, ethyl or H. In further embodiments R⁸ and R¹⁸ are methyl or H.

In some embodiments R⁸ is identical R¹⁸.

In some embodiments R⁸ and R¹⁸ are H.

In some embodiments R⁸ and R¹⁸ are methyl.

In some embodiments R⁸ and R¹⁸ are ethyl.

### R^{B1} and R^{B2}

In some embodiments R^{B1} and R^{B2} are each independently selected from (-CH₂-)₁₋₂ or a bond.

When R^{B1} or R^{B2} are a bond, this is a bond connected directly from the N to B'.

When R^{B1} or R^{B2} are (-CH₂-)₁₋₂ they can each independently be -CH₂- or -CH₂CH₂-.

In some embodiments R^{B1} is a bond and R^{B2} is -CH₂- or -CH₂CH₂-. In some embodiments R^{B1} is a bond and R^{B2} is -CH₂- or -CH₂CH₂-. In other embodiments R^{B2} is a bond and R^{B1} is -CH₂-. In other embodiments R^{B1} is a bond and R^{B2} is -CH₂-.

In some embodiments R^{B1} and R^{B2} are both a bond.

In some embodiments R^{B1} and R^{B2} are both -CH₂- or both -CH₂-CH₂- or R^{B1} is -CH₂- and R^{B2} is -CH₂-CH₂-In some embodiments R^{B1} is identical to R^{B2}.

In some embodiments R^{B1} and R^{B2} are both -CH₂-.

In some embodiments R^{B1} and R^{B2} are both -CH₂CH₂-.

### B'

B' is the point of attachment to the Linker group L as follows:

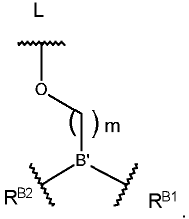

In some embodiments B' is CH, methyl, ethyl, propyl, butyl or pentyl. In further embodiments B is CH, methyl or ethyl.

In further embodiments B is CH or C₁₋₂ alkyl and B' together with R^{B1} and R^{B2} form a C₃₋₈ alkyl chain.

In further embodiments B is CH or C₁₋₂ alkyl and B' together with R^{B1} and R^{B2} form a C₅ alkyl chain.

In some embodiments m is 0.

In some embodiments m is 1.

In some embodiments m is 0 and therefore B' is bonded directly to O and B' is CH or C₁₋₂ alkyl.

In further embodiments m is 0 and therefore B' is bonded directly to O and B' together with R^{B1} and R^{B2} form a C₃₋₈ alkyl chain. In further embodiments m is 0 and therefore B' is bonded directly to O and B' together with R^{B1} and R^{B2} form a C₅ alkyl chain.

In some embodiments B' is of the formula: 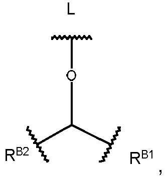
wherein R^{B1} and R^{B2} are each independently selected from (-CH₂-)_{1-2;};
wherein the wavy line indicates the point of attachment to the Linker (L), R^{B1} and R^{B2} as shown.

In further embodiments R^{B1} and R^{B2} are each -CH₂-CH₂- and together with B' have the following structure: 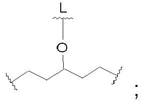 wherein the wavy line next to L represents the attachment to the linker and the wavy lines next to the alkyl groups represent the connection to the N atoms in the S1 structure.

### Symmetrical S1 compounds

In some embodiments R¹ is identical to R¹¹, R² is identical to R¹², R³ is identical to R¹³, R⁴ is identical to R¹⁴, R^{B1} is identical to R^{B2}, R⁸ is identical to R¹⁸ and R⁹ is identical to R¹⁹. In further embodiments R¹ is identical to R¹¹, R² is identical to R¹², R³ is identical to R¹³, R⁴ is identical to R¹⁴, R⁵ is identical to R¹⁵, R^{B1} is identical to R^{B2}, R⁸ is identical to R¹⁸, R⁶ is identical to R¹⁶ and R⁷ is identical to R¹⁷. Therefore, in some embodiments S1 is symmetrical.

### Further embodiments

In some embodiments, R⁴, R¹⁴, R², R¹², R¹ and R¹¹ are each independently selected from H, OH, OCH₃, halogen or C₁₋₃ alkyl.

In some embodiments, R⁷, R⁸, R¹⁷ and R¹⁸ are C₁₋₄alkyl.

In some embodiments,
R¹ and R¹¹ are selected from H, -OH or C₁₋₃ alkoxy;
R³ and R¹³ are selected from -C(=O)N(H)(C₁₋₃alkyl) or -C(=O)NH₂;
R⁴ and R¹⁴ are selected from H, halogen, OH or C₁₋₄ alkyl;
R⁷ and R¹⁷ are C₁₋₄ alkyl;
R⁵ and R¹⁵ are selected from H or C₁₋₄ alkyl;
R⁶ and R¹⁶ are H; and
R⁸ and R¹⁸ are methyl.

In further embodiments
R¹ and R¹¹ are H;
R³ and R¹³ are -C(=O)NH₂ or -C(=O)NH-CH₃;
R⁷ and R¹⁷ are ethyl; and
R⁵ and R¹⁵ are methyl.

In some embodiments
R¹, R¹¹, R⁶, R¹⁶, R⁴, R¹⁴, R² and R¹² are H;
R⁷ and R¹⁷ are ethyl;
R⁵, R¹⁵, R⁸ and R¹⁸ are methyl;
R³ and R¹³ are -C(=O)NH₂;
R^{B1} and R^{B2} are both -CH₂-;
b is 0;
a is 1;
X is -NR^{B3}R^{B4} or X is -OR^{B5};
R^{B5} is ethyl;
R^{B3} and R^{B4} are selected from H, ethyl, phenyl, pyrimidine, CH₂CH₂-O-CH₃, pyrazole substituted by methyl,
or R^{B3} and R^{B4} together form a group selected from a morpholine which is optionally substituted by phenyl, a 1,2,3,4-tetrahydroquinoline substituted with methoxy, a pyrrolidine substituted with a tetrazole or phenyl which phenyl is substituted with methoxy , or a 2-[(1,2,3,4-tetrahydro-2-isoquinolyl)carbonyl]pyrrolidine substituted with methoxy or a piperazine substituted with C(=O)CH₃.

In some embodiments compound (I) is of the Formula (I-2b): 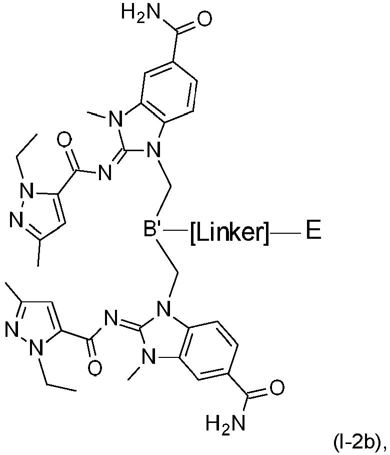 or a tautomer, or a pharmaceutically acceptable salt thereof wherein B' is a C₁₋₅ alkyl and is the connection point to the Linker.

In further embodiments compound of formula (I) is of the formula (I-2c) or (I-2d):

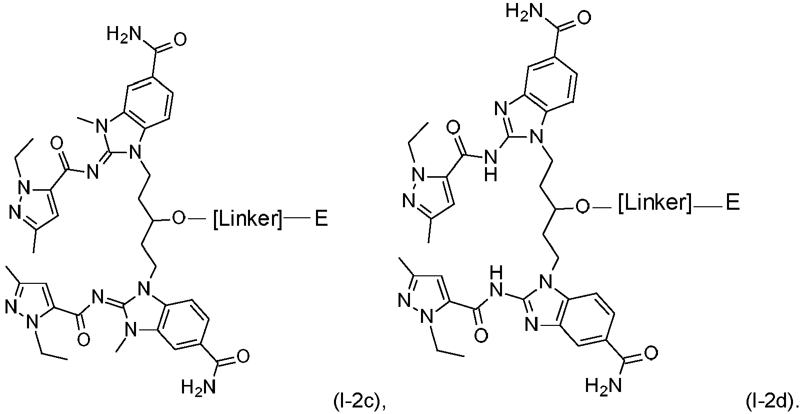

### E

In some embodiments the E3 ubiquitin ligase ligand is a Cereblon (CRBN) E3 ubiquitin ligase ligand. In some embodiments the E3 ubiquitin ligase ligand is a von Hippel-Lindau (VHL) E3 ubiquitin ligase ligand. In some embodiments the E3 ubiquitin ligase ligand is an Inhibitor of Apoptosis (IAP) E3 ubiquitin ligase ligand.

### E1

In some embodiments E has a structure according to Formula E1: 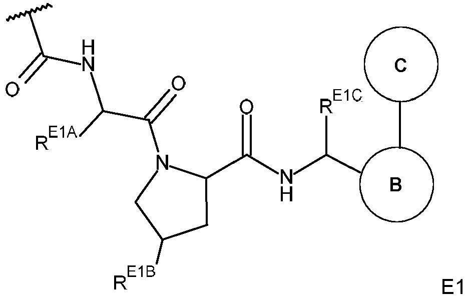 wherein
R^{E1A} is selected from optionally substituted C₁₋₆alkyl, optionally substituted C₃₋₇cycloalkyl, or optionally substituted C₄₋₁₀heterocyclyl, wherein the optional substituents are selected from OH, C₁₋₆alkyl, and halogen;
R^{E1B} is selected from H, OH, OR, NH₂, NHR, or NRR';
R^{E1C} is selected from H or C₁₋₆ alkyl;
Ring B is an optionally substituted C₆₋₁₀ carboaryl, or an optionally substituted C₅₋₁₀ heteroaryl group wherein the optional substituents are selected from C₁₋₆ alkyl, halogen or OH;
Ring C is an optionally substituted C₆₋₁₀ carboaryl, an optionally substituted C₅₋₁₀ heteroaryl group, or an optionally substituted C₅₋₁₀ heterocyclic group, wherein the optional substituents are selected from C₁₋₆ alkyl, halogen or OH;
wherein R and R' are C₁₋₆ alkyl, C₂₋₆ alkenyl, C₂₋₆ alkynyl, C₃₋₇ cycloalkyl, C₄₋₇ heterocyclyl, and 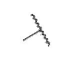 represents attachment point to L.

In some embodiments when R^{E1A} is optionally substituted C₁₋₆alkyl, optionally substituted C₃₋₇ cycloalkyl, or optionally substituted C₅₋₁₀ heterocyclyl, the optional substituents are selected from methyl, ethyl, OH, F or Cl.

When R^{E1A} is optionally substituted C₃₋₇ cycloalkyl, in some embodiments it is cyclopropyl, cyclobutyl or cyclopentyl.

When R^{E1A} is C₁₋₆ alkyl, in some embodiments it is methyl, ethyl, propyl, isopropyl, butyl, isobutyl, secbutyl or tert-butyl. When R^{E1A} is C₁₋₆alkyl, in some embodiments it is a branched C₁₋₄ alkyl.

In some embodiments, R^{E1A} is optionally substituted C₁₋₆ alkyl,

In some embodiments, R^{E1A} is a branched butyl group.

In some embodiments R^{E1A} is tert-butyl.

In some embodiments when R^{E1B} is OR, NHR or NRR', R and R' are independently selected from methyl, ethyl, propyl, cyclopropyl or cyclobutyl. In some embodiments when R^{E1B} is OR it is O-methyl or O-ethyl. In some embodiments when R^{E1B} is NHR or NRR', it is NHCH₃, NHCH₂CH₃, N(CH₃)(CH₃), N(CH₂CH₃)(CH₃) or N(CH₂CH₃)(CH₂CH₃).

In some embodiments R^{E1B} is H.

In some embodiments R^{E1B} is OH.

In some embodiments R^{E1C} is H.

In some embodiments R^{E1C} is C₁₋₃ alkyl. In further embodiments R^{E1C} is methyl.

In some embodiments Ring B is an optionally substituted C₆₋₁₀ carboaryl, or an optionally substituted C₅₋₁₀ heteroaryl group, wherein the optional substituents are selected from C₁₋₆alkyl, halogen or OH.

In some embodiments, Ring B is an optionally substituted C₆₋₁₀carboaryl, wherein the optional substituents are selected from C₁₋₆ alkyl, halogen or OH.

When Ring B is an optionally substituted C₅₋₁₀ heteroaryl group, in some embodiments it is an optionally substituted C₅₋₆ heteroaryl group which comprises 1 or 2 heteroatoms selected from O, S and N. In some embodiments the optional substituents are selected from methyl, ethyl, F, Cl and OH.

When Ring B is an optionally substituted C₆₋₁₀ carboaryl, or an optionally substituted C₅₋₁₀ heteroaryl group, in some embodiments the optional substituents are selected from methyl, ethyl, F, Cl and OH.

In some embodiments when Ring B is an optionally substituted C₆₋₁₀ carboaryl it is an optionally substituted phenyl wherein the optional substituents are selected from methyl, ethyl, F, Cl and OH.

In some embodiments Ring B is phenyl.

In further embodiments Ring B is phenyl linked in positions 1 and 4.

In some embodiments Ring C is an optionally substituted C₆₋₁₀ carboaryl, or an optionally substituted C₅₋₁₀ heteroaryl group, wherein the optional substituents are selected from C₁₋₆alkyl, halogen or OH.

In some embodiments when Ring C is an optionally substituted C₆₋₁₀carboaryl, or an optionally substituted C₅₋₁₀ heteroaryl group, the optional substituents are selected from methyl, ethyl, OH, F or Cl. In one embodiment there are 1 or 2 optional substituents. In some embodiments the optional substituent is methyl.

In some embodiments when Ring C is an optionally substituted C₆₋₁₀carboaryl it is an optionally substituted phenyl wherein the optional substituents are selected from methyl, ethyl, OH, F or Cl. In some embodiments Ring C is an unsubstituted phenyl.

In some embodiments Ring C is an optionally substituted C₅₋₁₀ heteroaryl group, wherein the optional substituents are selected from C₁₋₆ alkyl, halogen or OH. In some embodiments the optional substituents are selected from methyl, ethyl, OH, F or Cl.

When Ring C is an optionally substituted C₅₋₁₀ heteroaryl group, in some embodiments it is an optionally substituted C₅₋₆ heteroaryl group. In some embodiments Ring C has 1 or 2 heteroatoms selected from O, S and N. In some embodiments Ring C is selected from azolyl, pyridiyl, furanyl, thiophenyl, oxazolyl, isoxazolyl, isoxazinyl, thiazolyl, isothiazolyl, imidazolyl, pyrazoyl, pyridazinyl, pyrimidinyl or pyrazinyl. In some embodiments Ring C has 1 or 2 heteroatoms selected from S and N.

When Ring C is an optionally substituted C₅₋₁₀ heteroaryl group, in some embodiments it is an optionally substituted C₅ heteroaryl group. In some embodiments Ring C has two heteroatoms one being N and the other being S. In some embodiments the optional substituents are methyl. In some embodiments there is one methyl substituent on the C₅ heteroaryl.

In some embodiments Ring C is a 1,3-thiazole optionally substituted by methyl. In further embodiments Ring C is 4-methyl-1,3-thiazol-5-yl.

In some embodiments Ring C is

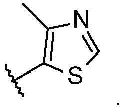

In some embodiments E has the formula E1 wherein R^{E1A} is a branched C₁₋₄alkyl, R^{E1B} is selected from OH, O-methyl or O-ethyl, R^{E1C} is H or methyl, Ring B is an optionally substituted phenyl wherein the optional substituents are selected from methyl, ethyl, F, Cl and OH and Ring C is an optionally substituted C₅₋₁₀heteroaryl group wherein the optional substituents are selected from methyl, ethyl, OH, F or Cl. In further embodiments E has the formula E1 wherein R^{E1A} is tert-butyl, R^{E1B} is OH, Ring B is phenyl and Ring C is a 1,3-thiazole substituted by methyl.

In some embodiments E has the following structure E1-1: 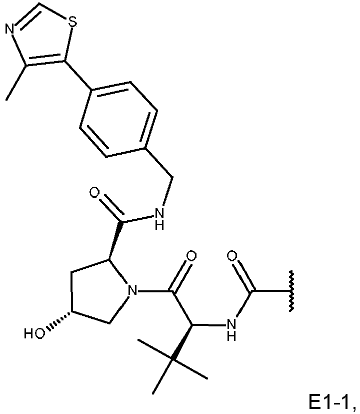 wherein 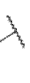 represents attachment point to L.

In some embodiments E has the following structure E1-2: 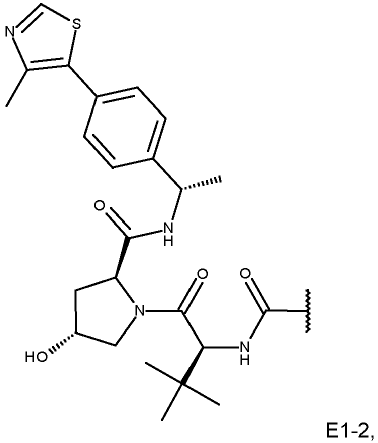 wherein 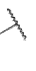 represents attachment point to L.

### E2

In some embodiments E has a structure according to Formula E2: 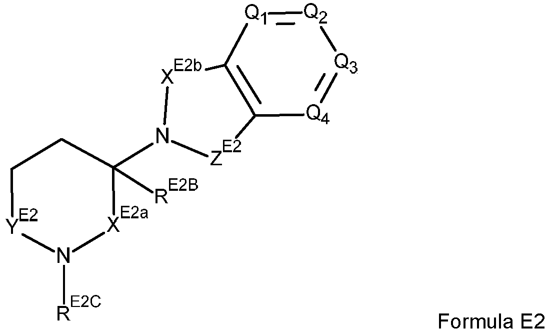 wherein
X^{E2a} is selected from C=O and C=S;
X^{E2b} is selected from C=O, C=S, or CH₂;
Y^{E2} is selected from C=O and C=S;
Z^{E2} is selected from CH₂, CHR, C=O, SO₂, NH, and NR;
Q₁, Q₂, Q₃, and Q₄ are independently N, CH, CR^{E2D}, or CR^{E2A}, wherein one of Q1, Q2, Q3, and Q4 is CR^{E2A};
R^{E2A} is selected from a bond, -O-, -C(=O)-, -CH₂-, -NR-,-NH-, and -SO₂-;
R^{E2B} is selected from H, halogen, C₁₋₆ alkyl, and C₃₋₇ cycloalkyl;
R^{E2C} is selected from H, OH, and C₁₋₆ alkyl;
R^{E2D} is selected from OH, halogen, C₁₋₆ alkoxy, C₁₋₆ alkyl, C₂₋₆ alkenyl, C₂₋₆ alkynyl, C₃₋₇ cycloalkyl, and C₄₋₇ heterocyclyl;
R is selected from H, OH, C₁₋₆ alkoxy, C₁₋₆ alkyl, C₂₋₆ alkenyl, C₂₋₆ alkynyl, C₃₋₇ cycloalkyl, and C₄₋₇ heterocyclyl,
and E is joined to L by the group R^{E2A}.

In some embodiments when R is C₁₋₆ alkyl it is methyl, ethyl or propyl. In some embodiments when R is C₁₋₆ alkenyl it is ethenyl or propenyl. In some embodiments when R is C₃₋₇ cycloalkyl it is cyclopropyl or cyclobutyl. In some embodiments R is H, OH or methyl. In some embodiments R is H. In some embodiments when R is C₁₋₆ alkoxy it is methoxy or ethoxy. In further embodiments when R is C₁₋₆ alkoxy it is OMe.

In some embodiments X^{E2a} is C=O. In some embodiments X^{E2b} is C=O. In some embodiments, X^{E2a} and X^{E2b} are C=O.

In some embodiments Y^{E2} is C=O.

In some embodiments Z^{E2} is C=O.

In some embodiments X^{E2a} is C=O.

In some embodiments X^{E2a} is C=O, X^{E2b} is C=O, Y^{E2} is C=O and Z^{E2} is C=O.

In some embodiments when R^{E2B} is C₁₋₆ alkyl it is methyl, ethyl or propyl. In some embodiments R^{E2B} is selected from H, F, Cl or methyl. In some embodiments R^{E2B} is H or F. In some embodiments R^{E2B} is H.

In some embodiments when R^{E2C} is C₁₋₆alkyl it is methyl, ethyl or propyl. In some embodiments R^{E2C} is selected from H, OH, or methyl. In further embodiments R^{E2C} is H.

In some embodiments Y^{E2} and X^{E2a} are each C=O and R^{E2C} is H.

In some embodiments one of Q₁, Q₂, Q₃, and Q₄ is CR^{E2A}, and no more than one of Q¹, Q², Q³, or Q⁴ are N.

In some embodiments, R^{E2D} is selected from OH, halogen, C₁₋₆alkoxy, and C₁₋₆ alkyl. In some embodiments, R^{E2D} is C₁₋₆ alkoxy.

In some embodiments when one of Q₁, Q₂, Q₃, and Q₄ is CR^{E2A}, the remaining are CH or CR^{E2D}. In further embodiments one of Q₁, Q₂, Q₃, and Q₄ is CR^{E2A}, and the remaining are CH. In some embodiments, one of Q₁, Q₂, Q₃, and Q₄ is CR^{E2A}, another is CR^{E2D}, and the remaining are CH.

In some embodiments, one of Q₁, Q₂, Q₃, and Q₄ is CR^{E2A}, another is COMe, and the remaining are CH.

When one of Q₁, Q₂, Q₃, and Q₄ is CR^{E2A}, and the remaining are CH or CR^{E2D} it is Q₄ is CR^{E2A}, and Q₁, Q₂ and Q₃ are CH or CR^{E2D}. In further embodiments Q₄ is CR^{E2A}, and Q₁, Q₂ and Q₃ are CH.

In some embodiments when one of Q₁, Q₂, Q₃, and Q₄ is CR^{E2A2}, another is Ome and the remaining are CH. In further embodiments Q₄ is CR^{E2A}. In further embodiments Q₄ is CR^{E2A}, Q₂ and Q₃ are CH and Q₁ is CH or COMe.

In some embodiments R^{E2A} is -O- or -NR- wherein R is H, OH, C₁₋₆ alkyl, C₂₋₆ alkenyl, C₂₋₆ alkynyl, C₃₋₇ cycloalkyl, C₄₋₇ heterocyclyl. When R^{E2A} is -NR and R is C₁₋₆ alkyl, R can be methyl or ethyl. When R^{E2A} is -NR and R is C₃₋₇ cycloalkyl R can be cyclopropyl. In some embodiments when R^{E2A} is -NR, R is H.

In some embodiments R^{E2A} is -NR-. In further embodiments R^{E2A} is -NH-. Therefore, in some embodiments E is joined to R^{E2A} via an -N(H)- bond.

In some embodiments R^{E2A} is a bond.

In some embodiments, Q₄ is CR^{E2A}. In some embodiments, Q₄ is CR^{E2A}, Q₂ and Q₃ are CH and Q₁ is CH or COMe. In some embodiments Q₄ is CR^{E2A}, and R^{E2A} is -NH- and Q₁, Q₂ and Q₃ are CH. Therefore, in some embodiments E is joined to R^{E2A} at the Q₄ position via an -N(H)- bond.

In some embodiments Q₄ is CR^{E2A}, and R^{E2A} is a bond and Q₁, Q₂ and Q₃ are CH.

In some embodiments X^{E2a} is C=O, X^{E2b} is C=O, Y^{E2} is C=O, Z^{E2} is C=O, Q₁, Q₂ and Q₃ are CH and Q₄ is CR^{E2A}, and R^{E2A} is a bond, R^{E2B} is H, and R^{E2C} is H.

In some embodiments E has the following structure E2-1: 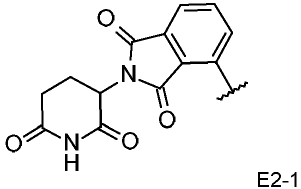 wherein 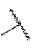 represents attachment point to L.

In some embodiments E has the following structure E2-2: 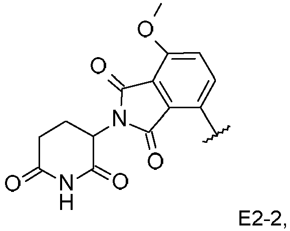 wherein 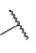 represents attachment point to L.

### E3

In some embodiments E has a structure according to Formula E3 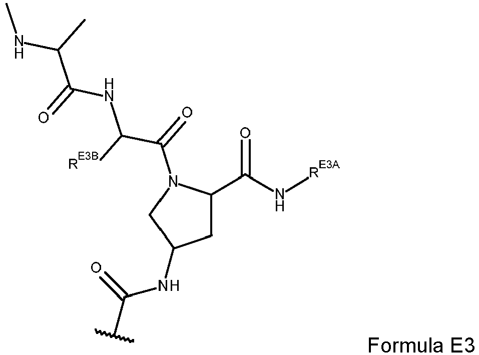 wherein
R^{E3A} is optionally substituted C₅₋₁₀ cycloalkyl or optionally substituted C₆₋₁₀ carboaryl, wherein the optional substituents are selected from C₁₋₆ alkyl, halogen and OH;
R^{E3B} is H, C₁₋₆ alkyl, or C₃₋₇ cycloalkyl; and 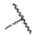 represents attachment point to L.

In some embodiments when R^{E3A} is optionally substituted C₅₋₁₀cycloalkyl, optionally substituted C₆₋₁₀ carboaryl the optional substituents are selected from methyl, ethyl, F, Cl and OH.

When R^{E3A} is an optionally substituted C₅₋₁₀ cycloalkyl it is selected from cyclopentyl, cyclohexyl, cycloheptyl, methylcyclopropyl, dimethylcyclopropyl, methylcyclobutyl, dimethylcyclobutyl, methylcyclopentyl, dimethylcyclopentyl, methylcyclohexyl, cyclooctyl, cyclononayl, cyclodecyl, decalinyl and 2,3,3a,4,5,6,7,7a-octahydro-1H-indenyl.

When R^{E3A} is an optionally substituted C₆₋₁₀carboaryl in some embodiments it is a single ring selected from phenyl, naphthyl and azulyl.

When R^{E3A} is an optionally substituted C₆₋₁₀ carboaryl in some embodiments it is fused rings, at least one of which is an aromatic ring, selected from indanyl (e.g. 2,3-dihydro-1H-indene), indenyl, isoindenyl (C₉) and tetralin-1-yl (e.g. 1,2,3,4-tetrahydronaphthalene).

In some embodiments, R^{E3A} is an unsubstituted tetralin. In some embodiments, R^{E3A} is (*1R*)-1,2,3,4-tetrahydronaphthalen-1-yl.

In some embodiments R^{E3A} is an unsubstituted tetralin-1-yl which has the following structure:

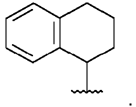

In some embodiments, R^{E3B} is C₃₋₇ cycloalkyl.

When R^{E3B} is C₃₋₇cycloalkyl it is selected from cyclopropyl, cyclobutyl, cyclopentyl, cyclohexyl, cycloheptyl, methylcyclopropyl, dimethylcyclopropyl, methylcyclobutyl, dimethylcyclobutyl, methylcyclopentyl, dimethylcyclopentyl methylcyclohexyl. In some embodiments R^{E3B} is cyclopentyl, cyclohexyl or cycloheptyl.

In some embodiments R^{E3B} is cyclohexyl.

In some embodiments R^{E3B} is cyclohexyl and R^{E3A} is an unsubstituted tetralin-1-yl.

In some embodiments E has the structure E3-1: 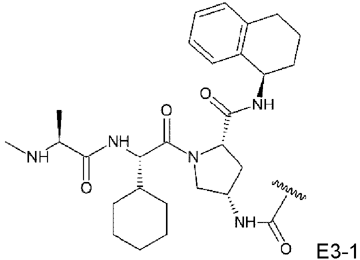 wherein 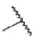 represents attachment point to L.

### E4

In some embodiments E has a structure according to Formula E4 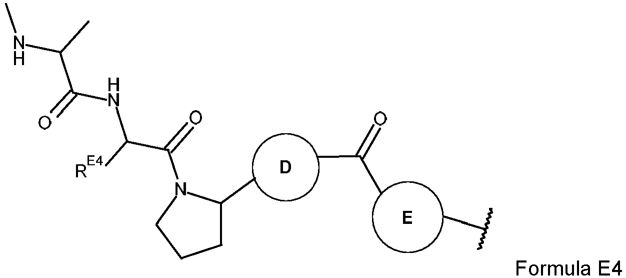 wherein
R^{E4} is selected from H, C₁₋₆ alkyl, or C₃₋₇ cycloalkyl;
Ring D is C₅₋₇ heterocyclyl or C₅₋₇ heteroaryl;
Ring E is C₆₋₁₀ carboaryl, or C₅₋₁₀ heteroaryl; and 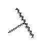 represents attachment point to L.

When R^{E4} is C₃₋₇ cycloalkyl in some embodiments it is selected from cyclopropyl, cyclobutyl, cyclopentyl, cyclohexyl, cycloheptyl, methylcyclopropyl, dimethylcyclopropyl, methylcyclobutyl, dimethylcyclobutyl, methylcyclopentyl, dimethylcyclopentyl methylcyclohexyl. In some embodiments R^{E4} is cyclopentyl, cyclohexyl or cycloheptyl.

In some embodiments R^{E4} is a C₃₋₇ cycloalkyl.

In some embodiments R^{E4} is a C₅₋₆ cycloalkyl.

In some embodiments R^{E4} is cyclohexyl.

In some embodiments Ring D is a C₅₋₇ heteroaryl.

When Ring D is a C₅₋₇ heteroaryl in some embodiments it contains 1 to 4 heteroatoms selected from O, N and S. In some embodiments it is a 5 or 6 membered heteroaryl with 1 or 2 heteroatoms. In some embodiments it is a 5 membered heteroaryl with 2 heteroatoms. In some embodiments it is a 5 membered heteroaryl with 2 heteroatoms one being N and the other being S.

In some embodiments Ring D is an N containing C₅₋₇ heteroaryl group. In some embodiments Ring D a C₅₋₇ heteroaryl group containing one N atom and one S atom.

When Ring D is a C₅₋₇ heteroaryl in some embodiments it is a pyrrolyl, pyridyl, furanyl, thiophenyl, oxazolyl, isoxazolyl, isoxainyl, oxadiazolyl, oxatirazolyl, thiazolyl, isothiazolyl, imidazolyl, pyrazolyl, pyridazinyl, pyrimidinyl, pyrazinyl, triazolyl, triazinyl or tetrazolyl.

In some embodiments Ring D is a thiazolyl. In some embodiments Ring D is a 1,3-thiazole linked in position 2 to the pyrrolidine ring and in position 4 to C=O.

In some embodiments Ring D is

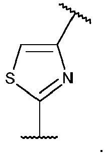

In some embodiments Ring E is a C₆ heteroaryl containing 1 heteroatom selected from O, N or S.

In some embodiments Ring E is C₆₋₁₀ carboaryl.

When Ring E is C₆₋₁₀ carboaryl in some embodiments it is phenyl.

In some embodiments Ring E is

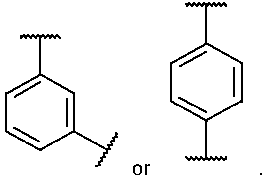

In some embodiments R^{E4} is C₅₋₆ cycloalkyl, Ring D is a C₅₋₇ heteroaryl group containing one S and one N and Ring E is phenyl.

In some embodiments E has the following structure E4-1: 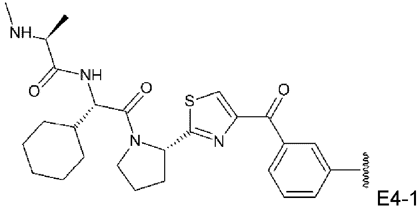 wherein 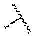 represents attachment point to L.

### E5

In some embodiments E has a structure according to E5: 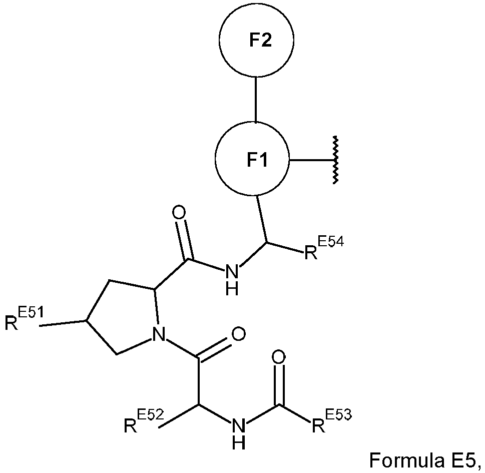
wherein Ring F1 is optionally substituted C₆₋₁₀ carboaryl, or optionally substituted C₅₋₁₀ heteroaryl group wherein the optional substituents are selected from C₁₋₆ alkyl, halogen, and OH;
Ring F2 is an optionally substituted C₆₋₁₀ carboaryl, an optionally substituted C₅₋₁₀ heteroaryl group an or an optionally substituted C₅₋₁₀ heterocyclic group wherein the optional substituents are selected from C₁₋₆ alkyl, halogen, and OH;
R^{E51} is selected from H, OH, OR, NH₂, NHR, or NRR';
wherein R and R' are C₁₋₆ alkyl, C₂₋₆ alkenyl, C₂₋₆ alkynyl, C₃₋₇ cycloalkyl, C₄₋₇ heterocyclyl;
R^{E52} is selected from optionally substituted C₁₋₆ alkyl, optionally substituted C₃₋₇ cycloalkyl, or optionally substituted C₅₋₁₀ heterocyclyl, wherein the optional substituents are OH, C₁₋₆ alkyl, or halogen;
R^{E53} is selected from optionally substituted C₃₋₆ cycloalkyl, optionally substituted C₁₋₆ alkyl, optionally substituted C₄₋₆ heterocycloalkyl, H or OH, wherein the optional substituents are selected from halogen, CN, OH and C₁₋₆ alkyl;
R^{E54} is selected from H and C₁₋₆ alkyl; and 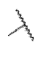 represents attachment to the Linker L.

In some embodiments Ring F2 is an optionally substituted C₆₋₁₀carboaryl, or an optionally substituted C₅₋₁₀ heteroaryl group, wherein the optional substituents are selected from C₁₋₆ alkyl, halogen or OH.

In some embodiments when Ring F2 is an optionally substituted C₆₋₁₀ carboaryl, or an optionally substituted C₅₋₁₀ heteroaryl group, wherein the optional substituents are selected from methyl, ethyl, propyl, OH or halogen. In some embodiments the optional substituents are selected from methyl, ethyl, OH, F or Cl. In one embodiment there are 1 or 2 optional substituents. In some embodiments the optional substituent is methyl.

In some embodiments when Ring F2 is an optionally substituted C₆₋₁₀ carboaryl it is an optionally substituted phenyl wherein the optional substituents are selected from methyl, ethyl, propyl, OH, F or Cl. In some embodiments Ring F2 is an unsubstituted phenyl.

In some embodiments Ring F2 is an optionally substituted C₅₋₁₀ heteroaryl group, wherein the optional substituents are selected from C₁₋₆ alkyl, halogen or OH. In some embodiments the optional substituents are selected from methyl, ethyl, OH, F or Cl.

In some embodiments, Ring F2 is an optionally substituted C₅₋₆ heteroaryl group, wherein the optional substituents are selected from methyl, ethyl, propyl, halogen or OH.

When Ring F2 is an optionally substituted C₅₋₁₀ heteroaryl group, in some embodiments it is an optionally substituted C₅₋₆ heteroaryl group. In some embodiments Ring F2 has 1 or 2 heteroatoms selected from O, S and N. In some embodiments Ring F2 is selected from azolyl, pyridiyl, furanyl, thiophenyl, oxazolyl, isoxazolyl, isoxazinyl, thiazolyl, isothiazolyl, imidazolyl, pyrazoyl, pyridazinyl, pyrimidinyl or pyrazinyl. In some embodiments Ring F2 has 1 or 2 heteroatoms selected from S and N.

When Ring F2 is an optionally substituted C₅₋₁₀ heteroaryl group, in some embodiments it is an optionally substituted C₅ heteroaryl group. In some embodiments Ring F2 has two heteroatoms one being N and the other being S. In some embodiments the optional substituents are methyl. In some embodiments there is one methyl substituent on the C₅ heteroaryl.

In some embodiments Ring F2 is a 1,3-thiazole optionally substituted by methyl. In further embodiments Ring F2 is 4-methyl-1,3-thiazol-5-yl.

In some embodiments Ring F2 is

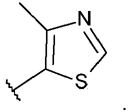

In some embodiments Ring F1 is an optionally substituted C₆₋₁₀carboaryl, or an optionally substituted C₅₋₁₀ heteroaryl group, wherein the optional substituents are selected from C₁₋₆ alkyl, halogen or OH.

When Ring F1 is an optionally substituted C₅₋₁₀ heteroaryl group it is an optionally substituted C₅₋₆ heteroaryl group which comprises 1 or 2 heteroatoms selected from O, S and N. In some embodiments the optional substituents are selected from methyl, ethyl, F, Cl and OH.

In some embodiments, Ring F1 is an optionally substituted C₆₋₁₀ carboaryl, wherein the optional substituents are selected from C₁₋₆ alkyl, halogen or OH.

When Ring F1 is an optionally substituted C₆₋₁₀ carboaryl, or an optionally substituted C₅₋₁₀ heteroaryl group, in some embodiments the optional substituents are selected from methyl, ethyl, F, Cl and OH.

In some embodiments when Ring F1 is an optionally substituted C₆₋₁₀ carboaryl it is an optionally substituted phenyl wherein the optional substituents are selected from methyl, ethyl, F, Cl and OH.

In further embodiments Ring F1 is phenyl.

In some embodiments R^{E51} is H, OH, OR or NH₂, wherein R is selected from C₁₋₆ alkyl or C₃₋₇ cycloalkyl. In some embodiments when R^{E51} is OR, R is methyl, ethyl or cyclopropyl. In some embodiments R^{E51} is H, OH or OMe. In further embodiments R^{E51} is OH.

In some embodiments R^{E52} is C₁₋₆ alkyl. In some embodiments R^{E52} is C₃₋₅ alkyl. In further embodiments R^{E52} is tert-butyl.

In some embodiments when R^{E53} is C₃₋₆ cycloalkyl it is optionally substituted cyclopropyl or optionally substituted cyclobutyl. In some embodiments when R^{E53} is C₁₋₆ alkyl it is methyl, ethyl or propyl. In some embodiments when R^{E53} is C₃₋₆ heterocycloalkyl it is selected from aziridine, 2H-azirine, oxirane, thiirane, azetidine, oxetane, pyrrolidine or tetrahydrofuran. In some embodiments R^{E53} is H. In some embodiments R^{E53} OH.

In some embodiments R^{E53} is optionally substituted C₃₋₆ cycloalkyl. In further embodiments R^{E53} is optionally substituted cyclopropyl. In some embodiments, R^{E53} is methyl.

In some embodiments when R^{E53} is optionally substituted with halogen it is substituted with F, Cl or Br. In some embodiments R^{E53} is unsubstituted. In some embodiments R^{E53} is substituted with CN.

In some embodiments R⁵³ is optionally substituted with F, Cl, Br, CN, OH or methyl. In some embodiments R⁵³ is optionally substituted with F or Cl. In further embodiments R⁵³ is substituted with F.

In some embodiments R^{E54} is H. In some embodiment R^{E54} is methyl, ethyl, propyl or butyl.

In some embodiments E5 is of the following formula E5-1: 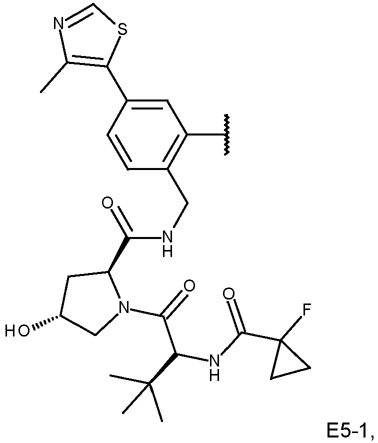 wherein ' 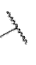 represents attachment point to L.

### E6

In some embodiments E has a structure according to Formula E6: 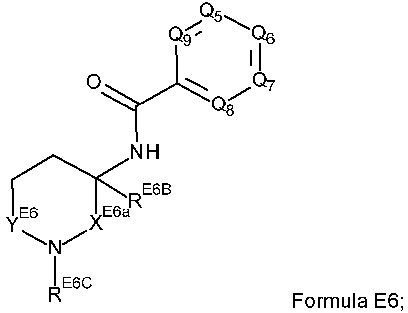 wherein
X^{E6a} is selected from C=O or C=S;
Y^{E6} is selected from C=O or C=S;
Q₅, Q₆, Q₇, Q₈, and Q₉ are independently N, CH, CR^{E6D}, or CR^{E6A} wherein one of Q₅, Q₆, Q₇, and Q₈ is CR^{E6A};
R^{E6A} is selected from a bond, -C(=O)-, -CH₂-, -O-, -NR-, -NH-, and -SO₂-;
R^{E6B} is selected from H, halogen, C₁₋₆ alkyl, and C₃₋₇ cycloalkyl;
R^{E6C} is selected from H, OH, and C₁₋₆ alkyl; and
R^{E6D} is selected from OH, halogen, C₁₋₆ alkoxy, C₁₋₆ alkyl, C₂₋₆ alkenyl, C₂₋₆ alkynyl, C₃₋₇ cycloalkyl, C₄₋₇ heterocyclyl;
R is selected from H, C₁₋₆ alkoxy, C₁₋₆ alkyl, C₂₋₆ alkenyl, C₂₋₆ alkynyl, C₃₋₇ cycloalkyl, C₄₋₇ heterocyclyl; and E is joined to L by the group R^{E6A}.

In some embodiments X^{E6a} is C=O.

In some embodiments Y^{E6} is C=O.

In some embodiments Y^{E6} and X^{E6a} are each C=O and R^{E6C} is H.

In some embodiments one of Q₅, Q₆, Q₇, Q₈, and Q₉ is CR^{E6A}, and no more than one of Q₅, Q₆, Q₇, or Q₈ are N.

In some embodiments one of Q₅, Q₆, Q₇, Q₈, and Q₉ is CR^{E6A}, and the remaining are CH or CR^{E6D}.

In some embodiments, R^{E6D} is selected from halogen, C₁₋₆ alkoxy, and C₁₋₆ alkyl.

In some embodiments R^{E6D} is selected from C₁₋₆ alkoxy, and C₁₋₆ alkyl.

In some embodiments R^{E6D} is selected from F, Cl, Br, and OMe

In some embodiments Q₆ is CR^{E2A}.

In some embodiments Q₆ is CR^{E2A}, Q₅, Q₇, and Q₉ are CH and Q₈ is selected from CH, C-halogen, and C-OCH₃.

In some embodiments R^{E6A} is a bond, or -NR- wherein R is H, C₁₋₆ alkyl, C₂₋₆ alkenyl, C₂₋₆ alkynyl, C₃₋₇ cycloalkyl, C₄₋₇ heterocyclyl. In some embodiments, R^{E6A} is a bond. In some embodiments, R^{E6A} is -NR-wherein R is H, OH, C₁₋₆alkyl, C₂₋₆ alkenyl, C₂₋₆ alkynyl, C₃₋₇ cycloalkyl, C₄₋₇ heterocyclyl. In some embodiments R^{E6A} is -NR- wherein R is H or C₁₋₆ alkyl. In some embodiments R^{E6A} is -NH-.

In some embodiments R^{E6B} is H, F, Cl, or Br. In some embodiments R^{E6B} is C₁₋₃alkyl. In some embodiments, R^{E6B} is H or fluorine. In some embodiments R^{E6B} is H.

In some embodiments wherein R^{E6C} is selected from H, OH, and C₁₋₆ alkyl. In some embodiments R^{E6C} is H.

In some embodiments, R^{E6D} is selected from OH, halogen, C₁₋₆alkoxy, and C₁₋₆ alkyl. In some embodiments, R^{E2D} is C₁₋₆ alkoxy.

In some embodiments E6 is formula E6-1 and has the following structure: 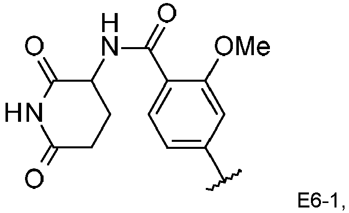 wherein 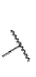 represents attachment point to L

In some embodiments E6 is formula E6-2 and has the following structure: 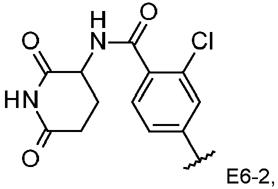 wherein 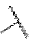 represents attachment point to L

In some embodiments E6 is formula E6-3 and has the following structure: 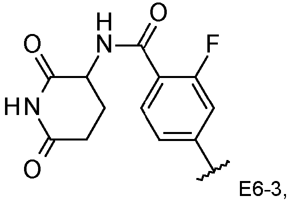 wherein 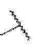 represents attachment point to L

In some embodiments E is selected from one of the following structures:

| **E** | **Structure** | **E** | **Structure** |
|---|---|---|---|
| E1-1 | 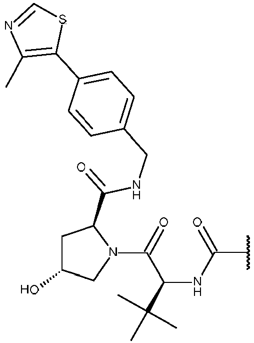 | E4-1 | 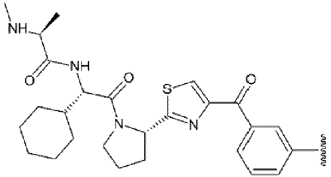 |
| E1-2 | 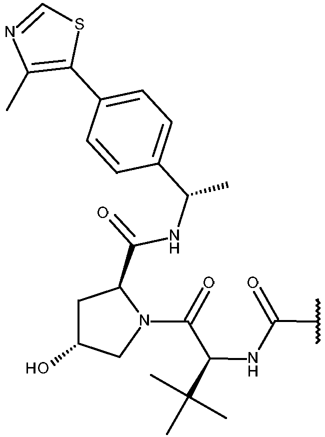 | E5-1 | 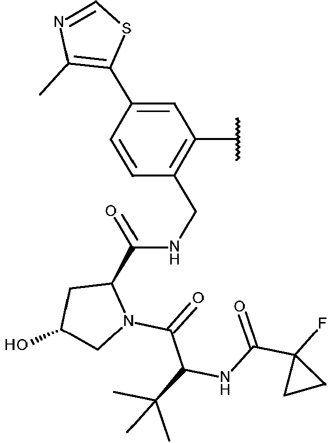 |
| E2-1 | 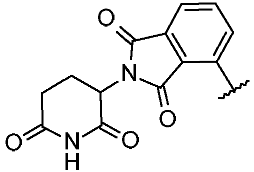 | E6-1 | 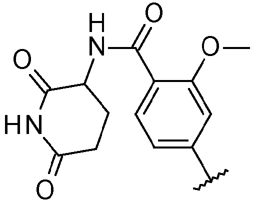 |
| E2-2 | 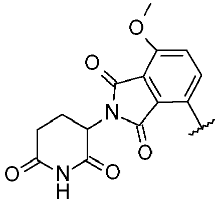 | E6-2 | 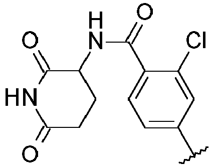 |
| E3-1 | 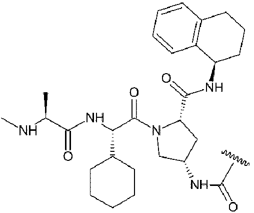 | E6-3 | 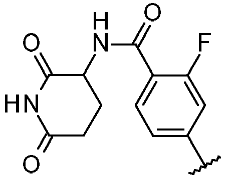 |

### Linker (L)

L is a linker. L is a linker which connects S1 to E.

In some embodiments, L is a saturated or a partially or fully unsaturated framework comprising C and H atoms and at least one heteroatom, wherein said framework has a minimum length of from 10-40 atoms between B' and E; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-CO-R^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, Cl, and CN;
wherein each R^{L} is independently selected from C₁₋₆ alkyl, C₂₋₆ alkenyl, C₂₋₆ alkynyl, C₃₋₆ cycloalkyl, C₃₋₆ carbocyclyl, C₅₋₆ heterocyclyl, C₅₋₆ aryl, and C₅₋₆ heteroaryl
wherein said Linker is attached via its point of attachment to an available C, N, O, or S atom at E and the O atom of S1.

In some embodiments, L is a saturated or a partially or fully unsaturated framework comprising C and H atoms and at least one heteroatom, wherein said framework has a shortest length of 10-40 atoms; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-COR^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, Cl, and CN;
wherein each R^{L} is independently selected from C₁₋₃ alkyl and cyclopropyl, and wherein the number of optional substituents is below 10;
wherein said Linker is attached via its point of attachment to an available C, N, O, or S atom at E and the O atom of S1.

In some embodiments L is a saturated or a partially or fully unsaturated framework comprising C and H atoms and at least one heteroatom, wherein said framework has a shortest length of 10-40 atoms; wherein said framework may include one or more straight and/or branched chains and/or rings and is optionally substituted on any available C atom(s) by one or more =O, OH, C₁₋₃ alkyl, F, C₁₋₃ alkoxy, NR^{L}₂, wherein each R^{L} is selected from H, C₁₋₆ alkyl;
wherein said Linker is attached via its point of attachment to an available C, N, O, or S atom at E or the O atom of S1.

In some embodiments L connects the STING ligand to the E3 ubiquitin ligase ligand via a chain of atoms having 10-40 atoms in shortest length. In further embodiments L connects the STING ligand to the E3 ubiquitin ligase ligand via a chain of atoms having 15-36 atoms in shortest length.

In some embodiments, L is a saturated or a partially or fully unsaturated framework comprising C and H atoms, and at least one heteroatom wherein said framework may include one or more of the groups selected from -O-, -C(=O)-, -N(R^{L1})-, -N(R^{L1})-C(=O)-, -C(=O)-N(R^{L1})-, -C₁₋₆alkyl-N(R^{L1}), -N(R^{L1})-C(=O)-C₁₋₆alkyl, - C₁₋₆alkyl-C(=O)-N(R^{L1})-, -C(=O)-N(R^{L1})-C₁₋₆alkyl-, -C₁₋₆alkyl-N(R^{L})-C(=O)-, and/or heterocyclic rings or heteroaryl rings,
wherein R^{L1} is selected from H, C₁₋₆alkyl.

In some embodiments L has the following structure:

*-L_{A}-[X]_{X}-L_{B}-*

wherein
L_{A} and L_{B} are each independently selected from a bond, *-C₁₋₆alkyl-, *-O-C₁₋₆alkyl-, *-C₃₋₇cycloalkyl, *-C₄₋₇heterocyclyoalkyl-, *-C₃₋₇cycloalkyl-C₃₋₇cycloalkyl-, *-C₄₋₇heterocyclyoalkyl-C₄₋₇heterocyclyoalkyl-, *-C₁-salkyl-O-, *-O- , *-N(R^{L1})-, *-N(R^{L1})-C(=O)-, *-C(=O)-N(R^{L1})-, *-O-CH₂-C(=O)-N(R^{L1})-, *-N(R^{L1})-C(=O)-CH₂-O- *-N(R^{L1})-C₁₋₆alkyl-, *-C₁₋₆alkyl-N(R^{L1})-, *-N(R^{L1})-C(=O)-C₁₋₆alkyl-, *-C₁₋₆alkyl-C(=O)-N(R^{L1})-, *-C(=O)-, `-C(=O)-C₁₋₆alkyl-,*-C(=O)-N(R^{L1})-C₁₋₆alkyl-, *-C₁₋₆alkyl-N(R^{L1})-C(=O)-, and a functional group selected from carbonyl, ester, amide, carbamate and thiourea;
wherein R^{L1} is selected from H, C₁₋₆alkyl;
X is selected from: a bivalent, saturated or unsaturated, straight or branched, C₁₋₄₅ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, -N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, -S(=O)₂N(R^{L})-, - S(=O)₂N(H)-, C₅₋₇ carbocyclyl, a C₅₋₇ heterocyclyl, a C₆₋₁₀ carboaryl, or a C₅₋₁₀ heteroaryl group;
wherein R^{L} is a C₁₋₆ alkyl group; and
* denotes the attachment to S1 or E.

In some embodiments X is selected from: a bivalent, saturated or unsaturated, straight or branched, C₆₋₃₆ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, - N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, - S(=O)₂N(R^{L})-, -S(=O)₂NH), C₅₋₇ carbocyclyl, a C₅₋₇ heterocyclyl, a C₆ carboaryl, or a C₅₋₇ heteroaryl group, 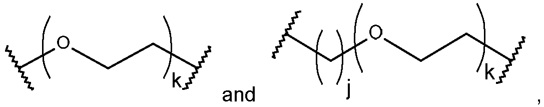 wherein k and j are each individually integers from 1 to 8;
wherein R^{L} is a C₁₋₆alkyl group.

In further embodiments X contains one or more -N(H)-C(=O)- or -C(=O)-N(H)- groups and 1 to 10 of the following group:

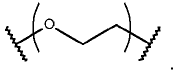

In some embodiments L has the structure:

E-(L7)ᵢ-(L6)ₕ-(L5)_{g}-(OC₂H₄)_{f}-(L4)ₑ-(L3)_{d}-(L2)_{c}-(OC₂H₄)_{b-}(NR^{X}C(=O))-(L1)ₐ-S1

wherein:
L1 is C₁₋₆ alkyl;
L2 is C₁₋₆ alkyl;
L3 is - N(methyl)-C(=O)-, -NH-C(=O)-, -C(=O)NH- or C(=O)N(methyl)-;
L4 is -C(=O)-C₅₋₇ heterocyclyl-C(=O)- or -C(=O)-C₅₋₇ heteroaryl-C(=O)-;
L5 is C₁₋₁₂ alkyl wherein one or more -CH₂- groups is optionally replaced by one or more groups selected from -O-, -NH-, -NMe-, -C(=O)NH, -C(=O)NMe, --NHC(=O)-, NMeC(=O)- group;
L6 is C₁₋₆ alkyl optionally substituted by a =O group;
L7 is -NH-, -NMe-, C₃₋₆cycloalkyl, or C₄₋₆heterocyclyl;
R^{X} is H or methyl.
a is 0 or 1;
b is 0 to 8;
c is 0 or 1;
d is 0 or 1;
e is 0 or 1;
f is 0 to 8;
g is 0 or 1;
h is 0 or 1;
i is 0 to 2;
E is the ubiquitin ligase as defined in any of the preceding claims; and
S1 is S1 as defined in any of the preceding claims.

In some embodiments when L1 is C₁₋₆ alkyl it is methyl, ethyl or propyl. In some embodiments L1 is methyl.

In some embodiments a is 0. In some embodiments a is 1.

In some embodiments L1 is methyl and a is 1.

In some embodiments b is 0 to 6. In some embodiments b is 1 to 5. In some embodiments b is 2 to 5. In some embodiments b is 2, 3, 4 or 5.

In some embodiments when L2 is C₁₋₆ alkyl it is methyl, ethyl or propyl. In some embodiments L1 is methyl or ethyl. In some embodiments L2 is ethyl.

In some embodiments c is 0. In some embodiments c is 1.

In some embodiments L2 is ethyl and c is 1.

In some embodiments L3 is NHC(=O) or N(methyl)C(=O).

In some embodiments d is 0. In some embodiments d is 1.

In some embodiments L3 is NHC(=O) or N(methyl)C(=O) and d is 1.

In some embodiments e is 0.

In some embodiments e is 1.

In some embodiments when e is 1 L4 is -C(=O)-C₆ heterocyclyl-C(=O)- wherein the C₆ heterocyclyl contains 1 or 2 heteroatoms selected from O, N or S. In some embodiments the C₆ heterocyclyl is selected from piperidine, piperazine or morpholine.

In some embodiments when e is 1 L4 is -C(=O)-C₆ heteroaryl-C(=O)- wherein the C₆ heteroaryl contains 1 or 2 heteroatoms selected from O, N or S. In some embodiments the C₆ heteroaryl is selected from pyridine, pyrimidine or pyrazine.

In some embodiments when e is 1 L4 is:

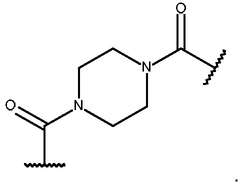

In some embodiments f is 0 to 5. In some embodiments f is 0. In some embodiments f is 1. In some embodiments f is 2. In some embodiments f is 3. In some embodiments f is 4. In some embodiments f is 5.

In some embodiments when g is 1, L5 is a C₄₋₁₀ alkyl optionally interrupted by one or more -O- groups and a C(=O)NH- group. In some embodiments when g is 1, L5 is a C₄₋₈ alkyl optionally interrupted by one or two -O- groups and a C(=O)NH- group. In some embodiments L5 is a C₆ alkyl interrupted by one -O-group and one C(=O)NH- group. In some embodiments L5 is a C₈ alkyl interrupted by two -O- groups and a C(=O)NH- group.

In some embodiments, L5 is -O-C₄H₈-NHC(=O)-C₂H₄- or -O-C₄H₈-NH-C(=O)-C₂H₄-O-C₂H₄-. In some embodiments L5 is -O-C₄H₈-NHC(=O)-C₂H₄- or -O-C₄H₈-NH-C(=O)-C₂H₄-O-C₂H₄- and g is 1.

In some embodiments g is 0.

In some embodiments h is 0.

In some embodiments when h is 1 L6 is a C₁₋₄ alkyl optionally substituted by =O.

In some embodiments L6 is methyl, ethyl, butyl or propyl substituted by =O and h is 1.

In some embodiments when h is 1 L6 is methyl. In some embodiments when h is 1 L6 is ethyl, In some embodiments when h is 1 L6 is propyl optionally substituted by =O. In some embodiments when h is 1 L6 is butyl.

In some embodiments L7 is C₃₋₆heterocyclyl. In some embodiments L7 is piperidinyl or piperazinyl.

In some embodiments, L7 is -NH- or C₃₋₆heterocyclyl, and i is 1 or 2.

In some embodiments, L7 is -NH-, and i is 1.

In some embodiments i is 0. In some embodiments i is 1. In some embodiments i is 2.

In some embodiments linker L is selected from one of the following structures:

| **L** | **Structure** |
|---|---|
| LK1 | 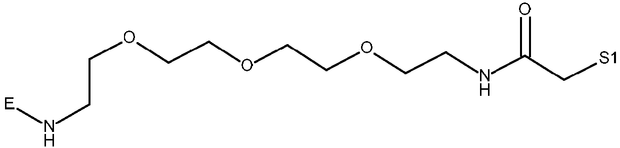 |
| LK2 | 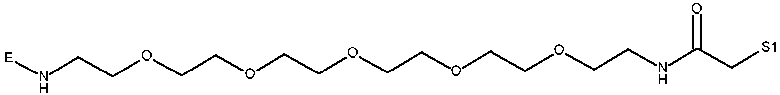 |
| LK3 | 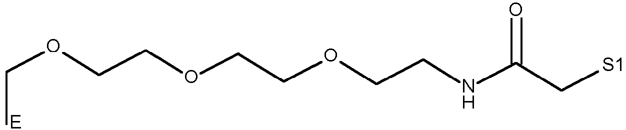 |
| LK4 | 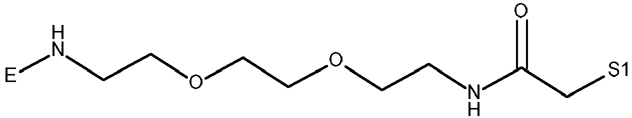 |
| LK5 | 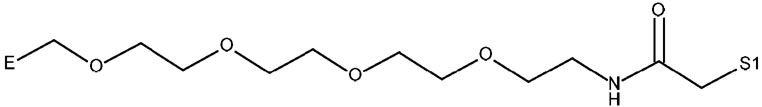 |
| LK6 | 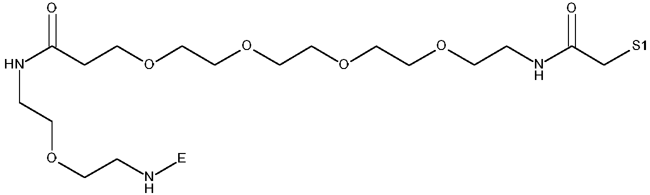 |
| LK7 | 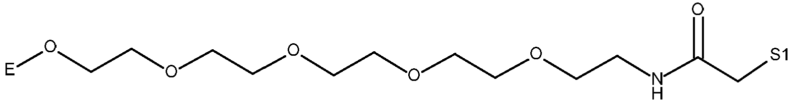 |
| LK8 | 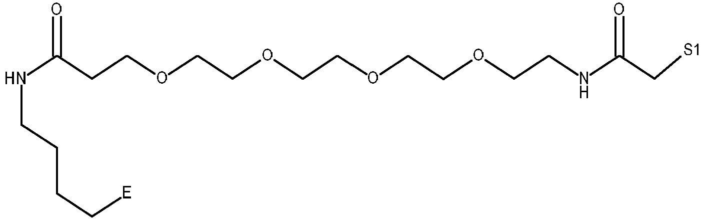 |
| LK9 | 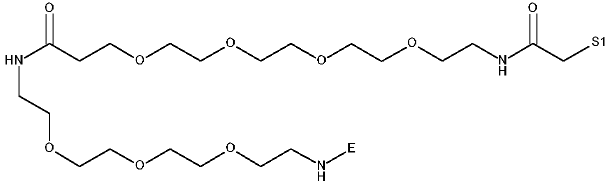 |
| LK10 | 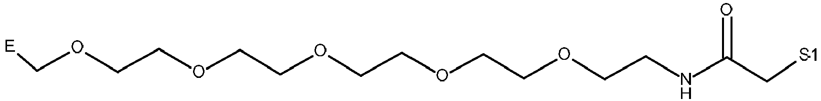 |
| LK11 | 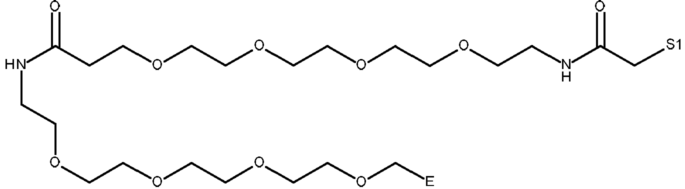 |
| LK12 | 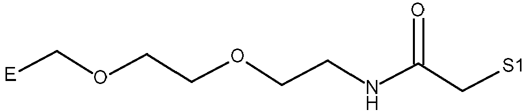 |
| LK13 | 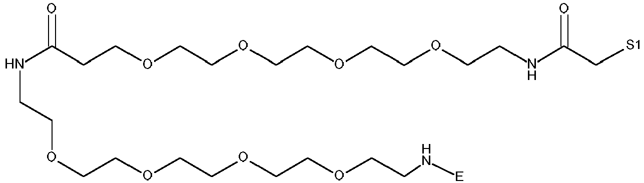 |
| LK14 | 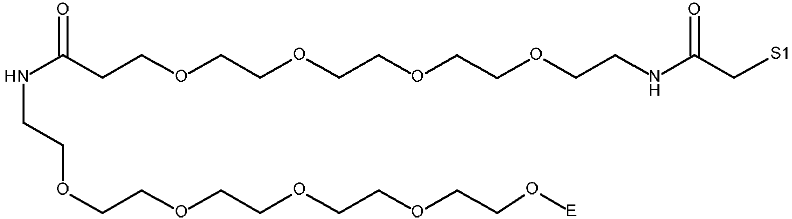 |
| LK15 | 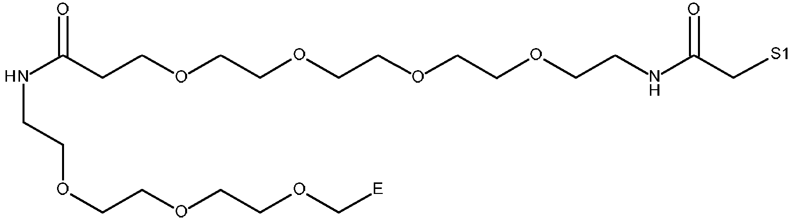 |
| LK16 | 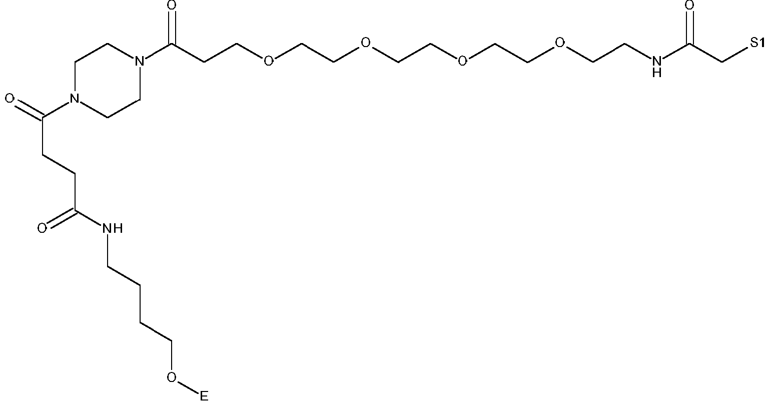 |
| LK17 | 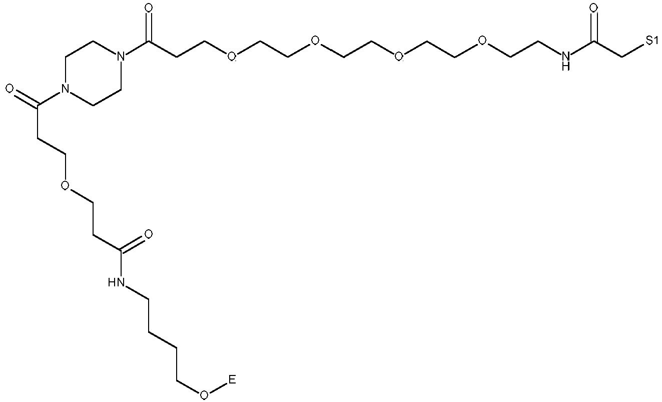 |
| LK18 | 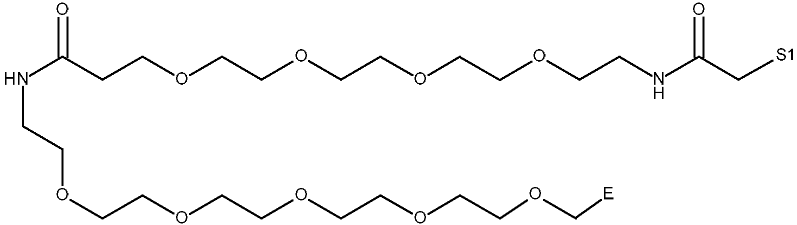 |
| LK19 | 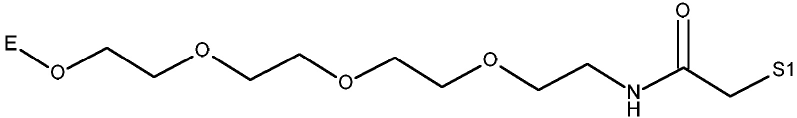 |
| LK20 | 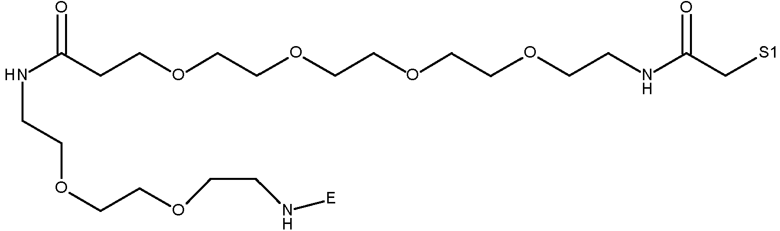 |
| LK21 | 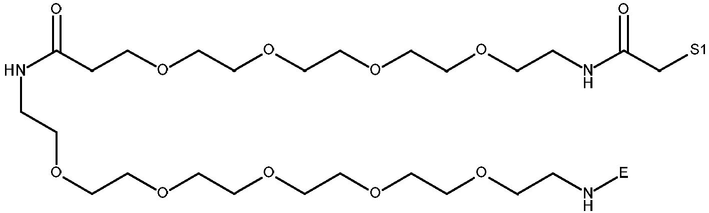 |
| LK22 | 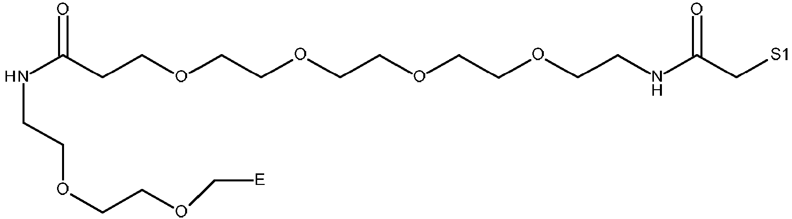 |
| LK23 | 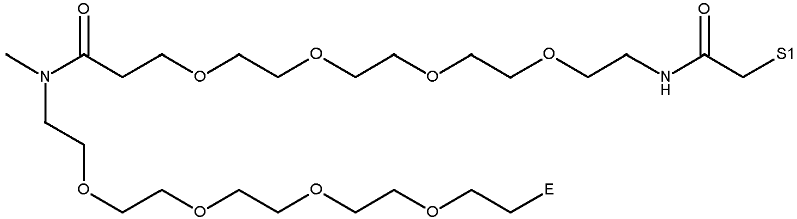 |
| LK24 | 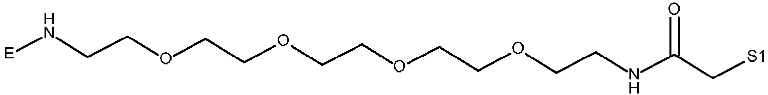 |
| LK25 | 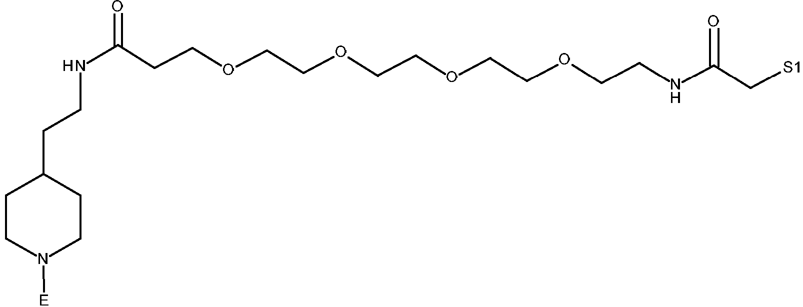 |
| LK26 | 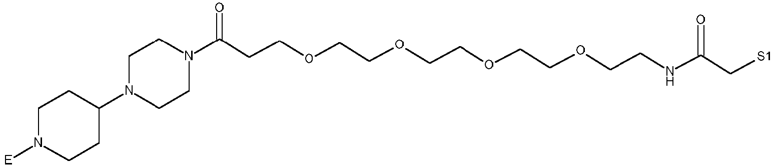 |

### S1-L-E

In some embodiments S1-L-E is selected from one of the following formulae: 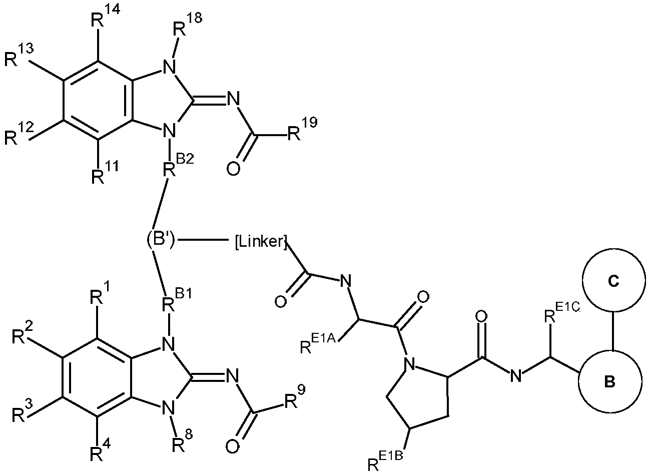 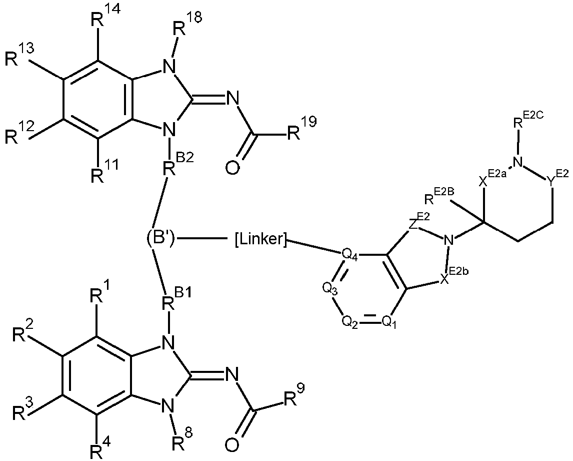 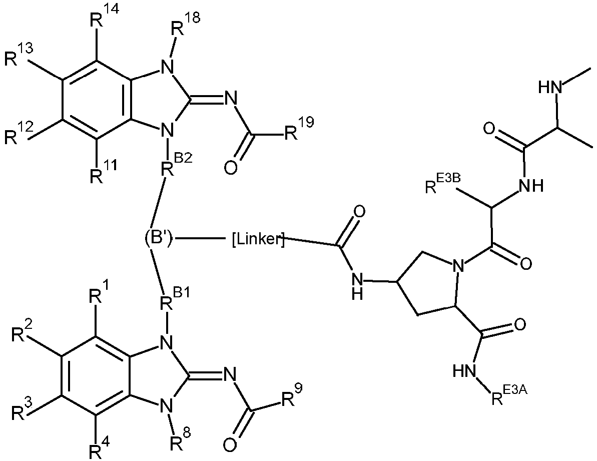 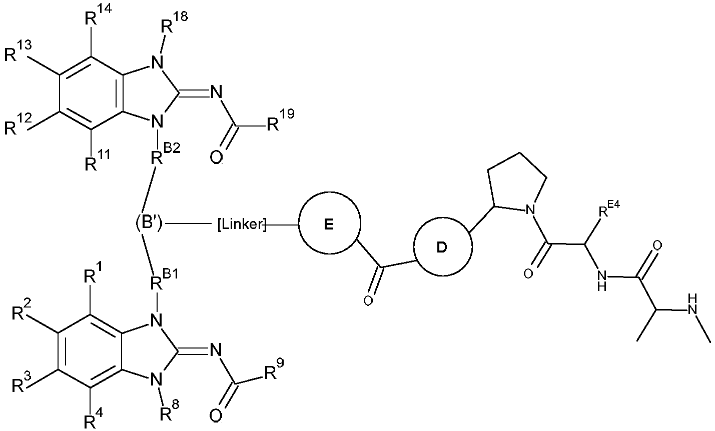 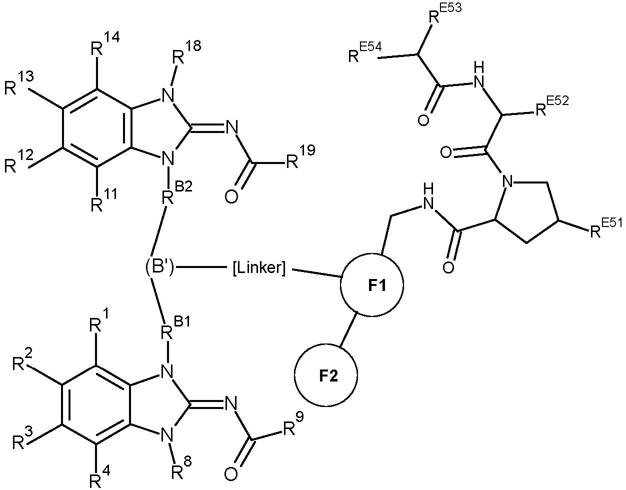 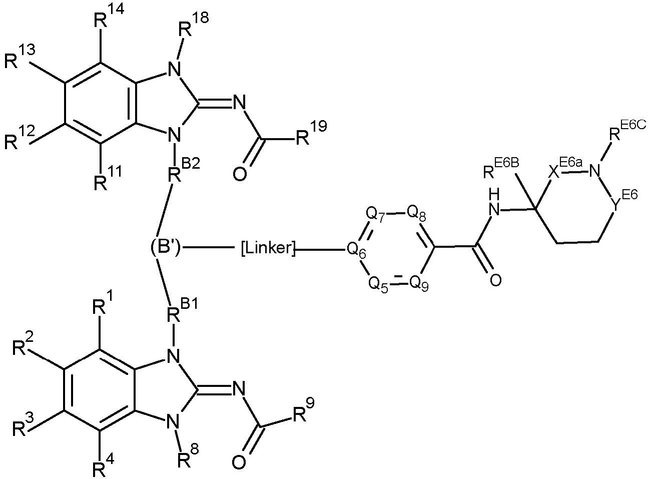 or tautomers thereof, wherein the substituents R¹, R², R³, R⁴, R⁸, R⁹, R^{B1}, B', Linker, R^{B2}, R¹¹, R¹², R¹³, R¹⁴, R¹⁸, R¹⁹ R^{E1A}, R^{E1B}, R^{E1C}, Rings B, C, D, E, F1, F2, Q₁, Q₂, Q₃, Q₄, Q₅, Q₆, Q₇, Q₈, Q₉, Z^{E2}, X^{E2b}, R^{E2B}, X^{E2a}, R^{E2C}, R^{E2D}, Y^{E2}, X^{E6a}, Y^{E6}, R^{E6B}, R^{E6C}, R^{E3A}, R^{E3B}, R^{E4}, R^{E51}, R^{E52}, R^{E53}, and R^{E54} are as defined above.

In some embodiments, R⁹ is: 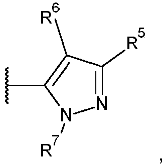 and R¹⁹ is:

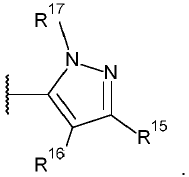

In some embodiments, R⁹ and R¹⁹ are each:

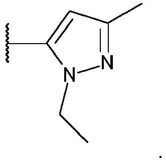

In some embodiments S1-L-E is selected from one of the following formulae:

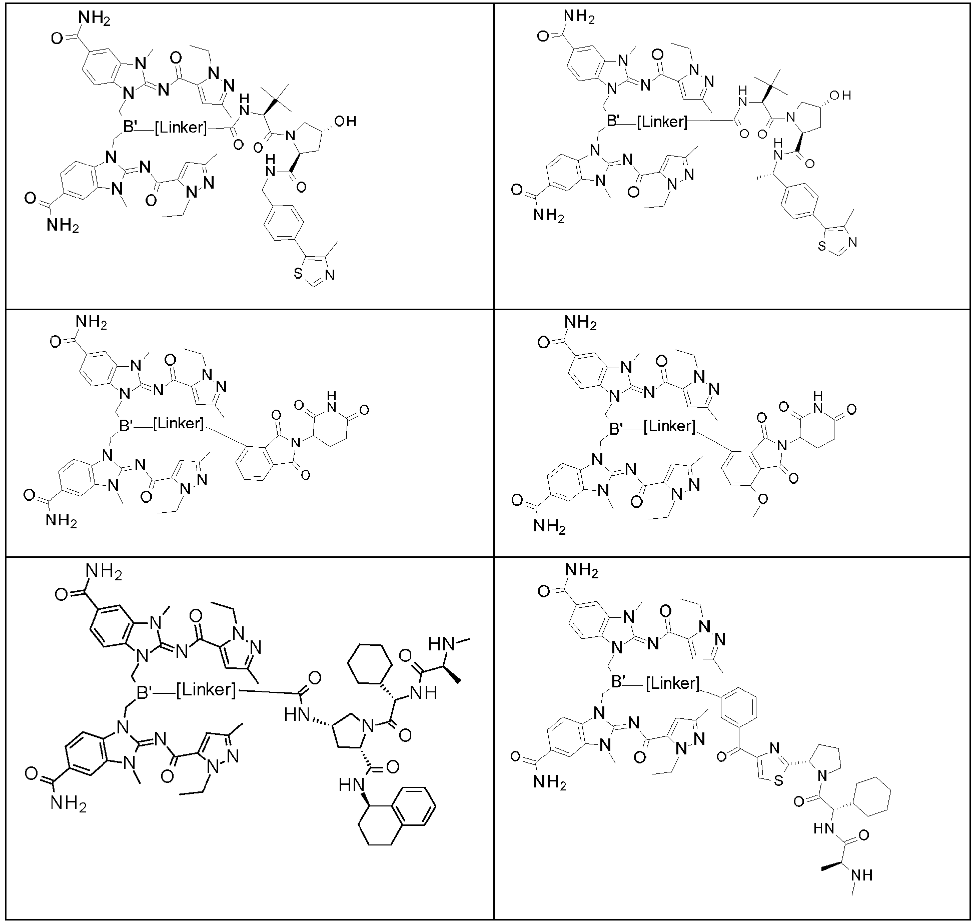

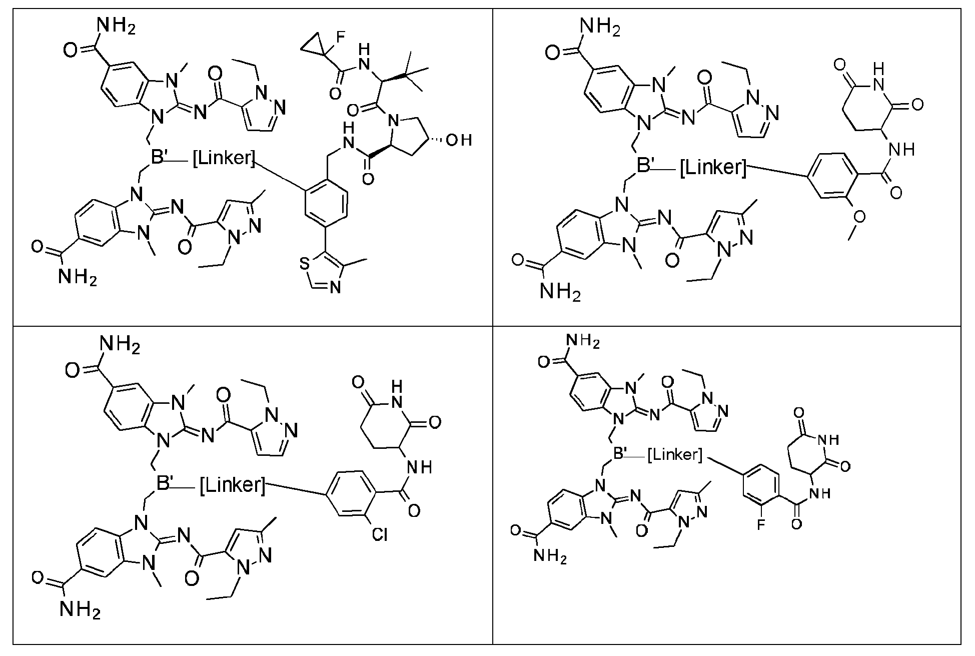

In some embodiments the compound is selected from one of the following compounds 1-38 in Table 1.

**Table 1**

| **Example number** | **Structure** | **IUPAC Name** |
|---|---|---|
| 1 | 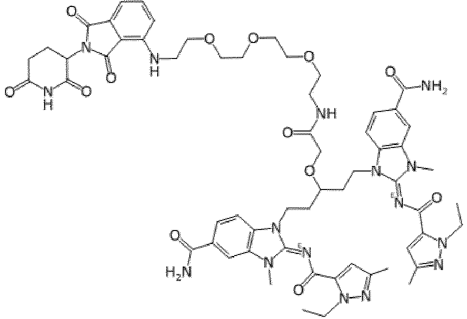 | 1-[3-({N-2-[2-(2-{2-[2-(2,6-dioxo-3-piperidyl)-1,3-dioxo-4-isoindolinylamino]ethoxy}ethox y)ethoxy]ethylcarbamoyl}metho xy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| 2 | 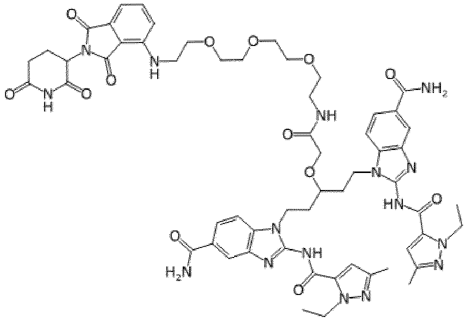 | N-{1-[3-({N-2-[2-(2-{2-[2-(2,6-dioxo-3-piperidyl)-1,3-dioxo-4-isoindolinylamino]ethoxy}ethox y)ethoxy]ethylcarbamoyl}metho xy)-5-[5-carbamoyl-2-(1-ethyl-3-methyl-5-pyrazolylcarbonylamino)-1H-1,3-benzimidazol-1-yl]pentyl]-5-carbamoyl-1H-1,3-benzimidazol-2-yl}-1-ethyl-3-methyl-5-pyrazolecarboxamide |
| 3 | 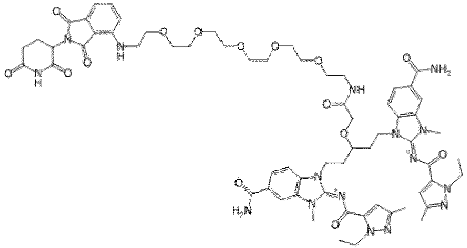 | 1-(3-{[N-2-(2-{2-[2-(2-{2-[2-(2,6-dioxo-3-piperidyl)-1,3-dioxo-4-isoindolinylamino]ethoxy}ethox y)ethoxy]ethoxy}ethoxy)ethylca rbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 4 | 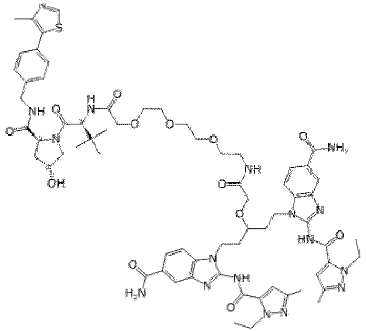 | N-[(S)-1 -{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropyl]11-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbon ylamino]-3,6,9-trioxaundecanamide |
| 5 | 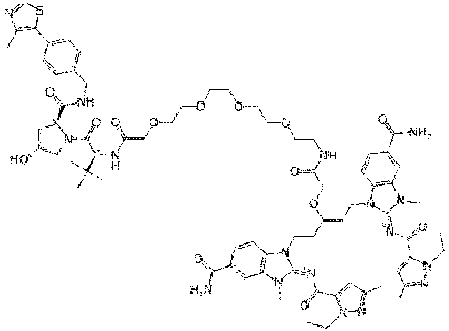 | N-[(S)-1 -{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropyl]14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecanamide |
| 6 | 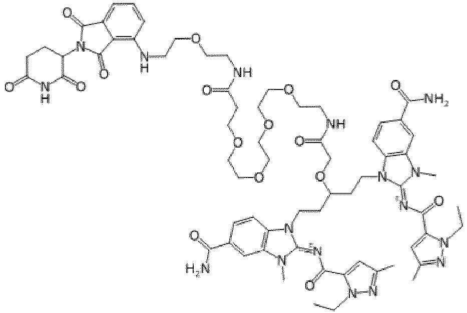 | N-(2-{2-[2-(2,6-dioxo-3-piperidyl)-1,3-dioxo-4-isoindolinylamino]ethoxy}ethyl) 15-{[(3-{5-carbamoyl-2-[(1 - ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 7 | 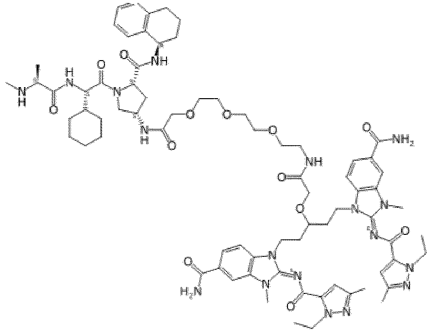 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidi nyl] 11-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9-trioxaundecanamide |
| 8 | 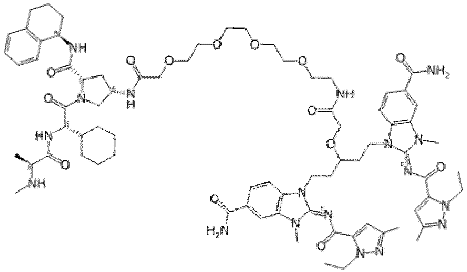 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidi nyl] 14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecanamide |
| 9 | 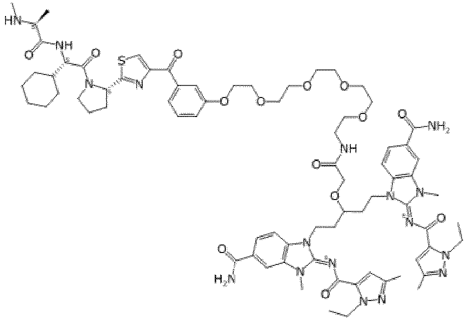 | 1-{3-[(N-2-{2-[2-(2-{2-[m-({2-[(S)-1-{(S)-2-[(S)-2-(methylamino)propionylamino]-2-cyclohexylacetyl}-2-pyrrolidinyl]-1,3-thiazol-4-yl}carbonyl)phenoxy]ethoxy}eth oxy)ethoxy]ethoxy}ethylcarbam oyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 10 | 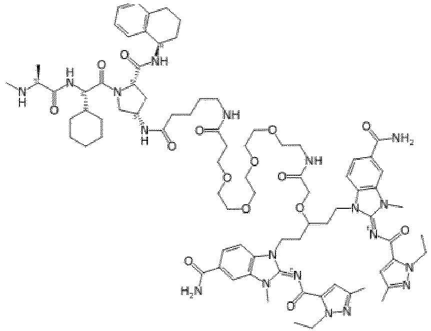 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]5-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecylcarbonylamin o)valeramide |
| 11 | 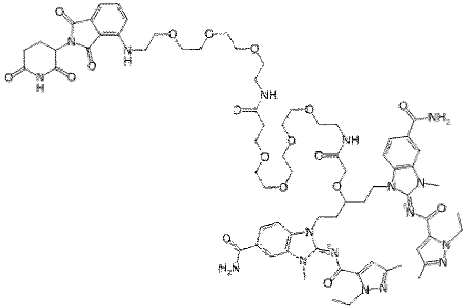 | N-{2-[2-(2-{2-[2-(2,6-dioxo-3-piperidyl)-1,3-dioxo-4-isoindolinylamino]ethoxy}ethox y)ethoxy]ethyl}15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 12 | 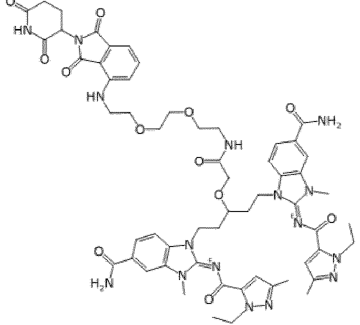 | 1-(3-{[N-2-(2-{2-[2-(2,6-dioxo-3-piperidyl)-1,3-dioxo-4-isoindolinylamino]ethoxy}ethox y)ethylcarbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 13 | 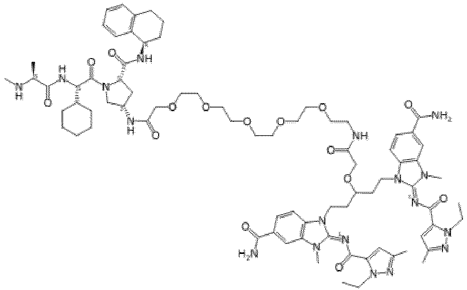 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]17-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9, 12, 15-pentaoxa heptadecana mid e |
| 14 | 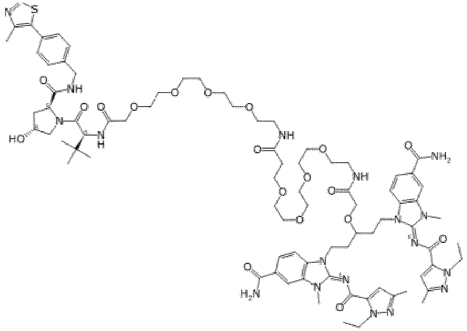 | N-[(S)-1 -{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropyl] 14-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecylcarbonylamin o)-3,6,9,12-tetraoxatetradecanamide |
| 15 | 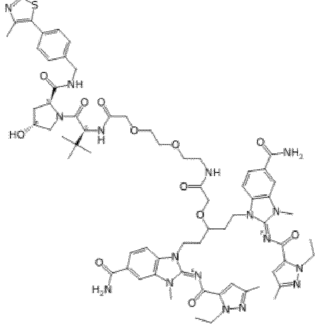 | 1-(3-{[N-2-(2-{[N-(S)-1-{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropylcarbamoyl]meth oxy}ethoxy)ethylcarbamoyl]met hoxy}-5-{5-carbamoyl-2-[(1 - ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 16 | 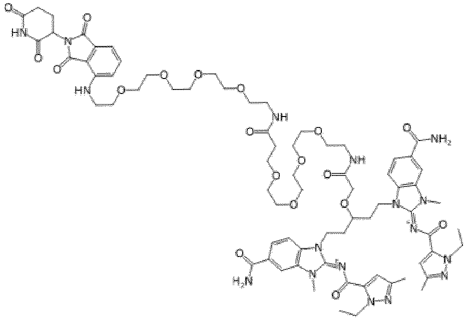 | N-(2-{2-[2-(2-{2-[2-(2,6-dioxo-3-piperidyl)-1,3-dioxo-4-isoindolinylamino]ethoxy}ethox y)ethoxy]ethoxy}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 17 | 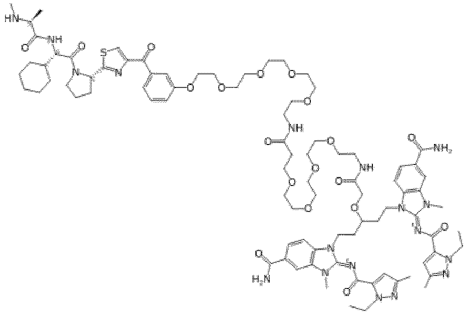 | N-(2-{2-[2-(2-{2-[m-({2-[(S)-1-{(S)-2-[(S)-2-(methylamino)propionylamino]-2-cyclohexylacetyl}-2-pyrrolidinyl]-1,3-thiazol-4-yl}carbonyl)phenoxy]ethoxy}eth oxy)ethoxy]ethoxy}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 18 | 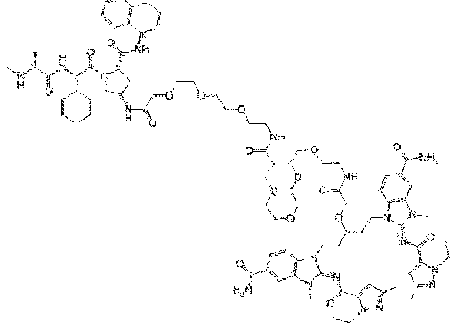 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]11-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecylcarbonylamin o)-3,6,9-trioxaundecanamide |
| 19 | 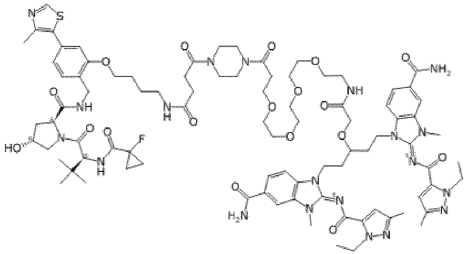 | N-{15-[4-(3-{N-4-[2-({(2S,4R)-1-[(S)-2-(1-fluorocyclopropanecarbonylami no)-3,3-dimethylbutyryl]-4-hydroxy-2-pyrrolidinylcarbonylamino}meth yl)-5-(4-methyl-1,3-thiazol-5-yl)phenoxy]butylcarbamoyl}pro pionyl)-1-piperazinyl]-15-oxo-3,6,9,12-tetraoxapentadecyl}(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide |
| 20 | 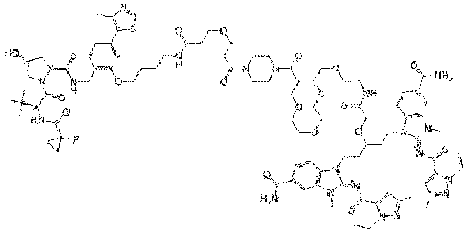 | N-(15-{4-[3-(2-{N-4-[2-({(2S,4R)-1-[(S)-2-(1-fluorocyclopropanecarbonylami no)-3,3-dimethylbutyryl]-4-hydroxy-2-pyrrolidinylcarbonylamino}meth yl)-5-(4-methyl-1,3-thiazol-5-yl)phenoxy]butylcarbamoyl}eth oxy)propionyl]-1-piperazinyl}-15-oxo-3,6,9,12-tetraoxapentadecyl)(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide |
| 21 | 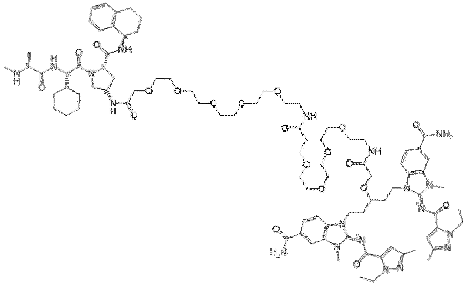 | N-{16-[N-(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinylcarbamoyl]-3,6,9,12,15-pentaoxahexadecyl}15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 22 | 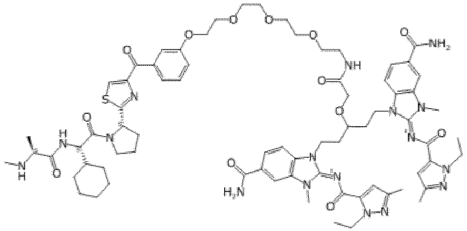 | 1-[3-({N-2-[2-(2-{2-[m-({2-[(S)-1-{(S)-2-[(S)-2-(methylamino)propionylamino]-2-cyclohexylacetyl}-2-pyrrolidinyl]-1,3-thiazol-4-yl}carbonyl)phenoxy]ethoxy}eth oxy)ethoxy]ethylcarbamoyl}met hoxy)-5-{5-carbamoyl-2-[(1 - ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| 23 | 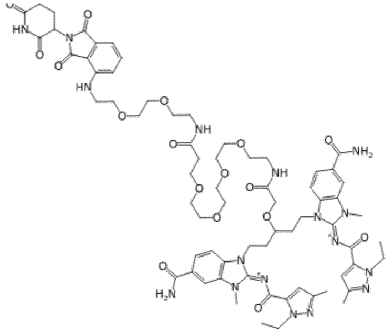 | N-[2-(2-{2-[2-(2,6-dioxo-3-piperidyl)-1,3-dioxo-4-isoindolinylamino]ethoxy}ethox y)ethyl]15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 24 | 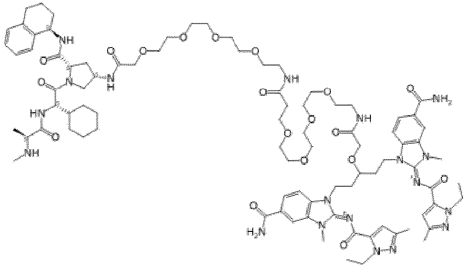 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]14-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecylcarbonylamin o)-3,6,9,12-tetraoxatetradecanamide |
| 25 | 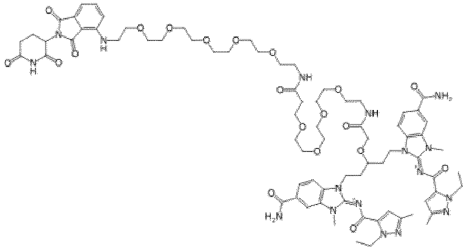 | N-[2-(2-{2-[2-(2-{2-[2-(2,6-dioxo-3-piperidyl)-1,3-dioxo-4-isoindolinylamino]ethoxy}ethox y)ethoxy]ethoxy}ethoxy)ethyl] 1 5-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 26 | 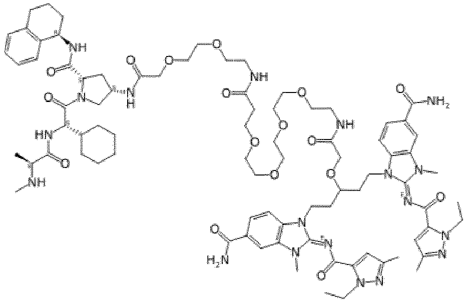 | N-[2-(2-{[N-(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinylcarbamoyl] methoxy} ethoxy)ethyl]15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 27 | 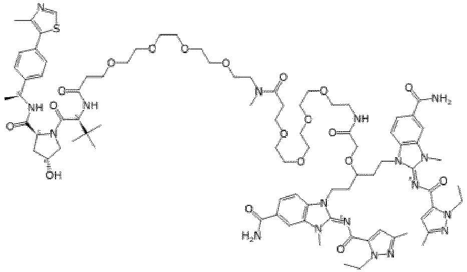 | N-{14-[N-(S)-1-{[(2S,4R)-2-{N-(S)-1-[p-(4-methyl-1,3-thiazol-5-yl)phenyl]ethylcarbamoyl}-4-hydroxy-1-pyrrolidinyl]carbonyl}-2,2-dimethylpropylcarbamoyl]-3,6,9,12-tetraoxatetradecyl}-N-methyl-1-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxa-14-tetradecanecarboxamide |
| 28 | 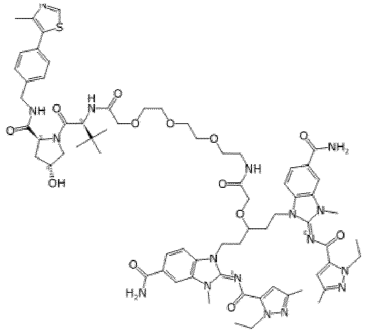 | N-[(S)-1 -{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropyl]11-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylaminoj-3,6,9-trioxaundecanamide |
| 29 | 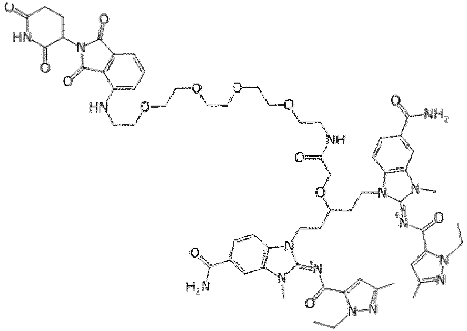 | 1-{3-[(N-2-{2-[2-(2-{2-[2-(2,6-dioxo-3-piperidyl)-1,3-dioxo-4-isoindolinylamino]ethoxy}ethox y)ethoxy]ethoxy}ethylcarbamoy I)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 30 | 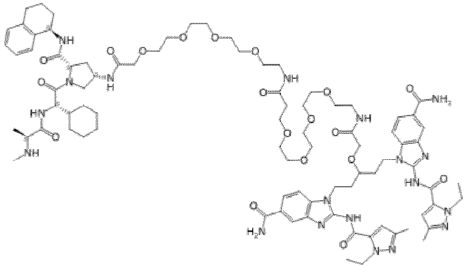 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]14-{14-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbon ylamino]-3,6,9,12-tetraoxatetradecylcarbonylamin o}-3,6,9,12-tetraoxatetradecanamide |
| 31 | 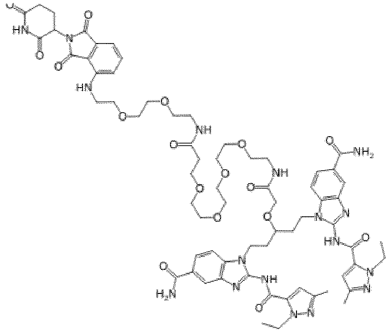 | N-[2-(2-{2-[2-(2,6-dioxo-3-piperidyl)-1,3-dioxo-4-isoindolinylamino]ethoxy}ethox y)ethyl] 15-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbon ylamino]-4,7,10,13-tetraoxapentadecanamide |
| 32 | 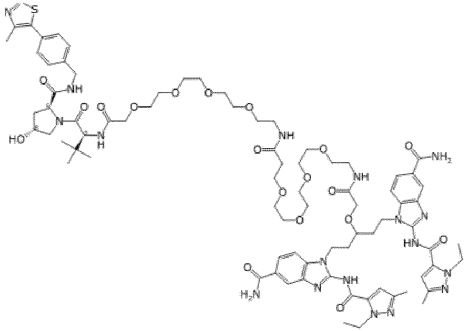 | N-[(S)-1-{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropyl] 14-{14-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbon ylamino]-3,6,9,12-tetraoxatetradecylcarbonylamin o}-3,6,9,12-tetraoxatetradecanamide |
| 33 | 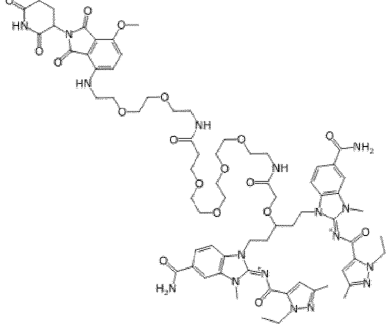 | N-[2-(2-{2-[2-(2,6-dioxo-3-piperidyl)-7-methoxy-1,3-dioxo-4-isoindolinylamino]ethoxy}ethox y)ethyl]15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 34 | 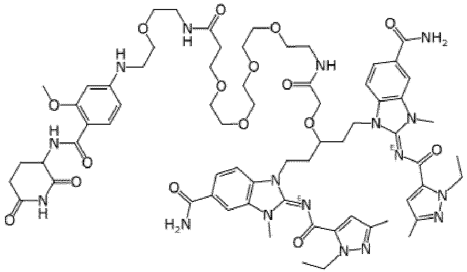 | N-(2-{2-[4-(N-2,6-dioxo-3-piperidylcarbamoyl)-3-anisidino]ethoxy}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 35 | 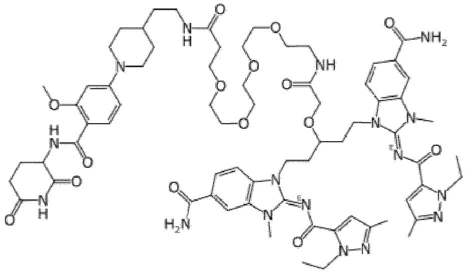 | N-(2-{1-[4-(N-2,6-dioxo-3-piperidylcarbamoyl)-3-methoxyphenyl]-4-piperidyl}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 36 | 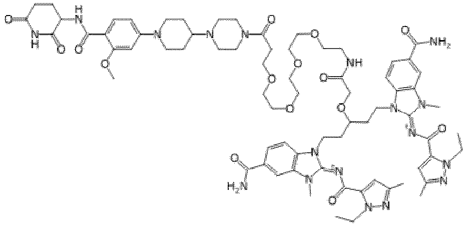 | N-[15-(4-{1 -[4-(N-2,6-dioxo-3-piperidylcarbamoyl)-3-methoxyphenyl]-4-piperidyl}-1-piperazinyl)-15-oxo-3,6,9,12-tetraoxapentadecyl](3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide |
| 37 | 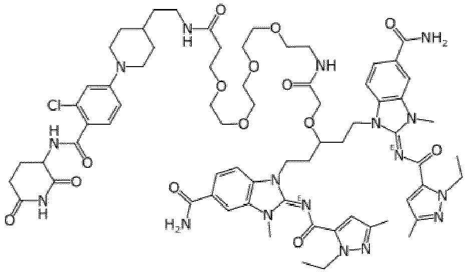 | N-(2-{1-[4-(N-2,6-dioxo-3-piperidylcarbamoyl)-3-chlorophenyl]-4-piperidyl}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 38 | 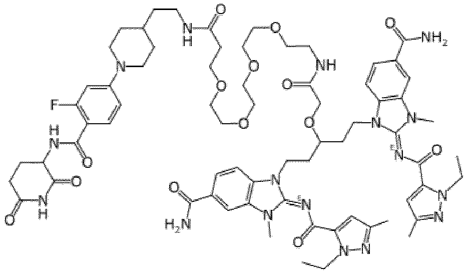 | N-(2-{1-[4-(N-2,6-dioxo-3-piperidylcarbamoyl)-3-fluorophenyl]-4-piperidyl}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

All compounds of the present invention can be synthesized by those skilled in the art of organic synthesis. Non-standard procedures and syntheses of special building blocks and intermediates are described below.

### Materials and methods

### List of abbreviations

| | |
|---|---|
| aq | aqueous |
| Boc | *tert*-butyloxycarbonyl |
| BuOH | butanol |
| cat.# | catalogue number |
| CV | column volumes |
| DCM | dichloromethane |
| diABZI | (E)-1-(4-(5-carbamoyl-2-(1-ethyl-3-methyl-1H-pyrazole-5-carboxamido)-7-(3-morpholinopropoxy)-1H-benzo[d]imidazol-1-yl)but-2-en-1-yl)-2-(1-ethyl-3-methyl-1H-pyrazole-5-carboxamido)-7-methoxy-1H-benzimidazole-5-carboxamide tris(hydrochloride); CAS-no. 2138299-34-8 |
| diABZI c3 | (E)-1-(4-(5-carbamoyl-2-(1-ethyl-3-methyl-1H-pyrazole-5-carboxamido)-7-(3-morpholinopropoxy)-1H-benzo[d]imidazol-1-yl)but-2-en-1-yl)-2-(1-ethyl-3-methyl-1H-pyrazole-5-carboxamido)-7-methoxy-1H-benzimidazole-5-carboxamide tris(hydrochloride); CAS-no. 2138299-34-8 |
| DIAD | diisopropyl azodicarboxylate |
| DIPEA | diisopropylethylamine; N-ethyl-N-(propan-2-yl)propan-2-aminel |
| DMAP | 4-(dimethylamino)pyridine |
| DMF | N,N-dimethylformamide |
| DMSO | dimethylsulfoxide |
| EDC | 1-(3-dimethylaminopropyl)-3-ethyl-carbodiimide hydrochloride |
| ESi | electrospray ionization |
| ESI | electrospray ionization |
| EtOAc | ethylacetate |
| EtOH | ethanol |
| FA | formic acid |
| HATU | 1-[bis(dimethylamino)methylene]-1H-1,2,3-triazolo[4,5-b]pyridinium 3-oxide hexafluorophosphate |
| HPLC | high-performance liquid chromatography |
| LCMS | liquid chromatography mass spectrometry |
| MeCN | acetonitrile |
| MeOH | methanol |
| MS | mass spectrometry; mass spectrum |
| NEt₃ | triethylamine |
| NMR | nuclear magnetic resonance spectroscopy |
| PDA | photo diode array |
| Ph | phenyl |
| PPh₃ | triphenylphosphine |
| rt | room temperature |
| Rt | retention time |
| SCX | strong cation exchange |
| SFC | supercritical fluid chromatography |
| TBAF | tetra-n-butylammonium fluoride |
| TBDMS | *tert*-butyldimethylsilyl |
| TBS | *tert*-butyldimethylsilyl |
| TLC | thin-layer chromatography |
| tBu | *tert*-butyl |
| TFA | trifluoroacetic acid |
| THF | tetrahydrofuran |
| TLC | thin-layer chromatography |
| TMS | tetramethylsilane or tetramethylsilyl |
| Ts | p-toluenesulfonyl |
| TsCl | p-toluenesulfonyl chloride |
| UPLC | ultra-performance liquid chromatography |

### LCMS methods

Analysis of products and intermediates was carried out using reverse phase analytical HPLC-MS using the parameters set out below.

**AnalpH2_MeCN_4min:** Agilent 1100 HPLC system with Waters ZQ mass detector; column: Waters Xbridge C18, 3.5 µm, 50 x 4.6 mm; column temperature 45 °C; A = water + 0.1% formic acid; B = MeCN + 0.1% formic acid; %B: 0.0 min 5%, 1.0 min 37.5%, 3.0 min 95%, 3.5 min 95%, 3.51 5%, 4.0 min 5%; flow 2.25 mL/min; PDA 210-400 nm and MS detection at 120-950 Da, ESi positive or negative.

**AnalpH9_MeCN_4min:** Agilent 1100 HPLC system with Waters ZQ mass detector; column: Waters Xbridge C18, 3.5 µm, 50 x 4.6 mm; column temperature 45 °C; A = 10 mM ammonium bicarbonate in water, pH 9; B = MeCN; %B: 0.0 min 5%, 1.0 min 37.5%, 3.0 min 95%, 3.5 min 95%, 3.51 5%, 4.0 min 5%; flow 2.25 mL/min; PDA 210-400 nm and MS detection at 120-950 Da, ESi positive or negative.

**UPLC_pH2_MeCN_2min:** Waters Acquity UPLC with Waters QDa mass detector; column: Waters BEH C18, 1.7 µm, 50 x 2.1 mm; A = water + 0.1% formic acid; B = MeCN + 0.1% formic acid; column temperature 50 °C; %B: 0.05 min 5%, 1.6 min 95%, 2.26min 5%; flow 0.6 mL/min; PDA 210-400 nm and MS detection at 120-1000 Da, ESi positive or negative.

**UPLC_pH9_MeCN_2min:** Waters Acquity UPLC with Waters QDa mass detector; column: Waters BEH C18, 1.7 µm, 50 x 2.1 mm; A = 10 mM ammonium bicarbonate in water, pH 9; B = MeCN; column temperature 50 °C; %B: 0.05 min 5%, 1.6 min 95%, 2.26 min 5%; flow 0.6 mL/min; PDA 210-400 nm and MS detection at 120-1000 Da, ESi positive or negative.

**UPLC_pH2_MeCN_QC_V1:** Waters Acquity UPLC with Waters QDa mass detector; column: Waters CSH C18, 1.7 µm, 100 x 2.1 mm; A = water + 0.1% formic acid; B = MeCN + 0.1% formic acid; column temperature 50 °C; %B: 0.05 min 5%, 5.00 min 95%, 6.60 min 5%; flow 0.35 mL/min; PDA 210-400 nm and MS detection at 120-1000 Da, ESi positive or negative.

**UPLC_pH2_MeCN_QC_V2:** Waters Acquity UPLC with Waters QDa mass detector; column: Waters CSH C18, 1.7 µm, 100 x 2.1 mm; A = water + 0.1% formic acid; B = MeCN + 0.1% formic acid; column temperature 50 °C; %B: 0.05 min 5%, 5.00 min 95%, 6.60 min 5%; flow 0.35 mL/min; PDA 210-400 nm and MS detection at 120-1250 Da, ESi positive or negative.

**UPLC_pH2_MeCN_QC_V3:** Waters Acquity UPLC with Waters QDa mass detector; column: Waters CSH C18, 1.7 µm, 100 x 2.1 mm; A = water + 0.1% formic acid; B = MeCN + 0.1% formic acid; column temperature 50 °C; %B: 0.05 min 5%, 5.00 min 95%, 6.60 min 5%; flow 0.35 mL/min; PDA 210-400 nm and MS detection at 250-2000 Da, ESi positive or negative.

**UPLC_pH9_MeCN_QC_V1:** Waters Acquity UPLC with Waters QDa mass detector; column: Waters CSH C18, 1.7 µm, 100 x 2.1 mm; A = 10 mM ammonium bicarbonate in water, pH 9; B = MeCN; column temperature 50 °C; %B: 0.05 min 5%, 5.00 min 95%, 6.60 min 5%; flow 0.35 mL/min; PDA 210-400 nm and MS detection at 120-1000 Da, ESi positive or negative.

**UPLC_pH9_MeCN_QC_V2:** Waters Acquity UPLC with Waters QDa mass detector; column: Waters CSH C18, 1.7 µm, 100 x 2.1 mm; A = 10 mM ammonium bicarbonate in water, pH 9; B = MeCN; column temperature 50 °C; %B: 0.05 min 5%, 5.00 min 95%, 6.60 min 5%; flow 0.35 mL/min; PDA 210-400 nm and MS detection at 120-1250 Da, ESi positive or negative.

**UPLC_pH9_MeCN_QC_V3:** Waters Acquity UPLC with Waters QDa mass detector; column: Waters CSH C18, 1.7 µm, 100 x 2.1 mm; A = 10 mM ammonium bicarbonate in water, pH 9; B = MeCN; column temperature 50 °C; %B: 0.05 min 5%, 5.00 min 95%, 6.60 min 5%; flow 0.35 mL/min; PDA 210-400 nm and MS detection at 250-2000, ESi positive or negative.

### ¹H-NMR spectroscopy

¹H NMR spectra were recorded at 400 MHz and 294 K if not stated otherwise. Chemical shifts (δ-values) are reported in parts per million (ppm), referenced to either tetramethylsilane (TMS) (0.0 ppm) or the corresponding solvent residual signal of DMSO (2.50 ppm in DMSO-d₆), chloroform (7.26 ppm in CDCl₃) or methanol (3.31 ppm in deuterated methanol). Coupling constants (J) are reported in Hertz (Hz), splitting patterns are designated as singlet (s), doublet (d), triplet (t), quadruplet (q), multiplet or more overlapping signals (m), broad signal (br); solvent is given in parentheses. ¹H-NMR integrations have been quantified when visible.

### Syntheses of building blocks, intermediates and probes

Methods for preparing molecules similar to some of the compounds described below can be found in WO2017/175147, WO2019/069270 and J. M. Ramanjulu et al., Nature (2018), 564(7736), 439-443, Supplementary Information.

### Intermediate 3

### 1-Ethyl-3-methyl-1H-pyrazole-5-carbonyl isothiocyanate

### Synthesis:

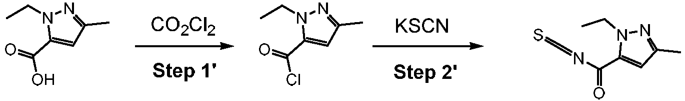

**Step 1':** To a stirred suspension of 1-ethyl-3-methyl-1H-pyrazole-5-carboxylic acid (4 g, 25.95 mmol) in anhydrous DCM (80 mL) at 0 °C were added oxalyl chloride (2.67 mL, 31.1 mmol) and DMF (0.2 mL, 2.59 mmol) dropwise. The reaction mixture was stirred at 0 °C for 15 min and then allowed to warm to rt (after 15 min the slurry turned into a light yellow solution). The reaction mixture was stirred for 1 h at rt. LCMS analysis of a sample dissolved in MeOH showed formation of the methyl ester and no starting material, indicating completed formation of the acid chloride.
LCMS (AnalpH2_MeCN_4min): Rt = 2.19 min, m/z 169.2 ([M+H]⁺ of methyl ester)

Solvents were removed under reduced pressure. 20 mL of DCM was added and the mixture was concentrated again to dryness, affording 1-ethyl-3-methyl-1H-pyrazole-5-carbonyl chloride (4.50 g, 100%) as a yellow oil, which was taken forward to the next step without further purification.

**Step 2':** 1-Ethyl-3-methyl-1H-pyrazole-5-carbonyl chloride (4.48 g, 25.95 mmol) was placed under nitrogen and dry acetone (66 mL) was added. The reaction mixture was cooled down to 0 °C and potassium thiocyanate (3.03 g, 31.1 mmol) was added portionwise. The reaction mixture was stirred for 15 min at 0 °C and then for 1 h at rt. 50 mL of isohexane was added and the reaction mixture was concentrated in vacuo to dryness. To the black residue 100 mL of isohexane were added and the resulting dark brown solid was collected by filtration and washed with isohexane twice. The yellow filtrate was concentrated in vacuo affording 1-ethyl-3-methyl-1H-pyrazole-5-carbonyl isothiocyanate (Intermediate 3; 4.60 g, 91%) as a brown oil that was stored in a freezer to avoid degradation.
LCMS (AnalpH2_MeCN_4min): Rt = 2.06 min, m/z 196.2 [M+H]⁺ and Rt = 2.85 min, m/z 196.2 [M+H]⁺ (1:1 ratio of both peaks).
¹H-NMR (400 MHz, CDCl₃): δ 6.75 (s, 1H), 4.52 (q, J = 7.2 Hz, 2H), 2.31 (s, 4H), 1.42 (t, J = 7.1 Hz, 3H)

### Intermediate L-1

### tert-Butyl N-[(1S)-1-{[(1S)-2-[(2S)-2-{4-[3-(2-{2-[2-(2-aminoethoxy)ethoxy]ethoxy}ethoxy)benzoyl]-1,3-thiazol-2-yl}pyrrolidin-1-yl]-1-cyclohexyl-2-oxoethyl]carbamoyl}ethyl]-N-methylcarbamate

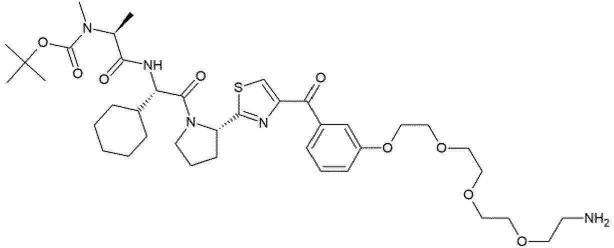

### Synthesis:

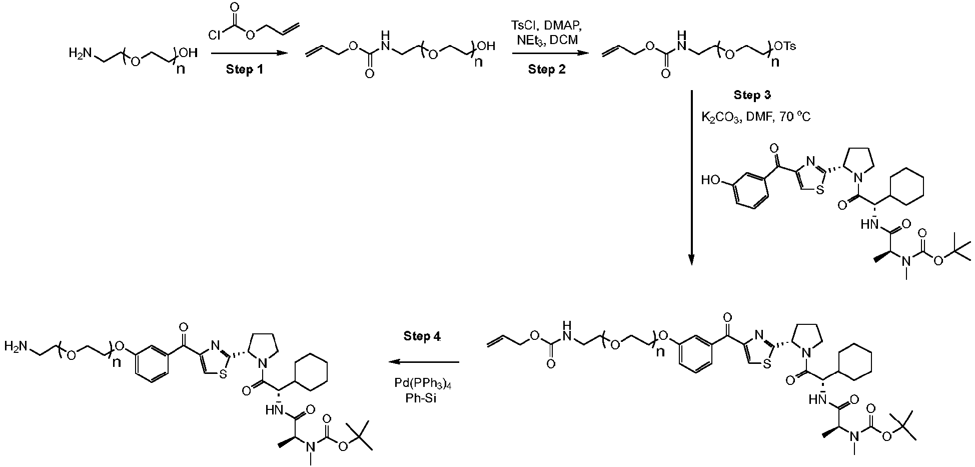

**Step** 1: To a stirred solution of 2-(2-(2-(2-aminoethoxy)ethoxy)ethoxy)ethanol (538 mg, 2.78 mmol) in acetone (7.0 mL) was added a solution of K₂CO₃ (770 mg, 5.57 mmol) in water (7.0 mL). The mixture was cooled to 0 °C. Allylchloroformate (0.30 mL, 2.78 mmol) was added. The reaction mixture was stirred at rt for several hours until consumption of starting material was observed by TLC (DCM-MeOH 9:1). Acetone was removed under reduced pressure. DCM was added and the aqueous phase was extracted with DCM (3x 8 mL). The organic phases were combined, dried over MgSO4, filtered and concentrated under reduced pressure, affording allyl N-(2-{2-[2-(2-hydroxyethoxy)ethoxy]ethoxy}ethyl)carbamate (750 mg, 2.70 mmol, 97 %) as a colourless oil.
1H-NMR (400 MHz, CDCl3) δ 6.00 (m, 2H), 5.36-5.30 (m, 1H), 5.22 (dd, J = 10.5, 1.4 Hz, 1H), 4.59 (d, J = 5.5 Hz, 2H), 3.77-3.73 (m, 4H), 3.69-3.63 (m, 8H), 3.58 (t, J = 4.8 Hz, 2H), 3.41 (q, J = 5.2 Hz, 2H)

**Step 2:** To a stirred solution of the product of Step 1 (300 mg, 1.08 mmol) in dry DCM (4.3 mL) at 0 °C were added TsCI (247 mg, 1.30 mmol), DMAP (13 mg, 0.108 mmol) and triethylamine (0.23 mL, 1.62 mmol). The reaction mixture was stirred at rt overnight. Saturated aqueous sodium bicarbonate solution was added and the organic phase was separated. The aqueous phase was further extracted with DCM (2x 20 mL). The combined organic phases were concentrated under reduced pressure. The crude residue was purified by column chromatography (SiO₂, Biotage, sfar, 25 g, dry loading), eluting from 100% isohexane to 100% EtOAc, affording 2-(2-{2-[2-(allyloxycarbonylamino)ethoxy]ethoxy}ethoxy)ethyl 4-methylbenzenesulfonate (382 mg, 82 %) as a colourless oil.
LCMS (UPLC_pH9_MeCN_2min): Rt = 1.67 min, m/z 432.1 [M+H]⁺

**Step 3:** A mixture of 2-(2-{2-[2-(allyloxycarbonylamino)ethoxy]ethoxy}ethoxy)ethyl 4-methylbenzenesulfonate (26 mg, 0.0602 mmol), tert-butyl ((S)-1-(((S)-1-cyclohexyl-2-((S)-2-(4-(3-hydroxybenzoyl)thiazol-2-yl)pyrrolidin-1-yl)-2-oxoethyl)amino)-1-oxopropan-2-yl)(methyl)carbamate (30 mg, 0.0501 mmol; available e.g. from Tocris Bioscience, cat.no. 7178) and potassium carbonate (14 mg, 0.101 mmol) was placed under nitrogen and DMF (0.50 mL) was added. The reaction mixture was stirred at 70 °C. After 2 h, another portion of the tosylate (20 mg, 0.0463 mmol) was added and stirring at 70 °C was continued. After totally 5 h, the mixture was cooled down to rt and 5 mL of water and 5 mL of DCM were added. The organic phase was separated and the aqueous phase was extracted with DCM (2 x 5 mL). The organic phases were combined and concentrated under reduced pressure, affording 2-[2-(2-{2-[m-({2-[(S)-1-[(S)-2-{(S)-2-[(tert-butyl)(methyl)(oxycarbonylamino)]propionylamino}-2-cyclohexylacetyl]-2-pyrrolidinyl]-1,3-thiazol-4-yl}carbonyl)phenoxy]ethoxy}ethoxy)ethoxy]ethyl 2-propene-1-carbamate (43 mg, 100 %) as a pale yellow oil that was used for the next step without further purification.
LCMS (UPLC_pH9_MeCN_2min): Rt = 2.04 min, m/z 858.5 [M+H]⁺

**Step 4:** A stirred solution of the crude product of Step 3 (43 mg, 0.0501 mmol) and phenylsilane (0.019 mL, 0.154 mmol) in dry DCM (0.60 mL) was degassed and purged five times with nitrogen. Then, tetrakis(triphenylphosphine)palladium(0) (5.8 mg, 0.0050 mmol) was added and the reaction mixture was stirred at rt for 50 min. The reaction mixture was concentrated under reduced pressure, then re-dissolved in a minimum amount of MeOH and loaded to an SCX cartridge (sorbent bed weight 0.5 g). The cartridge was flushed with MeOH (3 CV). The product was eluted with 2M NH₃ in MeOH (3 CV). The resulting eluate was concentrated under reduced pressure, affording tert-butyl N-[(1S)-1-{[(1S)-2-[(2S)-2-{4-[3-(2-{2-[2-(2-aminoethoxy)ethoxy]ethoxy}ethoxy)benzoyl]-1,3-thiazol-2-yl}pyrrolidin-1-yl]-1-cyclohexyl-2-oxoethyl]carbamoyl}ethyl]-N-methylcarbamate (Intermediate L-1; 40 mg, assumed quantitative) as a colourless oil.
LCMS (UPLC_pH9_MeCN_2min): Rt = 1.98 min, m/z 774.5 [M+H]⁺

### Intermediate L-2

### tert-Butyl N-[(1S)-1-{[(1S)-2-[(2S)-2-(4-{3-[(14-amino-3,6,9,12-tetraoxatetradecan-1-yl)oxy]benzoyl}-1,3-thiazol-2-yl)pyrrolidin-1-yl]-1-cyclohexyl-2-oxoethyl]carbamoyl}ethyl]-N-methylcarbamate

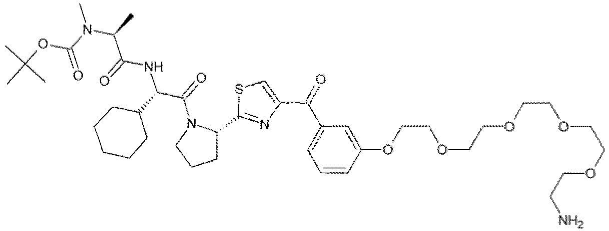

Prepared analogously to Intermediate L-1 from the corresponding aminoalcohol 2-(2-{2-[2-(2-aminoethoxy)ethoxy]ethoxy}ethoxy)ethanol.
LCMS (UPLC_pH9_MeCN_2min): Rt = 2.00 min, m/z 818.5 [M+H]⁺

### Intermediate L-3

### 3-(4-{2-[2-(2-Aminoethoxy)ethoxy]ethylamino}-7-methoxy-1,3-dioxo-2-isoindolinyl)-2,6-piperidinedione

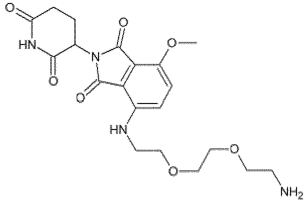

Prepared following the procedures described in: H. Bouguenina et al., A Degron Blocking Strategy Towards Improved CRL4CRBN Recruiting PROTAC Selectivity, ChemBioChem, 2023, e202300351, Supporting Information, pages 14-15 and 33-34, with the following modification: The final Boc deprotection step was performed with TFA in DCM, instead of HCl in dioxane and DCM, affording the TFA salt of Intermediate L-3.
LCMS (UPLC_pH2_MeCN_2min): Rt = 1.09 min, m/z 435.2 [M+H]⁺

### Intermediate L-4

### N-(2,6-Dioxo-3-piperidyl)-4-[2-(2-aminoethoxy)ethylamino]-2-anisamide

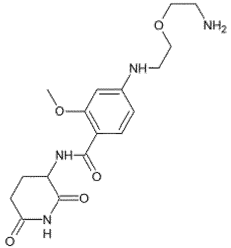

### Synthesis:

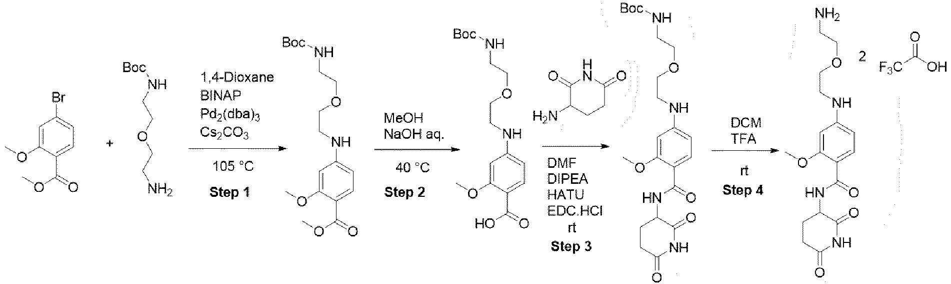

**Step 1:** Methyl 4-bromo-2-methoxybenzoate (250 mg, 1.02 mmol), tris(dibenzylideneacetone) dipalladium (0) (93 mg, 0.102 mmol), 2,2'-bis(diphenylphosphino)-1,1'-binaphthalene (64 mg, 0.102 mmol), cesium carbonate (997 mg, 3.06 mmol) and tert-butyl 2-(2-aminoethoxy)ethylcarbamate (229 mg, 1.12 mmol) were placed under nitrogen and suspended in anhydrous 1,4-dioxane (2.6 mL). The reaction mixture was purged with nitrogen for 5 min and then heated at 105 °C overnight. The reaction mixture was filtered through a celite cartridge (2.5 g), eluting with EtOAc. The filtrate was washed with brine (25 mL), passed through a hydrophobic frit, and concentrated under reduced pressure. The resulting material was purified via flash column chromatography (SiO₂; 5-95% EtOAc in isohexane), affording methyl 4-(2-{2-[tert-butyl(oxycarbonylamino)]ethoxy}ethylamino)-2-anisate (318 mg, 85 %) as a yellow gum.
LCMS (UPLC_pH9_MeCN_2min): Rt = 1.69 min, m/z 369.3 [M+H]⁺
1H-NMR (400 MHz, DMSO-d₆) δ 7.53 (d, J = 8.7 Hz, 1H), 6.78 (t, J = 5.5 Hz, 1H), 6.41 (t, J = 5.5 Hz, 1H), 6.21-6.17 (m, 2H), 3.73 (s, 3H), 3.65 (s, 3H), 3.53 (t, J = 5.7 Hz, 2H), 3.40 (t, J = 6.0 Hz, 2H), 3.24 (q, J = 5.6 Hz, 2H), 3.08 (q, J = 6.0 Hz, 2H), 1.36 (s, 9H).

**Step 2:** To a solution of methyl 4-(2-{2-[tert-butyl(oxycarbonylamino)]ethoxy}ethylamino)-2-anisate (318 mg, 0.863 mmol) in methanol (3 mL) was added sodium hydroxide (345 mg, 8.63 mmol) in H₂O (2 mL). The reaction was left to stir at 40 °C for 3 h. The pH was adjusted to pH 2 with 1 M HCl, and the mixture diluted with DCM (15 mL). Organic layer was washed with H₂O (5 mL) and brine (10 mL), then dried over MgSO₄, filtered, and the solvent evaporated under reduced pressure to give 4-(2-{2-[tert-butyl(oxycarbonylamino)]ethoxy}ethylamino)-2-anisic acid (276 mg, 90 %) as a yellow gum.
LCMS (UPLC_pH9_MeCN_2min): Rt = 1.23 min, m/z 355.3 [M+H]⁺

**Step 3:** To a stirred solution of 4-(2-{2-[tert-butyl(oxycarbonylamino)]ethoxy}ethylamino)-2-anisic acid (276 mg, 0.779 mmol) and 3-aminopiperidine-2,6-dione hydrochloride (385 mg, 2.34 mmol) in dry DMF (6.8 mL) was added DIPEA (0.54 mL, 3.10 mmol) followed by EDC (149 mg, 0.779 mmol) and HATU (444 mg, 1.17 mmol). The reaction mixture was stirred at rt for 16 h. Saturated aq NH₄Cl (100 mL) was added. The aqueous layer was extracted with 10 % MeOH in EtOAc (2x 100 mL). Organic layers were combined, washed with H₂O (100 mL) and brine (100 mL), dried over Na₂SO₄, filtered, and concentrated under reduced pressure to give a yellow gum (420 mg). 200 mg of the gum was purified by flash column chromatography (SiO₂, 0-20 % MeOH in DCM) to give a clear oil (166 mg). The oil was purified by preparative HPLC (column: Waters Xbridge C18 250 mm x 19 mm, 5 µm; method: increasing gradient of 8% to 48% MeCN in water containing 0.1% formic acid at pH 2), affording the Boc-protected intermediate 2-{2-[3-methoxy-4-(N-2,6-dioxo-3-piperidylcarbamoyl)phenylamino]ethoxy}ethyl 2-methyl-2-propanecarbamate (74 mg, 43 % based on 200 mg crude material purified) as a beige solid.
LCMS (UPLC_pH2_MeCN_2min): Rt = 1.51 min, m/z 465.3 [M+H]⁺

**Step 4:** To a solution of 2-{2-[3-methoxy-4-(N-2,6-dioxo-3-piperidylcarbamoyl)phenylamino]ethoxy}ethyl 2-methyl-2-propanecarbamate (66 mg, 0.142 mmol) in DCM (1.6 mL) was added TFA (0.72 mL). The mixture was stirred at rt for 30 min and then concentrated under reduced pressure to give the TFA salt of Intermediate L-4 (84 mg, 100 %) as a colourless oil.
LCMS (UPLC_pH2_MeCN_2min): Rt = 0.98 min, m/z 365.2 [M+H]⁺

### Intermediate L-5

### N-(2,6-Dioxo-3-piperidyl)-4-[4-(2-aminoethyl)-1-piperidyl]-2-anisamide

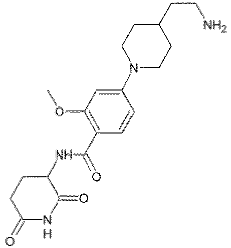

### Synthesis:

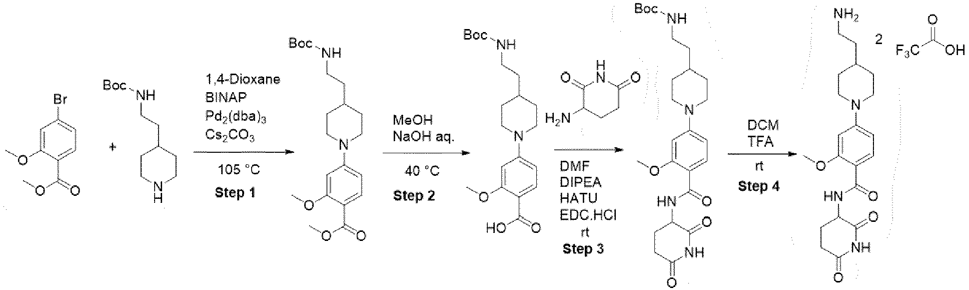

Prepared similarly to Intermediate L-4 from the corresponding amine tert-butyl [2-(piperidin-4-yl)ethyl]carbamate.
LCMS (UPLC_pH2_MeCN_2min): Rt = 1.03 min, m/z 389.2 [M+H]⁺

### Intermediate L-6

### N-(2,6-Dioxo-3-piperidyl)-2-methoxy-4-[4-(1-piperazinyl)-1-piperidyl]benzamide

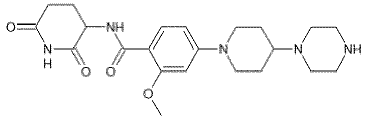

Prepared similarly to Intermediate L-4 from the corresponding amine tert-butyl 4-(piperidin-4-yl)piperazine-1-carboxylate (CAS-no. 205059-24-1).
LCMS (UPLC_pH2_MeCN_2min): Rt = 0.89 min, m/z 430.4 [M+H]⁺

### Intermediate L-7

### N-(2,6-Dioxo-3-piperidyl)-4-[4-(2-aminoethyl)-1-piperidyl]-2-chlorobenzamide

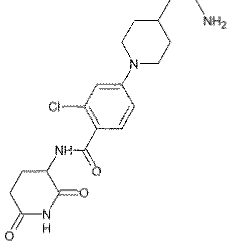

### Synthesis:

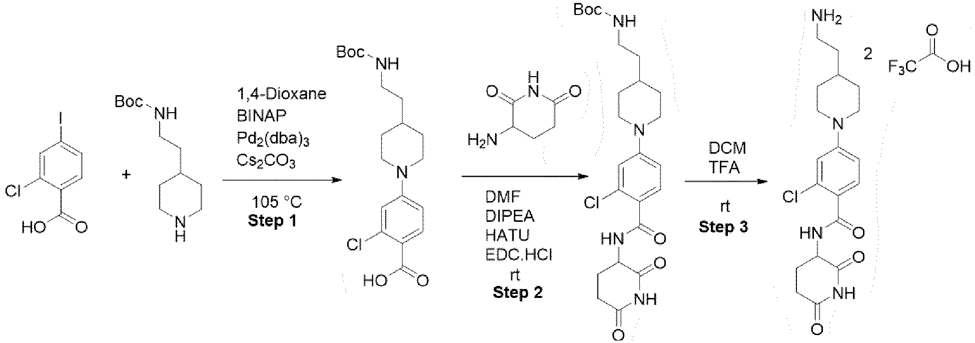

**Step 1:** 2-Chloro-4-iodobenzoic acid (250 mg, 0.867 mmol), tris(dibenzylideneacetone) dipalladium (0) (79 mg, 0.087 mmol), 2,2'-bis(diphenylphosphino)-1,1'-binaphthalene (54 mg, 0. 0.087 mmol), cesium carbonate (848 mg, 2.60 mmol) and tert-butyl [2-(piperidin-4-yl)ethyl]carbamate (218 mg, 0.954 mmol) were placed under nitrogen and suspended in anhydrous 1,4-dioxane (2.6 mL). The reaction mixture was purged with nitrogen for 5 min and then heated at 105 °C overnight. The reaction mixture was filtered through a celite cartridge (2.5 g), eluting with EtOAc. The filtrate was washed with brine (25 mL), passed through a hydrophobic frit, and concentrated under reduced pressure. The resulting material was purified via flash column chromatography (SiO₂; 0-10% MeOH in DCM), affording 4-(4-{2-[tert-butyl(oxycarbonylamino)]ethyl}-1-piperidyl)-2-chlorobenzoic acid (32 mg, 10 %) as a yellow gum.
LCMS (UPLC_pH2_MeCN_2min): Rt = 1.40 min, m/z 383.3 [M+H]⁺

**Step 2:** To a stirred solution of 4-(4-{2-[tert-butyl(oxycarbonylamino)]ethyl}-1-piperidyl)-2-chlorobenzoic acid (32 mg, 0.084 mmol) and 3-aminopiperidine-2,6-dione hydrochloride (42 mg, 0.254 mmol) in dry DMF (0.68 mL) was added DIPEA (0.059 mL, 0.339 mmol) followed by EDC (16 mg, 0.083 mmol) and HATU (48 mg, 0.127 mmol). The reaction mixture was stirred at rt for 30 min. Saturated aq NH₄Cl (10 mL) was added. The aqueous layer was extracted with 10 % MeOH in EtOAc (2x 10 mL). Organic layers were combined, washed with H₂O (10 mL) and brine (10 mL), passed through a hydrophobic frit and concentrated to give a yellow solid. The solid was dissolved in DMSO and was purified by preparative HPLC (column: Waters Xbridge C18 250 mm x 19 mm, 5 µm; method: 30-70 % MeCN in water containing 10 mM ammonium bicarbonate at pH 9), affording the Boc-protected intermediate 2-{1-[3-chloro-4-(N-2,6-dioxo-3-piperidylcarbamoyl)phenyl]-4-piperidyl}ethyl 2-methyl-2-propanecarbamate (9.6 mg, 23 %) as a white solid.
LCMS (UPLC_pH9_MeCN_2min): Rt = 1.68 min, m/z 493.2 [M+H]⁺

**Step 3:** The product of Step 2 (9.6 mg, 0.019 mmol) was dissolved DCM (0.23 mL) and TFA (0.8 mL). The mixture was stirred at rt for 2 h and then concentrated under reduced pressure to give the TFA salt of Intermediate L-7 (12.1 mg, 100 %) as a colourless oil.
LCMS (UPLC_pH9_MeCN_2min): Rt = 1.15 min, m/z 393.2 [M+H]⁺

### Intermediate L-8

### N-(2,6-Dioxo-3-piperidyl)-4-[4-(2-aminoethyl)-1-piperidyl]-2-fluorobenzamide

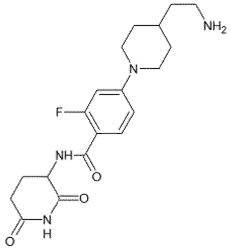

Prepared similarly to Intermediate L-4, but using methyl 4-bromo-2-fluorobenzoate and tert-butyl [2-(piperidin-4-yl)ethyl]carbamate in Step 1.
LCMS (UPLC_pH2_MeCN_2min): Rt = 1.26 min, m/z 377.2 [M+H]⁺

### Probe 1 (fluorescein-labelled STING ligand)

### N-{1-[4-(5-{N-3-(3',6'-Dihydroxy-3-oxospiro[isobenzofuran-1,9'-xanthen]-5-ylcarbonylamino)propylcarbamoyl}-2-(1-ethyl-3-methyl-5-pyrazolylcarbonylamino)-1H-1,3-benzimidazol-1-yl)butyl]-5-carbamoyl-1H-1,3-benzimidazol-2-yl}-1-ethyl-3-methyl-5-pyrazolecarboxamide

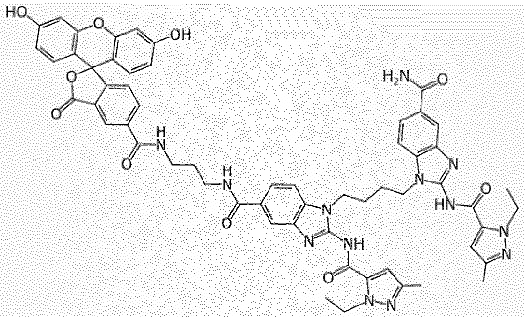

### Synthesis:

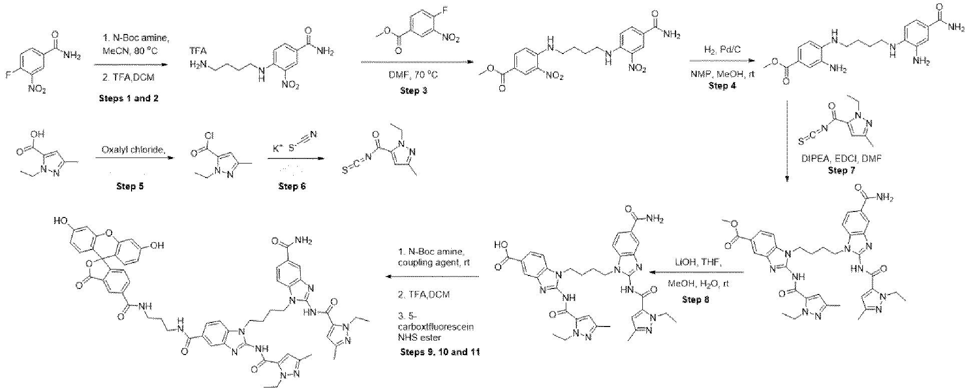

**Step 1:** To a suspension of 4-fluoro-3-nitrobenzamide (50 mg, 0.272 mmol) and 1-Boc-1,4-diaminobutane (51 mg, 0.272 mmol) in MeCN (1.4 mL) was added DIPEA (0.14 mL, 0.804 mmol). The reaction mixture was stirred at 80 °C for 6 h. The reaction mixture was concentrated under reduced pressure, affording 104 mg of crude *tert*-butyl (4-((4-carbamoyl-2-nitrophenyl)amino)butyl)carbamate (96 mg, 0.272 mmol, 100 %) as a yellow solid.
LCMS (UPLC_pH2_MeCN_2min): Rt = 1.57 min, m/z 353.1 [M+H]⁺

**Step 2:** To a stirred suspension of *tert*-butyl (4-((4-carbamoyl-2-nitrophenyl)amino)butyl)carbamate (96 mg, 0.272 mmol) in DCM (2.2 mL) was added TFA (0.54 mL) dropwise. The reaction mixture was stirred at rt for 4 h (slurry became a solution after TFA addition, but the compound precipitated again). The reaction mixture was concentrated under reduced pressure, affording the product as the TFA salt that was taken to the next step without further purification. Assumed quantitative, furnished 4-((4-aminobutyl)amino)-3-nitrobenzamide TFA salt as a yellow gum (100 mg, 0.272 mmol, 100 %).
LCMS (UPLC_pH9_MeCN_2min): Rt = 1.02 min, m/z 253.1 [M+H]⁺

**Step 3:** To a stirred solution of 4-(4-aminobutylamino)-3-nitro-benzamide;2,2,2-trifluoroacetic acid (796 mg, 2.17 mmol) in DMF (11 mL) were added methyl 4-fluoro-3-nitrobenzoate (433 mg, 2.17 mmol) and DIPEA (1.9 mL, 10.9 mmol). The reaction mixture was stirred at 70 °C for 6 h and then cooled down to rt and poured into ice-cold water. The resulting solid was isolated by filtration and dried under vacuum, affording methyl 4-((4-((4-carbamoyl-2-nitrophenyl)amino)butyl)amino)-3-nitrobenzoate (865 mg, 2.01 mmol, 92 %) as an orange solid.
LCMS (UPLC_pH9_MeCN_2min): Rt = 1.68 min, m/z 432.1 [M+H]⁺
¹H-NMR (400 MHz, DMSO-d₆): δ 8.64-8.57 (m, 3H), 8.40 (t, J = 5.7 Hz, 1H), 7.97 (ddd, J = 14.0, 9.2, 2.1 Hz, 3H), 7.27 (s, 1H), 7.14 (dd, J = 22.4, 9.2 Hz, 2H), 3.83 (s, 3H), 3.49-3.46 (m, 4H), 1.77-1.72 (m, 4H).

**Step 4:** Methyl 4-((4-((4-carbamoyl-2-nitrophenyl)amino)butyl)amino)-3-nitrobenzoate (300 mg, 0.695 mmol) was suspended in 1-methyl-2-pyrrolidinone (36 mL) and MeOH (2.2 mL). The reaction was vac / filled with nitrogen three times. Then, palladium on carbon (74 mg, 0.695 mmol) was added under nitrogen and the reaction mixture was vac / filled with hydrogen three times leaving a balloon of hydrogen. The reaction mixture was stirred at rt overnight. The reaction mixture was filtered through celite, eluting with MeOH. The filtrate was concentrated under reduced pressure. To the residue was added diethylether and the resulting solid was isolated by filtration and dried under vacuum, furnishing methyl 3-amino-4-((4-((2-amino-4-carbamoylphenyl)amino)butyl)amino)benzoate (224 mg, 0.603 mmol, 87 %) as a dark grey solid.
LCMS (UPLC_pH9_MeCN_2min): Rt = 1.37 min, m/z 372.2 [M+H]⁺

**Step 7:** To a stirred solution of methyl 3-amino-4-((4-((2-amino-4-carbamoylphenyl)amino)butyl)amino)benzoate (50 mg, 0.135 mmol) in DMF (0.67 mL) at 0 °C was added 1-ethyl-3-methyl-1H-pyrazole-5-carbonyl isothiocyanate (Intermediate 3; 53 mg, 0.269 mmol) dropwise. The reaction mixture was stirred at 0 °C for 20 min, and an LCMS sample was taken to confirm the formation of the thiourea intermediate. To the reaction mixture at 0 °C were then EDC (77 mg, 0.404 mmol) and DIPEA (0.14 mL, 0.804 mmol) dropwise. The reaction mixture was stirred at rt overnight. The reaction mixture was poured into ice-cold water dropwise. The resulting solid was collected by filtration, affording a gum that was redissolved in DCM/MeOH and concentrated under reduced pressure, affording methyl 1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxylate (30 mg, 0.0432 mmol, 32 %) as a yellow solid.
LCMS (UPLC_pH9_MeCN_2min): Rt = 1.60 min, m/z 694.3 [M+H]⁺

**Step 8:** To a stirred solution of methyl 1-(4-(5-carbamoyl-2-(1-ethyl-3-methyl-1H-pyrazole-5-carboxamido)-1H-benzo[d]imidazol-1-yl)butyl)-2-(1 -ethyl-3-methyl-1H-pyrazole-5-carboxamido)-1H-benzo[d]imidazole-5-carboxylate (516 mg, 0.744 mmol) in MeOH (3.0 mL) and THF (3.0 mL) was added 1M aq LiOH (1.5 mL, 7.44 mmol) dropwise. The reaction mixture was stirred at rt for 48 h. THF and MeOH were removed under reduced pressure and the residue was acidified to pH 4 with aq 1M HCl. The resulting solid was collected by filtration, affording 1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxylic acid (500 mg, 0.664 mmol, 89 %) as a grey solid.
LCMS (UPLC_pH9_MeCN_2min): Rt = 1.25 min, m/z 680.3 [M+H]⁺
¹H-NMR (400 MHz, DMSO-d₆): δ δ 8.07 (s, 1H), 7.97 (s, 1H), 7.94 (s, 1H), 7.82 (d, J = 6.9 Hz, 1H), 7.76 (d, J = 9.6 Hz, 1H), 7.57-7.53 (m, 2H), 7.30 (s, 1H), 6.59 (s, 1H), 6.58 (s, 1H), 4.56 (q, J = 6.9 Hz, 4H), 4.31-4.23 (s, 4H), 2.09 (s, 6H), 1.90-1.82 (s, 4H), 1.30 (t, J = 7.1 Hz, 6H).

**Step 9:** To a stirred solution of 1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxylic acid (50 mg, 0.0736 mmol) and N-Boc-1,3-diaminopropane (15 mg, 0.0883 mmol) in DMSO (2.0 mL) was added DIPEA (0.077 mL, 0.442 mmol). The reaction mixture was stirred for 10 min and then HATU (34 mg, 0.0883 mmol) was added. The reaction mixture was diluted with DMSO and purified by preparative HPLC, affording tert-butyl (3-(1-(4-(5-carbamoyl-2-(1-ethyl-3-methyl-1H-pyrazole-5-carboxamido)-1H-benzo[d]imidazol-1-yl)butyl)-2-(1 -ethyl-3-methyl-1H-pyrazole-5-carboxamido)-1H-benzo[d]imidazole-5-carboxamido)propyl)carbamate (36 mg, 0.0388 mmol, 53 %) as a white solid.
LCMS (UPLC_pH9_MeCN_2min): Rt = 1.62 min, m/z 836.4 [M+H]⁺

**Step 10:** To a stirred solution/suspension of tert-butyl (3-(1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamido)propyl)carbamate (36 mg, 0.0388 mmol) in DCM (7.1 mL) at rt was added TFA (0.71 mL) dropwise. The reaction mixture was stirred at rt for 1 h. The solvent was removed under reduced pressure, affording a crude containing N-(3-aminopropyl)-1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamide TFA salt (42 mg, 0.0388 mmol, assumed 100 %) as a light yellow gum.
UPLC-MS, pH9 MeCN, 2.6 min method:
LCMS (UPLC_pH9_MeCN_2min): Rt = 1.47 min, m/z 736.3 [M+H]⁺

**Step 11:** To N-(3-aminopropyl)-1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamide TFA salt (42 mg, 0.0388 mmol) in DMF (0.40 mL) was added triethylamine (0.032 mL, 0.233 mmol). Then, 5-carboxyfluorescein-N-hydroxysuccinimide ester (18 mg, 0.0388 mmol) in DMF (0.40 mL) was added dropwise, and the reaction mixture was stirred at rt for 1 h. The reaction mixture was purified by preparative HPLC, affording N-{1-[4-(5-{N-3-(3',6'-dihydroxy-3-oxospiro[isobenzofuran-1,9'-xanthen]-5-ylcarbonylamino)propylcarbamoyl}-2-(1-ethyl-3-methyl-5-pyrazolylcarbonylamino)-1H-1,3-benzimidazol-1-yl)butyl]-5-carbamoyl-1H-1,3-benzimidazol-2-yl}-1-ethyl-3-methyl-5-pyrazolecarboxamide (Probe 1; 37 mg, 0.0334 mmol, 86 %) as an orange solid.
LCMS (UPLC_pH2_QC_V1): Rt = 4.20 min, m/z 1094.2 [M+H]⁺
¹H-NMR (400 MHz, DMSO-d₆): δ 8.85 (t, J = 5.6 Hz, 1H), 8.47-8.51 (m, 2H), 8.18 (d, J = 8.0 Hz, 1H), 7.95-7.96 (s with shoulder, 3H), 7.73 (t, J = 8.8 Hz, 2H), 7.53 (t, J = 9.2 Hz, 2H), 7.34 (d, J = 8.0 Hz with shoulder, 2H), 6.58-6.63 (m, 6H), 6.50 (d, J = 8.8 Hz, 2H), 4.56 (q, J = 7.0 Hz, 4H), 4.26 (br s, 4H), 3.25-3.45 (m, overlapping with H₂O peak at 3.30 ppm), 2.09 (s, 6H), 1.80-1.91 (m, 6H), 1.30 (t, J = 7.1 Hz, 6H).

### Building Block G-1

### Ethyl {3-[5-carbamoyl-2-(1-ethyl-3-methyl-5-pyrazolylcarbonylamino)-1H-1 ,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate

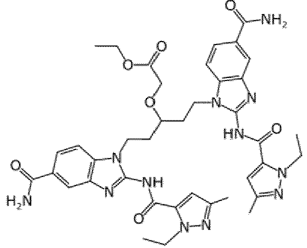

### Synthesis:

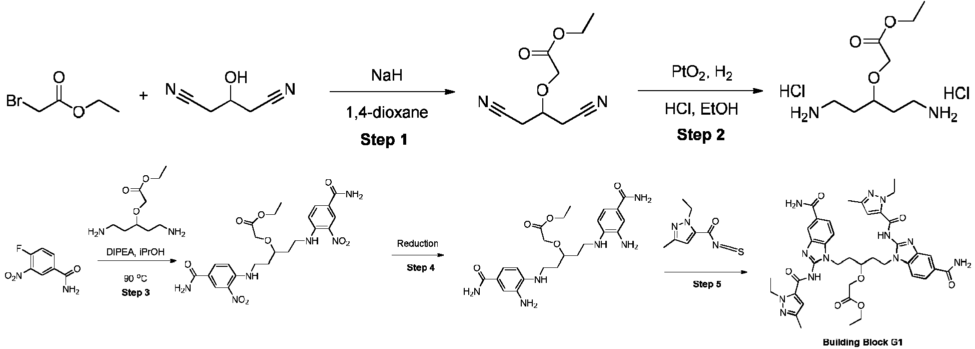

**Step** 1: To a stirred solution of 3-hydroxypentanedinitrile (5.00 g, 45.4 mmol) in dry 1 ,4-dioxane (200 mL) at 5 °C was added sodium hydride (60% dispersion in oil) (1816 mg, 45.4 mmol) portion-wise. The reaction mixture was stirred at rt for 1 h. Then, the reaction mixture was cooled down to 5 °C and ethyl bromoacetate (5.1 mL, 45.4 mmol) was added dropwise. The reaction mixture was stirred at rt overnight. The reaction mixture was cooled down to 5 °C and EtOH was added to quench any residual NaH. The reaction mixture was concentrated under reduced pressure and purified by column chromatography (SiO₂, Biotage, sfar, 350 g), eluting from 100% isohexane to 100% EtOAc, affording ethyl [2-cyano-1-(cyanomethyl)ethoxy]acetate (2.65 g, 13.5 mmol, 30%) as a light yellow oil.
¹H-NMR (400 MHz, CDCl₃): δ 4.33 (s, 2H), 4.26 (q, J = 7.2 Hz, 2H), 4.06-4.12 (m, 1H), 2.89 (s, 4H), 1.33 (t, J = 7.3 Hz, 3H).

**Step 2:** To a stirred solution of ethyl [2-cyano-1-(cyanomethyl)ethoxy]acetate (474 mg, 2.42 mmol) in ethanol (24 mL) was added hydrochloric acid (5 M solution in water, 1.4 mL, 7.0 mmol). The reaction mixture was vac / filled with nitrogen three times. Then, platinum (IV) oxide, Adam's catalyst (110 mg, 0.483 mmol) was added under N₂ and the reaction mixture was vac / filled with hydrogen three times. The reaction mixture was stirred at rt under 1 atm of hydrogen. Consumption of starting material was monitored by TLC. After reaction overnight, TLC showed complete consumption of starting material (EtOAc/isohexane 1:1) and the reaction mixture was filtered through a pad of celite eluting with EtOH and DCM. The filtrate was concentrated under reduced pressure, affording ethyl [3-amino-1-(2-aminoethyl)propoxy]acetate dihydrochloride (670 mg, 2.42 mmol, 100%) as a light yellow gum.
¹H-NMR (400 MHz, DMSO-d₆): δ 8.06 (4H, br s), 4.11-4.17 (m, 4H), 3.64-3.69 (m, 1H), 2.87 (t, J = 7.1 Hz, 4H), 1.81-1.73 (m, 4H), 1.22 (t, J = 7.1 Hz, 3H)

**Step 3:** To a stirred solution of 4-fluoro-3-nitrobenzamide (891 mg, 4.84 mmol) and ethyl [3-amino-1-(2-aminoethyl)propoxy]acetate dihydrochloride (670 mg, 2.42 mmol) in isopropanol (12 mL) was added DIPEA (4.2 mL, 24.1 mmol). The reaction mixture was stirred at 90 °C. After 1 h, the reaction mixture turned orange and a solid precipitate was observed. The reaction mixture was cooled down to 0 °C, diethylether was added and the solid was isolated by filtration, affording ethyl {3-(4-carbamoyl-2-nitrophenylamino)-1-[2-(4-carbamoyl-2-nitrophenylamino)ethyl]propoxy}acetate (626 mg, 1.18 mmol, 49%) as an orange solid.
LCMS (UPLC_pH9_MeCN_2min): Rt = 1.46 min, m/z 533.1 [M+H]⁺

**Step 4:** A stirred solution of ethyl {3-(4-carbamoyl-2-nitrophenylamino)-1-[2-(4-carbamoyl-2-nitrophenylamino)ethyl]propoxy}acetate (3.10 g, 5.82 mmol) in DMF (531 mL) was treated with 35% aq ammonia (9.7 mL) and then a solution of sodium dithionite (10.14 g, 58.2 mmol) in water (317 mL) was added dropwise at 0 °C. The reaction mixture was stirred at rt until consumption of the starting material was observed by LCMS. The resulting white precipitate was removed by filtration and discarded. The filtrate was concentrated under reduced pressure. MeOH and diethylether were added, the solid formed was collected by filtration and triturated further with DCM to give ethyl {3-(2-amino-4-carbamoylphenylamino)-1-[2-(2-amino-4-carbamoylphenylamino)ethyl]propoxy}acetate (2.75 g, 5.82 mmol, 99%) as a light brown solid.
LCMS (UPLC_pH9_MeCN_2min): Rt = 0.97-1.22 min, m/z 473.2 [M+H]⁺

**Step 5:** To a stirred solution of ethyl {3-(2-amino-4-carbamoylphenylamino)-1-[2-(2-amino-4-carbamoylphenylamino)ethyl]propoxy}acetate (2.75 g, 5.82 mmol) in DMF (29 mL) was added 1-ethyl-3-methyl-1H-pyrazole-5-carbonyl isothiocyanate (Intermediate 3; 2.5 g, 12.8 mmol) at 0 °C. The mixture was stirred for 30 min at 0 °C and formation of the thiourea intermediate was confirmed by LCMS. EDC (3.35 g, 17.46 mmol) and DIPEA (6.08 mL, 34.91 mmol) were added and the mixture was stirred at rt overnight. The resulting mixture was dropped into half saturated aqueous NH₄Cl solution and stirred for 1 h. The resulting precipitate was collected by filtration, washed with water and dried, affording ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate (Building Block G-1; 2.60 g, 3.27 mmol, 56%) as an off white solid.
LCMS (UPLC_pH2_MeCN_2min): Rt = 1.48 min, m/z 795.4 [M+H]⁺
¹H-NMR (400 MHz, DMSO-d₆): δ 8.00 (d, *J* = 1.6 Hz, 2H), 7.95 (s, 2H), 7.78 (dd, *J* = 8.5, 1.6 Hz, 2H), 7.52 (d, *J* = 8.2 Hz, 2H), 7.31 (s, 2H), 6.60 (s, 2H), 4.58 (q, *J* = 7.2 Hz, 4H), 4.25-4.40 (m, 4H), 4.17 (s, 2H), 4.10 (q, *J* = 7.2 Hz, 2H), 3.68-3.74 (m, 1H), 2.08-2.13 (m, 4H), 2.07 (s, 6H), 1.31 (t, *J* = 7.1 Hz, 6H), 1.16 (t, *J* = 7.1 Hz, 3H)

### Building Block G-2

### Ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate

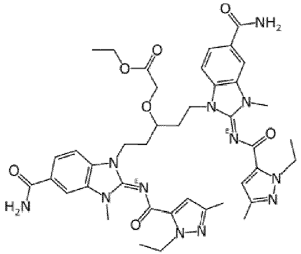

To a stirred solution of ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate (Building Block G-1; 700 mg, 0.881 mmol) in DMF (4.2 mL) were added cesium carbonate (1435 mg, 4.40 mmol) and iodomethane (0.14 mL, 2.20 mmol). The mixture was stirred overnight. The solid was filtered off and the filtrate was purified by preparative HPLC (column: Waters Xbridge C18 250 mm x 19 mm, 5 µm; method: increasing gradient of 20% to 60% MeCN in water containing 0.1% formic acid at pH 2), affording ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate (Building Block G-2; 307 mg, 42%), as an off white solid.
LCMS (UPLC_pH2_MeCN_2min): Rt = 1.40 min, m/z 823.4 [M+H]⁺
¹H-NMR (400 MHz, DMSO-d₆): δ 8.06 (d, *J* = 1.6 Hz, 2H), 8.02 (s, 2H), 7.84 (dd, *J* = 8.5, 1.4 Hz, 2H), 7.55 (d, *J* = 8.2 Hz, 2H), 7.42 (s, 2H), 6.47 (s, 2H), 4.49 (q, *J* = 7.2 Hz, 4H), 4.14-4.28 (m, 4H), 3.99-4.05 (m, 4H), 3.51-3.60 (m, 7H), 2.10 (s, 6H), 1.95-1.99 (m, 4H), 1.27 (t, *J=* 7.1 Hz, 6H), 1.13 (t, *J* = 7.1 Hz, 3H)

### Building Block G-3

### {3-[5-Carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetic acid

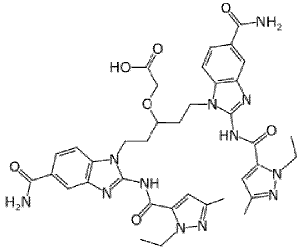

To a stirred solution of ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate (Building Block G-1; 200 mg, 0.252 mmol) in EtOH (0.63 mL) and THF (0.63 mL) was added was added 1M aqueous lithium hydroxide (1.26 mL, 1.26 mmol) dropwise. The mixture was stirred overnight at rt. pH was adjusted to 4-6 by careful addition of 1M aq HCl and the reaction mixture was concentrated under reduced pressure. One third of the mixture was purified by preparative HPLC (column: SunFire C18, 250 mm × 19 mm, 5 µm; method: 10-50% MeCN in water containing 0.1% formic acid at pH 2). Fractions containing the desired product were combined and freeze-dried, affording {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetic acid (Building Block G-3; 25 mg, 0.0326 mmol) as an off-white solid.
LCMS (UPLC_pH2_MeCN_QC_V2): Rt = 3.47 min, m/z+ 767.4 [M+H]⁺
¹H-NMR (400 MHz, DMSO-d₆): δ 7.99 (d, *J* = 1.6 Hz, 2H), 7.95 (s, 2H), 7.77 (dd, *J* = 8.2, 1.4 Hz, 2H), 7.54 (d, *J* = 8.2 Hz, 2H), 7.31 (s, 2H), 6.60 (s, 2H), 4.58 (q, *J* = 7.2 Hz, 4H), 4.25-4.41 (m, 4H), 4.10 (s, 2H), 3.69-3.75 (m, 1H), 2.07-2.11 (m, 4H), 2.07 (s, 3H), 1.31 (t, *J* = 7.1 Hz, 6H)

### Building Block G-3L

### 14-{14-[({3-[5-Carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbonylamino]-3,6,9,12-tetraoxatetradecylcarbonylamino}-3,6,9,12-tetraoxatetradecanoic acid

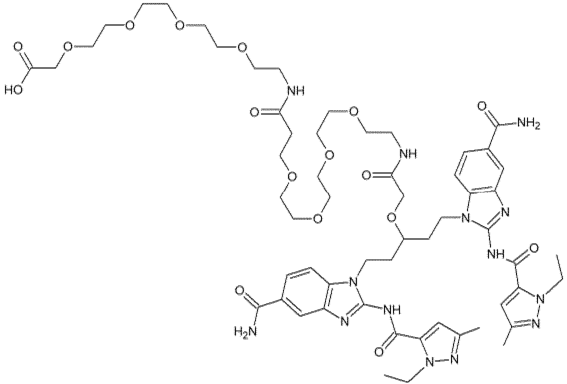

Building Block G-3L may be prepared from Building Block G-3 in a similar manner as described below for the synthesis of Building Block G-4L from Building Block G-4. In brief, amide coupling of Building Block G-3 with tert-butyl 15-amino-4,7,10,13-tetraoxapentadecanoate followed by reaction with excess TFA affords Building Block G-3L.

### Building Block G-4

### (3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)acetic acid

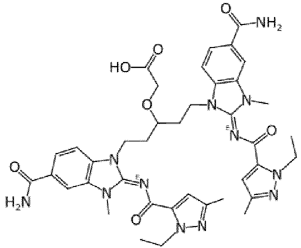

To a stirred solution of ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate (Building Block G-2; 35 mg, 0.0425 mmol) in EtOH (0.21 mL) and THF (0.21 mL) was added 1M aqueous lithium hydroxide (0.213 mL, 0.213 mmol) dropwise. The mixture was stirred overnight at rt. pH was adjusted to 4-6 by careful addition of 1M aq HCl and the reaction mixture was concentrated under reduced pressure, affording (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetic acid (Building Block G-4; 33.8 mg) as an off-white gum.
LCMS (UPLC_pH2_MeCN_2min): Rt = 1.29 min, m/z 795.3 [M+H]⁺
Batch purified by preparative HPLC (column: SunFire C18, 250 mm × 19 mm, 5 µm; method: 10-50% MeCN in water containing 0.1% formic acid at pH 2), affording an off-white solid.
¹H-NMR (400 MHz, DMSO-d₆): δ 8.05 (s, 2H), 8.02 (s, 2H), 7.84 (d, *J* = 8.4 Hz, 2H), 7.58 (d, *J* = 8.4 Hz, 2H), 7.41 (s, 2H), 6.48 (s, 2H), 4.49 (q, J = 6.9 Hz, 4H), 4.15-4.30 (m, 4H), 3.92 (s, 2H), 3.55-3.59 (m, 1H), 3.55 (s, 6H), 2.09 (s, 6H), 1.92-1.99 (m, 4H), 1.26 (t, *J* = 7.2 Hz, 6H)

### Building Block G-4L

### 15-{[(3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanoic acid

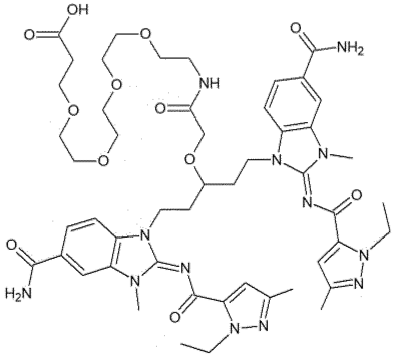

**Step 1:** To a stirred solution of Building Block G-4 (300 mg, 0.377 mmol) and tert-butyl 15-amino-4,7,10,13-tetraoxapentadecanoate (121 mg, 0.377 mmol) in DMSO were added DIPEA (0.33 mL, 1.89 mmol) and HATU (144 mg, 0.377 mmol). The reaction mixture was stirred for 1 h and then purified by preparative HPLC (column: Waters Xbridge C18 250 mm x 19 mm, 5 µm; method: increasing gradient of 30% to 70% MeCN in water containing 10 mM ammonium bicarbonate at pH 9). Fractions containing the desired intermediate product were combined and concentrated under reduced pressure, affording the expected tert-butyl ester of Building Block G-4L (266 mg, 0.242 mmol, 64 %) as a colourless gum.
LCMS (UPLC_pH9_MeCN_2min): Rt = 1.55 min, m/z 550.0 [(M+2H)/2]⁺

**Step 2:** To a stirred solution of the product of Step 1 (266 mg, 0.242 mmol) in DCM (13 mL) was added TFA (2.5 mL) dropwise. The reaction mixture was stirred overnight at rt. The reaction mixture was concentrated under reduced pressure and the residue was freeze-dried, affording 15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanoic acid (Building Block G-4L; 252 mg, 0.242 mmol, 100 %) as a colourless gum.
LCMS (UPLC_pH9_MeCN_2min): Rt = 1.18 min, m/z 522.1 [(M+2H)/2]⁺

### Intermediate 4

### N-[15-Oxo-15-(1-piperazinyl)-3,6,9,12-tetraoxapentadecyl](3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide

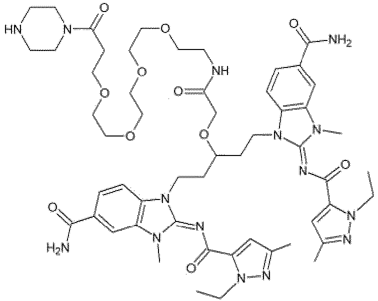

### Synthesis:

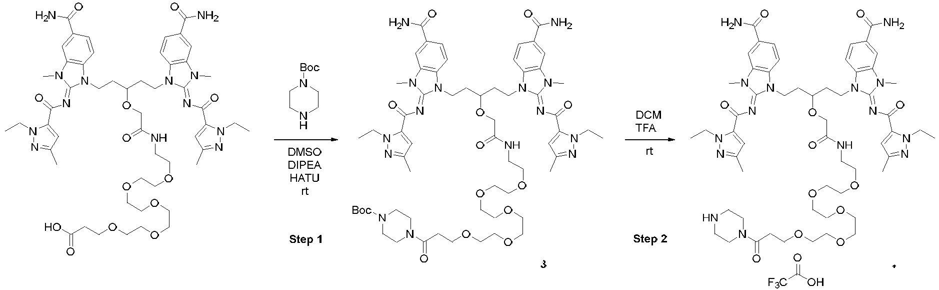

**Step** 1: To a solution of Building Block G-4L (283 mg, 0.272 mmol) and 1-Boc-piperazine (51 mg, 0.274 mmol) in anhydrous DMSO (2.2 mL) was added DIPEA (0.24 mL, 1.38 mmol) followed by HATU (116 mg, 0.305 mmol). The reaction was stirred for 16 h at rt and then diluted and purified by preparative HPLC (column: SunFire C18, 250 mm x 19 mm, 5 µm; method: 30-70% MeCN in water containing 0.1% formic acid at pH 2). Fractions containing the desired product were combined and concentrated under reduced pressure, affording the expected Boc-protected intermediate (254 mg, 77 %) as an off-white solid.
LCMS (UPLC_pH2_MeCN_2min): Rt = 1.45 min, m/z 606.1 [(M+2H)/2]⁺

**Step 2:** The product obtained from Step 1 (254 mg, 0.210 mmol) was treated with DCM (2.3 mL) and TFA (1.2 mL). The resulting solution was stirred for 30 min and then concentrated under reduced pressure, affording the TFA salt of Intermediate 4 (266 mg, assumed 100 %) as a clear colourless gum.
LCMS (UPLC_pH2_MeCN_2min): Rt = 1.18 min, m/z 556.0 [(M+2H)/2]⁺

### Intermediate 5

### 4-[4-(15-{[(3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanoyl)-1-piperazinyl]-4-oxobutyric acid

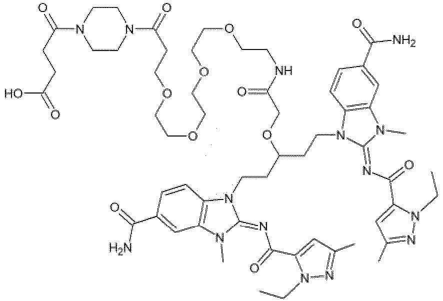

To a stirred solution of N-[15-oxo-15-(1-piperazinyl)-3,6,9,12-tetraoxapentadecyl](3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide TFA salt (Intermediate 4; 86 mg, 0.0702 mmol) in dry DCM (1.4 mL) were added triethylamine (0.059 mL, 0.422 mmol)) and succinic anhydride (7.0 mg, 0.070 mmol). The reaction mixture was left to stir at rt for 72 h. Succinic anhydride (7.0 mg, 0.070 mmol) was then added and stirring was continued for 1 h. The reaction mixture was concentrated under reduced pressure, dissolved in DMSO making up to a 25 mg/mL concentration and purified by preparative HPLC (column: SunFire C18, 250 mm x 19 mm, 5 µm; method: 10-50% MeCN in water containing 0.1% formic acid at pH 2). Fractions containing the desired product were combined and concentrated under reduced pressure, affording Intermediate 5 (77 mg, 91 %) as a colourless solid.
LCMS (UPLC_pH2_MeCN_2min): Rt = 1.26 min, m/z 606.0 [(M+2H)/2]⁺

### Intermediate 6

### 3-{3-[4-(15-{[(3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanoyl)-1-piperazinyl]-3-oxopropoxy}propionic acid

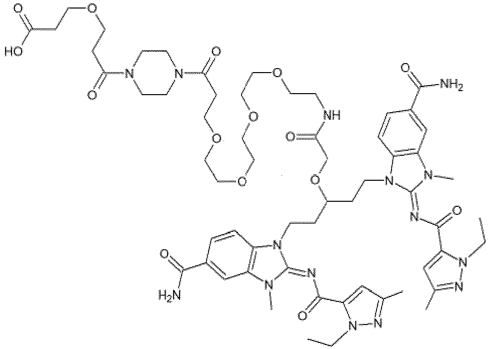

**Step 1:** To a stirred solution of N-[15-oxo-15-(1-piperazinyl)-3,6,9,12-tetraoxapentadecyl](3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide TFA salt (Intermediate 4; 114 mg, 0.093 mmol) and 3-(3-(tert-butoxy)-3-oxopropoxy)propanoic acid (CAS-no. 2086689-08-7; 20 mg, 0.092 mmol) in dry DMSO (0.74 mL) were added DIPEA (0.097 mL, 0.557 mmol) and HATU (39 mg, 0.103 mmol). The reaction mixture was stirred at rt for 1 h. Additional portions of DIPEA (0.291 ml, 1.67 mmol), 3-(3-(tert-butoxy)-3-oxopropoxy)propanoic acid (20 mg, 0.092 mmol) and HATU (20 mg, 0.053 mmol) were added and stirring was continued for 17 h. The reaction mixture was diluted with DMSO to a concentration of about 25 mg/mL and purified by preparative HPLC (column: SunFire C18, 250 mm x 19 mm, 5 µm; method: 20-60% MeCN in water containing 0.1% formic acid at pH 2). Fractions containing the desired product were combined and concentrated under reduced pressure, affording the expected tert-butyl-protected intermediate (74 mg, 60 %) as a clear colourless solid film.
LCMS (UPLC_pH2_MeCN_2min): Rt = 1.44 min, m/z 656.1 [(M+2H)/2]⁺

**Step 2:** The product obtained from Step 1 (74 mg, 0.056 mmol) was treated with DCM (1.0 mL) and TFA (0.5 mL). The resulting solution was stirred for 1 h and then concentrated under reduced pressure, affording Intermediate 6 (71 mg, assumed 100 %) as a clear colourless oil.
LCMS (UPLC_pH2_MeCN_2min): Rt = 1.29 min, m/z 628.1 [(M+2H)/2]⁺

### Intermediate 7

### 15-(N-Methyl{14-[({3-[(E)-5-carbamoyl-2-[(1-ethyl-3-methyl-5-pyrazolyl)carbonylimino]-3-methyl-2,3-dihydro-1H-1 ,3-benzimidazol-1-yl]-1-{2-[(E)-5-carbamoyl-2-[(1-ethyl-3-methyl-5-pyrazolyl)carbonylimino]-3-methyl-2,3-dihydro-1H-1,3-benzimidazol-1-yl]ethyl}propoxy}methyl)carbonylamino]-3,6,9,12-tetraoxatetradecyl}carbonylamino)-4,7,10,13-tetraoxapentadecanoic acid

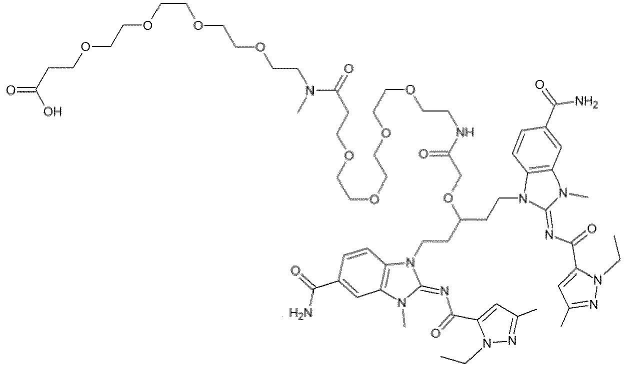

**Step 1:** To a stirred solution of Building Block G-4L (60 mg, 0.058 mmol) and tert-butyl 5,8,11,14-tetraoxa-2-azaheptadecan-17-oate (CAS-no. 1621616-14-5; 19 mg, 0.057 mmol) in dry DMSO (0.58 mL) was added DIPEA (0.060 mL, 0.344 mmol). After 10 min, HATU (24 mg, 0.063 mmol) was added. The reaction mixture was stirred at rt for 1 h. The reaction mixture was purified by preparative HPLC (column: SunFire C18, 250 mm x 19 mm, 5 µm; method: 30-70% MeCN in water containing 0.1% formic acid at pH 2). Fractions containing the desired product were combined and concentrated under reduced pressure, affording the expected tert-butyl-protected intermediate (57 mg, 0.042 mmol, 74 %) as a clear colourless gum.
LCMS (UPLC_pH2_MeCN_2min): Rt = 1.58 min, m/z 680.6 [(M+2H)/2]⁺
LCMS (UPLC_pH9_MeCN_2min): Rt = 1.54 min, m/z 680.7 [(M+2H)/2]⁺

**Step 2:** The product obtained from Step 1 (57 mg, 0.042 mmol) was dissolved in DCM (2.2 mL), and TFA (0.44 mL) was added. The resulting solution was stirred at rt overnight and then concentrated under reduced pressure, affording Intermediate 7 (55 mg, 100 %) as a colourless gum.
LCMS (UPLC_pH2_MeCN_2min): Rt = 1.36 min, m/z 652.7 [(M+2H)/2]⁺
LCMS (UPLC_pH9_MeCN_2min): Rt = 1.23 min, m/z 652.7 [(M+2H)/2]⁺

### Bifunctional degrader/PROTAC^{®} compounds

### Example 1

### 1-[3-({N-2-[2-(2-{2-[2-(2,6-Dioxo-3-piperidyl)-1,3-dioxo-4-isoi ndoli nylami no] eth oxy}ethoxy)ethoxy] ethylcarbamoyl}methoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1 ,3-benzimidazole-5-carboxamide

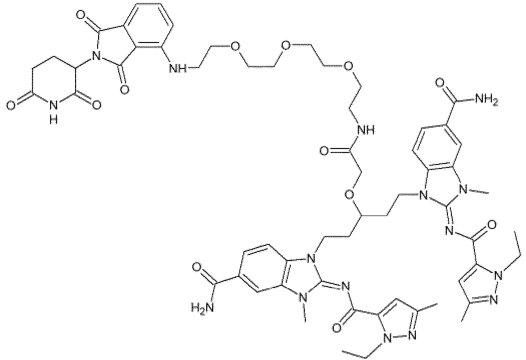

### Synthesis:

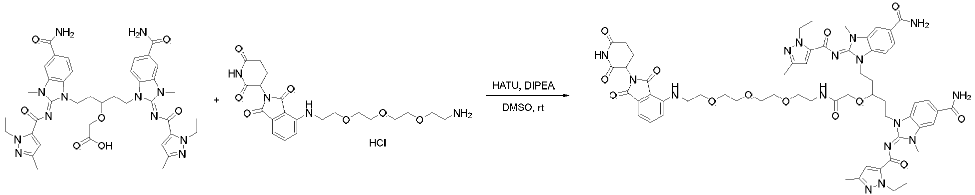

Building Block G-4 (30 mg, 0.0377 mmol) and 3-[4-(2-{2-[2-(2-aminoethoxy)ethoxy]ethoxy}ethylamino)-1 ,3-dioxo-2-isoindolinyl]-2,6-piperidinedione hydrochloride (18 mg, 0.0371 mmol) were dissolved in dry DMSO (0.38 mL). DIPEA (0.033 mL, 0.189 mmol) was added followed by HATU (17 mg, 0.0453 mmol). The reaction mixture was stirred at rt overnight. The reaction mixture was purified directly by preparative HPLC (column: Waters Xbridge C18 250 mm x 19 mm, 5 µm; method: 20-60 % MeCN in water containing 10 mM ammonium bicarbonate at pH 9). Fractions containing the desired product were concentrated giving a yellow film. The material was freeze-dried overnight, affording 1-[3-({N-2-[2-(2-{2-[2-(2,6-dioxo-3-piperidyl)-1,3-dioxo-4-isoindolinylamino]ethoxy}ethoxy)ethoxy]ethylcarbamoyl}methoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide (22 mg, 48 %) as a yellow solid.
LCMS (UPLC_pH2_MeCN_QC_V2): Rt = 3.74 min, m/z 1225.7 [M+H)]⁺
LCMS (UPLC_pH9_MeCN_QC_V2): Rt = 3.78 min, m/z 1225.7 [M+H)]⁺

### Example 2

### N-{1-[3-({N-2-[2-(2-{2-[2-(2,6-Dioxo-3-pi peridyl)-1, 3-di oxo-4-isoi ndoli nylami no] eth oxy}ethoxy)ethoxy] ethylcarbamoyl}methoxy)-5-[5-carbamoyl-2-(1-ethyl-3-methyl-5-pyrazolylcarbonylamino)-1H-1,3-benzimidazol-1-yl]pentyl]-5-carbamoyl-1H-1,3-benzimidazol-2-yl}-1-ethyl-3-methyl-5-pyrazolecarboxamide

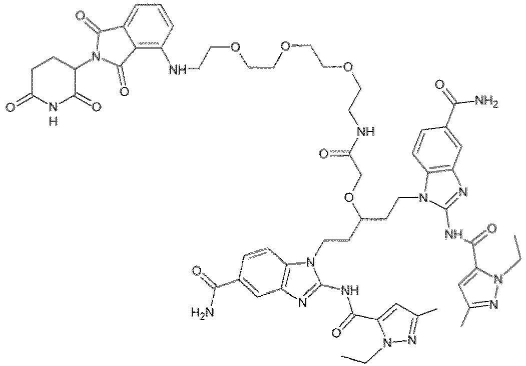

Prepared similarly to Example 1 from Building Block G-3.
LCMS (UPLC_pH2_MeCN_QC_V2): Rt = 3.85 min, m/z 1197.7 [M+H)]⁺

### Example 3

### 1-(3-{[N-2-(2-{2-[2-(2-{2-[2-(2,6-Dioxo-3-piperidyl)-1,3-dioxo-4-isoindolinylamino]ethoxy}ethoxy)ethoxy]ethoxy}ethoxy)ethylcarbamoyl]methoxy}-5-{5-carbamoyl-2-[(1-ethyl-3-methyl-5-pyrazolyl)carbonylimino]-3-methyl-2,3-dihydro-1H-1 ,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

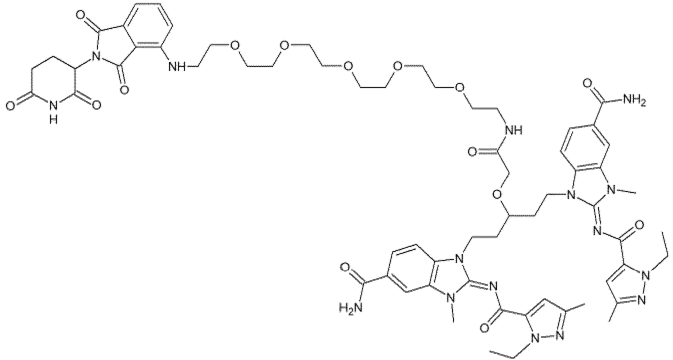

Prepared similarly to Example 1 from Building Block G-4 and the corresponding commercially available amine.
LCMS (UPLC_pH2_MeCN_QC_V3): Rt = 3.76 min, m/z 1313.9 [M+H]⁺

### Example 4

### N-[(S)-1-{[(2S,4R)-4-Hydroxy-2-({[p-(4-methyl-1,3-thiazol-5-yl)phenyl]methyl}carbamoyl)-1-pyrrolidinyl]carbonyl}-2,2-dimethylpropyl]11-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbonylamino]-3,6,9-trioxaundecanamide

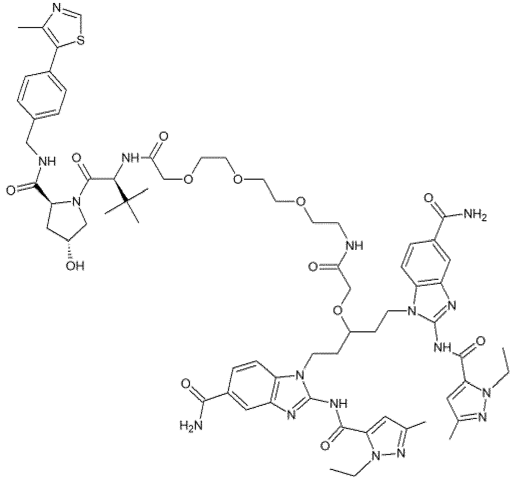

Prepared similarly to Example 1 from Building Block G-3 and the corresponding commercially available amine.
LCMS (UPLC_pH2_MeCN_QC_V2): Rt = 3.91 min, m/z 685.3 [(M+2H)/2]⁺

### Example 5

### N-[(S)-1-{[(2S,4R)-4-Hydroxy-2-({[p-(4-methyl-1 ,3-thiazol-5-yl)phenyl]methyl}carbamoyl)-1-pyrrolidinyl]carbonyl}-2,2-dimethylpropyl]14-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-3,6,9,12-tetraoxatetradecanamide

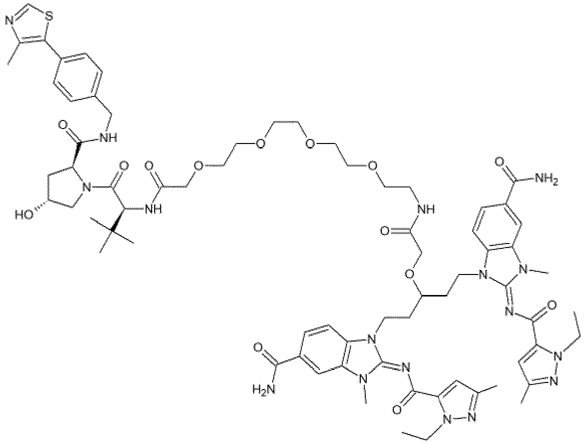

Prepared similarly to Example 1 from Building Block G-4 and the corresponding commercially available amine.
LCMS (UPLC_pH2_MeCN_QC_V2): Rt = 3.81 min, m/z 721.4 [(M+2H)/2]⁺
LCMS (UPLC_pH9_MeCN_QC_V2): Rt = 3.88 min, m/z 721.4 [(M+2H)/2]⁺

### Example 6

### N-(2-{2-[2-(2,6-Dioxo-3-piperidyl)-1,3-dioxo-4-isoindolinylamino]ethoxy}ethyl)15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-4,7,10,13-tetraoxapentadecanamide

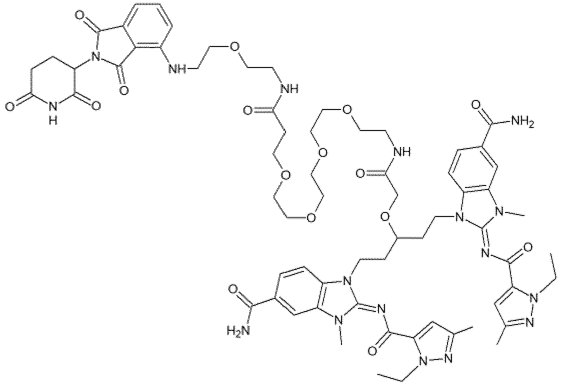

Prepared similarly to Example 1 from Building Block G-4L and the corresponding commercially available amine.
LCMS (UPLC_pH2_MeCN_QC_V2): Rt = 3.61 min, m/z 693.4 [(M+2H)/2]⁺
LCMS (UPLC_pH9_MeCN_QC_V2): Rt = 3.67 min, m/z 693.4 [(M+2H)/2]⁺

### Example 7

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]11-{[(3-{5-carbamoyl-2-[(1-ethyl-3-methyl-5-pyrazolyl)carbonylimino]-3-methyl-2,3-dihydro-1H-1 ,3-benzimidazol-1 -y I}-1 -(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-3,6,9-trioxaundecanamide

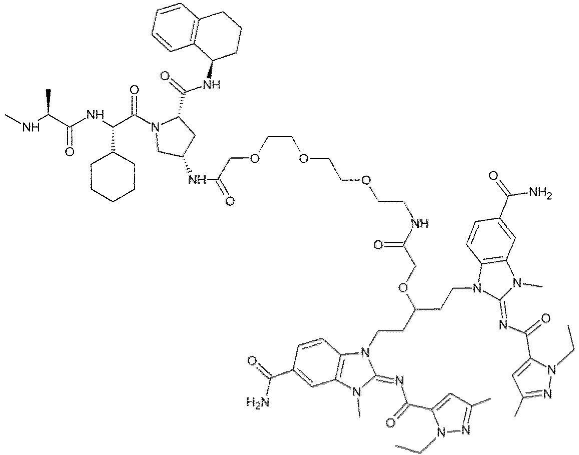

### Synthesis:

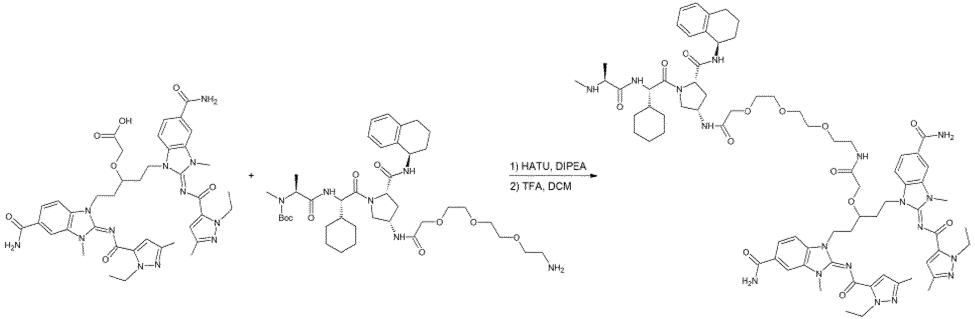

**Step** 1: A solution of crude Building Block G-4 lithium salt (32 mg, assumed 0.034 mmol with purity 85 %) and tert-butyl N-[(1S)-1-{[(1S)-2-[(2S,4S)-4-(2-{2-[2-(2-aminoethoxy)ethoxy]ethoxy}acetamido)-2-{[(1R)-1,2,3,4-tetrahydronaphthalen-1-yl]carbamoyl}pyrrolidin-1-yl]-1-cyclohexyl-2-oxoethyl]carbamoyl}ethyl]-N-methylcarbamate (25 mg, 0.0323 mmol) in DMSO was treated with DIPEA (0.028 mL, 0.1608 mmol) and stirred for 30 min. HATU (15 mg, 0.0394 mmol) was added. The reaction mixture was stirred overnight at rt and then purified by preparative HPLC (column: Waters Xbridge C18 250 mm x 19 mm, 5 µm; method: gradient of 40-75 % MeCN in water containing 10 mM ammonium bicarbonate at pH 9). Relevant clean fractions were combined and concentrated under reduced pressure, affording the expected Boc-protected product (25 mg, 50 %) as a white solid.
LCMS (UPLC_pH9_MeCN_2min): Rt = 1.78 min, m/z 1551.1 [M+H]⁺

**Step 2:** To a stirred solution of the Boc-protected product of Step 1 (25 mg, 0.0161 mmol) in DCM (0.85 mL) was added TFA (0.22 mL). The reaction mixture was stirred at rt for 45 min and then concentrated under reduced pressure, redissolved in MeOH and concentrated again. The resulting residue was dissolved in MeOH and loaded to an SCX cartridge (sorbent bed weight 0.5 g). The cartridge was flushed with MeOH (3 CV) and then the product was eluted with 2M NH₃ in MeOH. The resulting eluate was concentrated under reduced pressure. Subsequent freeze-drying afforded N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]1 1-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-3,6,9-trioxaundecanamide (19.9 mg, 85 %) as an off white solid.
LCMS (UPLC_pH2_MeCN_QC_V3): Rt = 3.36 min, m/z 1450.3 [M+H]⁺
LCMS (UPLC_pH9_MeCN_QC_V3): Rt = 4.08 min, m/z 1450.4 [M+H]⁺

### Example 8

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]14-{[(3-{5-carbamoyl-2-[(1-ethyl-3-methyl-5-pyrazolyl)carbonylimino]-3-methyl-2,3-dihydro-1H-1 ,3-benzimidazol-1 -y I}-1 -(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-3,6,9,12-tetraoxatetradecanamide

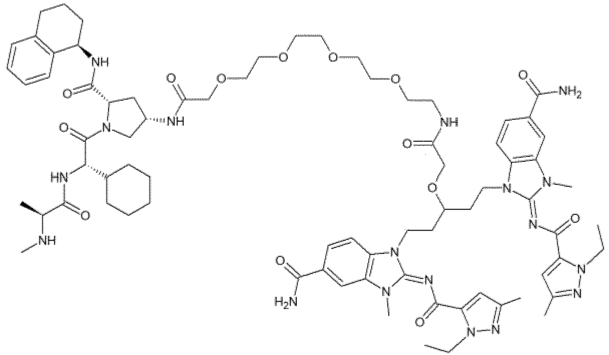

Prepared similarly to Example 7 from Building Block G-4 and the corresponding commercially available amine (CAS-no. 2446474-10-6).
LCMS (UPLC_pH2_MeCN_QC_V3): Rt = 3.39 min, m/z 1494.4 [M+H]⁺
LCMS (UPLC_pH9_MeCN_QC_V3): Rt = 4.12 min, m/z 1494.4 [M+H]⁺

### Example 9

### 1-{3-[(N-2-{2-[2-(2-{2-[m-({2-[(S)-1-{(S)-2-[(S)-2-(Methylamino)propionylamino]-2-cyclohexylacetyl}-2-pyrrolidinyl]-1,3-thiazol-4-yl}carbonyl)phenoxy]ethoxy}ethoxy)ethoxy]ethoxy}ethylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

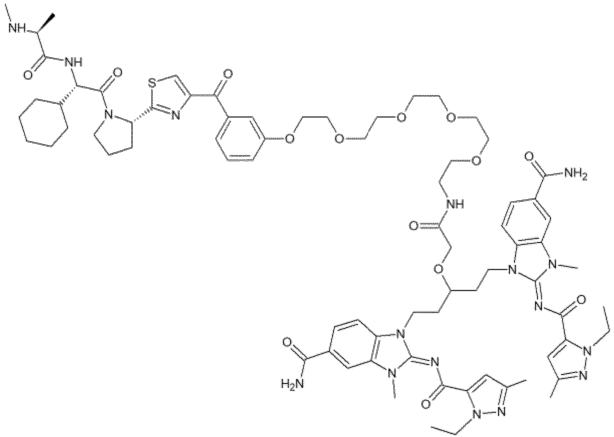

Prepared similarly to Example 7 from Building Block G-4 and the corresponding amine Intermediate L-2.
LCMS (UPLC_pH2_MeCN_QC_V3): Rt = 3.46 min, m/z 748.0 [(M+2H)/2]⁺
LCMS (UPLC_pH9_MeCN_QC_V3): Rt = 4.29 min, m/z 1495.6 [M+H]⁺

### Example 10

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]5-(14-{[(3-{5-carbamoyl-2-[(1-ethyl-3-methyl-5-pyrazolyl)carbonylimino]-3-methyl-2,3-dihydro-1H-1 ,3-benzimidazol-1 -y I}-1 -(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-3,6,9,12-tetraoxatetradecylcarbonylamino)valeramide

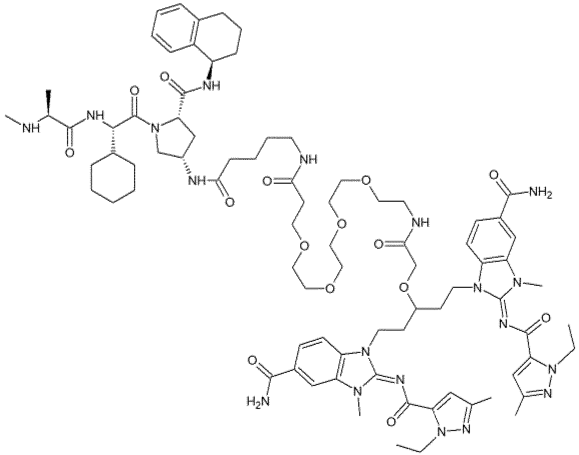

Prepared similarly to Example 7 from Building Block G-4L and the corresponding commercially available amine (CAS-no. 2415256-18-5).
LCMS (UPLC_pH2_MeCN_QC_V2): Rt = 3.33 min, m/z 804.6 [(M+2H)/2]⁺
LCMS (UPLC_pH9_MeCN_QC_V2): Rt = 4.09 min, m/z 804.3 [(M+2H)/2]⁺

### Example 11

### N-{2-[2-(2-{2-[2-(2,6-Dioxo-3-piperidyl)-1,3-dioxo-4-isoindolinylamino]ethoxy}ethoxy)ethoxy]ethyl}15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

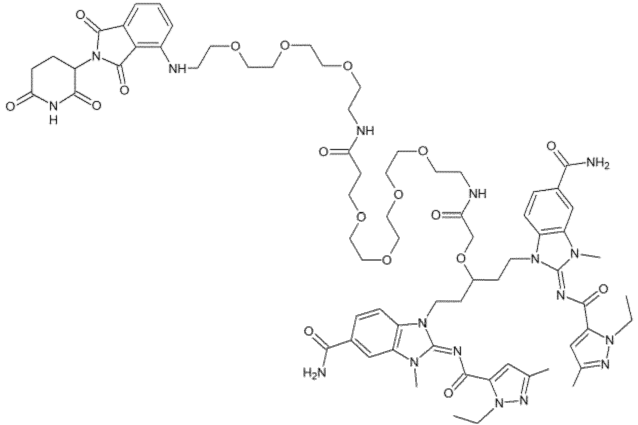

Prepared similarly to Example 1 from Building Block G-4L and the corresponding commercially available amine.
LCMS (UPLC_pH2_MeCN_QC_V3): Rt = 3.69 min, m/z 1473.4 [M+H]⁺
LCMS (UPLC_pH9_MeCN_QC_V3): Rt = 3.73 min, m/z 1473.2 [M+H]⁺

### Example 12

### 1-(3-{[N-2-(2-{2-[2-(2,6-Dioxo-3-piperidyl)-1,3-dioxo-4-isoindolinylamino]ethoxy}ethoxy)ethylcarbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1 ,3-benzimidazole-5-carboxamide

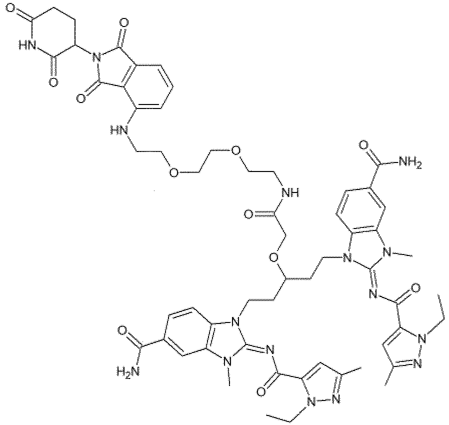

Prepared similarly to Example 1 from Building Block G-4 and the corresponding commercially available amine.
LCMS (UPLC_pH2_MeCN_QC_V3): Rt = 3.64 min, m/z 1181.9 [M+H]⁺
LCMS (UPLC_pH9_MeCN_QC_V3): Rt = 3.67 min, m/z 1181.9 [M+H]⁺

### Example 13

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]17-{[(3-{5-carbamoyl-2-[(1-ethyl-3-methyl-5-pyrazolyl)carbonylimino]-3-methyl-2,3-dihydro-1H-1 ,3-benzimidazol-1 -y I}-1 -(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-3,6,9, 12, 15-pentaoxaheptadecanamide

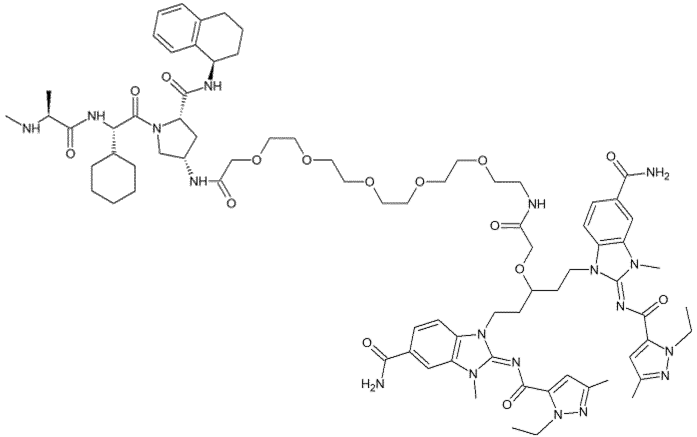

Prepared similarly to Example 7 from Building Block G-4 and the corresponding commercially available amine (CAS-no. 2446474-11-7).
LCMS (UPLC_pH2_MeCN_QC_V3): Rt = 3.41 min, m/z 769.6 [(M+2H)/2]⁺
LCMS (UPLC_pH9_MeCN_QC_V2): Rt = 4.20 min, m/z 770.0 [(M+2H)/2]⁺

### Example 14

### N-[(S)-1-{[(2S,4R)-4-Hydroxy-2-({[p-(4-methyl-1,3-thiazol-5-yl)phenyl]methyl}carbamoyl)-1-pyrrolidinyl]carbonyl}-2,2-dimethylpropyl]14-(14-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-3,6,9,12-tetraoxatetradecylcarbonylamino)-3,6,9,12-tetraoxatetradecanamide

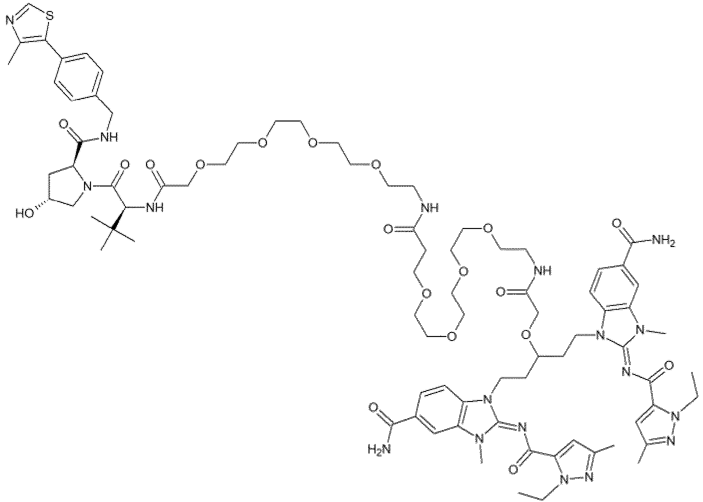

Prepared similarly to Example 1 from Building Block G-4L and the corresponding commercially available amine.
LCMS (UPLC_pH2_MeCN_QC_V2): Rt = 3.79 min, m/z 844.8 [(M+2H)/2]⁺
LCMS (UPLC_pH9_MeCN_QC_V2): Rt = 3.86 min, m/z 844.9 [(M+2H)/2]⁺

### Example 15

### 1-(3-{[N-2-(2-{[N-(S)-1-{[(2S,4R)-4-Hydroxy-2-({[p-(4-methyl-1,3-thiazol-5-yl)phenyl]methyl}carbamoyl)-1-pyrrolidinyl]carbonyl}-2,2-dimethylpropylcarbamoyl]methoxy}ethoxy)ethylcarbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

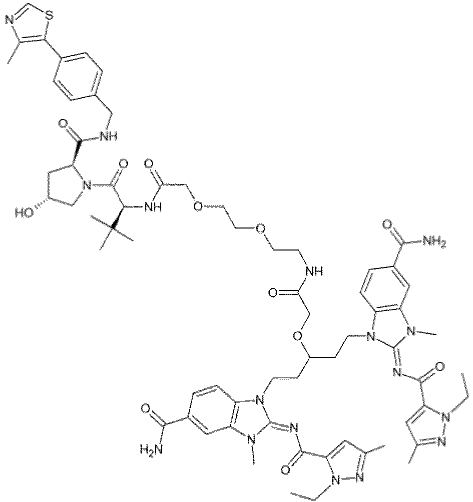

Prepared similarly to Example 1 from Building Block G-4 and the corresponding commercially available amine.
LCMS (UPLC_pH2_MeCN_QC_V3): Rt = 3.69 min, m/z 1353.2 [M+H]⁺
LCMS (UPLC_pH9_MeCN_QC_V2): Rt = 3.74 min, m/z 1353.2 [(M+2H)/2]⁺

### Example 16

### N-(2-{2-[2-(2-{2-[2-(2,6-Dioxo-3-piperidyl)-1,3-dioxo-4-isoindolinylamino]ethoxy}ethoxy)ethoxy]ethoxy}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

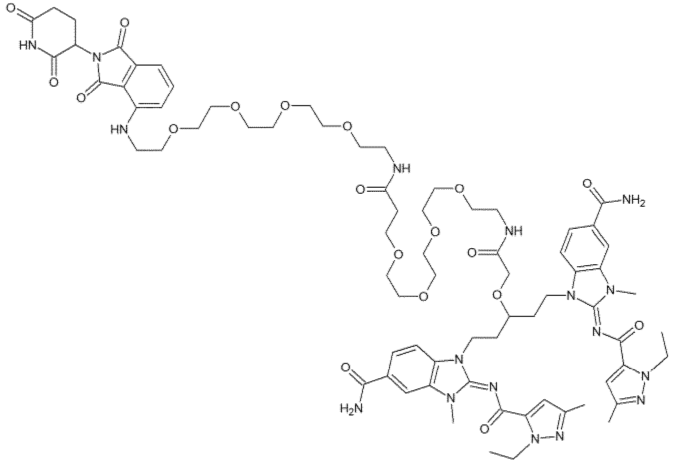

Prepared similarly to Example 1 from Building Block G-4L and the corresponding commercially available amine.
LCMS (UPLC_pH2_MeCN_QC_V2): Rt = 3.78 min, m/z 759.4 [(M+2H)/2]⁺
LCMS (UPLC_pH9_MeCN_QC_V2): Rt = 3.81 min, m/z 759.3 [(M+2H)/2]⁺

### Example 17

### N-(2-{2-[2-(2-{2-[m-({2-[(S)-1-{(S)-2-[(S)-2-(Methylamino)propionylamino]-2-cyclohexylacetyl}-2-pyrrolidinyl]-1,3-thiazol-4-yl}carbonyl)phenoxy]ethoxy}ethoxy)ethoxy]ethoxy}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

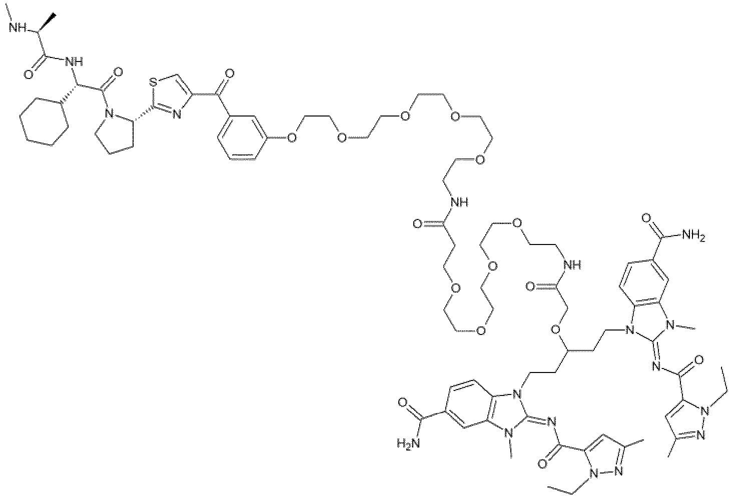

Prepared similarly to Example 7 from Building Block G-4L and the corresponding amine Intermediate L-2.
LCMS (UPLC_pH2_MeCN_QC_V3): Rt = 3.45 min, m/z 871.7 [(M+2H)/2]⁺
LCMS (UPLC_pH9_MeCN_QC_V3): Rt = 3.67 min, m/z 871.7 [(M+2H)/2]⁺

### Example 18

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]11-(14-{[(3-{5-carbamoyl-2-[(1-ethyl-3-methyl-5-pyrazolyl)carbonylimino]-3-methyl-2,3-dihydro-1H-1 ,3-benzimidazol-1 -y I}-1 -(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbonylamino}-3,6,9,12-tetraoxatetradecylcarbonylamino)-3,6,9-trioxaundecanamide

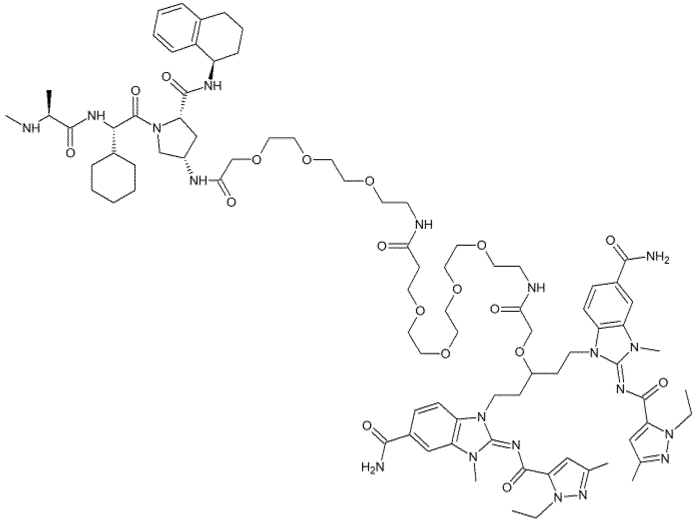

Prepared similarly to Example 7 from Building Block G-4L.
LCMS (UPLC_pH2_MeCN_QC_V3): Rt = 3.38 min, m/z 849.3 [(M+2H)/2]⁺
LCMS (UPLC_pH9_MeCN_QC_V2): Rt = 4.12 min, m/z 849.4 [(M+2H)/2]⁺

### Example 19

### N-{15-[4-(3-{N-4-[2-({(2S,4R)-1-[(S)-2-(1-Fluorocyclopropanecarbonylamino)-3,3-dimethylbutyryl]-4-hydroxy-2-pyrrolidinylcarbonylamino}methyl)-5-(4-methyl-1,3-thiazol-5-yl)phenoxy]butylcarbamoyl}propionyl)-1-piperazinyl]-15-oxo-3,6,9,12-tetraoxapentadecyl}(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide

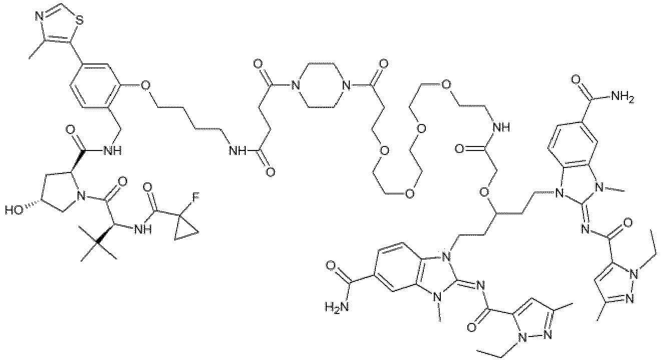

Prepared similarly to Example 1 from carboxylic acid Intermediate 5 and the corresponding commercially available amine hydrochloride (CAS-no. 2564467-03-2).
LCMS (UPLC_pH2_MeCN_QC_V2): Rt = 3.87 min, m/z 899.0 [(M+2H)/2]⁺
LCMS (UPLC_pH9_MeCN_QC_V2): Rt = 3.93 min, m/z 899.1 [(M+2H)/2]⁺

### Example 20

### N-(15-{4-[3-(2-{N-4-[2-({(2S,4R)-1-[(S)-2-(1-Fluorocyclopropanecarbonylamino)-3,3-dimethylbutyryl]-4-hydroxy-2-pyrrolidinylcarbonylamino}methyl)-5-(4-methyl-1,3-thiazol-5-yl)phenoxy]butylcarbamoyl}ethoxy)propionyl]-1-piperazinyl}-15-oxo-3,6,9,12-tetraoxapentadecyl)(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide

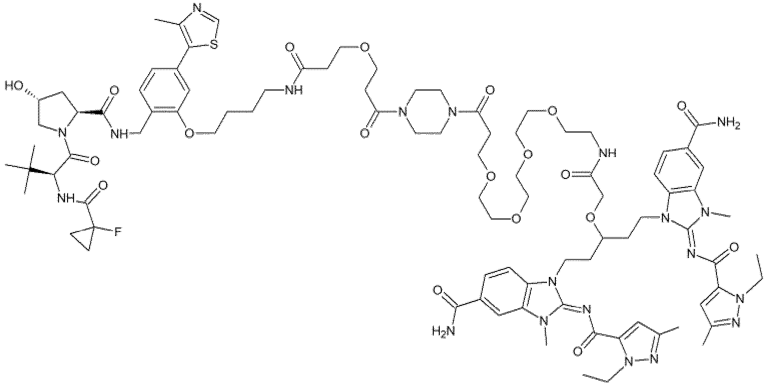

Prepared similarly to Example 1 from carboxylic acid Intermediate 6 and the corresponding commercially available amine hydrochloride.
LCMS (UPLC_pH2_MeCN_QC_V2): Rt = 3.85 min, m/z 921.1 [(M+2H)/2]⁺
LCMS (UPLC_pH9_MeCN_QC_V2): Rt = 3.90 min, m/z 921.1 [(M+2H)/2]⁺

### Example 21

### N-{16-[N-(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinylcarbamoyl]-3,6,9,12,15-pentaoxahexadecyl}15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

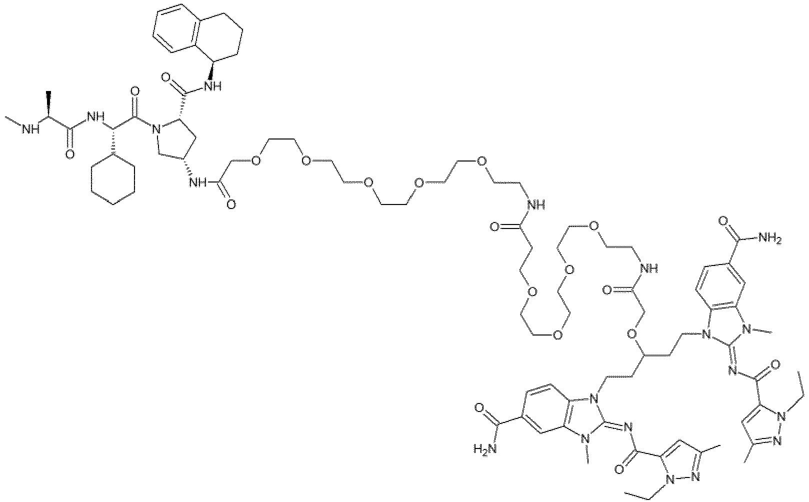

Prepared similarly to Example 7 from Building Block G-4L and the corresponding commercially available amine (CAS-no. 2446474-11-7).
LCMS (UPLC_pH2_MeCN_QC_V3): Rt = 3.76 min, m/z 1785.8 [M+H]⁺
LCMS (UPLC_pH9_MeCN_QC_V2): Rt = 4.13 min, m/z 893.7 [(M+2H)/2]⁺

### Example 22

### 1-[3-({N-2-[2-(2-{2-[m-({2-[(S)-1-{(S)-2-[(S)-2-(Methylamino)propionylamino]-2-cyclohexylacetyl}-2-pyrrolidinyl]-1,3-thiazol-4-yl}carbonyl)phenoxy]ethoxy}ethoxy)ethoxy]ethylcarbamoyl}methoxy)-5-{5-carbamoyl-2-[(1-ethyl-3-methyl-5-pyrazolyl)carbonylimino]-3-methyl-2,3-dihydro-1H-1 ,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide

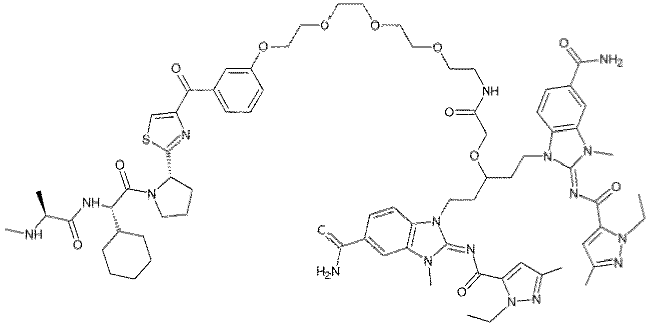

Prepared similarly to Example 7 from Building Block G-4 and the corresponding amine Intermediate L-1.
LCMS (UPLC_pH2_MeCN_QC_V2): Rt = 3.44 min, m/z 726.3 [(M+2H)/2]⁺
LCMS (UPLC_pH9_MeCN_QC_V2): Rt = 4.23 min, m/z 726.3 [(M+2H)/2]⁺

### Example 23

### N-[2-(2-{2-[2-(2,6-Dioxo-3-piperidyl)-1,3-dioxo-4-isoindolinylamino]ethoxy}ethoxy)ethyl]15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

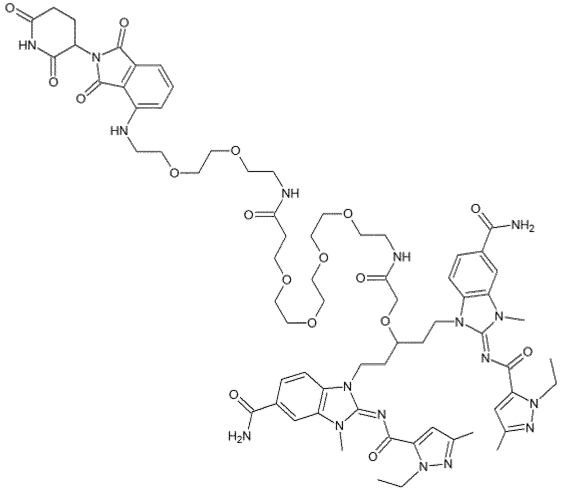

Prepared similarly to Example 1 from Building Block G-4L and the corresponding commercially available amine.
LCMS (UPLC_pH2_MeCN_QC_V3): Rt = 3.68 min, m/z 1429.4 [M+H]⁺
LCMS (UPLC_pH9_MeCN_QC_V3): Rt = 3.72 min, m/z 715.0 [(M+2H)/2]⁺

### Example 24

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]14-(14-{[(3-{5-carbamoyl-2-[(1-ethyl-3-methyl-5-pyrazolyl)carbonylimino]-3-methyl-2,3-dihydro-1H-1 ,3-benzimidazol-1 -y I}-1 -(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbonylamino}-3,6,9,12-tetraoxatetradecylcarbonylamino)-3,6,9,12-tetraoxatetradecanamide

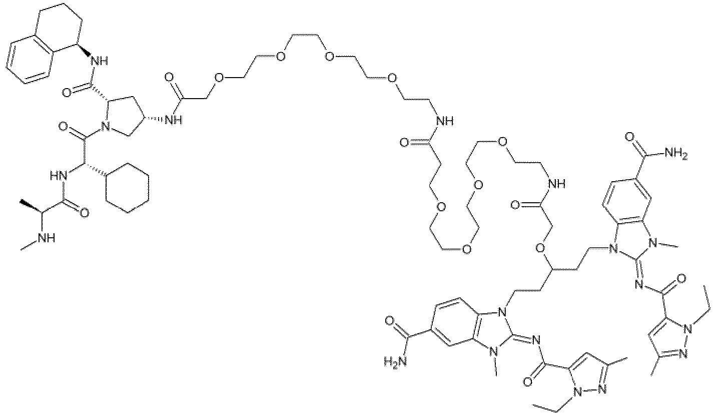

Prepared similarly to Example 7 from Building Block G-4L and the corresponding commercially available amine (CAS-no. 2446474-10-6).
LCMS (UPLC_pH2_MeCN_QC_V2): Rt = 3.37 min, m/z 871.6 [(M+2H)/2]⁺
LCMS (UPLC_pH9_MeCN_QC_V2): Rt = 4.09 min, m/z 871.6 [(M+2H)/2]⁺

### Example 25

### N-[2-(2-{2-[2-(2-{2-[2-(2,6-Dioxo-3-piperidyl)-1,3-dioxo-4-isoi ndoli nylami no] eth oxy}ethoxy)ethoxy] ethoxy}ethoxy)ethyl] 15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-4,7,10,13-tetraoxapentadecanamide

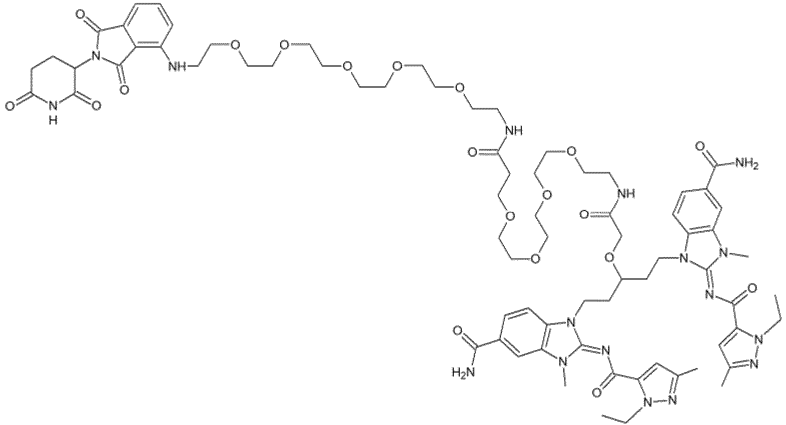

Prepared similarly to Example 1 from Building Block G-4L and the corresponding commercially available amine.
LCMS (UPLC_pH2_MeCN_QC_V3): Rt = 3.73 min, m/z 1561.8 [M+H]⁺
LCMS (UPLC_pH9_MeCN_QC_V3): Rt = 3.77 min, m/z 781.0 [(M+2H)/2]⁺

### Example 26

### N-[2-(2-{[N-(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinylcarbamoyl]methoxy}ethoxy)ethyl]15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

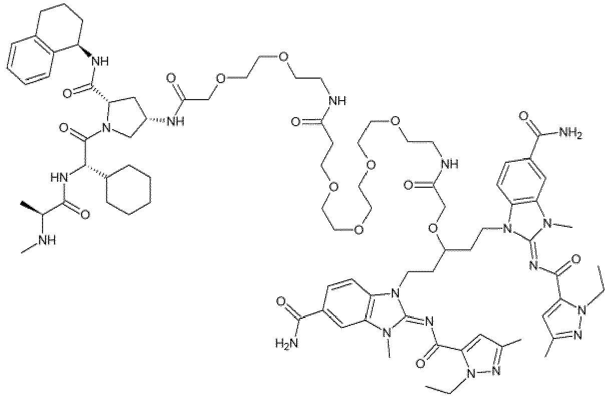

Prepared similarly to Example 7 from Building Block G-4L and the corresponding commercially available amine (CAS-no. 2415256-16-3).
LCMS (UPLC_pH2_MeCN_QC_V3): Rt = 3.36 min, m/z 827.3 [(M+2H)/2]⁺
LCMS (UPLC_pH9_MeCN_QC_V3): Rt = 4.10 min, m/z 827.2 [(M+2H)/2]⁺

### Example 27

### N-{14-[N-(S)-1-{[(2S,4R)-2-{N-(S)-1-[p-(4-Methyl-1,3-thiazol-5-yl)phenyl]ethylcarbamoyl}-4-hydroxy-1-pyrrolidinyl]carbonyl}-2,2-dimethylpropylcarbamoyl]-3,6,9,12-tetraoxatetradecyl}-N-methyl-1-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-3,6,9,12-tetraoxa-14-tetradecanecarboxamide

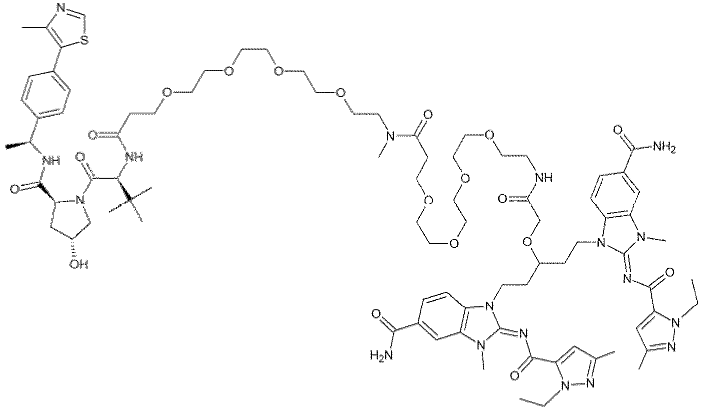

Prepared similarly to Example 1 from carboxylic acid Intermediate 7 and the corresponding commercially available amine.
LCMS (UPLC_pH2_MeCN_QC_V3): Rt = 3.87 min, m/z 865.7 [(M+2H)/2]⁺
LCMS (UPLC_pH9_MeCN_QC_V3): Rt = 3.92 min, m/z 865.7 [(M+2H)/2]⁺

### Example 28

### N-[(S)-1-{[(2S,4R)-4-Hydroxy-2-({[p-(4-methyl-1,3-thiazol-5-yl)phenyl]methyl}carbamoyl)-1-pyrrolidinyl]carbonyl}-2,2-dimethylpropyl]11-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-3,6,9-trioxaundecanamide

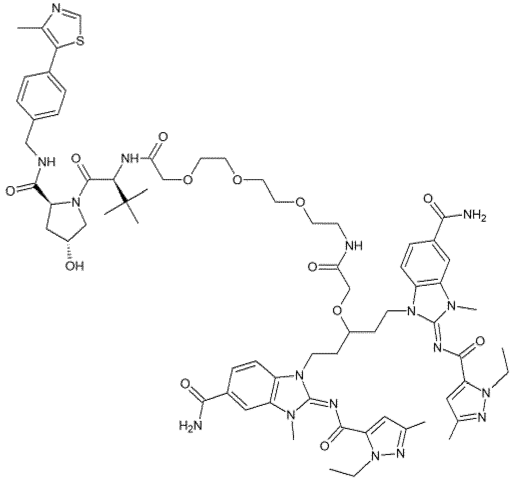

Prepared similarly to Example 1 from Building Block G-4 and the corresponding commercially available amine.
LCMS (UPLC_pH2_MeCN_QC_V2): Rt = 3.79 min, m/z 699.3 [(M+2H)/2]⁺
LCMS (UPLC_pH9_MeCN_QC_V2): Rt = 3.86 min, m/z 699.3 [(M+2H)/2]⁺

### Example 29

### 1-{3-[(N-2-{2-[2-(2-{2-[2-(2,6-Dioxo-3-piperidyl)-1,3-dioxo-4-isoindolinylamino]ethoxy}ethoxy)ethoxy]ethoxy}ethylcarbamoyl)methoxy]-5-{5-carbamoyl-2-[(1-ethyl-3-methyl-5-pyrazolyl)carbonylimino]-3-methyl-2,3-dihydro-1H-1 ,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

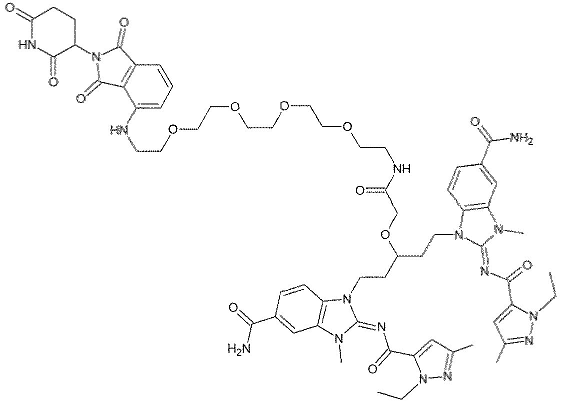

Prepared similarly to Example 1 from Building Block G-4 and the corresponding commercially available amine.
LCMS (UPLC_pH2_MeCN_QC_V2): Rt = 3.77 min, m/z 635.7 [(M+2H)/2]⁺
LCMS (UPLC_pH9_MeCN_QC_V2): Rt = 3.82 min, m/z 635.6 [(M+2H)/2]⁺

### Example 30

### N-[(3S,5S)-5-[N-(R)-1,2,3,4-Tetrahydro-1-naphthylcarbamoyl]-1-{(S)-2-[(S)-2-(methylamino)propionylamino]-2-cyclohexylacetyl}-3-pyrrolidinyl]14-{14-[({3-[5-carbamoyl-2-(1-ethyl-3-methyl-5-pyrazolylcarbonylamino)-1H-1 ,3-benzimidazol-1-yl]-1-{2-[5-carbamoyl-2-(1-ethyl-3-methyl-5-pyrazolylcarbonylamino)-1H-1 ,3-benzimidazol-1-yl]ethyl}propoxy}methyl)carbonylamino]-3,6,9,12-tetraoxatetradecylcarbonylamino}-3,6,9,12-tetraoxatetradecanamide

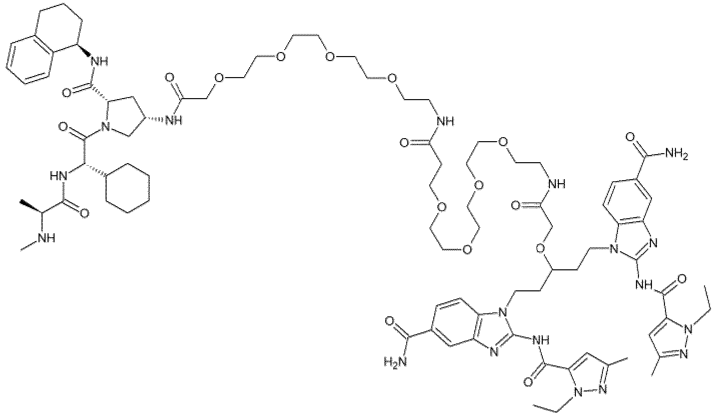

The compound may be prepared similarly to Example 7 from Building Block G-3L and the corresponding commercially available amine (CAS-no. 2446474-10-6).

### Example 31

### N-[2-(2-{2-[2-(2,6-Dioxo-3-pi peridyl)-1, 3-dioxo-4-isoi ndoli nylami no]ethoxy}eth oxy)ethyl] 15-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1 ,3-benzimidazol-1-yl]ethyl}propoxy}methyl)carbonylamino]-4,7,10,13-tetraoxapentadecanamide

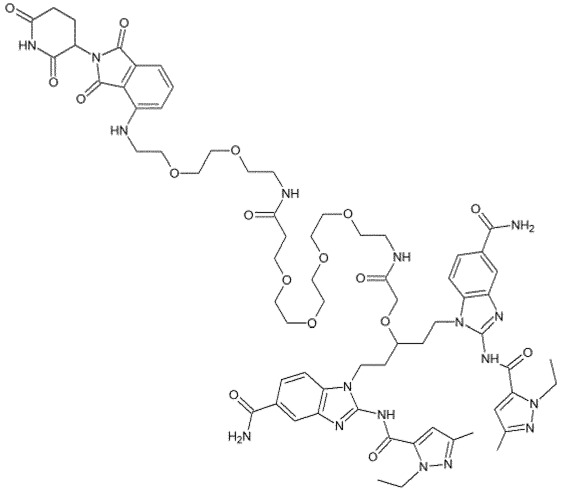

The compound is prepared similarly to Example 1 from Building Block G-3L and the corresponding commercially available amine.

### Example 32

### N-[(S)-1-{[(2S,4R)-4-Hydroxy-2-({[p-(4-methyl-1,3-thiazol-5-yl)phenyl]methyl}carbamoyl)-1-pyrrolidinyl]carbonyl}-2,2-dimethylpropyl]14-{14-[({3-[5-carbamoyl-2-(1-ethyl-3-methyl-5-pyrazolylcarbonylamino)-1 H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbonylamino]-3,6,9,12-tetraoxatetradecylcarbonylamino}-3,6,9,12-tetraoxatetradecanamide

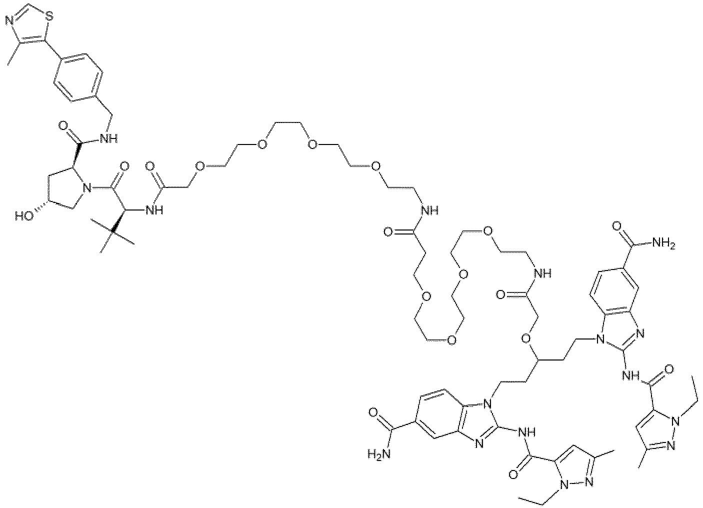

The compound is prepared similarly to Example 1 from Building Block G-3L and the corresponding commercially available amine.

### Example 33

### N-[2-(2-{2-[2-(2,6-Dioxo-3-piperidyl)-7-methoxy-1,3-dioxo-4-isoindolinylamino]ethoxy}ethoxy)ethyl] 15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbonylamino}-4,7,10,13-tetraoxapentadecanamide

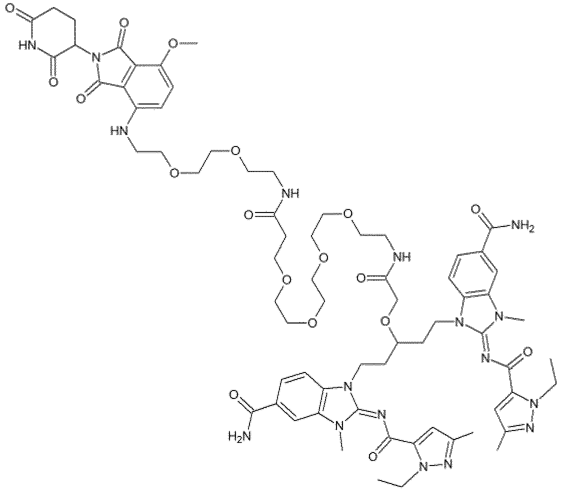

Prepared similarly to Example 1 from Building Block G-4L and the corresponding amine Intermediate L-3.
LCMS (UPLC_pH2_MeCN_QC_V2): Rt = 3.58 min, m/z 730.2 [(M+2H)/2]⁺
LCMS (UPLC_pH9_MeCN_QC_V2): Rt = 3.61 min, m/z 730.2 [(M+2H)/2]⁺

### Example 34

### N-(2-{2-[4-(N-2,6-Dioxo-3-piperidylcarbamoyl)-3-anisidino]ethoxy}ethyl)15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-4,7,10,13-tetraoxapentadecanamide

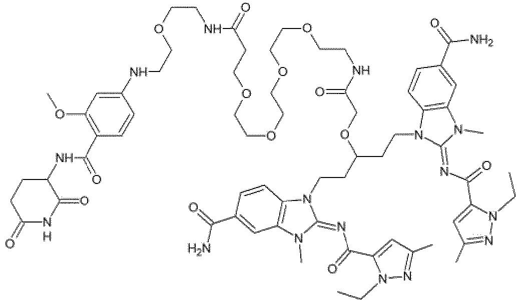

Prepared similarly to Example 1 from Building Block G-4L and the corresponding amine Intermediate L-4.
LCMS (UPLC_pH2_MeCN_QC_V2): Rt = 3.45 min, m/z 695.2 [(M+2H)/2]⁺
LCMS (UPLC_pH9_MeCN_QC_V2): Rt = 3.44 min, m/z 695.3 [(M+2H)/2]⁺

### Example 35

### N-(2-{1-[4-(N-2,6-Dioxo-3-piperidylcarbamoyl)-3-methoxyphenyl]-4-piperidyl}ethyl)15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-4,7,10,13-tetraoxapentadecanamide

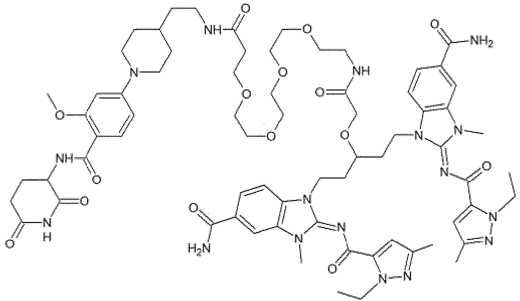

Prepared similarly to Example 1 from Building Block G-4L and the corresponding amine Intermediate L-5.
LCMS (UPLC_pH2_MeCN_QC_V3): Rt = 3.52 min, m/z 1413.5 [M+H]⁺
LCMS (UPLC_pH9_MeCN_QC_V2): Rt = 3.62 min, m/z 707.2 [(M+2H)/2]⁺

### Example 36

### N-[15-(4-{1-[4-(N-2,6-Dioxo-3-piperidylcarbamoyl)-3-methoxyphenyl]-4-piperidyl}-1-piperazinyl)-15-oxo-3,6,9,12-tetraoxapentadecyl](3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1 H-1 ,3-benzimidazol-1-yl}ethyl)propoxy)acetamide

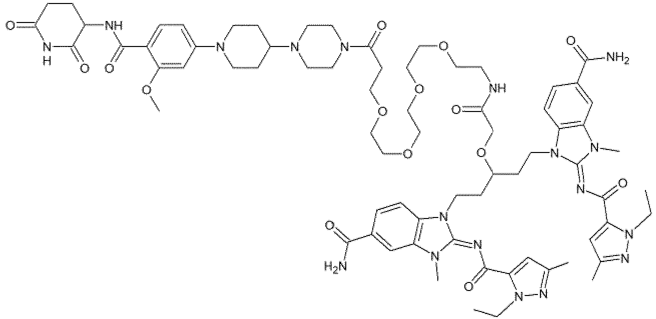

Prepared similarly to Example 1 from Building Block G-4L and the corresponding amine Intermediate L-6.
LCMS (UPLC_pH2_MeCN_QC_V3): Rt = 2.87 min, m/z 1454.2 [M+H]⁺
LCMS (UPLC_pH9_MeCN_QC_V3): Rt = 3.50 min, m/z 727.5 [(M+2H)/2]⁺

### Example 37

### N-(2-{1-[4-(N-2,6-Dioxo-3-piperidylcarbamoyl)-3-chlorophenyl]-4-piperidyl}ethyl)15-{[(3-{5-carbamoyl-2-[(1-ethyl-3-methyl-5-pyrazolyl)carbonylimino]-3-methyl-2,3-dihydro-1H-1 ,3-benzimidazol-1-yl}-1-(2-{5-carbamoyl-2-[(1-ethyl-3-methyl-5-pyrazolyl)carbonylimino]-3-methyl-2,3-dihydro-1H-1 ,3-benzimidazol-1-yl}ethyl)propoxy)methyl]carbonylamino}-4,7,10,13-tetraoxapentadecanamide

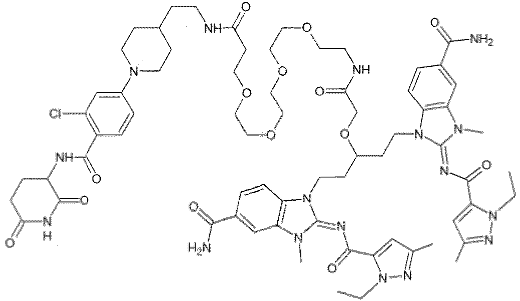

Prepared similarly to Example 1 from Building Block G-4L and the corresponding amine Intermediate L-7.
LCMS (UPLC_pH2_MeCN_QC_V2): Rt = 3.58 min, m/z 709.5 [(M+2H)/2]⁺
LCMS (UPLC_pH9_MeCN_QC_V2): Rt = 3.65 min, m/z 709.4 [(M+2H)/2]⁺

### Example 38

### N-(2-{1-[4-(N-2,6-Dioxo-3-piperidylcarbamoyl)-3-fluorophenyl]-4-piperidyl}ethyl)15-{[(3-{5-carbamoyl-2-[(1-ethyl-3-methyl-5-pyrazolyl)carbonylimino]-3-methyl-2,3-dihydro-1 H-1 ,3-benzimidazol-1-yl}-1-(2-{5-carbamoyl-2-[(1-ethyl-3-methyl-5-pyrazolyl)carbonylimino]-3-methyl-2,3-dihydro-1 H-1 ,3-benzimidazol-1-yl}ethyl)propoxy)methyl]carbonylamino}-4,7,10,13-tetraoxapentadecanamide

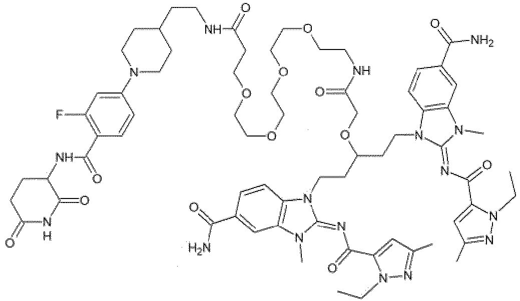

Prepared similarly to Example 1 from Building Block G-4L and the corresponding amine Intermediate L-8.
LCMS (UPLC_pH2_MeCN_QC_V2): Rt = 3.60 min, m/z 701.3 [(M+2H)/2]⁺
LCMS (UPLC_pH9_MeCN_QC_V2): Rt = 3.63 min, m/z 701.2 [(M+2H)/2]⁺

### Biological assays and data

As stated above, the compounds of the present invention are inhibitors of STING. As stated above, the compounds of the present invention are not STING agonists. Biological activities of the compounds of the present invention can be determined using any suitable *in vitro* assay as well as tissue and *in vivo* models.

As demonstrated below, the STING-binding portion of the compounds of the invention are not STING agonists and may function as STING antagonists.

### Example 39 - STING binding FP assay I

The interaction between molecules and the C-terminal domain (CTD) of human STING was evaluated using a competition binding assay. In this assay, recombinant human STING protein (155-379) and a STING binding fluorescent probe were employed. Upon binding of the probe to STING, the rotation of the fluorescent probe is slowed due to the larger molecular size of the protein-bound complex. As a result, emitted light retains its plane of polarization, leading to an increase in fluorescence polarization. Molecules competing for the probe binding site displace the probe, which leads to rapid randomization of the polarization plane of the emitted light and a subsequent decrease in measured fluorescence polarization signal. Change in polarization was measured, allowing a comparative evaluation of the binding affinities of different compounds to the target protein.

The assay was performed in 96-well plates (ThermoFisher, cat.# 237108) containing 100µl : 10 µL test compound in 10% DMSO and buffer A (150 mM NaCl, 50 mM HEPES pH 7.5, 1 mM DTT) + 90 µL prepared master mix solution of: 4 nM fluorescein-labelled STING ligand (Probe 1; synthesis described above) in buffer A + 0.055% Tween-20 (v/v) and 35 nM STING in buffer A. Post mixing, plates were agitated at 600rpm and incubated for 20 min at room temperature. Subsequent measurements of the fluorescence polarization signal were made on a BioTek Synergy-H1 plate reader, using a polarization filter; Exc: 485/20 nm, Em: 528/20 nM) with a gain-setting of 75. IC50 values were determined using a standard non-linear regression four-parameter curve fit in Graphpad Prism.

Using the STING binding FP assay I described above, the following IC50 values of Example compounds and selected relevant Building Blocks were determined:

| **Example number** | **STING binding FP assay I, IC50 (nM)** |
|---|---|
| 1 | <30 |
| 5 | <30 |
| 28 | <30 |
| 29 | <30 |

| **Building Block** | **STING binding FP assay I, IC50 (nM)** |
|---|---|
| G-2 | <30 |
| G-4 | <30 |

### Example 40 - STING binding FP assay II

The procedure for STING binding FP assay II is the same as described for STING binding FP assay I, except for a lower STING concentration. In STING binding FP assay II, a concentration of 7 nM STING in buffer A was used.

Using the STING binding FP assay II described above, the following IC50 values of Example compounds were determined:

| **Example number** | **STING binding FP assay II, IC50 (nM)** |
|---|---|
| 5 | 2.4 |
| 7 | 3.0 |
| 8 | 2.5 |
| 9 | 8.4 |
| 12 | 2.1 |
| 14 | 5.7 |
| 15 | 1.7 |
| 16 | 4.5 |
| 17 | 7.9 |
| 29 | 1.8 |

### Example 41 - STING binding FP assay III

The procedure for STING binding FP assay III is the same as described for STING binding FP assay I, except for a lower STING concentration. In STING binding FP assay III, a concentration of 10 nM STING in buffer A was used.

Using the STING binding FP assay III described above, the following IC50 values of Example compounds were determined:

| **Example number** | **STING binding FP assay II, IC50 (nM)** |
|---|---|
| 4 | 2.2 |
| 6 | 2.6 |
| 10 | 4.5 |
| 11 | 5.3 |
| 13 | 4.1 |
| 18 | 3.5 |
| 19 | 7.1 |
| 20 | 2.7 |
| 21 | 5.1 |
| 23 | 2.9 |
| 24 | 8.6 |
| 25 | 3.1 |
| 26 | 4.4 |
| 27 | 4.5 |
| 34 | 3.7 |
| 35 | 10.3 |
| 36 | 10.0 |
| 37 | 3.7 |
| 38 | 2.6 |

### Example 42 - STING binding TR-FRET assay

The affinity of molecules to the C-terminal domain (CTD) of human STING was determined using a commercial Human STING WT Binding Kit (CisBio, cat.# 64BDSTGPEG). In this time-resolved FRET assay, a recombinant human STING protein construct with a His-tag was employed. When bound to STING, a D2-labeled active site probe accepts the 620 nm emission from Anti-HIS-Tb-STING and an increase in fluorescence is measured at 665 nm. Molecules that compete for the probe binding site will result in a low 665 nm signal. The assay was run in CisBio low-volume white 96-well plates (catalog# 66PL96005) containing 5 µL compounds in Diluent Buffer (supplied with the CisBio kit). A solution of 1xSTING, 1x Anti-HIS-Tb and 1X D2-probe in TB detection buffer (kit supplied) was added to the plate. Plates were centrifuged for 2 min at 500 rpm, incubated for 60 min at room temperature in the dark, and then fluorescence emission at 665 nm following 330 nm xenon flash excitation on an BioTek synergy H1 multimode reader (Agilent) was measured. The plC50 values were determined using a standard four parameter curve fit in GraphPad Prism.

Using the STING binding TR-FRET assay described above, the following IC50 values of Building Block G-2 was determined:

| **Building block number** | **STING binding TR-FRET assay, IC50 (nM)** |
|---|---|
| G-2 | 0.452 |

### Example 43 - Western Blot analysis for STING degradation (DC50) in HFF-1 cells

To assess STING degradation in human foreskin fibroblasts HFF-1 cells (ATCC, SCRC-1041) cells were seeded at a density of 60.000 cells/well in in 24-well Nunclon Delta surface treated culture plate in DMEM growth medium (supplemented with 10% FBS, 1% Pen-strep). Cells were incubated over-night at 37°C 5% CO₂ to allow adhesion. Next day growth medium was renewed and a 5x concentration of test compounds (2.5% DMSO) diluted in growth medium were added to the appropriate well. The final DMSO concentration were 0.5%. Cell culture plates were returned to the incubator for additional 24h.

For cell lysing for Western Blot analysis cell culture plates were brought to ice, supernatant was removed by aspiration and cells washed 2x in ice-cold DPBS. Cells were lysed in 65 µL of RIPA buffer supplemented with cOmplete ULTRA Tablets protease inhibitor cocktail, PIERCE Phosphatase inhibitor mini tablet, Benzonase and 10mM NaF for 20 min on ice. Lysates were collected, cleared by centrifugation (13000xg for 10 min at 4°C). Samples were either analysed directly or stored at -80°C until analysis.

For Western Blotting, total protein concentration in each lysate was determined using PIERCE Gold BCA Assay kit according to manufactures protocol. 6 µg of total protein was mixed with 2x Laemmli buffer containing DTT, boiled for 5 min at 95°C, cooled on ice and loaded into each well of a 4-20% Criterion TGX gel. Proteins were separated by electrophoresis, by running for 1h at 150V. After separation proteins were blotted onto a PVDF membrane by semi-dry transfer using Trans-Blot turbo system at pre-defined BioRad MIDI protocol; 25V, 1A (fixed) for 30 min. Membranes were blocked for 1h in 5% skim milk in TBS-T20 on a rocker at room temperature followed by washing and exposure to primary antibodies against human STING 1:1000 (Cell Signalling, #13647) or loading controls Vinculin 1:1000 (Cell Signalling, #13901) or beta-Actin 1:10000 (Sigma, A5441) all diluted in 5% BSA in TBS-T20. Membranes were probed over night at 4°C on a rocker. Membranes were washed 3x in TBS-T20 followed by exposure to secondary HRP-conjugated anti-rabbit antibody or HRP-conjugated anti-mouse antibody diluted 1:10000 in 5% skim milk in TBS-T20 as appropriate. Membranes were incubated for 1h at room temperature on a rocker, then washed 3x in TBS-T20. Signals were revealed using clarity ECL mixed 1:1 for 1 min and imaged using ImageQuant800.

For generating DC₅₀ values, the intensity of individual bands was measured using imaged and expression levels were calculated as a ratio of STING intensities vs. Reference protein intensities and normalized to DMSO control. The normalized ratios were plotted on a XY curve in GraphPad Prism where DC₅₀ was estimated using a 4-parameter non-linear regression fitting curve.

Using the Western Blot analysis described above, the following DC50 values of Example compounds were determined (DC50 < 200 nM = +++; 200 nM ≤ DC50 < 1000 nM = ++; 1000 nM ≤ DC50 < 5000 nM = +):

| **Example number** | **STING degradation in HFF-1 cells, DC50 (nM)** | **Reference protein used for DC50 determination** |
|---|---|---|
| 3 | ++ | beta-actin |
| 6 | ++ | beta-actin |
| 10 | +++ | vinculin |
| 11 | +++ | beta-actin |
| 16 | +++ | beta-actin |
| 23 | ++ | beta-actin |
| 24 | ++ | vinculin |
| 25 | +++ | beta-actin |
| 26 | +++ | vinculin |

### Example 44 - Western Blot analysis for STING degradation (DC50) in H596, H1650 and HaCat cells

To assess STING degradation in H596 cells (ATCC, HTB-178) cells were seeded at a density of 100.000 cells/well in in 24-well Nunclon Delta surface treated culture plate in RPMI-1640 growth medium (supplemented with 10% FBS, 1% Pen-strep). Cells were incubated over-night at 37°C 5% CO₂ to allow adhesion. Next day growth medium was renewed and a 5x concentration of test compounds (2.5% DMSO) diluted in growth medium were added to the appropriate well. Final DMSO concentration was 0.5%. Cell culture plates were returned to the incubator for additional 24h. Final test compound concentrations were 0.22 µM, 0.67 µM, 2 µM and 6 µM.

For cell lysis, western blotting and data analysis, the methods described above for HFF-1 cells were applied.

Representative western blots are shown in Figure 1 (middle panel) for Example compounds 14, 16 and 24 and demonstrate induction of STING degradation using either compound. Example compounds 16 and 24 showed a more potent effect of induction of STING degradation than Example compound 14, with Example compound 14 inducing visible degradation in H1650 and H596 at the higher concentrations tested but did not induce visible STING degradation in HaCat cells.

Using the Western Blot analysis described above, the following DC50 values of Example compounds were determined (DC50 < 200 nM = +++; 200 nM ≤ DC50 < 1000 nM = ++; 1000 nM ≤ DC50 < 5000 nM = +):

| **Example number** | **STING degradation in H596 cells, DC50 (nM)** | **Reference protein used for DC50 determination** |
|---|---|---|
| 14 | + | beta-actin |
| 19 | +++ | beta-actin |
| 20 | +++ | beta-actin |
| 27 | +++ | beta-actin |

Similar protocols were carried out to assess STING degradation in H1650 cells (Figure 1, top panel) and HaCat cells (Figure 1, bottom panel) using Example compounds 14, 16 and 24, except that the seeding density was 150,000 cells/well and 250,000 cells/well for H1650 cells and HaCat cells, respectively. As shown in H596 cells, Example compounds 14, 16 and 24 induced STING degradation, with Example compounds 16 and 24 having a more potent effect than Example compound 14.

### Example 45 - Western Blot analysis for STING degradation in mouse RAW264.7 cells

To assess STING degradation in RAW264.7 cells, cells were seeded at a density of 60.000 cells/well in in 24-well Nunclon Delta surface treated culture plate in RPMI-1640 growth medium (supplemented with 10% FBS, 1% Pen-strep). Cells were incubated over-night at 37°C 5% CO₂ to allow adhesion. Next day growth medium was renewed and a 5x concentration of test compounds (2.5% DMSO) diluted in growth medium were added to the appropriate well. Final DMSO concentration was 0.5%. Cells were treated with a titration of test compound from 19.5nM to 10 µM 24 hours prior to cells lysis and subsequently analyzed by WB using anti-STING (1:1000) and anti-VCL (1:1000) antibodies. Controls included UT (DMSO) and 1.8µM diABZI treated cells.

For cell lysis, western blotting and data analysis, the methods described above for HFF-1 cells were applied.

Representative western blots are shown in Figure 2A-C for compounds Example compounds 14, 16 and 24, respectively, with increasing concentrations of example compound from 19.5nM to 10 µM.

Figures 2A-C demonstrate that each compound tested induced STING degradation in mouse cells in a dose-dependent manner.

### Example 46 - Western Blot analysis for STING recovery following Example compound washout in HFF-1 cells

Again, the methods described above for HFF-1 cells were applied for cell culture, lysis and western blotting. HFF1 cells were treated with 1µM Compound E16 or E24 or left untreated (UT) for 24h prior washout of test compound, and then incubated for 0-48 hours in DMEM without test compound. At the different time points cell lysates were prepared and assessed by western blot using anti-STING (1:1000) and anti-VCL (1:1000) antibodies. Controls included cells left untreated.

Figure 3 shows that following incubation with either Example compound 16 or Example compound 24, STING levels failed to recover to pre-treatment levels after 48 hours following removal of Example compound. Slow rebound of STING protein after washout suggests the intracellular half-life of the Example compounds will allow for a prolonged protein degradation effect and that this prolonged effect will be seen after in vivo exposure.

### Example 47 - Western Blot analysis for STING degradation in STING-associated vasculopathy with onset in infancy (SAVI) patient cells

For this study, STING degradation was assessed in primary fibroblast cells with constitutively active STING (comprising a N154S mutation), derived from SAVI patients, and compared to healthy donor primary fibroblast cells.

Cells were seeded at 50,000 cells/well and cultured under standard conditions for 24 hours. Media was replaced with test compound and incubated for 20 hours prior to analysis.

Cell culture medium was replaced with fresh medium containing 1 µM Example compound and incubated for 20 hours. For cell lysis and western blotting, the methods described above for HFF-1 cells were applied.

Figure 4 shows Example compounds 16 and 24 induced STING degradation in health donor cells and SAVI patient cells, demonstrating that these compounds can induce degradation of not only wild-type STING but constitutively active STING expressed in patients.

### Example 48 - Western Blot analysis for S1 STING ligand antagonism

To assess whether S1 STING ligands inhibit STING activation, primary human skin fibroblasts (Figure 5) or HFF-1 cells (Figure 6) were treated with various compounds which comprise only an S1 ligand domain and a Linker domain before undergoing Western Blot analysis as described below. Compounds A9, A11, A26 and A30 used in the following experiments share a common S1 ligand, which is also shared by Example compounds 1, 3, 5-29, 33-38.

| **S1 ligand** | **Structure** | **IUPAC name** |
|---|---|---|
| A9 | 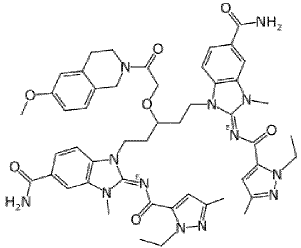 | 1-(5-{5-Carbamoyl-2-[(1-ethyl-3-methyl-5-pyrazolyl)carbonylimino]-3-methyl-2,3-dihydro-1H-1,3-benzimidazol-1-yl}-3-[2-(6-methoxy-1,2,3,4-tetrahydro-2-isoquinolyl)-2-oxoethoxy]pentyl)-2-[(1-ethyl-3-methyl-5-pyrazolyl)carbonylimino]-3-methyl-2,3-dihydro-1H-1,3-benzimidazole-5-carboxamide |
| A11 | 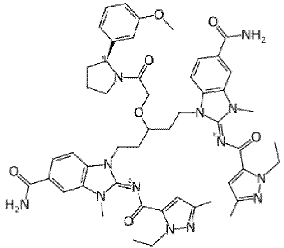 | 1-(3-{2-[(S)-2-(3-Methoxyphenyl)-1-pyrrolidinyl]-2-oxoethoxy}-5-{5-carba moyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| A25 | 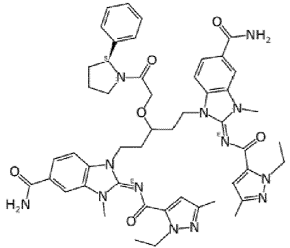 | 1-(3-{2-[(S)-2-Phenyl-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| A26 | 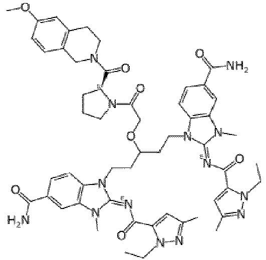 | 1-[3-(2-{(S)-2-[(6-Methoxy-1,2,3,4-tetrahydro-2-isoquinolyl)carbonyl]-1-pyrrolidinyl}-2-oxoethoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| A30 | 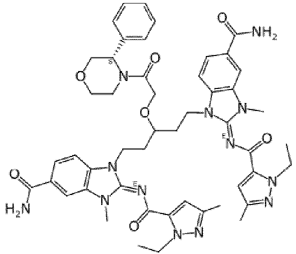 | 1-(3-{2-[(S)-3-Phenyl-4-morpholinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |

Figure 5 shows the results of Western Blots analysis of human skin fibroblasts treated with 5 µM of A9 or A11 compound either before, simultaneously with, or after activation with the 0.3 µM diABZI. Cells were incubated with A9 or A11 for 1 or 2 hours before addition of diABZI c3, treated with test compound and diABZI c3 simultaneously, or treated with A9 or A11 after 15 minutes incubation with diABZI c3. As a control, cells were also treated with diABZI c3 alone.

Figure 5 shows that under all conditions tested, A9 and A11 inhibited activation of STING, as demonstrated by a reduced intensity of P-STING. In addition, the downstream effector of active STING, TBK1, also showed reduced levels of activation as demonstrated by reduced P-TBK1 intensity compared to cells only treated with diABZI c3. Likewise, activation of the downstream effector LC3B is also reduced following treatment with Compounds A9 and A11 as demonstrated by reduced LC3B lipidation.

In Figure 6A, HFF-1 cells were treated with 5 µM of compounds A11, A25, A26, or A30, or 0.5 µM STING antagonist control for 15 min followed prior to addition of 300 nM diABZI and incubation for 2 h or 5 h. The cells were lysed and analysed on WB. As additional controls, cells were left untreated (UT), treated with Lipofectamine (Lipo control) or with compounds A26 or A30 as single agent. The WB membrane was probed for p-STING, STING, p-IRF3, p-TBK1, and Vinculin.

As expected, in cells treated only with diABZI c3 (-), STING was phosphorylated and activated as were the downstream STING effectors IRF3 and TBK1. In contrast, cells treated with STING antagonist control, STING, TBK1 and IRF3 remained largely unphosphorylated. In cells treated with compounds A11, A25, A26 and A30 prior to diABZI c3 activation, STING, TBK1 and IRF1 phosphorylation was lower compared to cells treated with diABZI c3 alone, which may indicate that these compounds are STING antagonists. In addition, in cells treated with only A26 or A30, no phosphorylation of STING, IRF1 or TBK1 was observed indicating that these compounds are not STING agonists.

In Figure 6B, similar experiments were performed as for Figure 6A, except that cells were activated with 8 µg/mL cGAMP instead of diABZI c3. In addition, the controls used in this set of experiments was A11, A25, A26 and A30 as a single agent with no cGAMP activation. In cells treated with cGAMP only (-), STING was phosphorylated and activated, as were the downstream STING effectors IRF3 and TBK1. In contrast, cells treated with STING antagonist control, STING, TBK1 and IRF3 remained largely unphosphorylated, with the exception of IRF3 after 5 hours of incubation with cGAMP. In cells treated with compounds A11, A25, A26 and A30 prior to cGAMP activation, STING, TBK1 and IRF1 phosphorylation was lower compared to cells treated with cGAMP alone (with the exception of TBK1 in lane A25), which indicates that these compounds are STING antagonists. In addition, in cells treated with only A11, A25, A26 or A30, no phosphorylation of STING, IRF1 or TBK1 was observed indicating that these compounds are not STING agonists.

It is clear from these results that the S1 ligand domain is not a STING agonist.

### Example 49 - Building Blocks act as STING antagonists and do not act as STING agonists: Human FB STING antagonist assay

Human skin fibroblasts were pre-treated for 2h with a serial dilution of test compound followed by stimulation with mock or 300 nM GSK STING agonist (CAS number: 2138299-34-8). Supernatants were harvested after 20 h of STING agonist stimulation. Release of IFN-a/b was determined using HEK-Blue IFN-α/β bioassay (Invivogen). Cellular viability was assessed using Alamar Blue HS assay. IC50 values were estimated using a non-linear regression fitting, variable slope (4 parameters) in GraphPad Prism 9.3.1.

Using the Human FB STING STING antagonist assay described above, the following IC50 values of building block compounds were determined:

| **Building block number** | **Human FB STING antagonist assay, IC50 (µM)** |
|---|---|
| G-1 | 3.2 |
| G-2 | 2.5 |

### Example 50 - Example compounds do not act as STING agonists

Human fibroblast were treated with a titration of E22, E9, E14 and E16 (7.2µM-3.6nM) for 24h prior to cell lysis and subsequently total STING, pSTING and Vinculin were assessed by western blot using rabbit anti-STING (1:1000), anti-pSTING (1:1000), anti-Vinculin (1:1000) and 2nd anti-rabbit-HRP (1:10.000) or anti-mouse-HRP (1:7500). Proteins were visualized using ImageQuant800. Control cells included untreated cells and cells treated with 300nM diABZI C3.

As shown in Figures 7A and 7B, in cells treated with diABZI c3 alone, as expected, STING was phosphorylated. In contrast, cells treated with increasing concentrations of E22 or E9 (Figure 7A), or increasing concentration of E14 or E16 (Figure 7B), no STING phosphorylation was observed. In addition, cells treated with either E14 or E16, total STING protein levels were observed to decrease indicating these compounds induce STING degradation. Together, these data show that the Example compounds do act as STING agonists because they do not induce STING phosphorylation following binding.

### Example 51 - Inhibition of STING-agonist-induced IFN-β release in HFF-1 cells (EC50) and mouse RAW264.7 cells

To evaluate cellular effects of test compounds, we assessed inhibition of STING-agonist-induced IFN-β release in human foreskin fibroblasts HFF-1 cells (ATCC, SCRC-1041). Cells were seeded at a density of 10.000 cells/well in in 96-well Nunclon Delta surface treated culture plate in DMEM growth medium (supplemented with 10% FBS, 1% Pen-strep). Cells were incubated over-night at 37°C 5% CO₂ to allow adhesion. Next morning growth medium was renewed and a 7x concentration 3-step dilution series of test compounds (2,1% DMSO) diluted in growth medium were added to the appropriate well. Final DMSO concentration was 0.3%. Cells were further incubated for 24h followed by addition of 8x concentration of GSK STING agonist (CAS number: 2138299-34-8; diABZI c3; diABZI), final concentration was 0.3 µM. Cells culture plates were further incubated, and supernatants were harvested after 6h and immediately stored at -20°C until determination of IFN-β release.

Cell supernatants were analysed for IFN-β release using DuoSet human IFN-β ELISA (R&D systems, DY-814) following manufactures protocol except using 96-well, half-volume plates from Corning and subsequently use half volume for all steps throughout the protocol. In brief, plates were coated o/n with a 130x dilution of capturing antibody in PBS. Wells were washed 3x with PBS-T20 followed by blocking with 1% BSA diluted in TBS-T20 for 1h. Wells were washed 3x with PBS-T20 and standards (n=2) and samples (n=3) were added to the plate and incubated for 2h at rt. Wells were washed 3x with PBS-T20 and detection antibody diluted 1:60 in 1% BSA in PBS was added to each well and incubated for 2h at rt. Plates were washed 3x with PBS-T20 and Streptavidin-HRP diluted 1:40 in 1% BSA in PBS was added pr well and incubated for 30 min at rt. The wells were washed 4x with PBS-T20 and TMB One Substrate (Promega) was added and incubated for 5-10 min in the dark. When fully developed, 1 mmol/L Sulfuric acid was added to stop the reaction. Signals were measured using BioTek Synergy H1 plate reader at 450nm and 570nm (reference).

Raw data values were quantified based on the standard curve using blank subtracted Δ450nm-570nm values generated using the Gen5 software connected to the plate reader. Concentrations were plotted into GraphPad Prism to calculate EC₅₀ values using a 4-parameter non-linear regression fitting curve, bottom constrains set for >0.

Using the HFF-1 STING inhibition assay described above, the following EC50 values of Example compounds were determined (EC50 < 200 nM = +++; 200 nM ≤ EC50 < 1000 nM = ++; 1000 nM ≤ EC50 < 5000 nM = +):

| **Example number** | **Inhibition of STING-agonist-induced IFN-β release in HFF-1 cells, EC50 (nM)** |
|---|---|
| 14 | + |
| 16 | +++ |
| 24 | ++* |

| | |
|---|---|
| * incomplete inhibition | |

Figure 8 also demonstrates that Example compounds disclosed herein inhibit STING-agonist induced IFN-β release in mouse RAW264.7 cells.

As expected, RAW264.7 cells treated with 1.8 µM diABZI c3 increased IFN-β secretion, resulting in a media concentration of around 1000 pg/mL. In contrast, cells treated with 1.8 µM diABZI c3 and 0.05 µM Example compounds 14, 16 or 24 reduced this expression to approximately 500 pg/mL. This inhibitory effect was even more pronounced in cells treated with either 0.5 µM or 5 µm of either one of Example compounds 14, 16 or 24.

HFF-1 cells were also assessed for inhibition of STING-agonist-induced IFN-β and CXCL10 release in cells treated with Compounds A9 and A11. In the first set of experiments (Figure 9A, unfilled bars), cells treated with only diABZI c3 resulted in ~ 200 U/mL IFN media concentration. In contrast, pre-treating cells for 1 to 2 hours prior to diABZI c3, simultaneous treatment with A9 and diABZI c3, or pre-treating cells for 15 minutes with diABZI c3 followed by A9 treatment led to a decrease in the IFN media concentration to ~100 U/mL. Similar results were observed for CXCL10 secretion: cells treated with diABZI c3 alone resulted in a CXCL10 media concentration of ~ 60 pg/mL. In contrast, cells pre-treated for 1 to 2 hours prior to diABZI c3, simultaneous treatment with A9 and diABZI c3, or pre-treating cells for 15 minutes with diABZI c3 followed by A9 treatment led to a decrease in the CXCL10 media concentration to ~30 U/mL (Figure 9B, unfiled bars).

In the second set of experiments (Figure 9A; filled bars), cells treated with only diABZI c3 resulted in ~ 100 U/mL IFN media concentration. In contrast, pre-treating cells for 1 to 2 hours prior to diABZI c3, simultaneous treatment with A11 and diABZI c3, or pre-treating cells for 15 minutes with diABZI c3 followed by A11 treatment led to a decrease in the IFN media concentration to -10 - 20 U/mL. Similar results were observed for CXCL10 secretion: cells treated with diABZI c3 alone resulted in a CLCL10 media concentration ~ 60 pg/mL. In contrast, cells pre-treated for 1 to 2 hours prior to diABZI c3, simultaneous treatment with A11 and diABZI c3, or pre-treating cells for 15 minutes with diABZI c3 followed by A11 treatment led to a decrease in the CXCL10 media concentration to -10 - 20 pg/mL (Figure 9B; filled bars).

Together, these data further demonstrate that the S1 ligand of the Example compounds do not act as STING agonists and may act as STING antagonists.

### Example 52 - Inhibition of STING-agonist-induced IFN-β release in H596 cells (EC50)

To determine cellular effects of test compounds, we assessed inhibition of STING-agonist-induced IFN-β release in H596 cells (ATCC, HTB-178). Cells were seeded at a density of 25.000 cells/well in in 96-well Nunclon Delta surface treated culture plate in RPMI-1640 growth medium (supplemented with 10% FBS, 1% Pen-strep). Cells were incubated over-night at 37°C 5% CO₂ to allow adhesion. Next morning growth medium was renewed and a 10x concentration, 3-step dilution series of test compounds diluted in growth medium (5% DMSO) were added to the appropriate well. Final DMSO concentration was 0.5%. Cells were further incubated for 24h followed by incubation with a 10x concentration of GSK STING agonist (CAS number: 2138299-34-8), final concentration was 0.3 µM. Cells culture plates were further incubated, and supernatants were harvested after 6h and immediately stored at -20°C until determination of IFN-β release.

For ELISA and data analysis, the methods described above for HFF-1 cells were applied.

Using the H596 STING inhibition assay described above, the following EC50 values of Example compounds were determined (EC50 < 200 nM = +++; 200 nM ≤ EC50 < 1000 nM = ++; 1000 nM ≤ EC50 < 5000 nM = +):

| **Example number** | **Inhibition of STING-agonist-induced IFN-β release in H596 cells, EC50 (nM)** |
|---|---|
| 10 | ++ |
| 16 | + |
| 19 | +++ |
| 20 | +++ |
| 24 | +++ |

### Example 53 - Pharmacokinetic profiling of Example compounds in mouse

Mice were administered with either 10 mg/kg Example compound 16 (Figure 10A) or 8 mg/kg Example compound 24 (Figure 10B) by subcutaneous injection. Plasma concentration of test compound was measured over the course of 8 hours.

Subcutaneous injection with either Example compound 16 or Example compound 24 leads to prolonged plasma exposure in mice. 8 hours after injection, the plasma concentration of Example compound 16 is approximately 100 nM, while the plasma concentration of Example compound 24 is approximately 1000 nM. These data are also consistent with plasma concentrations following intravenous injection which show a lower clearance rate of Example compound 24 vs Example compound 16 after 8 hours.

### Example 54 - In vivo effects on cytokine production following STING inhibition/degradation

Mice were pre-treated with either Example compound 16 or Example compound 24 in three dose arms; i) pre-treatment at t = -16 hours with 10 mg/kg Example compound and again at t = -2 hours with 10 mg/kg

Example compound; ii) pre-treatment at t = -16 hours only with 10 mg/kg Example compound, or iii) pre-treatment at t = -8 hours only with 10 mg/kg Example compound, prior to treatment with 1 mg/kg diABZi at t = 0 (Figure 11A). IFN-β and CXCL10 (IP-10) levels were analysed 6 hours after treatment with diABZi. One outlier in mice receiving pre-treatment with Example compound 16 was present in treatment arm iii) and is shown in Figures 11B and 11C as an open circle.

Figure 11B shows that in mice pre-treated with either Example compound 16 or Example compound 24 IFN-β in dose arm i), IFN-β expression appears to be completely abolished. Similar results were seen in mice treated with SP-0150 in dose arms ii) and iii), with IFN-β expression IFN-β expression appearing to be completely abolished. Similar, but slightly less pronounced results, are observed in mice treated with Example compound 16 in dose arms i) and ii) and show a significant inhibition of IFN-β.

Figure 11C shows that in mice pre-treated with Example compound 16 in each dose are i)-iii) show significantly reduced CXCL10 expression. Similarly, mice pre-treated with Example compound 24 in each dose arm also show reduced CXCL10 expression, with dose arm ii) reaching statistical significance.

These data together indicate that degradation of STING using the Example compounds is an effective approach for reducing STING-induced inflammation. In addition, dose arm iii) of each treatment arm also suggests that the mechanism of action of the Example compounds goes beyond induction of STING degradation through recruitment of E3 Ubiquitin ligase. Instead, the rapid reduction in secreted CLCL10 and, in particular IFN-β compared to cells treated with STING agonist suggest that the Example compounds are also acting as a 'classical' antagonist of STING separate from their function as inducers of protein degradation.

The S1 portion of the above compounds (e.g. Building blocks G-1, G-2 and G4, and Compounds A9, A11, A25, A26 and A30) have been demonstrated to bind to STING and, in the case of G-1, G-2, Compounds A9, A11, A25, A26 and A30, do not act as STING agonists. Each S1 region of the Example compounds of the invention share the core of at least one of G-1, G-2, A9, A11, A25, A26 and A30 chemical structure. Viewing the data as a whole, it is clear that the compounds of the invention have a prolonged STING protein degradation effect, which is consistent with the PK profiles discussed above. In addition, based on the shared structure of the S1 portions of the Example compounds and that of the G-1 or G-2 building blocks, or that of Compounds A9, A11, A25, A26 and A30, it is predicted that the Example compounds also do not act as STING agonists. This is supported by the data in Figure 7 which demonstrates that none of Compounds E9, E14, E16 or E22 induce STING phosphorylation.

### References

A number of publications are cited above in order to more fully describe and disclose the invention and the state of the art to which the invention pertains. Full citations for these references are provided below. The entirety of each of these references is incorporated herein.
Békés et al., PROTAC targeted protein degraders: the past is prologue; Nat. Rev. Drug Discov., 2022, 21, 181-200.
Decout et al., The cGAS-STING pathway as a therapeutic target in inflammatory diseases; Nat. Rev. Immunol., 2021, 21, 548-569
Konig et al. Familial chilblain lupus due to a gain-of-function mutation in STING; Ann. Rheum. Dis., 2017, 76, 468-472
Mutlu, M., Schmidt, I., Morrison, A.I. et al. Small molecule induced STING degradation facilitated by the HECT ligase HERC4. Nat Commun 15, 4584 (2024). https://doi.org/10.1038/s41467-024-48922-w
Paul et al, Centromere defects, chromosome instability, and cGAS-STING activation in systemic sclerosis; Nat Commun. 2022, 13(1):7074.
Rodero et al., Type I interferon-mediated autoinflammation due to DNase II deficiency; Nat. Comms., 2017, 8, 2176
Seok, J.K., Kim, M., Kang, H.C. et al. Beyond DNA sensing: expanding the role of cGAS/STING in immunity and diseases. Arch. Pharm. Res. 46, 500-534 (2023). https://doi.org/10.1007/s12272-023-01452-3
Siu et al., Discovery of a Novel cGAMP Competitive Ligand of the Inactive Form of STING; ACS Med. Chem. Lett. 2019, 10, 1, 92-97

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

**1.** A compound of formula (I):
*S1-L-E* (I),
wherein:
S1 is a STING ligand;
L is a Linker;
E is a E3 ubiquitin ligase ligand,
and pharmaceutically acceptable salts thereof,
wherein the STING ligand does not form a covalent bond with STING and
wherein the STING ligand does not exhibit STING agonist activity.

**2.** The compound according to claim 1, or a pharmaceutically acceptable salt thereof, wherein the compound is suitable for the degradation of Stimulator of Interferon Genes (STING).

**3.** The compound of claim 1 or 2, or a pharmaceutically acceptable salt thereof, wherein the E3 ubiquitin ligase ligand is a Cereblon (CRBN) E3 ubiquitin ligase ligand.

**4.** The compound of claim 1 or 2, or a pharmaceutically acceptable salt thereof, wherein the E3 ubiquitin ligase ligand is a von Hippel-Lindau (VHL) E3 ubiquitin ligase ligand

**5.** The compound of claim 1 or 2, or a pharmaceutically acceptable salt thereof, wherein the E3 ubiquitin ligase ligand is an Inhibitor of Apoptosis (IAP) E3 ubiquitin ligase ligand.

**6.** The compound of any one of the preceding claims, or a pharmaceutically acceptable salt thereof, wherein L connects the STING ligand to the E3 ubiquitin ligase ligand via a chain of atoms having 10-40 atoms in shortest length.

**7.** The compound of any one of the preceding claims, wherein the compound is of formula (I-1): 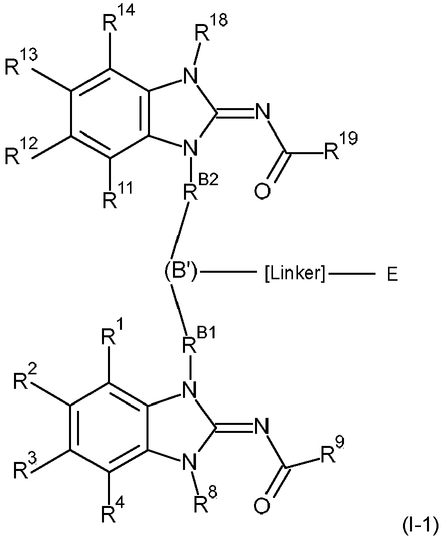
or a tautomer or pharmaceutically acceptable salt thereof,
wherein R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl);
R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -O-P(O)(OH)₂, C₁₋₄ alkoxy, NH₂, NH(C₁₋₆ alkyl), N(C₁₋₆ alkyl)₂, CO₂(C₁₋₆ alkyl) and halogen;
R³ and R¹³ are each independently selected from H, C(=O)NH₂; optionally substituted - C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, NH(C₁₋₆ alkyl), N(C₁₋₆ alkyl)₂, NH₂, CO₂(C₁₋₆ alkyl) and halogen;
R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH(C₁₋₆ alkyl), NH₂, CO₂(C₁₋₆ alkyl) and halogen;
R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -O-P(O)(OH)₂, C₁₋₄ alkoxy, N(C₁₋₆ alkyl)₂, NH(C₁₋₆ alkyl), NH₂, CO₂(C₁₋₆ alkyl) and halogen;
R⁸ and R¹⁸ are each independently selected from H, and C₁₋₄ alkyl;
B' taken together with R^{B1} and R^{B2} forms a linking group having 3-7 atoms in shortest length;
B' is the point of attachment to the Linker group L as follows: 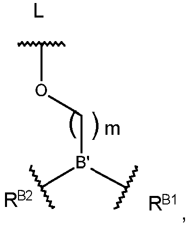
wherein m is 0 or 1;
the Linker (L) is a saturated or a partially or fully unsaturated framework comprising C and H atoms and at least one heteroatom, wherein said framework has a minimum length of from 10 to 40 atoms between B' and E; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-CO-R^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, C!, and CN;
wherein each R^{L} is independently selected from C₁₋₆ alkyl, C₂₋₆ alkenyl, C₂₋₆ alkynyl, C₃₋₆ cycloalkyl, C₃₋₆ carbocyclyl, C₅₋₆ heterocyclyl, C₅₋₆ aryl, and C₅₋₆ heteroaryl; and
E is an E3 ubiquitin ligase ligand.

**8.** The compound of claim 7, or a tautomer, or a pharmaceutically acceptable salt thereof, wherein R^{B1} and R^{B2} are each independently selected from (-CH₂-)₁₋₂ or a bond and B' taken together with R^{B1} and R^{B2} forms an alkyl linking group having 3-7 atoms in shortest length; and
B' is the point of attachment to the Linker group L as follows: 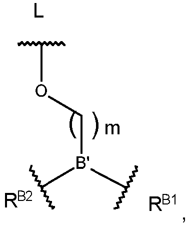
wherein B' is CH or C₁₋₅ alkyl;
wherein m is 0 or 1.

**9.** The compound of claim 7 or 8, or a tautomer, or a pharmaceutically acceptable salt thereof, wherein
R⁹ and R¹⁹ are each independently selected from an optionally substituted C₅₋₆ heteroaryl or optionally substituted C₅₋₆ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl).

**10.** The compound of any one of claims 7 to 9, or a tautomer, or a pharmaceutically acceptable salt thereof, wherein the compound of formula (I) is of formula (I-2): 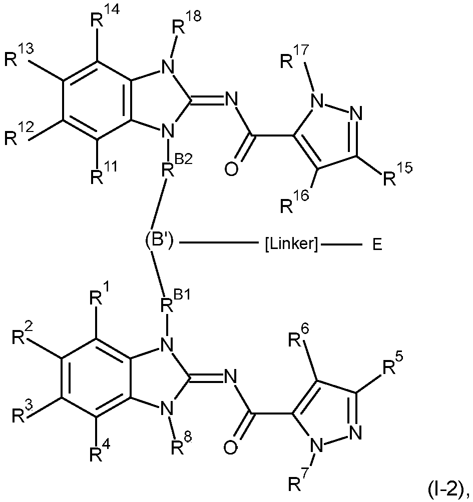
wherein R¹, R², R³, R⁴, R⁸, R¹¹, R¹², R¹³, R¹⁴, R¹⁸, R^{B1}, R^{B2}, B', Linker and E are as defined in claim 7,
wherein R⁷ and R¹⁷ are each independently selected from optionally substituted C₁₋₄alkyl wherein the optional substituents are independently selected from halogen, -halo(C₁₋₄alkyl), OH, and C₁₋₄ alkoxy;
R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy; and
R⁵ and R¹⁵ are each independently selected from H, cyclopropyl and C₁₋₄ alkyl.

**11.** The compound of any of claims 7 to 10, or a tautomer, or a pharmaceutically acceptable salt thereof, wherein R⁴, R¹⁴, R², R¹², R¹ and R¹¹ are each independently selected from H, OH, OCH₃, halogen or C₁₋₃ alkyl.

**12.** The compound of claim 10 or 11, or a pharmaceutically acceptable salt thereof, wherein R⁶ and R¹⁶ are each independently selected from H, OCH₃, halogen or C₁₋₃ alkyl.

**13.** The compound of any of claims 7 to 12, or a tautomer, or a pharmaceutically acceptable salt thereof, wherein R¹ and R¹¹ are H.

**14.** The compound of any of claims 7 to 13, or a tautomer, or a pharmaceutically acceptable salt thereof, wherein R⁶ and R¹⁶ are H.

**15.** The compound of any one of claims 7 to 14, or a tautomer, or a pharmaceutically acceptable salt thereof, wherein R⁴ and R¹⁴ are H.

**16.** The compound of any one of claims 7 to 15, or a tautomer, or a pharmaceutically acceptable salt thereof, wherein R² and R¹² are H.

**17.** The compound of any one of claims 10 to 16, or a tautomer, or a pharmaceutically acceptable salt thereof, wherein R⁵ and R¹⁵ are each independently selected from C₁₋₄ alkyl or C₁₋₄ alkoxy.

**18.** The compound of any of claims 10 to 17, or a tautomer, or a pharmaceutically acceptable salt thereof, wherein R⁷, R⁸, R¹⁷ and R¹⁸ are C₁₋₄ alkyl.

**19.** The compound of any of claims 10 to 18, or a tautomer, or a pharmaceutically acceptable salt thereof, wherein R⁷ and R¹⁷ are ethyl.

**20.** The compound of any one of claims 10 to 19, or a tautomer, or a pharmaceutically acceptable salt thereof, wherein R⁵ and R¹⁵ are methyl.

**21.** The compound of any one of claims 7 to 20, or a tautomer, or a pharmaceutically acceptable salt thereof, wherein R⁸ and R¹⁸ are methyl.

**22.** The compound of any one of claims 7 to 21, or a tautomer, or a pharmaceutically acceptable salt thereof, wherein R³ and R¹³ are selected from -C(=O)NH(C₁₋₃ alkyl) and -C(=O)NH₂.

**23.** The compound of any one of claims 7 to 22, or a tautomer, or a pharmaceutically acceptable salt thereof, wherein R³ and R¹³ are -C(=O)NH₂.

**24.** The compound of any one of claims 7 to 23, or a tautomer, or a pharmaceutically acceptable salt thereof, wherein:
R¹ and R¹¹ are selected from H, -OH or C₁₋₃ alkoxy;
R³ and R¹³ are selected from -C(=O)N(H)(C₁₋₃ alkyl) or -C(=O)NH₂;
R⁴ and R¹⁴ are selected from H, halogen, OH or C₁₋₄ alkyl;
R⁷ and R¹⁷ are C₁₋₄ alkyl;
R⁵ and R¹⁵ are selected from H or C₁₋₄ alkyl;
R⁶ and R¹⁶ are H; and
R⁸ and R¹⁸ are methyl.

**25.** The compound of claim 24, or a tautomer, or a pharmaceutically acceptable salt thereof, wherein:
R¹ and R¹¹ are H;
R³ and R¹³ are -C(=O)NH₂ or -C(=O)-NH-CH₃;
R⁷ and R¹⁷ are ethyl; and
R⁵ and R¹⁵ are methyl.

**26.** The compound of any one of claims 7 to 25, or a tautomer, or a pharmaceutically acceptable salt thereof, wherein R¹ is identical to R¹¹, R² is identical to R¹², R³ is identical to R¹³, R⁴ is identical to R¹⁴, R^{B1} is identical to R^{B2}, R⁸ is identical to R¹⁸ and R⁹ is identical to R¹⁹.

**27.** The compound any one of claims 10 to 26, or a tautomer, or a pharmaceutically acceptable salt thereof, wherein R¹ is identical to R¹¹, R² is identical to R¹², R³ is identical to R¹³, R⁴ is identical to R¹⁴, R^{B1} is identical to R^{B2}, R⁸ is identical to R¹⁸, R⁵ is identical to R¹⁵, R⁶ is identical to R¹⁶ and R⁷ is identical to R17.

**28.** The compound of any one of claims 7 to 27, or a tautomer, or a pharmaceutically acceptable salt thereof, wherein B' is of the formula: 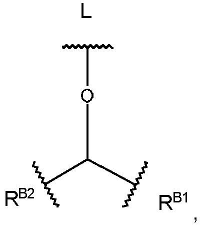
wherein R^{B1} and R^{B2} are each independently selected from (-CH₂-)₁₋₂;
wherein the wavy line indicates the point of attachment to the Linker (L), R^{B1} and R^{B2} as shown, optionally wherein R^{B1} and R^{B2} are each -CH₂-CH₂- and together with B' have the following structure: 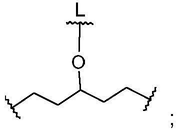
wherein the wavy line next to L represents the attachment to the linker and the wavy lines next to the alkyl groups represent the connection to the N atoms in the S1 structure.

**29.** The compound of any one of claims 7 to 28, or a tautomer, or a pharmaceutically acceptable salt thereof, wherein compound (I) is of the Formula (I-2b): 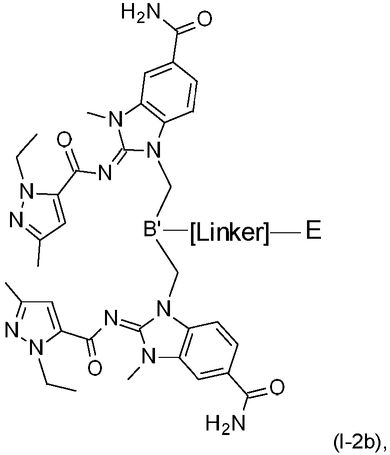 or a tautomer, or a pharmaceutically acceptable salt thereof wherein B' is a C₁₋₅ alkyl and is the connection point to the Linker.

**30.** The compound of any one of claims 7 to 29, or a tautomer, or a pharmaceutically acceptable salt thereof, wherein the compound of formula (I) is of the formula (I-2c) or (I-2d):

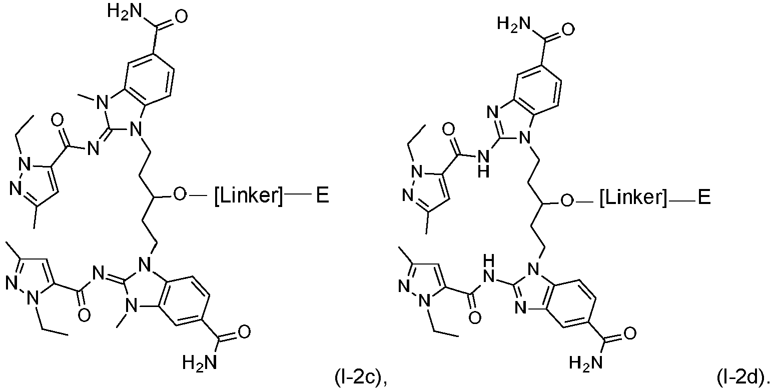

**31.** The compound of any one of claims 1 to 30, or a tautomer, or a pharmaceutically acceptable salt thereof, wherein E has a structure according to Formula E1: 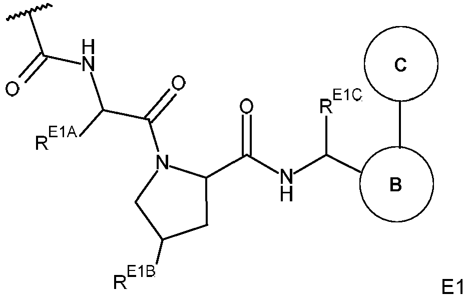 wherein
R^{E1A} is selected from optionally substituted C₁₋₆alkyl, optionally substituted C₃₋₇cycloalkyl, or optionally substituted C₄₋₁₀heterocyclyl, wherein the optional substituents are selected from OH, C₁₋₆alkyl, and halogen;
R^{E1B} is selected from H, OH, OR, NH₂, NHR, or NRR';
R^{E1C} is selected from H or C₁₋₆ alkyl;
Ring B is an optionally substituted C₆₋₁₀ carboaryl, or an optionally substituted C₅₋₁₀ heteroaryl group wherein the optional substituents are selected from C₁₋₆ alkyl, halogen or OH;
Ring C is an optionally substituted C₆₋₁₀ carboaryl, or an optionally substituted C₅₋₁₀ heteroaryl group, or an optionally substituted C₅₋₁₀ heterocyclic group, wherein the optional substituents are selected from C₁₋₆ alkyl, halogen or OH;
wherein R and R' are C₁₋₆ alkyl, C₂₋₆ alkenyl, C₂₋₆ alkynyl, C₃₋₇ cycloalkyl, C₄₋₇ heterocyclyl, and 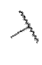 represents attachment point to L.

**32.** The compound according to claim 31, or a tautomer, or a pharmaceutically acceptable salt thereof, wherein R^{E1A} is C₁₋₆ alkyl.

**33.** The compound according to claim 32, or a tautomer, or a pharmaceutically acceptable salt thereof, wherein R^{E1A} is tert-butyl.

**34.** The compound according to any one of claims 31 to 33, or a tautomer, or a pharmaceutically acceptable salt thereof, wherein R^{E1B} is OH.

**35.** The compound according to any one of claims 31 to 33, or a tautomer, or a pharmaceutically acceptable salt thereof, wherein R^{E1C} is H.

**36.** The compound according to any one of claims 31 to 34, or a tautomer, or a pharmaceutically acceptable salt thereof, wherein R^{E1C} is C₁₋₃ alkyl.

**37.** The compound according to any one of claims 31 to 34, or a tautomer, or a pharmaceutically acceptable salt thereof, wherein R^{E1C} is methyl.

**38.** The compound according to any one of claims 31 to 37, or a tautomer, or a pharmaceutically acceptable salt thereof, wherein Ring B is an optionally substituted C₆₋₁₀carboaryl, or an optionally substituted C₅₋₁₀ heteroaryl group, wherein the optional substituents are selected from C₁₋₆ alkyl, halogen or OH.

**39.** The compound according to claim 38, or a tautomer, or a pharmaceutically acceptable salt thereof, wherein Ring B is an optionally substituted C₆₋₁₀ carboaryl, wherein the optional substituents are selected from C₁₋₆ alkyl, halogen or OH.

**40.** The compound according to claim 39, or a tautomer, or a pharmaceutically acceptable salt thereof, wherein Ring B is phenyl.

**41.** The compound according to claim 40, or a tautomer, or a pharmaceutically acceptable salt thereof, wherein ring B is phenyl linked in positions 1 and 4.

**42.** The compound according to any one of claims 31 to 41, or a tautomer, or a pharmaceutically acceptable salt thereof, wherein Ring C is an optionally substituted C₆₋₁₀ carboaryl, or an optionally substituted C₅₋₁₀ heteroaryl group, wherein the optional substituents are selected from C₁₋₆ alkyl, halogen or OH.

**43.** The compound according to claim 42, or a tautomer, or a pharmaceutically acceptable salt thereof, wherein Ring C is an optionally substituted C₅₋₁₀ heteroaryl group, wherein the optional substituents are selected from C₁₋₆ alkyl, halogen or OH.

**44.** The compound according to claim 43, or a tautomer, or a pharmaceutically acceptable salt thereof, wherein Ring C is a 1,3-thiazole optionally substituted by methyl, optionally wherein Ring C is 4-methyl-1,3-thiazol-5-yl.

**45.** The compound according to any one of claims 31 to 44, or a tautomer, or a pharmaceutically acceptable salt thereof, wherein E has the following structure E1-1: 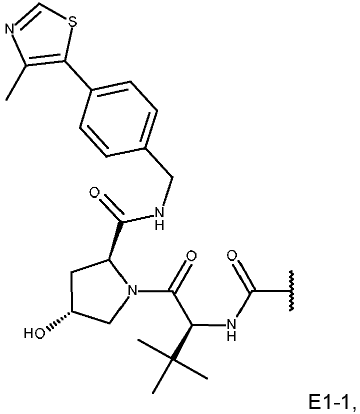 wherein 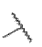 represents attachment point to L.

**46.** The compound according to any one of claims 31 to 44, or a tautomer, or a pharmaceutically acceptable salt thereof, wherein E has the following structure E1-2: 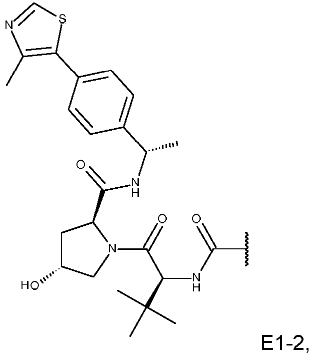 wherein 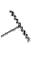 represents attachment point to L.

**47.** The compound according to any one of claims 1 to 30, or a tautomer, or a pharmaceutically acceptable salt thereof, wherein E has a structure according to Formula E2: 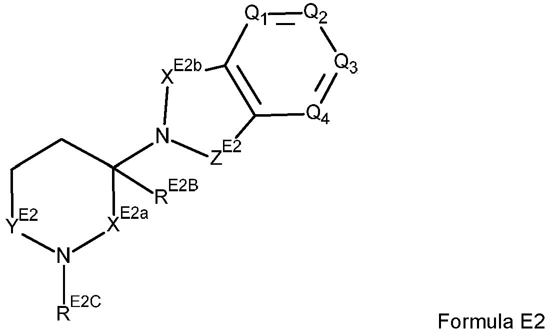 wherein
X^{E2a} is selected from C=O and C=S;
X^{E2b} is selected from C=O, C=S, or CH₂;
Y^{E2} is selected from C=O and C=S;
Z^{E2} is selected from CH₂, CHR, C=O, SO₂, NH, and NR;
Q₁, Q₂, Q₃, and Q₄ are independently N, CH, CR^{E2D}, or CR^{E2A} wherein one of Q₁, Q₂, Q₃, and Q₄ is CR^{E2A}; R^{E2A} is selected from a bond, -O-, -C(=O)-, -CH₂-, -NR-, -NH-, and -SO₂-;
R^{E2B} is selected from H, halogen, C₁₋₆alkyl, and C₃₋₇ cycloalkyl;
R^{E2C} is selected from H, OH, and C₁₋₆alkyl;
R^{E2D} is selected from OH, halogen, C₁₋₆ alkoxy, C₁₋₆ alkyl, C₂₋₆ alkenyl, C₂₋₆ alkynyl, C₃₋₇ cycloalkyl, and C₄₋₇ heterocyclyl;
R is independently selected from H, C₁₋₆ alkoxy, C₁₋₆ alkyl, C₂₋₆ alkenyl, C₂₋₆ alkynyl, C₃₋₇ cycloalkyl, and C₄₋₇ heterocyclyl,
and E is joined to L by the group R^{E2A}.

**48.** The compound according to claim 47, or a tautomer, or a pharmaceutically acceptable salt thereof, wherein X^{E2a} and X^{E2b} are C=O.

**49.** The compound according to either claim 47 or 48, or a tautomer, or a pharmaceutically acceptable salt thereof, wherein Y^{E2} is C=O.

**50.** The compound according to any one of claims 47 to 49, or a tautomer, or a pharmaceutically acceptable salt thereof, wherein Z^{E2} is C=O.

**51.** The compound according to any one of claims 47 to 50, or a tautomer, or a pharmaceutically acceptable salt thereof, wherein Y^{EZ} and X^{E2a} are each C=O and R^{E2C} is H.

**52.** The compound according to any one of claims 47 to 51, or a tautomer, or a pharmaceutically acceptable salt thereof, wherein one of Q₁, Q₂, Q₃, and Q₄ is CR^{E2A}, and no more than one of Q¹, Q², Q³, or Q⁴ are N.

**53.** The compound according to any one of claims 47 to 51, or a tautomer, or a pharmaceutically acceptable salt thereof, wherein one of Q₁, Q₂, Q₃, and Q₄ is CR^{E2A}, and the remaining are CH or CR^{E2D}.

**54.** The compound according to any one of claims 47 to 51, or a tautomer, or a pharmaceutically acceptable salt thereof, wherein one of Q₁, Q₂, Q₃, and Q₄ is CR^{E2A}, and the remaining are CH.

**55.** The compound according to any one of claims 47 to 51, or a tautomer, or a pharmaceutically acceptable salt thereof, wherein one of Q₁, Q₂, Q₃, and Q₄ is CR^{E2A}, another is CR^{E2D}, and the remaining are CH.

**56.** The compound according to any one of claims 47 to 55, or a tautomer, or a pharmaceutically acceptable salt thereof, wherein Q₄ is CR^{E2A}.

**57.** The compound according to any one of claims 47 to 55, or a tautomer, or a pharmaceutically acceptable salt thereof, wherein Q₄ is CR^{E2A}, Q₂ and Q₃ are CH and Q₁ is CH or COMe.

**58.** The compound according to any one of claims 47 to 57, or a tautomer, or a pharmaceutically acceptable salt thereof, wherein R^{E2A} is -O- or -NR- wherein R is H, OH, C₁₋₆ alkyl, C₂₋₆ alkenyl, C₂₋₆ alkynyl, C₃₋₇cycloalkyl, C₄₋₇ heterocyclyl.

**59.** The compound according to claim 58, or a tautomer, or a pharmaceutically acceptable salt thereof, wherein R^{E2A} is -NH-.

**60.** The compound according to claim 58, or a tautomer, or a pharmaceutically acceptable salt thereof, wherein R^{E2A} is a bond.

**61.** The compound according to any one of claims 47 to 59, or a tautomer, or a pharmaceutically acceptable salt thereof, wherein R^{E2B} is H or fluorine.

**62.** The compound according to claim 61, or a tautomer, or a pharmaceutically acceptable salt thereof, wherein R^{E2B} is H.

**63.** The compound according to any one of claims 47 to 62, or a tautomer, or a pharmaceutically acceptable salt thereof, wherein R^{E2C} is H.

**64.** The compound according to any one of claims 47 to 63, or a tautomer, or a pharmaceutically acceptable salt thereof, wherein E has the following structure E2-1: 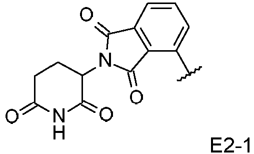 wherein 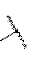 represents attachment point to L.

**65.** The compound according to any one of claims 47 to 63, or a tautomer, or a pharmaceutically acceptable salt thereof, wherein E has the following structure E2-2: 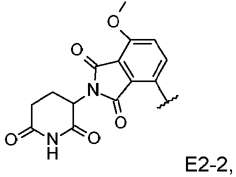 wherein 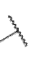 represents attachment point to L.

**66.** The compound according to any one of claims 1 to 30, or a tautomer, or a pharmaceutically acceptable salt thereof, wherein E has a structure according to Formula E3 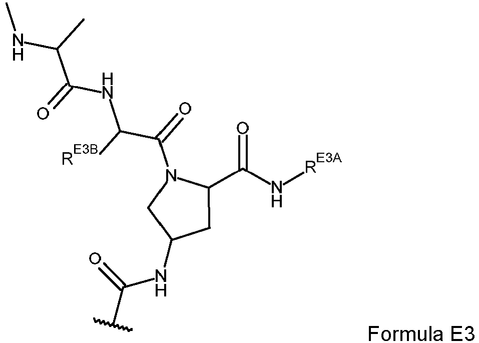 wherein
R^{E3A} is optionally substituted C₅₋₁₀ cycloalkyl or optionally substituted C₆₋₁₀ carboaryl, wherein the optional substituents are selected from C₁₋₆ alkyl, halogen and OH;
R^{E3B} is H, C₁₋₆ alkyl or C₃₋₇ cycloalkyl; and 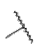 represents attachment point to L.

**67.** The compound according to claim 66, or a tautomer, or a pharmaceutically acceptable salt thereof, wherein R^{E3A} is an unsubstituted tetralin, optionally wherein R^{E3A} is (*1R*)-1,2,3,4-tetrahydronaphthalen-1-yl.

**68.** The compound according to either claim 66 or 67, or a tautomer, or a pharmaceutically acceptable salt thereof, wherein R^{E3B} is C₃₋₇ cycloalkyl.

**69.** The compound according to claim 68, or a tautomer, or a pharmaceutically acceptable salt thereof, wherein R^{E3B} is cyclohexyl.

**70.** The compound according to any one of claims 66 to 69, or a tautomer, or a pharmaceutically acceptable salt thereof, wherein E has the following structure: 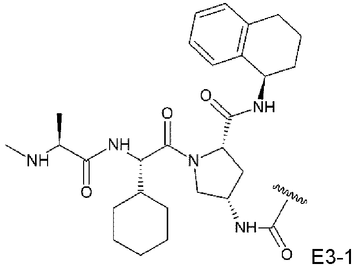 wherein 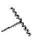 represents attachment point to L.

**71.** The compound according to any one of claims 1 to 30, or a tautomer, or a pharmaceutically acceptable salt thereof, wherein E has a structure according to Formula E4: 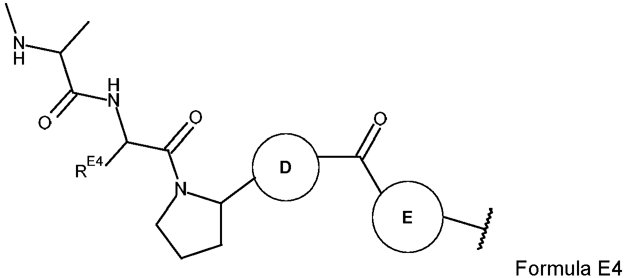 wherein
R^{E4} is selected from H, C₁₋₆ alkyl, or C₃₋₇ cycloalkyl;
Ring D is C₅₋₇ heterocyclyl or C₅₋₇ heteroaryl;
Ring E is C₆₋₁₀ carboaryl, or C₅₋₁₀ heteroaryl; and 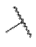 represents attachment point to L.

**72.** The compound according to claim 71, or a tautomer, or a pharmaceutically acceptable salt thereof, wherein R^{E4} is C₃₋₇ cycloalkyl.

**73.** The compound according to claim 72, or a tautomer, or a pharmaceutically acceptable salt thereof, wherein R^{E4} is cyclohexyl.

**74.** The compound according to any one of claims 71 to 73, or a tautomer, or a pharmaceutically acceptable salt thereof, wherein Ring D is C₅₋₇ heteroaryl.

**75.** The compound according to claim 74, or a tautomer, or a pharmaceutically acceptable salt thereof, wherein Ring D is a C₅₋₇ heteroaryl group containing one N atom and one S atom.

**76.** The compound according to claim 75, or a tautomer, or a pharmaceutically acceptable salt thereof, wherein Ring D is a 1,3-thiazole linked in position 2 to the pyrrolidine ring and in position 4 to C=O.

**77.** The compound according to any one of claims 71 to 76, or a tautomer, or a pharmaceutically acceptable salt thereof, wherein Ring E is C₆₋₁₀ carboaryl.

**78.** The compound according to claim 77, or a tautomer, or a pharmaceutically acceptable salt thereof, wherein Ring E is:

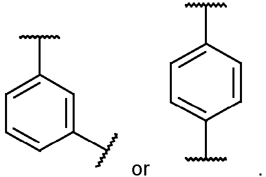

**79.** The compound according to any one of claims 71 to 78, or a tautomer, or a pharmaceutically acceptable salt thereof, wherein E has the following structure: 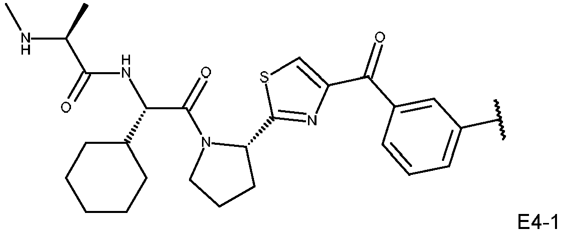 wherein 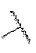 represents attachment point to L.

**80.** The compound according to any one of claims 1 to 30, or a tautomer, or a pharmaceutically acceptable salt thereof, wherein E has a structure according to Formula E5: 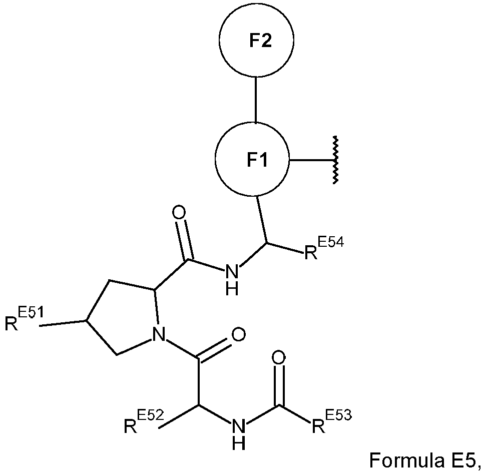
wherein Ring F1 is an optionally substituted C₆₋₁₀ carboaryl, or optionally substituted C₅₋₁₀ heteroaryl group wherein the optional substituents are selected from C₁₋₆ alkyl, or halogen, and OH; Ring F2 is an optionally substituted C₆₋₁₀ carboaryl, an optionally substituted C₅₋₁₀ heteroaryl group, or an optionally substituted C₅₋₁₀ heterocyclic group wherein the optional substituents are selected from C₁₋₆ alkyl, halogen, and OH;
R^{E51} is selected from H, OH, OR, NH₂, NHR, or NRR';
wherein R and R' are C₁₋₆ alkyl, C₂₋₆ alkenyl, C₂₋₆ alkynyl, C₃₋₇ cycloalkyl, C₄₋₇ heterocyclyl;
R^{E52} is selected from optionally substituted C₁₋₆ alkyl, optionally substituted C₃₋₇ cycloalkyl, or optionally substituted C₄₋₁₀ heterocyclyl, wherein the optional substituents are OH, C₁₋₆alkyl, or halogen;
R^{E53} is selected from optionally substituted C₃₋₆ cycloalkyl, optionally substituted C₁₋₆ alkyl, optionally substituted C₄₋₆ heterocycloalkyl, H or OH, wherein the optional substituents are selected from halogen, CN, OH and C₁₋₆ alkyl;
R^{E54} is selected from H and C₁₋₆ alkyl; and 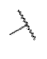 represents attachment to the Linker L.

**81.** The compound according to claim 80 or a tautomer, or a pharmaceutically acceptable salt thereof, wherein Ring F2 is an optionally substituted C₆₋₁₀ carboaryl, or an optionally substituted C₅₋₁₀ heteroaryl group, wherein the optional substituents are selected from C₁₋₆alkyl, halogen or OH.

**82.** The compound according to claim 81, or a tautomer, or a pharmaceutically acceptable salt thereof, wherein Ring F2 is an optionally substituted C₅₋₆ heteroaryl group, wherein the optional substituents are selected from methyl, ethyl, propyl, halogen or OH.

**83.** The compound according to claim 82, or a tautomer, or a pharmaceutically acceptable salt thereof, wherein Ring F2 is a 1,3-thiazole optionally substituted by methyl, optionally wherein Ring F2 is 4-methyl-1,3-thiazol-5-yl.

**84.** The compound according to any one of claims 80 to 83, or a tautomer, or a pharmaceutically acceptable salt thereof, wherein Ring F1 is an optionally substituted C₆₋₁₀ carboaryl, or an optionally substituted C₅₋₁₀ heteroaryl group, wherein the optional substituents are selected from C₁₋₆ alkyl, halogen or OH.

**85.** The compound according to claim 84, or a tautomer, or a pharmaceutically acceptable salt thereof, wherein Ring F1 is an optionally substituted C₆₋₁₀ carboaryl, wherein the optional substituents are selected from C₁₋₆ alkyl, halogen or OH.

**85.** The compound according to claim 85, or a tautomer, or a pharmaceutically acceptable salt thereof, wherein Ring F1 is phenyl.

**87.** The compound according to any one of claims 80 to 86, or a tautomer, or a pharmaceutically acceptable salt thereof, wherein R^{E51} is OH.

**88.** The compound according to claim any one of claims 80 to 87, or a tautomer, or a pharmaceutically acceptable salt thereof, wherein R^{E52} is C₁₋₆alkyl.

**89.** The compound according to claim 88, or a tautomer, or a pharmaceutically acceptable salt thereof, wherein R^{E52} is tert-butyl.

**90.** The compound according to any one of claims 80 to 89, or a tautomer, or a pharmaceutically acceptable salt thereof, wherein R^{E53} is optionally substituted C₃₋₆ cycloalkyl.

**91.** The compound according to claim 90, or a tautomer, or a pharmaceutically acceptable salt thereof, wherein R^{E53} is optionally substituted cyclopropyl.

**92.** The compound according to any one of claims 80 to 91, or a tautomer, or a pharmaceutically acceptable salt thereof, wherein R^{E53} is optionally substituted with F, Cl, Br, CN, OH or methyl.

**93.** The compound according to claim 91, or a tautomer, or a pharmaceutically acceptable salt thereof, wherein R^{E53} is optionally substituted with F.

**94.** The compound according to any one of claims 80 to 93, or a tautomer, or a pharmaceutically acceptable salt thereof, wherein E5 is of the following formula: 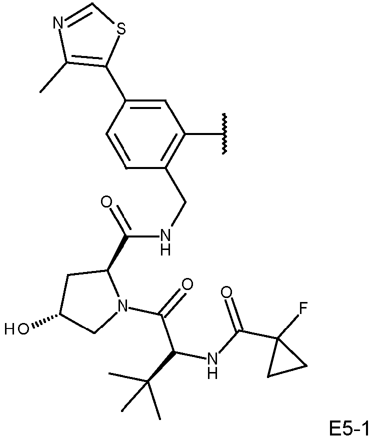 wherein 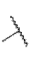 represents attachment point to L.

**95.** The compound according to any one of claims 1 to 30, or a tautomer, or a pharmaceutically acceptable salt thereof, wherein E has a structure according to Formula E6: 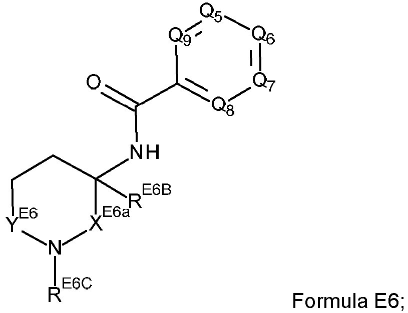 wherein
X^{E6a} is selected from C=O or C=S;
Y^{E6} is selected from C=O or C=S;
Q₅, Q₆, Q₇, Q₈, and Q₉ are independently N, CH, CR^{E6D}, or CR^{E6A} wherein one of Q₅, Q₆, Q₇, and Q₈ is CR^{E6A};
R^{E6A} is selected from a bond, -C(=O)-, -CH₂-, -O-, -NR-, -NH-, and -SOz-;
R^{E6B} is selected from H, halogen, C₁₋₆alkyl, and C₃₋₇ cycloalkyl;
R^{E6C} is selected from H, OH, and C₁₋₆ alkyl; and
R^{E6D} is selected from OH, halogen, C₁₋₆ alkoxy, C₁₋₆ alkyl, C₂₋₆ alkenyl, C₂₋₆ alkynyl, C₃₋₇ cycloalkyl, C₄₋₇ heterocyclyl;
R is selected from H, C₁₋₆ alkoxy, C₁₋₆ alkyl, C₂₋₆ alkenyl, C₂₋₆ alkynyl, C₃₋₇ cycloalkyl, C₄₋₇ heterocyclyl;
and E is joined to L by the group R^{E6A}.

**96.** The compound according to claim 95, or a tautomer, or a pharmaceutically acceptable salt thereof, wherein X^{E6a} is C=O.

**97.** The compound according to claim 95 or 96, or a tautomer, or a pharmaceutically acceptable salt thereof, wherein Y^{E6} is C=O.

**98.** The compound according to any one of claims 95 to 97, or a tautomer, or a pharmaceutically acceptable salt thereof, wherein Y^{E6} and X^{E6a} are each C=O and R^{E6C} is H.

**99.** The compound according to any one of claims 95 to 98, or a tautomer, or a pharmaceutically acceptable salt thereof, wherein one of Q₅, Q₆, Q₇, and Q₈ is CR^{E6A}, and no more than one of Q₅, Q₆, Q₇, or Q₈ are N.

**100.** The compound according to any one of claims 95 to 98, or a tautomer, or a pharmaceutically acceptable salt thereof, wherein one of Q₅, Q₆, Q₇, and Q₈ is CR^{E6A}, and the remaining are CH or CR^{E6D}.

**101.** The compound according to any one of claims 95 to 100, or a tautomer, or a pharmaceutically acceptable salt thereof, wherein R^{E6D} is selected from halogen, C₁₋₆ alkoxy, and C₁₋₆ alkyl.

**102.** The compound according to any one of claims 95 to 100, or a tautomer, or a pharmaceutically acceptable salt thereof, wherein R^{E6D} is selected from F, Cl, Br, and OMe

**103.** The compound according to any one of claims 95 to 102, or a tautomer, or a pharmaceutically acceptable salt thereof, wherein Q₆ is CR^{E2A}.

**104.** The compound according to any one of claims 95 to 102, or a tautomer, or a pharmaceutically acceptable salt thereof, wherein Q₆ is CR^{E2A}, Q⁵, Q⁷, and Q⁹ are CH and Q₈ is selected from CH, C-halogen, and C-OCH₃.

**105.** The compound according to any one of claims 95 to 104, or a tautomer, or a pharmaceutically acceptable salt thereof, wherein R^{E6A} is a bond, or -NR- wherein R is H, C₁₋₆ alkyl, C₂₋₆ alkenyl, C₂₋₆ alkynyl, C₃₋₇ cycloalkyl, C₄₋₇ heterocyclyl.

**106.** The compound according to any one of claims 95 to 105, or a tautomer, or a pharmaceutically acceptable salt thereof, wherein R^{E6B} is H or fluorine.

**107.** The compound according to claim 106, or a tautomer, or a pharmaceutically acceptable salt thereof, wherein R^{E6B} is H.

**108.** The compound according to any one of claims 95 to 107, or a tautomer, or a pharmaceutically acceptable salt thereof, wherein R^{E6C} is H.

**109.** The compound according to claim 95, or a tautomer, or a pharmaceutically acceptable salt thereof, wherein E6 is selected from one of the following structures E6-1, E6-2, or E6-3: 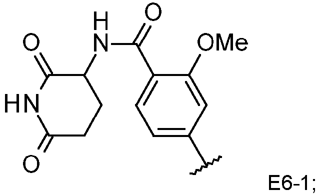 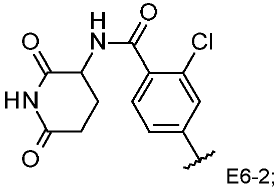 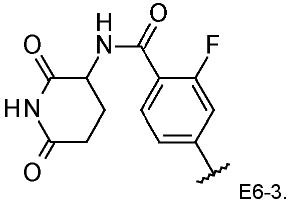 wherein 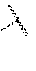 represents attachment point to L.

**110.** The compound according to any of claims 1 to 30, or a tautomer, or a pharmaceutically acceptable salt thereof, wherein E is selected from one of the following structures:
| **E** | **Structure** | **E** | **Structure** |
|---|---|---|---|
| E1-1 | 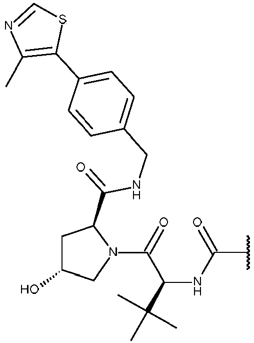 | E4-1 | 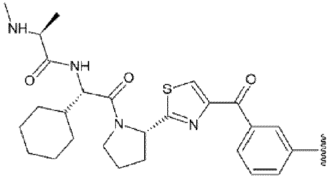 |
| E1-2 | 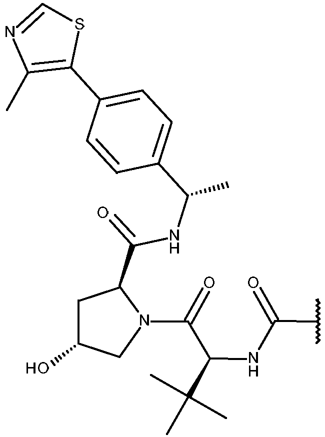 | E5-1 | 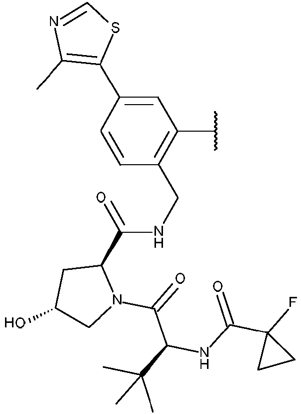 |
| E2-1 | 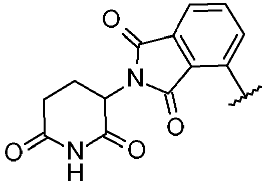 | E6-1 | 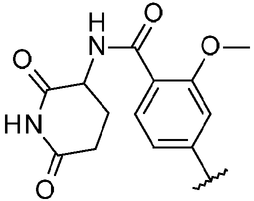 |
| E2-2 | 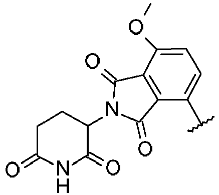 | E6-2 | 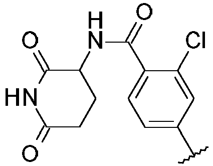 |
| E3-1 | 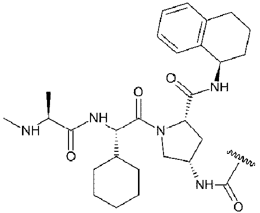 | E6-3 | 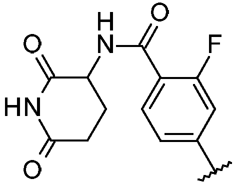 |

**111.** The compound of any one of claims 1 to 110, or a tautomer, or a pharmaceutically acceptable salt thereof, wherein L is a saturated or a partially or fully unsaturated framework comprising C and H atoms and at least one heteroatom, wherein said framework has a shortest length of 10 to 40 atoms; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-COR^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, Cl, and CN;
wherein each R^{L} is independently selected from C₁₋₃ alkyl and cyclopropyl, and wherein the number of optional substituents is below 10;
wherein said Linker is attached via its point of attachment to an available C, N, O, or S atom at E and the O atom of S1.

**112.** The compound of any one of claims 1 to 111, or a tautomer, or a pharmaceutically acceptable salt thereof, wherein L connects the STING ligand to the E3 ubiquitin ligase ligand via a chain of atoms having 15-36 atoms in shortest length.

**113.** The compound according to claim 111 or 112, or a tautomer, or a pharmaceutically acceptable salt thereof, wherein L is a saturated or a partially or fully unsaturated framework comprising C and H atoms, and at least one heteroatom wherein said framework may include one or more of the groups selected from -O-, -C(=O)-, -N(R^{L1})-, -N(R^{L1})-C(=O)-, -C(=O)-N(R^{L1})-, -C₁₋₆alkyl-N(R^{L1}), -N(R^{L1})-C(=O)-C₁-₆alkyl, - C₁₋₆alkyl-C(=O)-N(R^{L1})-, -C(=O)-N(R^{L1})-C₁₋₆alkyl-, -C₁₋₆alkyl-N(R^{L})-C(=O)-, and/or heterocyclic rings or heteroaryl rings,
wherein R^{L1} is selected from H, C₁₋₆alkyl.

**114.** The compound according to any one of claims 1 to 113, or a tautomer, or a pharmaceutically acceptable salt thereof, wherein L has the following structure:
*-L_{A}-[X]-L_{B}-*
wherein
L_{A} and L_{B} are each independently selected from a bond, *-C₁₋₆alkyl-, *-O-C₁₋₆alkyl-, *-C₃₋₇cycloalkyl, *-C₄₋₇heterocyclyoalkyl-, *-C₃₋₇cycloalkyl-C₃₋₇cycloalkyl-, *-C₄₋₇heterocyclyoalkyl-C₄₋₇heterocyclyoalkyl-, *-C₁₋₆alkyl-O-, *-O- , *-N(R^{L1})-, *-N(R^{L1})-C(=O)-, *-C(=O)-N(R^{L1})-, *-O-CH₂-C(=O)-N(R^{L1})-, *-N(R^{L1})-C(=O)-CH₂-O- *-N(R^{L1})-C₁₋₆alkyl-, *-C₁₋₆alkyl-N(R^{L1})-, *-N(R^{L1})-C(=O)-C₁₋₆alkyl-, *-C₁₋₆alkyl-C(=O)-N(R^{L1})-, *-C(=O)-, *-C(=O)-C₁₋₆alkyl-,*-C(=O)-N(R^{L1})-C₁₋₆alkyl-, *-C₁₋₆alkyl-N(R^{L1})-C(=O)-, and a functional group selected from carbonyl, ester, amide, carbamate and thiourea;
wherein R^{L1} is selected from H, C₁₋₆alkyl;
X is selected from: a bivalent, saturated or unsaturated, straight or branched, C₁₋₄₅ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, -N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, -S(=O)₂N(R^{L})-, - S(=O)₂N(H)-, C₅₋₇ carbocyclyl, a C₅₋₇ heterocyclyl, a C₆₋₁₀ carboaryl, or a C₅₋₁₀ heteroaryl group;
wherein R^{L} is a C₁₋₆alkyl group; and
* denotes the attachment to S1 or E.

**115.** The compounds according to claim 114, or a tautomer, or a pharmaceutically acceptable salt thereof, wherein X is selected from: a bivalent, saturated or unsaturated, straight or branched, C₆₋₃₆ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, - N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, - S(=O)₂N(R^{L})-, -S(=O)₂NH), C₅₋₇ carbocyclyl, a C₅₋₇ heterocyclyl, a C₆ carboaryl, or a C₅₋₇ heteroaryl group, 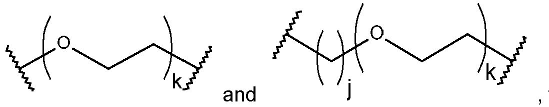 wherein k and j are each individually integers from 1 to 8;
wherein R^{L} is a C₁₋₆alkyl group.

**116.** The compounds according to claim 115, or a tautomer, or a pharmaceutically acceptable salt thereof, wherein X contains one or more -N(H)-C(=O)- or -C(=O)-N(H)- groups and 1 to 10 of the following group:

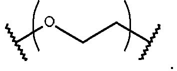

**117.** The compound of any of the preceding claims, or a tautomer, or a pharmaceutically acceptable salt thereof, wherein L connects the STING ligand to the E3 ubiquitin ligase ligand via the following linker structure:
E-(L7)ᵢ-(L6)ₕ-(L5)_{g}-(OC₂H₄)_{f}-(L4)ₑ-(L3)_{d}-(L2)_{c}-(OC₂H₄)_{b}-(NR^{X}C(=O))-(L1)ₐ-S1
wherein:
L1 is C₁₋₆ alkyl;
L2 is C₁₋₆ alkyl;
L3 is - N(methyl)-C(=O)-, -NH-C(=O)-, -C(=O)NH- or C(=O)N(methyl)-;
L4 is -C(=O)-C₅₋₇ heterocyclyl-C(=O)- or -C(=O)-C₅₋₇ heteroaryl-C(=O)-;
L5 is C₁₋₁₂ alkyl wherein one or more -CH₂- groups is optionally replaced by one or more groups selected from -O-, -NH-, -NMe-, -C(=O)NH, -C(=O)NMe, --NHC(=O)-, NMeC(=O)- group;
L6 is C₁₋₆ alkyl optionally substituted by a =O group;
L7 is -NH-, -NMe-, C₃₋₆cycloalkyl, C₄₋₆heterocyclyl;
R^{X} is H or methyl.
a is 0 or 1;
b is 0 to 8;
c is 0 or 1;
d is 0 or 1;
e is 0 or 1;
f is 0 to 8;
g is 0 or 1;
h is 0 or 1;
i is 0 to 2;
E is the ubiquitin ligase as defined in any of the preceding claims; and
S1 is S1 as defined in any of the preceding claims.

**118.** The compound of claim 117, or a tautomer, or a pharmaceutically acceptable salt thereof, wherein L1 is methyl and a is 1.

**119.** The compound of claim 117 or 118, or a tautomer, or a pharmaceutically acceptable salt thereof, wherein b is 2 to 5.

**120.** The compound of any one of claims 117 to 119, or a tautomer, or a pharmaceutically acceptable salt thereof, wherein L2 is ethyl.

**121.** The compound of any one of claims 117 to 120, or a tautomer, or a pharmaceutically acceptable salt thereof, wherein L3 is NHC(=O) or N(methyl)C(=O) and d is 1.

**122.** The compound of claim any one of claims 117 to 121, or a tautomer, or a pharmaceutically acceptable salt thereof, wherein L4 is 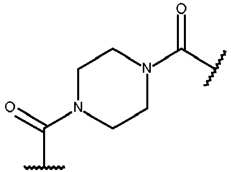 and e is 1.

**123.** The compound of any one of claims 117 to 121, or a tautomer, or a pharmaceutically acceptable salt thereof, wherein e is 0.

**124.** The compound of any one of claims 117 to 123, or a tautomer, or a pharmaceutically acceptable salt thereof, wherein f is 0 to 5.

**125.** The compound of any one of claims 117 to 124, or a tautomer, or a pharmaceutically acceptable salt thereof, wherein L5 is -O-C₄H₈-NHC(=O)-C₂H₄- or -O-C₄H₈-NH-C(=O)-C₂H₄-O-C₂H₄-.

**126.** The compound of any one of claims 117 to 125, or a tautomer, or a pharmaceutically acceptable salt thereof, wherein g is 0.

**127.** The compound of any one of claims 117 to 126, or a tautomer, or a pharmaceutically acceptable salt thereof, wherein L6 is methyl, ethyl, butyl or propyl substituted by =O and h is 1.

**128.** The compound of any one of claims 117 to 127, or a tautomer, or a pharmaceutically acceptable salt thereof, wherein i is 0.

**129.** The compound of any one of claims 117 to 127, or a tautomer, or a pharmaceutically acceptable salt thereof, wherein L7 is -NH- or C₃₋₆heterocyclyl, and i is 1 or 2.

**130.** The compound of any one of claims 1 to 109, or a tautomer, or a pharmaceutically acceptable salt thereof, wherein the linker L is selected from one of the following structures:
| **L** | **Structure** |
|---|---|
| LK1 | 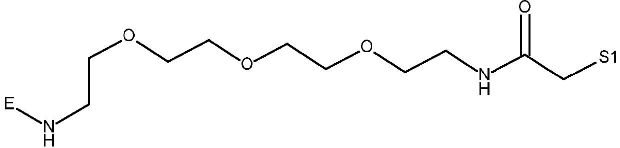 |
| LK2 | 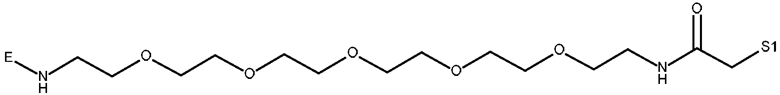 |
| LK3 | 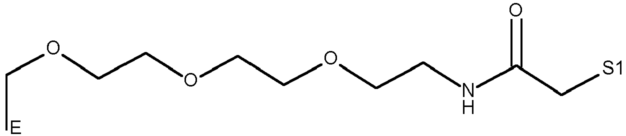 |
| LK4 | 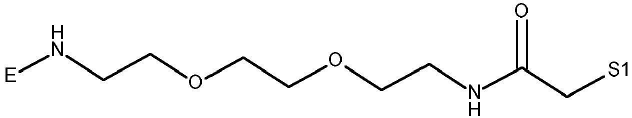 |
| LK5 | 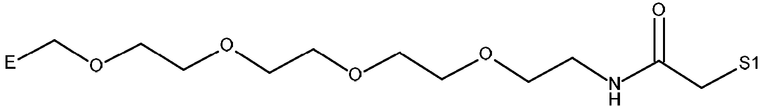 |
| LK6 | 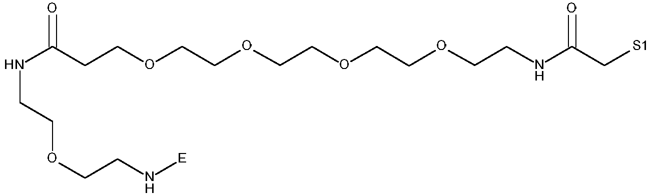 |
| LK7 | 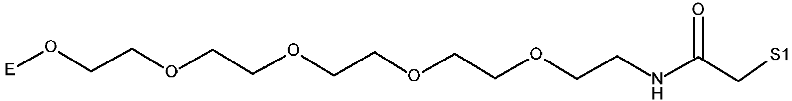 |
| LK8 | 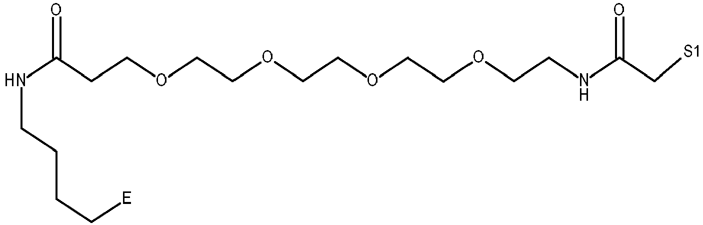 |
| LK9 | 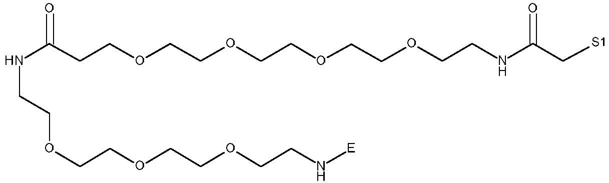 |
| LK10 | 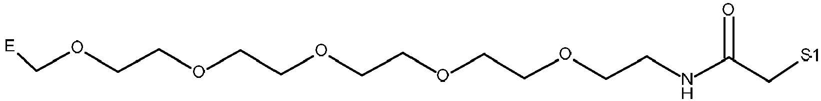 |
| LK11 | 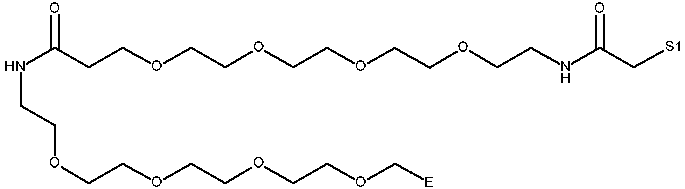 |
| LK12 | 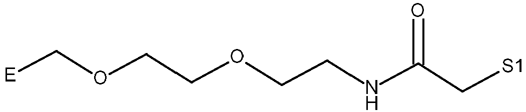 |
| LK13 | 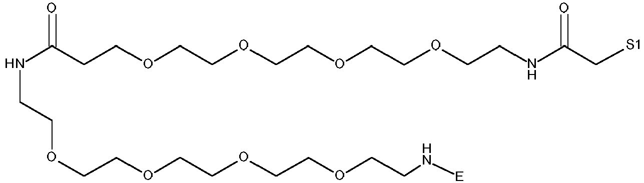 |
| LK14 | 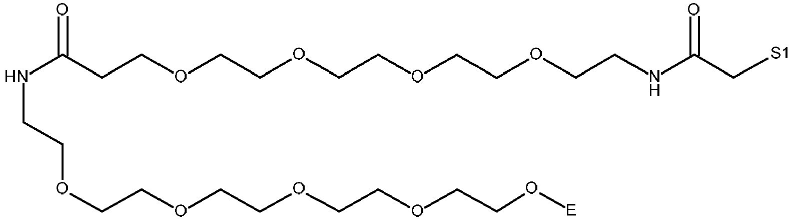 |
| LK15 | 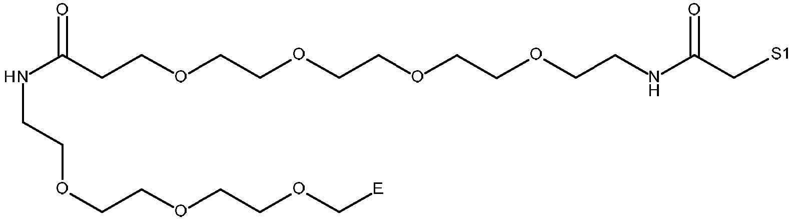 |
| LK16 | 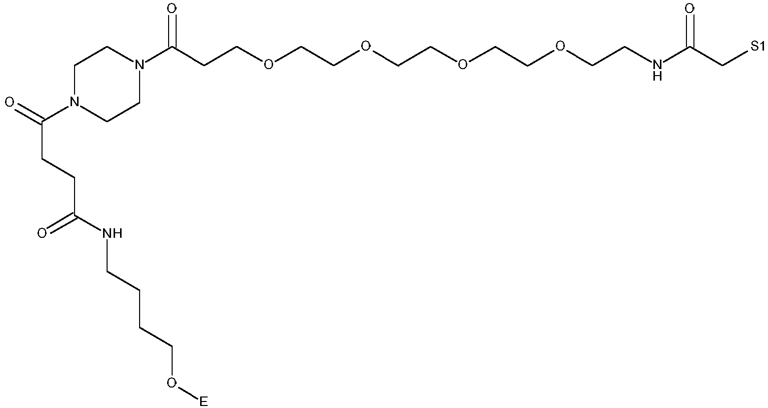 |
| LK17 | 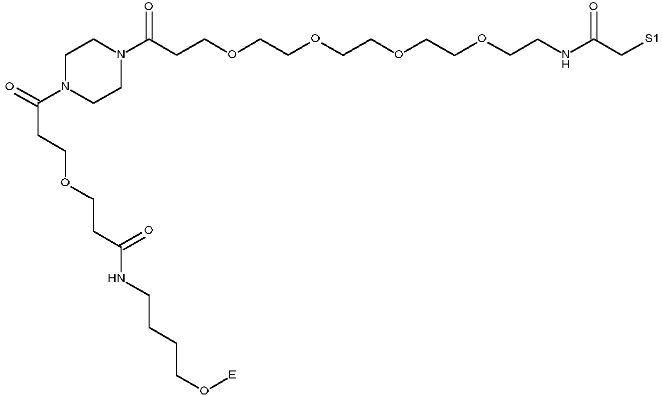 |
| LK18 | 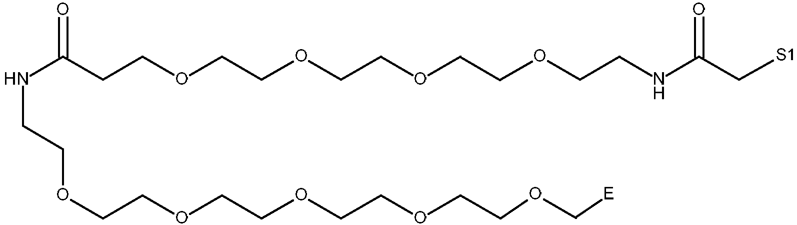 |
| LK19 | 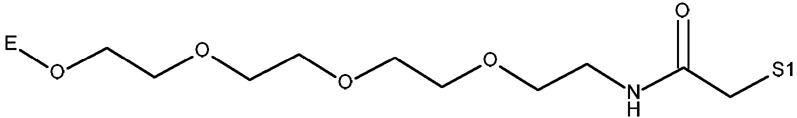 |
| LK20 | 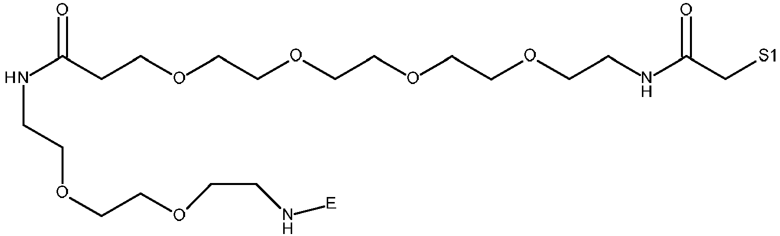 |
| LK21 | 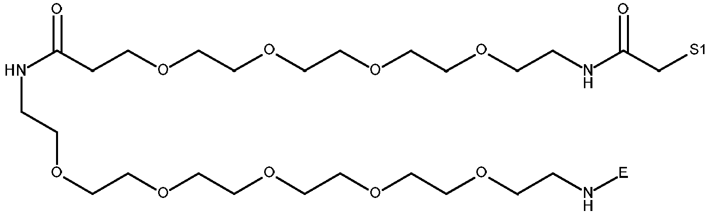 |
| LK22 | 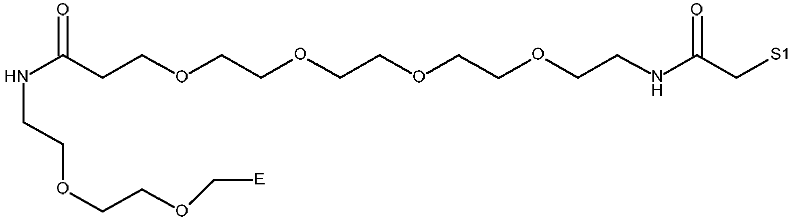 |
| LK23 | 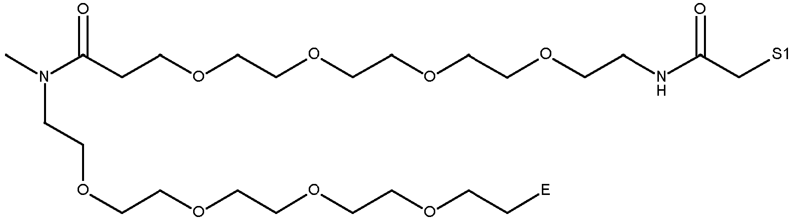 |
| LK24 | 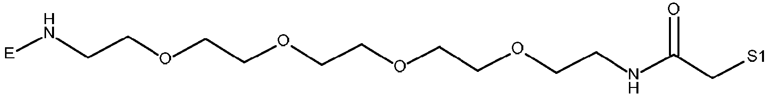 |
| LK25 | 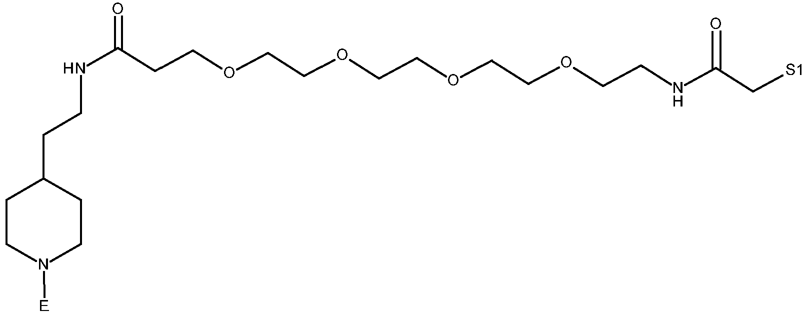 |
| LK26 | 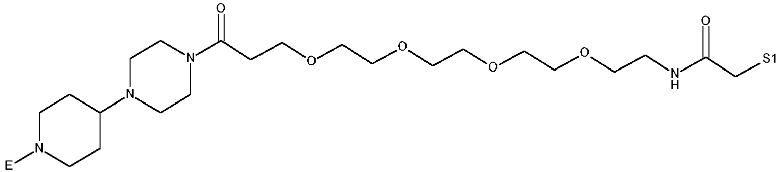 |

**131.** The compound of any one of claims 1 to 130, or a tautomer, or a pharmaceutically acceptable salt thereof, wherein S1-L-E is selected from one of the following formulae: 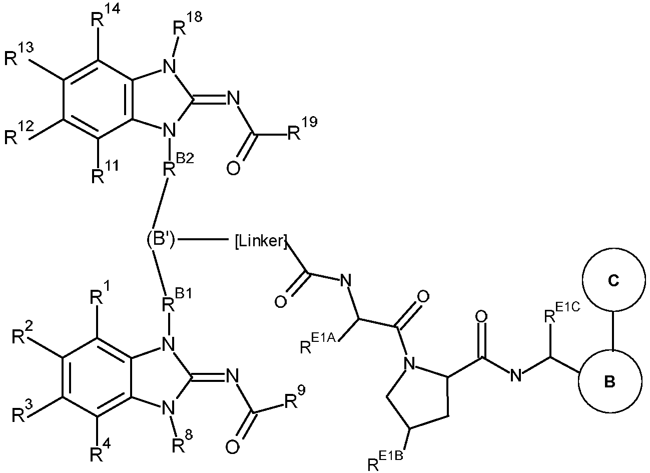 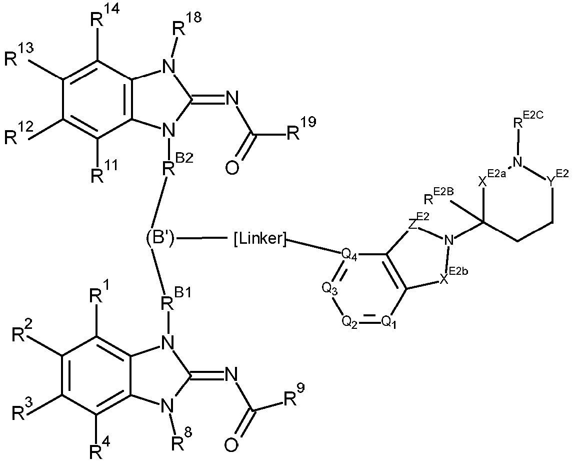 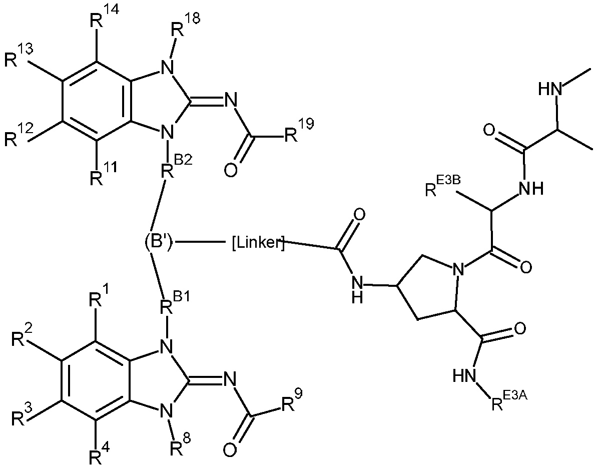 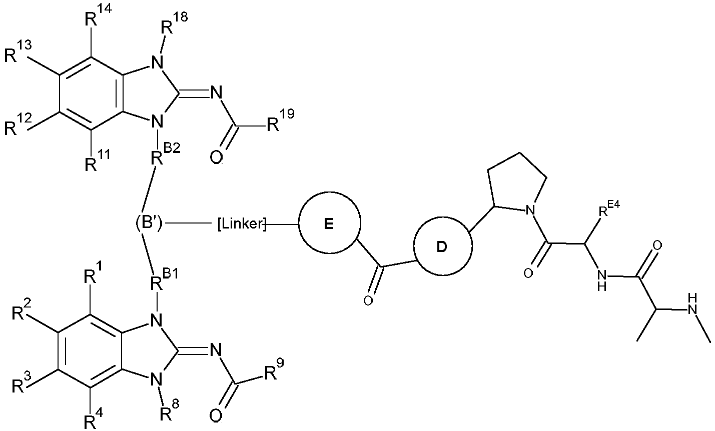 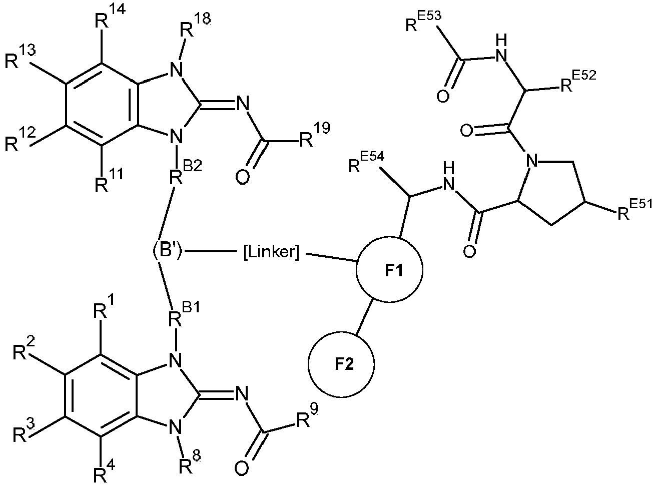 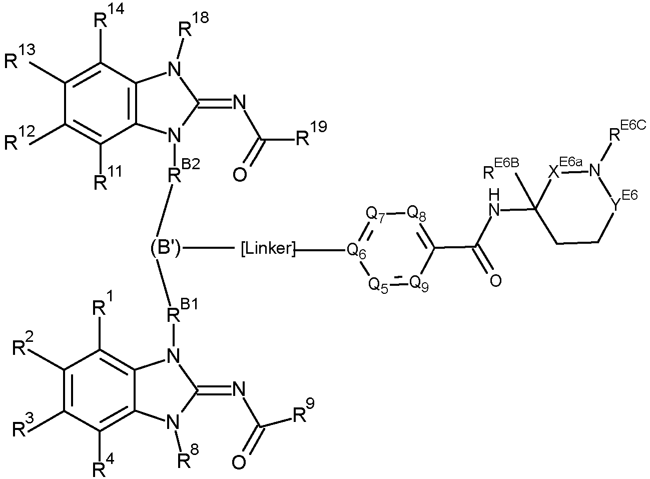 wherein the substituents R¹, R², R³, R⁴, R⁸, R⁹, R^{B1}, B', Linker, R^{B2}, R¹¹, R¹², R¹³, R¹⁴, R¹⁸, R¹⁹ R^{E1A}, R^{E1B}, R^{E1C}, Rings B, C, D, E, F1, F2, Q₁, Q₂, Q₃, Q₄, Q₅, Q₆, Q₇, Q₈, Q₉, Z^{E2}, X^{E2b}, R^{E2B}, X^{E2a}, R^{E2C}, Y^{E2}, X^{E6a}, Y^{E6} R^{E6B}, R^{E6C}, R^{E3A}, R^{E3B}, R^{E4}, R^{E51}, R^{E52}, R^{E53} and R^{E54} are as defined in any of the preceding claims.

**132.** The compound of Table 1 or a pharmaceutically acceptable salt thereof.

**133.** The compound of any one of the preceding claims, or a pharmaceutically acceptable salt thereof, wherein incubating cells with 1 µM of the compound for 16 hours reduces relative STING abundance by at least 35%, at least 40%, at least 45%, 50%, at least 55%, at least 60%, at least 65%, at least 70%, at least 75%, at least 80%, at least 85%, at least 90%, at least 95%, at least 96%, at least 97%, at least 98%, at least 99% as compared to cells not treated with the compound.

**134.** The compound of any one of the preceding claims, or a pharmaceutically acceptable salt thereof, for use in a method of treatment of a disorder or condition in a subject, the method comprising administering a therapeutically effective amount of the compound to a subject in need thereof.

**135.** The compound for the use according to claim 134, wherein the disorder or condition is associated with overactivation of STING.

**136.** The compound for the use according to claim 134 or 135, wherein the disorder or condition is a monogenic autoinflammatory syndrome.

**137.** The compound for the use according to claim 136, wherein the disorder or condition is STING-associated vasculopathy with onset in infancy (SAVI), Aicardi-Goutieres Syndrome, COPA Syndrome, Familial Chilblain Lupus, DNase II deficiency, or Trex1 deficiency.

**138.** The compound for the use according to claim 134 or 135, wherein the disorder or condition is an autoimmune disease.

**139.** The compound for the use according to claim 138, wherein the disorder or condition is systemic lupus erythematosus (SLE) or Sjögren's syndrome.

**140.** The compound for the use according to claim 134 or 135, wherein the disorder or condition is an inflammatory disease.

**141.** A compound according to any one of the preceding claims, or a pharmaceutically acceptable salt thereof, for use as a medicament.

**142.** Use of a compound according to any one of the preceding claims, or a pharmaceutically acceptable salt thereof, for the manufacture of a medicament for the treatment of a disease or condition a subject in need thereof.

**143.** The compound for the use according to any one of claims 134 to 140, wherein upon non-covalent binding of S1 to STING and E to an E3 ubiquitin ligase, STING is degraded.

**144.** A method of treatment of a disease or condition in a subject comprising administering a therapeutically effective amount of a compound according to any one of claims 1 to 133, or a pharmaceutically acceptable salt thereof, to a subject in need thereof.

**145.** A use of the compound according to any one of the preceding claims, or a pharmaceutically acceptable salt thereof, in the manufacture of a medicament for the treatment of a disease or condition in a subject in need thereof.

**146.** The compound or pharmaceutically acceptable salt, for the use according to any one of claims 132 to 145, wherein the subject is a mammal.

**147.** The compound or pharmaceutically acceptable salt, for the use according to claim 146, wherein the mammal is a human.

**148.** A pharmaceutical composition comprising the compound according to any one of the preceding claims, or a pharmaceutically acceptable salt thereof, and a pharmaceutically acceptable excipient.
